# EUROPÄISCHE PATENTSCHRIFT

(11) **EP 4 308 664 B1**
(45) Veröffentlichungstag und Bekanntmachung des Hinweises auf die Patenterteilung: **17.06.2026**
(21) Anmeldenummer: 22710127.6
(22) Anmeldetag: 15.03.2022
(51) Int. Cl.: C09K 11/06, H10K 101/10, H10K 85/60

(54) **HETEROAROMATISCHE VERBINDUNGEN FÜR ORGANISCHE ELEKTROLUMINESZENZVORRICHTUNGEN**
HETEROAROMATIC COMPOUNDS FOR ORGANIC ELECTROLUMINESCENT DEVICES
COMPOSÉS HÉTÉROAROMATIQUES POUR DISPOSITIFS ÉLECTROLUMINESCENTS ORGANIQUES

(30) Priorität: 18.03.2021 EP 21163561
(43) Veröffentlichungstag der Anmeldung: 24.01.2024
(73) Patentinhaber: Merck Patent GmbH, 64293 Darmstadt (DE)
(72) Erfinder: PARHAM, Amir Hossain, 64293 DARMSTADT (DE); EHRENREICH, Christian, 64293 DARMSTADT (DE)
(74) Vertreter: Merck Patent Association
(86) Internationale Anmeldenummer: PCT/EP2022/056584
(87) Internationale Veröffentlichungsnummer: WO 2022/194799

(56) Entgegenhaltungen:
- WO-A1-2014/008982
- WO-A1-2016/124304
- DE-A1- 102009 048 791
- SOUKRI M ET AL: "Synthesis of novel 5a,10,14b,15-tetraaza-benzo[a]indeno[1,2-c]anthracen-5-one and benzimidazo[1,2-c]quinazoline derivatives under microwave irradiation", TETRAHEDRON LETTERS, ELSEVIER, AMSTERDAM , NL, vol. 41, no. 31, 29 July 2000 (2000-07-29), pages 5857 - 5860, XP004209574, ISSN: 0040-4039, DOI: 10.1016/S0040-4039(00)00987-4

## Beschreibung

Die vorliegende Erfindung betrifft heteroaromatische Verbindungen, die für die Verwendung in elektronischen Vorrichtungen, insbesondere in organischen Elektrolumineszenzvorrichtungen geeignet sind, sowie elektronische Vorrichtungen, insbesondere organische Elektrolumineszenzvorrichtungen, enthaltend diese Materialien.

In organischen Elektrolumineszenzvorrichtungen werden als emittierende Materialien häufig phosphoreszierende metallorganische Komplexe eingesetzt. Aus quantenmechanischen Gründen ist unter Verwendung metallorganischer Verbindungen als Phosphoreszenzemitter eine bis zu vierfache Energie- und Leistungseffizienz möglich. Generell gibt es bei Elektrolumineszenzvorrichtungen, insbesondere auch bei Elektrolumineszenzvorrichtungen, die Triplettemission (Phosphoreszenz) zeigen, immer noch Verbesserungsbedarf. Die Eigenschaften phosphoreszierender Elektrolumineszenzvorrichtungen werden nicht nur von den eingesetzten Triplettemittern bestimmt. Hier sind insbesondere auch die anderen verwendeten Materialien, wie Matrixmaterialien, von besonderer Bedeutung. Verbesserungen dieser Materialien können somit auch zu deutlichen Verbesserungen der Eigenschaften der Elektrolumineszenzvorrichtungen führen.

In der Veröffentlichung Tetrahedron Letters 41 (2000) 5857-5860 wird die Synthese von Chinazolin-Derivate beschrieben. Allerdings finden sich in dieser Druckschrift keine Hinweise auf deren Verwendung in einer elektronischen Vorrichtung. DE102009048791 A1 offenbart OLEDs, die heteroaromatische Verbindungen enthalten.

Generell besteht bei diesen Materialien, beispielsweise für die Verwendung als Matrixmaterialien noch Verbesserungsbedarf, insbesondere in Bezug auf die Lebensdauer, aber auch in Bezug auf die Effizienz und die Betriebsspannung der Vorrichtung.

Aufgabe der vorliegenden Erfindung ist daher die Bereitstellung von Verbindungen, welche sich für den Einsatz in einer organischen elektronischen Vorrichtung, insbesondere in einer organischen Elektrolumineszenzvorrichtung eignen, und welche bei Verwendung in dieser Vorrichtung zu guten Device-Eigenschaften führen, sowie die Bereitstellung der entsprechenden elektronischen Vorrichtung.

Insbesondere ist es die Aufgabe der vorliegenden Erfindung Verbindungen zur Verfügung zu stellen, die zu hoher Lebensdauer, guter Effizienz und geringer Betriebsspannung führen. Gerade auch die Eigenschaften der Matrixmaterialien haben einen wesentlichen Einfluss auf die Lebensdauer und die Effizienz der organischen Elektrolumineszenzvorrichtung.

Eine weitere Aufgabe der vorliegenden Erfindung kann darin gesehen werden, Verbindungen bereitzustellen, welche sich für den Einsatz in einer phosphoreszierenden oder fluoreszierenden Elektrolumineszenzvorrichtungen eignen, insbesondere als Matrixmaterial. Insbesondere ist es eine Aufgabe der vorliegenden Erfindung, Matrixmaterialien bereitzustellen, welche sich für rot und gelb phosphoreszierende Elektrolumineszenzvorrichtungen, insbesondere für rot und grün phosphoreszierende Elektrolumineszenzvorrichtungen und gegebenenfalls auch für blau phosphoreszierende Elektrolumineszenzvorrichtungen eignen.

Weiterhin sollten die Verbindungen, insbesondere bei ihrem Einsatz als Matrixmaterialien, als Elektronentransportmaterialien oder als Lochblockiermaterialien in organischen Elektrolumineszenzvorrichtung zu Vorrichtungen führen, die eine ausgezeichnete Farbreinheit aufweisen.

Eine weitere Aufgabe kann darin gesehen werden, elektronische Vorrichtungen mit einer ausgezeichneten Leistungsfähigkeit möglichst kostengünstig und in konstanter Qualität bereitzustellen
Weiterhin sollten die elektronischen Vorrichtungen für viele Zwecke eingesetzt oder angepasst werden können. Insbesondere sollte die Leistungsfähigkeit der elektronischen Vorrichtungen über einen breiten Temperaturbereich erhalten bleiben.

Überraschend wurde gefunden, dass bestimmte, unten näher beschriebene Verbindungen diese Aufgabe lösen, sich gut für die Verwendung in Elektrolumineszenzvorrichtungen eignen und zu Verbesserungen der organischen Elektrolumineszenzvorrichtung führen, insbesondere in Bezug auf die Lebensdauer, der Farbreinheit, der Effizienz und der Betriebsspannung. Diese Verbindungen sowie elektronische Vorrichtungen, insbesondere organische Elektrolumineszenzvorrichtungen, welche derartige Verbindungen enthalten, sind daher der Gegenstand der vorliegenden Erfindung.

Gegenstand der vorliegenden Erfindung ist eine Verbindung umfassend mindestens eine Struktur mit mindestens drei miteinander kondensierten Strukturelementen gemäß den Formeln (A), (B) und (C), vorzugsweise Verbindung bestehend aus drei miteinander kondensierten Strukturelementen gemäß den Formeln (A), (B) und (C), vorzugsweise Verbindung bestehend aus drei miteinander kondensierten Strukturelementen gemäß den Formeln (A), (B) und (C), 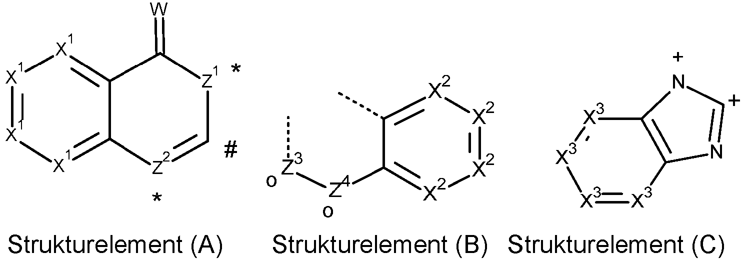 wobei das Strukturelement (A) mit Strukturelement (B) kondensiert ist und das Strukturelement (B) mit Strukturelement (C) kondensiert ist; wobei das Strukturelement (B) über die gestrichelt dargestellten Bindungen an das Strukturelement (A) bindet, wobei eine Bindung über die mit # markierte Bindungsstelle erfolgt und eine Bindung über eine mit * markierte Bindungsstelle erfolgt; wobei das Strukturelement (B) mit dem Strukturelement (C) über die mit o und + markierten Atome miteinander kondensiert ist und die jeweils markierten Atome von den Strukturelementen (B) und (C) geteilt sind, und
für die verwendeten Symbole und Indizes gilt:
   - W: steht für O oder S, vorzugsweise für O;
   - Z¹: steht für NAr oder N, für den Fall, dass das Strukturelement (A) mit dem Strukturelement (B) über Z¹ kondensiert ist;
   - Z²: steht für X oder C, für den Fall, dass das Strukturelement (A) mit dem Strukturelement (B) über Z² kondensiert ist;
   - Z³, Z⁴: steht für N oder C, wobei einer der Reste Z³, Z⁴ für N und einer der Reste Z³, Z⁴ für C steht;
   - X: steht für N oder CR, vorzugsweise für N;
   - X¹: steht bei jedem Auftreten gleich oder verschieden für N oder CR¹, vorzugsweise für CR¹, mit der Maßgabe, dass nicht mehr als zwei der Gruppen X¹ in einem Cyclus für N stehen;
   - X²: steht bei jedem Auftreten gleich oder verschieden für N oder CR², mit der Maßgabe, dass nicht mehr als zwei der Gruppen X² in einem Cyclus für N stehen;
   - X³: steht bei jedem Auftreten gleich oder verschieden für N oder CR³, vorzugsweise für CR³, mit der Maßgabe, dass nicht mehr als zwei der Gruppen X³ in einem Cyclus für N stehen;
   - Ar: ist bei jedem Auftreten gleich oder verschieden ein aromatisches oder heteroaromatisches Ringsystem mit 5 bis 40 aromatischen Ringatomen, welches Reste R aufweist, vorzugsweise eine Arylgruppe mit 6 bis 30 aromatischen Ringatomen oder eine Heteroarylgruppe mit 5 bis 14 aromatischen Ringatomen, welche Reste R aufweist;
   - R: ist bei jedem Auftreten gleich oder verschieden H, D, F, Cl, Br, I, R⁴C=C(R⁴)₂, N(R⁴)₂, N(Ar')₂, CN, NO₂, OR⁴, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, eine geradkettige Alkyl-, Alkoxy- oder Thioalkylgruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkylgruppe mit 3 bis 20 C-Atomen, wobei die Alkyl-, Alkoxy- oder Thioalkyl-, Alkenyl- oder Alkinylgruppe jeweils Reste R⁴ aufweist und wobei eine oder mehrere nicht benachbarte CH₂-Gruppen durch R⁴C=CR⁴, C≡C, Si(R⁴)₂, C=O, C=S, C=Se, C=NR⁴, -C(=O)O-, -C(=O)NR⁴-, NR⁴, P(=O)( R⁴), -O-, -S-, SO oder SO₂ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils Reste R⁴ aufweist, oder eine Aryloxy- oder Heteroaryloxygruppe mit 5 bis 40 aromatischen Ringatomen, die jeweils Reste R⁴ aufweist, oder eine Diarylamino-, Arylheteroarylamino-, Diheteroarylaminogruppe mit 5 bis 60 aromatischen Ringatomen im jeweiligen aromatischen oder heteroaromatischen Rest, wobei die Diarylamino-, Arylheteroarylamino-, Diheteroarylaminogruppe Reste R⁴ aufweist; dabei können zwei Reste R oder ein Rest R mit einem weiteren Rest, vorzugsweise einer Gruppe R¹, R², R³ ein aliphatisches, heteroaliphatisches, aromatisches oder heteroaromatisches Ringsystem, vorzugsweise ein aliphatisches, heteroaliphatisches oder heteroaromatisches Ringsystem bilden, besonders bevorzugt bildet der Rest R kein solches Ringsystem;
   - R¹: ist bei jedem Auftreten gleich oder verschieden H, D, F, Cl, Br, I, R⁴C=C(R⁴)₂, N(R⁴)₂, N(Ar')₂, CN, NO₂, OR⁴, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, eine geradkettige Alkyl-, Alkoxy- oder Thioalkylgruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkylgruppe mit 3 bis 20 C-Atomen, wobei die Alkyl-, Alkoxy- oder Thioalkyl-, Alkenyl- oder Alkinylgruppe jeweils Reste R⁴ aufweist und wobei eine oder mehrere nicht benachbarte CH₂-Gruppen durch R⁴C=CR⁴, C≡C, Si(R⁴)₂, C=O, C=S, C=Se, C=NR⁴, -C(=O)O-, -C(=O)NR⁴-, NR⁴, P(=O)( R⁴), -O-, -S-, SO oder SO₂ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils Reste R⁴ aufweist, oder eine Aryloxy- oder Heteroaryloxygruppe mit 5 bis 40 aromatischen Ringatomen, die jeweils Reste R⁴ aufweist, oder eine Diarylamino-, Arylheteroarylamino-, Diheteroarylaminogruppe mit 5 bis 60 aromatischen Ringatomen im jeweiligen aromatischen oder heteroaromatischen Rest, wobei die Diarylamino-, Arylheteroarylamino-, Diheteroarylaminogruppe Reste R⁴ aufweist; dabei können zwei Reste R¹ oder ein Rest R¹ mit einem weiteren Rest, vorzugsweise einer Gruppe R, R², R³ auch miteinander ein aromatisches, heteroaromatisches, aliphatisches oder heteroaliphatisches Ringsystem, vorzugsweise ein aliphatisches, heteroaliphatisches oder heteroaromatisches Ringsystem bilden, besonders bevorzugt bilden die Reste R¹ kein solches Ringsystem;
   - R²: ist bei jedem Auftreten gleich oder verschieden H, D, F, Cl, Br, I, R⁴C=C(R⁴)₂, N(R⁴)₂, N(Ar')₂, CN, NO₂, OR⁴, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, eine geradkettige Alkyl-, Alkoxy- oder Thioalkylgruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkylgruppe mit 3 bis 20 C-Atomen, wobei die Alkyl-, Alkoxy- oder Thioalkyl-, Alkenyl- oder Alkinylgruppe jeweils Reste R⁴ aufweist und wobei eine oder mehrere nicht benachbarte CH₂-Gruppen durch R⁴C=CR⁴, C=C, Si(R⁴)₂, C=O, C=S, C=Se, C=NR⁴, -C(=O)O-, -C(=O)NR⁴-, NR⁴, P(=O)( R⁴), -O-, -S-, SO oder SO₂ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils Reste R⁴ aufweist, oder eine Aryloxy- oder Heteroaryloxygruppe mit 5 bis 40 aromatischen Ringatomen, die jeweils Reste R⁴ aufweist, oder eine Diarylamino-, Arylheteroarylamino-, Diheteroarylaminogruppe mit 5 bis 60 aromatischen Ringatomen im jeweiligen aromatischen oder heteroaromatischen Rest, wobei die Diarylamino-, Arylheteroarylamino-, Diheteroarylaminogruppe Reste R⁴ aufweist; dabei können zwei Reste R² oder ein Rest R² mit einem weiteren Rest, vorzugsweise einer Gruppe R, R¹, R³ auch miteinander ein aromatisches, heteroaromatisches, aliphatisches oder heteroaliphatisches Ringsystem, vorzugsweise ein aliphatisches, heteroaliphatisches oder heteroaromatisches Ringsystem bilden, besonders bevorzugt bilden die Reste R² kein solches Ringsystem;
   - R³: ist bei jedem Auftreten gleich oder verschieden H, D, F, Cl, Br, I, R⁴C=C(R⁴)₂, N(R⁴)₂, N(Ar')₂, CN, NO₂, OR⁴, OAr', SR⁴, SAr', C(=O)OR⁴, C(=O)N(R⁴)², Si(R⁴)₃, B(OR⁴)₂, C(=O)R⁴, P(=O)(R⁴)₂, S(=O)R⁴, S(=O)₂R⁴, OSO₂R⁴, eine geradkettige Alkyl-, Alkoxy- oder Thioalkylgruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkylgruppe mit 3 bis 20 C-Atomen, wobei die Alkyl-, Alkoxy- oder Thioalkyl-, Alkenyl- oder Alkinylgruppe jeweils Reste R⁴ aufweist und wobei eine oder mehrere nicht benachbarte CH₂-Gruppen durch R⁴C=CR⁴, C≡C, Si(R⁴)₂, C=O, C=S, C=Se, C=NR⁴, -C(=O)O-, -C(=O)NR⁴-, NR⁴, P(=O)( R⁴), -O-, -S-, SO oder SO₂ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils Reste R⁴ aufweist, oder eine Aryloxy- oder Heteroaryloxygruppe mit 5 bis 40 aromatischen Ringatomen, die jeweils Reste R⁴ aufweist, oder eine Diarylamino-, Arylheteroarylamino-, Diheteroarylaminogruppe mit 5 bis 60 aromatischen Ringatomen im jeweiligen aromatischen oder heteroaromatischen Rest, wobei die Diarylamino-, Arylheteroarylamino-, Diheteroarylaminogruppe Reste R⁴ aufweist; dabei können zwei Reste R³ oder ein Rest R³ mit einem weiteren Rest, vorzugsweise einer Gruppe R, R¹, R² auch miteinander ein aromatisches, heteroaromatisches, aliphatisches oder heteroaliphatisches Ringsystem, vorzugsweise ein aliphatisches, heteroaliphatisches oder heteroaromatisches Ringsystem bilden, besonders bevorzugt bilden die Reste R³ kein solches Ringsystem;
   - Ar': ist bei jedem Auftreten gleich oder verschieden ein aromatisches oder heteroaromatisches Ringsystem mit 5 bis 40 aromatischen Ringatomen, welches Reste R⁴ aufweist, vorzugsweise eine Arylgruppe mit 6 bis 30 aromatischen Ringatomen oder eine Heteroarylgruppe mit 5 bis 14 aromatischen Ringatomen, welche Reste R⁴ aufweist;
   - R⁴: ist bei jedem Auftreten gleich oder verschieden H, D, F, Cl, Br, I, R⁵C=C(R⁵)₂, N(R⁵)₂, CN, NO₂, OR⁵, SR⁵, Si(R⁵)³, B(OR⁵)₂, C(=O)R⁵, P(=O)(R⁵)², S(=O)R⁵, S(=O)₂R⁵, OSO₂R⁵, eine geradkettige Alkyl-, Alkoxy- oder Thioalkylgruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkylgruppe mit 3 bis 20 C-Atomen, wobei die Alkyl-, Alkoxy- oder Thioalkyl-, Alkenyl- oder Alkinylgruppe jeweils Reste R⁵ aufweist, wobei eine oder mehrere nicht benachbarte CH₂-Gruppen durch R⁵C=CR⁵, C=C, Si(R⁵)₂, C=O, C=S, C=Se, C=NR⁵, -C(=O)O-, -C(=O)NR⁵-, NR⁵, P(=O)( R⁵), -O-, -S-, SO oder SO₂ ersetzt sein können, oder ein aromatisches oder heteroaromatisches Ringsystem mit 5 bis 40 aromatischen Ringatomen, das jeweils Reste R⁵ aufweist, oder eine Aryloxy- oder Heteroaryloxygruppe mit 5 bis 40 aromatischen Ringatomen, die jeweils Reste R⁵ aufweist, oder eine Diarylamino-, Arylheteroarylamino-, Diheteroarylaminogruppe mit 5 bis 60 aromatischen Ringatomen im jeweiligen aromatischen oder heteroaromatischen Rest, wobei die Diarylamino-, Arylheteroarylamino-, Diheteroarylaminogruppe Reste R⁵ aufweist; dabei können zwei oder mehrere Reste R⁴ miteinander ein aromatisches, heteroaromatisches, aliphatisches oder heteroaliphatisches Ringsystem bilden, vorzugsweise ein aliphatisches Ringsystem, besonders bevorzugt bilden die Reste R⁴ kein solches Ringsystem;
   - R⁵: ist bei jedem Auftreten gleich oder verschieden H, D, F oder ein aliphatischer, aromatischer oder heteroaromatischer organischer Rest, insbesondere ein Kohlenwasserstoffrest, mit 1 bis 20 C-Atomen, in dem auch ein oder mehrere H-Atome durch F ersetzt sein können, dabei können zwei oder mehrere Reste R⁵ miteinander ein Ringsystem bilden;
wobei mindestens einer der Reste R, R¹, R², R³ ausgewählt ist aus der Gruppe bestehend aus einem heteroaromatischen Ringsystem mit 6 bis 60 aromatischen Ringatomen, das Reste R⁴ aufweist, einem aromatischen Ringsystem mit 10 bis 60 aromatischen Ringatomen, das Reste R⁴ aufweist, einer Aryloxygruppe mit 10 bis 60 aromatischen Ringatomen oder Heteroaryloxygruppe mit 6 bis 40 aromatischen Ringatomen, die jeweils Reste R⁴ aufweist, einer Diarylaminogruppe mit 6 bis 60 aromatischen Ringatomen im jeweiligen aromatischen Rest, einer Arylheteroarylaminogruppe mit 6 bis 60 aromatischen Ringatomen im jeweiligen aromatischen oder heteroaromatischen Rest und einer Diheteroarylaminogruppe mit 6 bis 60 aromatischen Ringatomen mit 6 bis 60 aromatischen Ringatomen im jeweiligen heteroaromatischen Rest, wobei die Diarylamino-, Arylheteroarylamino-, Diheteroarylaminogruppe Reste R⁴ aufweist.

Die Gruppen R, R¹, R², R³ weisen Reste R⁴ auf, wobei der Rest R⁴ H sein kann. Für den Fall, dass R⁴ ungleich H ist, stellt der Rest R⁴ einen Substituenten dar, so dass die Gruppen R, R¹, R², R³ durch Reste R⁴ substituiert sein können. Diese Klarstellung gilt entsprechend für die weiteren Gruppen und Reste.

Zwei Reste R oder ein Rest R mit einem weiteren Rest, vorzugsweise einer Gruppe R¹, R², R³ können miteinander ein aromatisches, heteroaromatisches, aliphatisches oder heteroaliphatisches Ringsystem bilden. Falls ein Ringsystem gebildet wird, wird dies vorzugsweise durch zwei Reste R¹ gebildet, wobei ein kondensiertes Ringsystem entsteht. Dies gilt auch für die weiteren Reste, insbesondere für zwei Reste R¹, R², R³.

In einer weiterhin bevorzugten Ausgestaltung kann vorgesehen sein, dass die erfindungsgemäßen Verbindungen eine Struktur der Formeln (I-1) bis (I-7) umfassen, wobei die erfindungsgemäßen Verbindungen besonders bevorzugt ausgewählt sein können aus den Verbindungen der Formeln (I-1) bis (I-7), 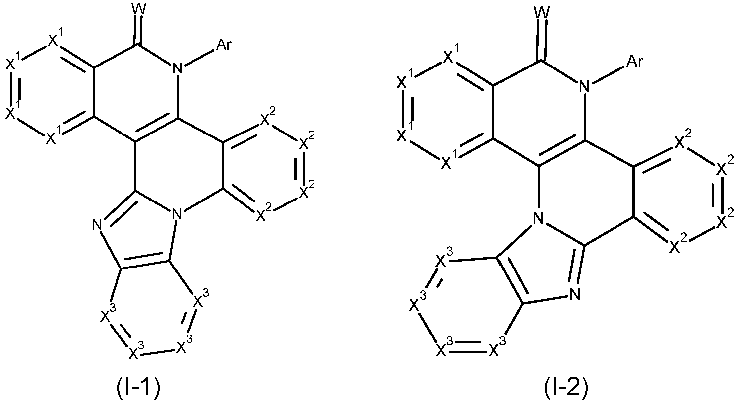 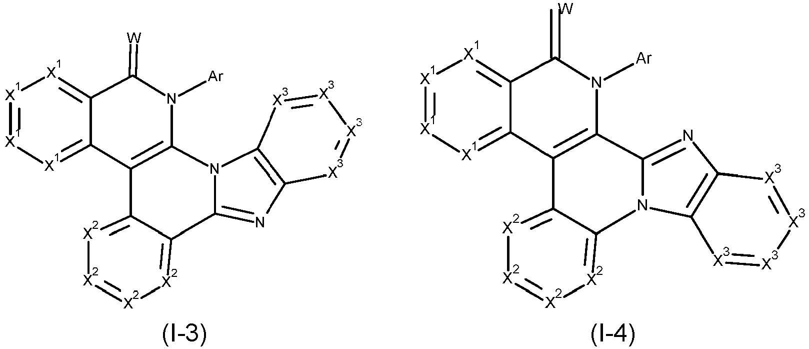 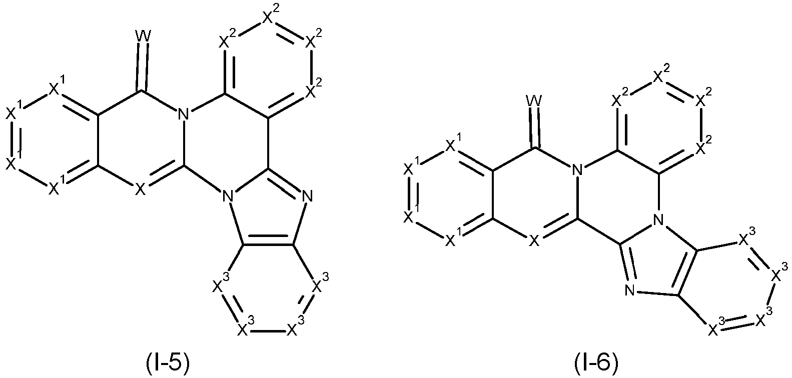 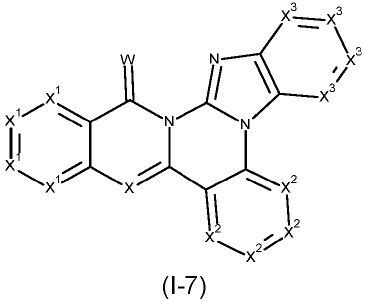 wobei die Symbole W, X, X¹, X², X³ und Ar, die zuvor, insbesondere für Strukturelemente (A), (B) und (C) genannten Bedeutungen aufweisen.

Ferner kann vorgesehen sein, dass in Strukturelement (A) und/oder in Verbindungen der Formeln (I-1) bis (I-7) nicht mehr als eine Gruppe X¹ für N steht, bevorzugt alle Gruppen X¹ für CR¹ stehen, wobei vorzugsweise höchstens 3, besonders bevorzugt höchstens 2 und speziell bevorzugt höchstens 1 der Gruppen CR¹, für die X¹ steht, ungleich der Gruppe CH ist.

In einer Ausführungsform der vorliegenden Erfindung kann vorgesehen sein, dass in Strukturelement (B) und/oder in Verbindungen der Formeln (I-1) bis (I-7) nicht mehr als eine Gruppe X² für N steht, bevorzugt alle Gruppen X² für CR² stehen, wobei vorzugsweise höchstens 3, besonders bevorzugt höchstens 2 und speziell bevorzugt höchstens 1 der Gruppen CR², für die X² steht, ungleich der Gruppe CH ist.

Weiterhin kann vorgesehen sein, dass in Strukturelement (C) und/oder in Verbindungen der Formeln (I-1) bis (I-7) nicht mehr als eine Gruppe X³ für N steht, bevorzugt alle Gruppen X³ für CR³ stehen, wobei vorzugsweise höchstens 3, besonders bevorzugt höchstens 2 und speziell bevorzugt höchstens 1 der Gruppen CR³, für die X³ steht, ungleich der Gruppe CH ist.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Eine elektronenarme Heteroarylgruppe im Sinne der vorliegenden Erfindung ist eine Heteroarylgruppe, die mindestens einen heteroaromatischen Sechsring mit mindestens einem Stickstoffatom aufweist. An diesen Sechsring können noch weitere aromatische oder heteroaromatische Fünfringe oder Sechsringe ankondensiert sein. Beispiele für elektronenarme Heteroarylgruppen sind Pyridin, Pyrimidin, Pyrazin, Pyridazin, Triazin, Chinolin, Chinazolin oder Chinoxalin.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind. Bevorzugt ist das aromatische Ringsystem gewählt aus Fluoren, 9,9'-Spirobifluoren, 9,9-Diarylamin oder Gruppen, in denen zwei oder mehr Aryl- und/oder Heteroarylgruppen durch Einfachbindungen miteinander verknüpft sind.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 20 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 60 bzw. 5 bis 40 aromatischen Ringatomen, welches noch jeweils mit den oben genannten Resten substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombinationen dieser Systeme.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

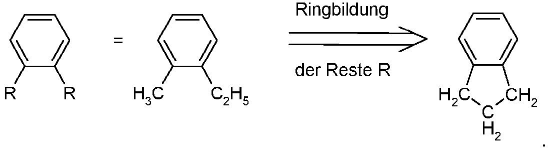

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

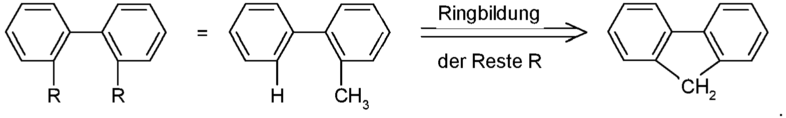

In einer bevorzugten Ausgestaltung können die erfindungsgemäßen Verbindungen vorzugsweise mindestens eine Struktur der Formeln (II-1) bis (II-30) umfassen und sind besonders bevorzugt ausgewählt aus den Verbindungen der Formeln (II-1) bis (II-30), 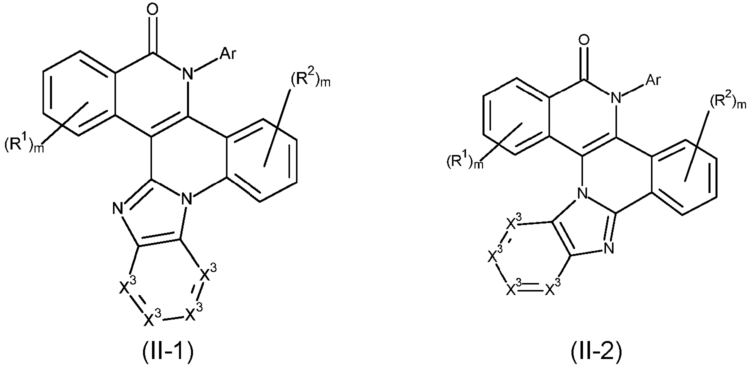 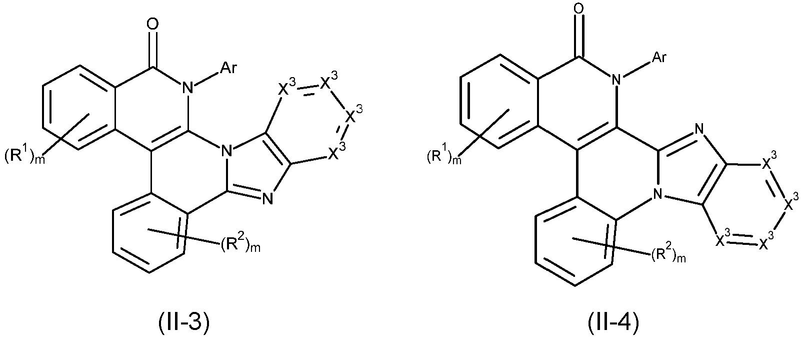 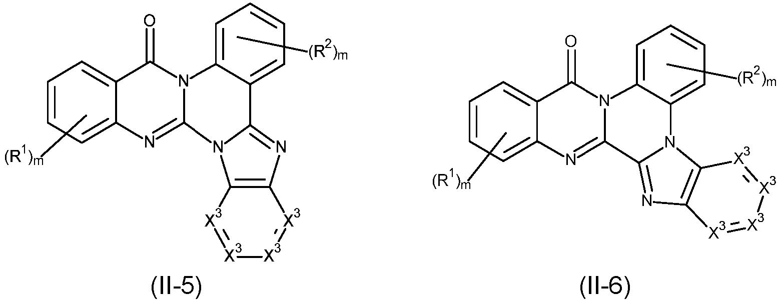 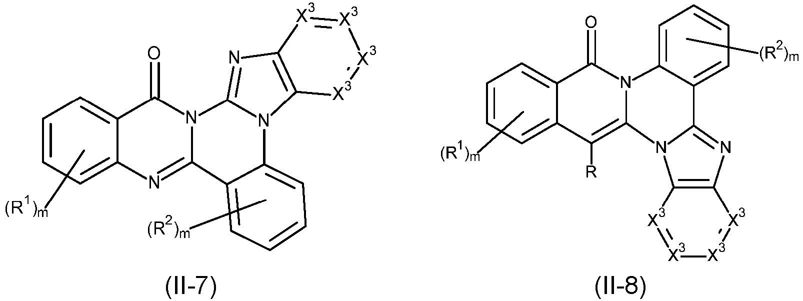 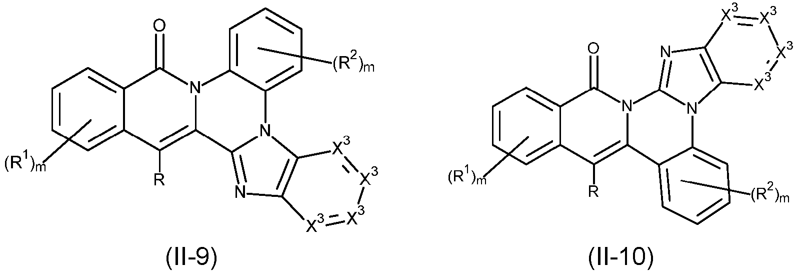 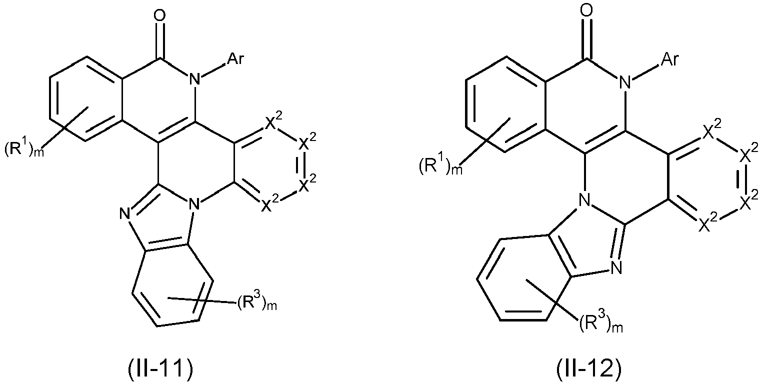 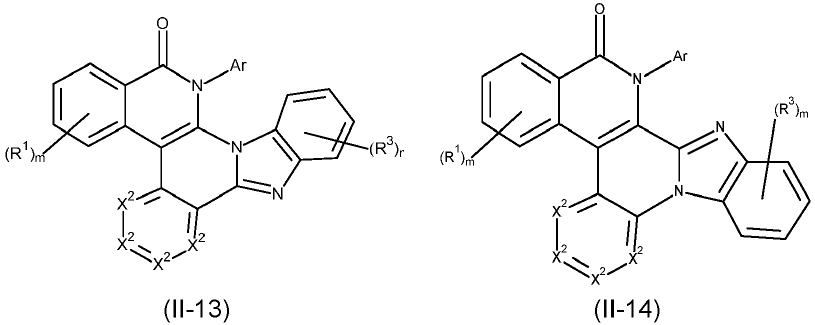 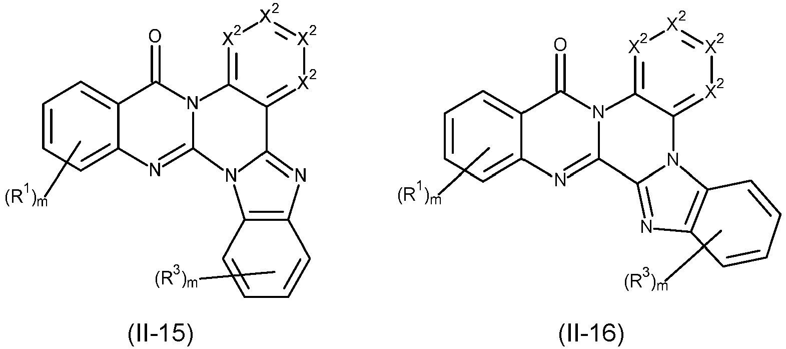 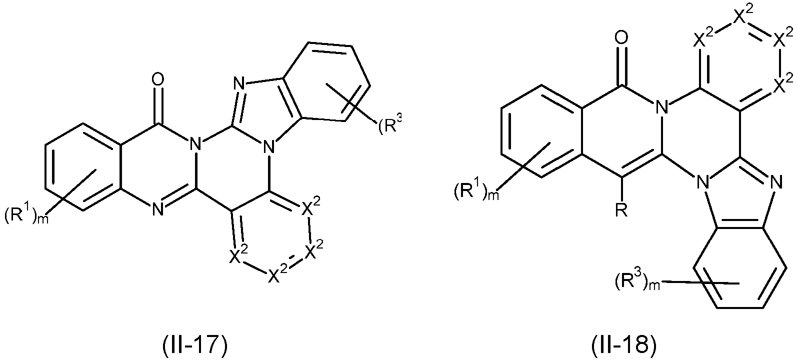 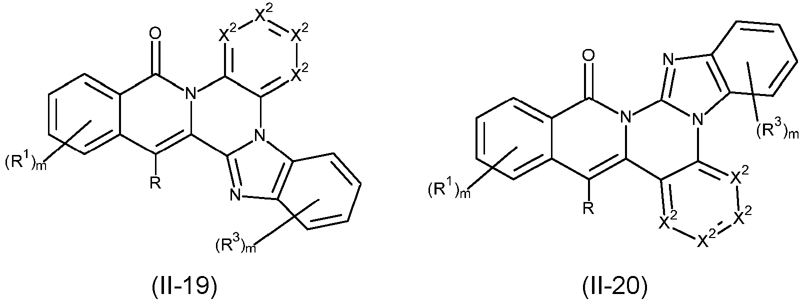 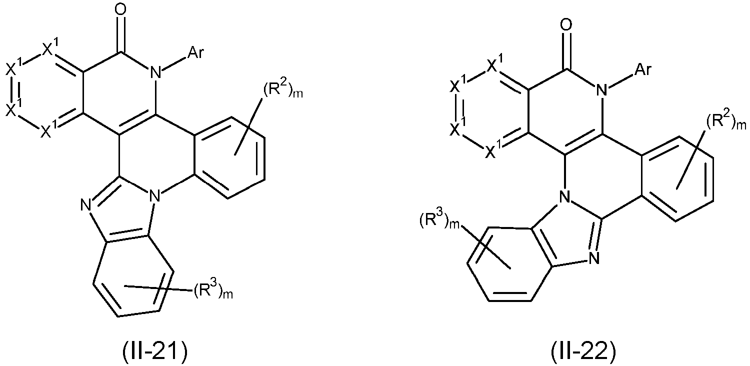 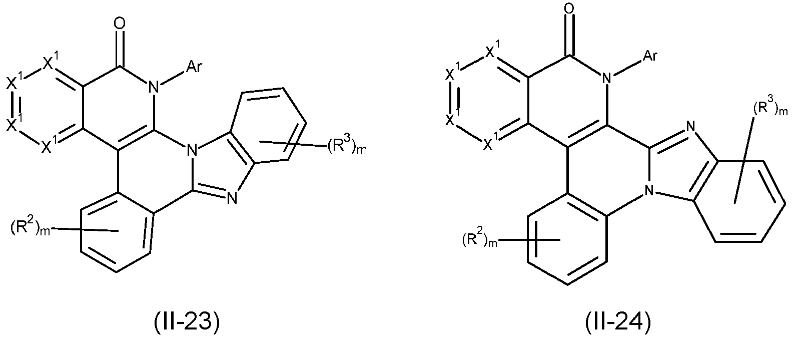 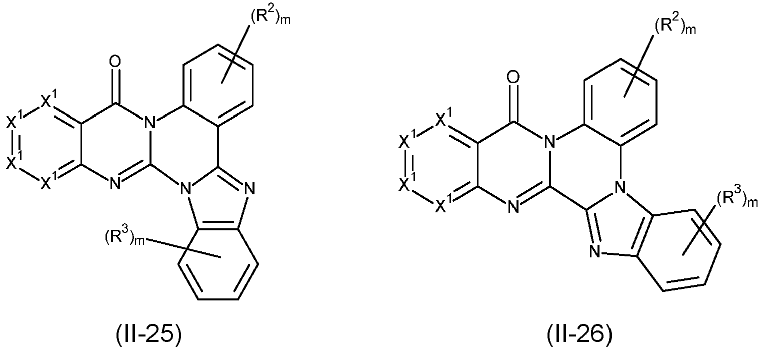 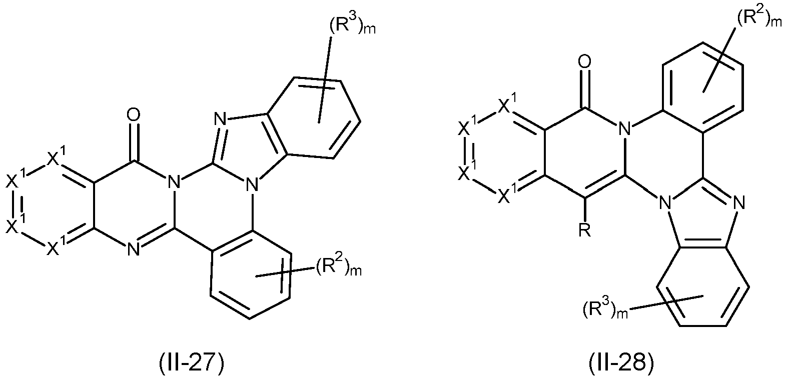 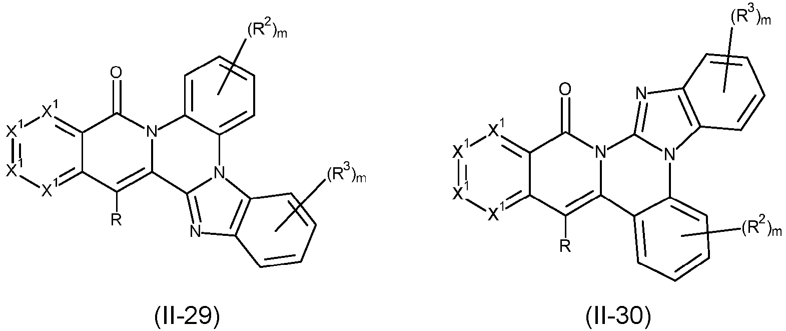 wobei die Symbole X¹, X², X³, R, R¹, R², R³ und Ar, die zuvor, insbesondere für Strukturelemente (A), (B) und (C) genannten Bedeutungen aufweisen und der Index m 0, 1, 2, 3 oder 4, vorzugsweise 0, 1 oder 2 ist. Hierbei sind Strukturen/Verbindungen der Formeln (II-1) bis (II-7), (II-11) bis (II-17) und/oder (II-21) bis (II-27) bevorzugt.

In einer weiterhin bevorzugten Ausgestaltung kann vorgesehen sein, dass die erfindungsgemäßen Verbindungen eine Struktur der Formeln (III-1) bis (III-10) umfassen, wobei die erfindungsgemäßen Verbindungen besonders bevorzugt ausgewählt sein können aus den Verbindungen der Formeln (III-1) bis (III-10), 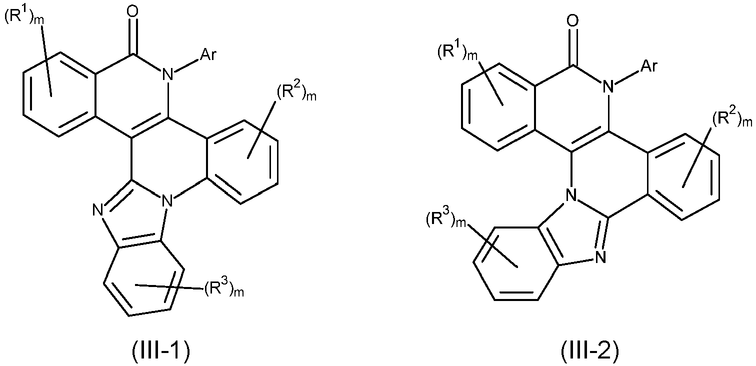 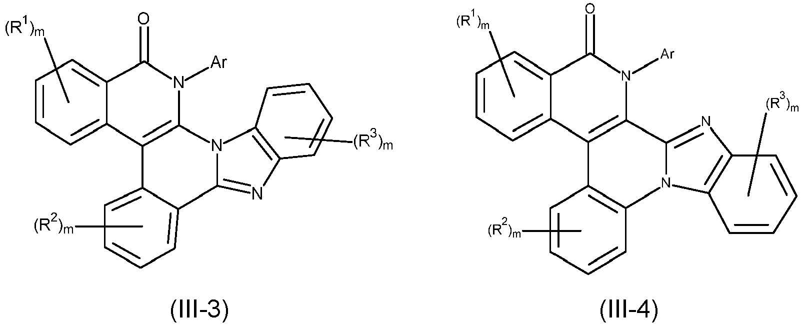 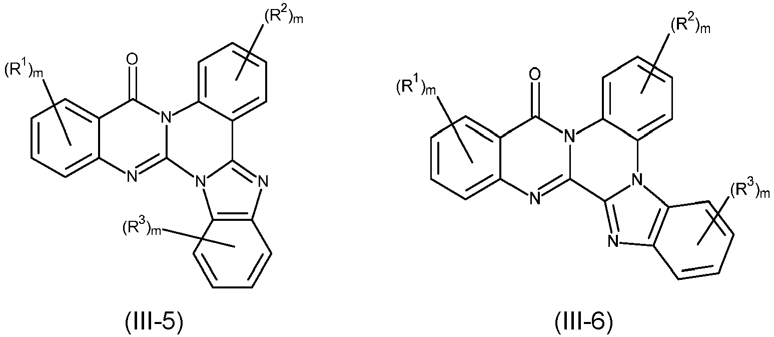 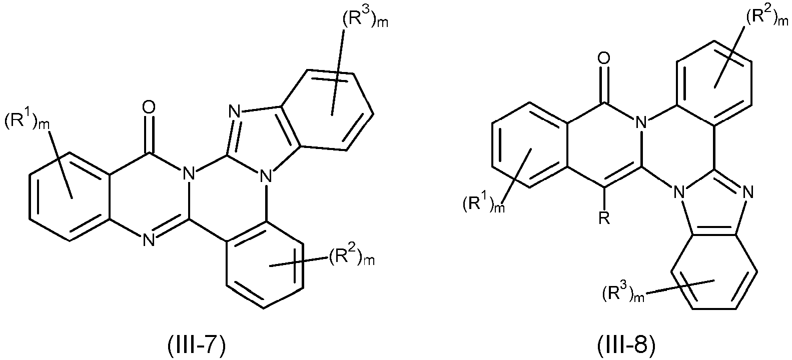 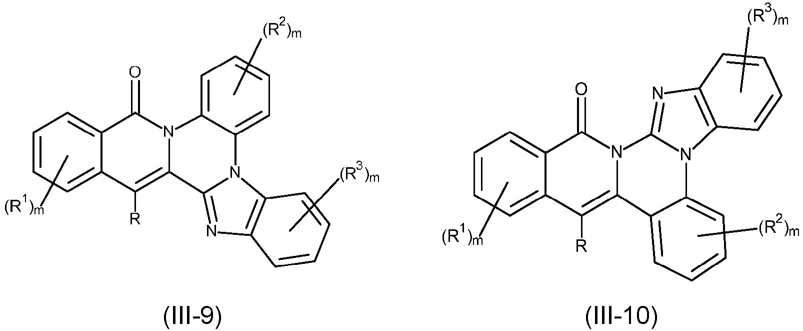 wobei die Symbole R, R¹, R², R³ und Ar, die zuvor, insbesondere für Strukturelemente (A), (B) und (C) genannten Bedeutungen aufweisen und der Index m 0, 1, 2, 3 oder 4, vorzugsweise 0, 1 oder 2 ist. Hierbei sind Strukturen/Verbindungen der Formeln (III-1) bis (III-7) bevorzugt.

Der Index m ist 0, 1, 2, 3 oder 4, vorzugsweise 0, 1 oder 2. Für den Fall, dass der Index m kleiner als 4 ist, weisen die jeweiligen Ringe eine entsprechende Anzahl an H-Atomen auf. Hier ist zu festzuhalten, dass die Gruppen R¹, R², R³ für H stehen können. Falls daher der Index m ungleich 0 ist, weisen diese Ringe vorzugsweise Substituenten R¹, R², R³ auf. Dies bedeutet, dass die entsprechenden Gruppen R¹, R², R³ vorzugsweise für einen Rest ungleich H stehen. Hierbei gelten die zuvor und nachfolgend dargelegten Bevorzugungen für die entsprechenden Gruppen R¹, R², R³. Diese Klarstellung gilt entsprechend für die weiteren Gruppen, Reste, wie zum Beispiel R, R⁴, R⁵, R⁶, R⁷, R⁸ und/oder R⁹, und Indices, insbesondere für n, l und r.

Vorzugsweise beträgt die Summe der Indices m höchstens 6, insbesondere bevorzugt höchstens 4 und besonders bevorzugt höchstens 2. Dies gilt unter anderem für Strukturen/Verbindungen der Formeln (II-1) bis (II-30) und (III-1) bis (III-10).

Weiterhin kann vorgesehen sein, dass die Substituenten R, R¹, R² und R³ gemäß obigen Formeln mit den Ringatomen des Ringsystems kein kondensiertes aromatisches oder heteroaromatisches Ringsystem, vorzugsweise kein kondensiertes Ringsystem bilden. Dies schließt die Bildung eines kondensierten Ringsystems mit möglichen Substituenten R⁴, R⁵ ein, die an die Reste R, R¹, R², R³ gebunden sein können.

Wenn zwei Reste, die insbesondere ausgewählt sein können aus R, R², R³, R⁴, R⁵, R⁶, R⁷, R⁸ und/oder R⁹, miteinander ein Ringsystem bilden, so kann dieses mono- oder polycyclisch, aliphatisch, heteroaliphatisch, aromatisch oder heteroaromatisch sein. Dabei können die Reste, die miteinander ein Ringsystem bilden, benachbart sein, d.h. dass diese Reste an dasselbe Kohlenstoffatom oder an Kohlenstoffatome, die direkt aneinander gebunden sind, gebunden sind, oder sie können weiter voneinander entfernt sein. Weiterhin können die mit den Substituenten R, R¹, R², R³, R⁴, R⁵, R⁶, R⁷, R⁸ und/oder R⁹ versehenen Ringsysteme auch über eine Bindung miteinander verbunden sein, so dass hierdurch ein Ringschluss bewirkt werden kann.

Vorzugsweise kann vorgesehen sein, dass mindestens eine Gruppe R, R¹, R² und/oder R³ gleich oder verschieden ausgewählt ist aus den Resten der folgenden Formeln SAr-1 bis SAr-18 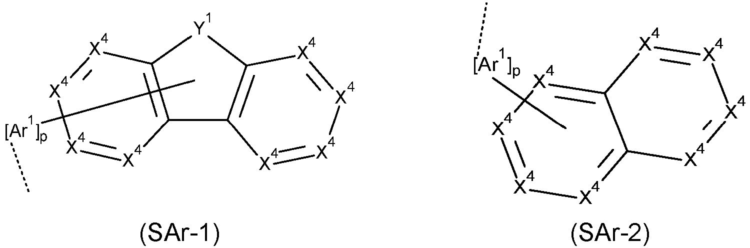 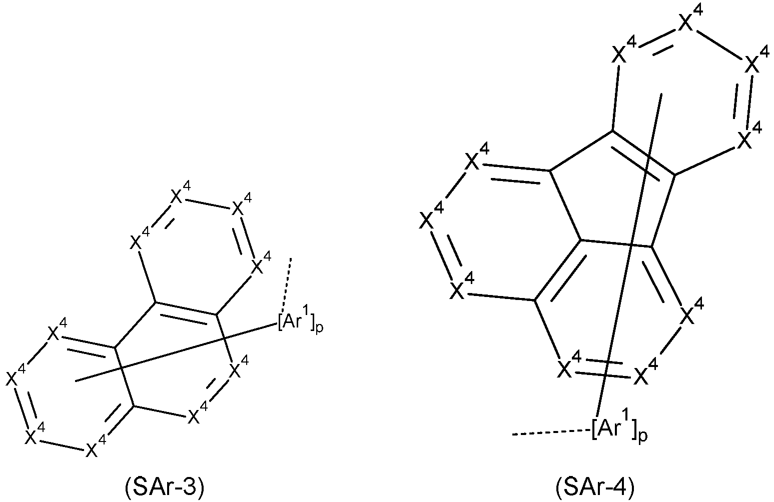 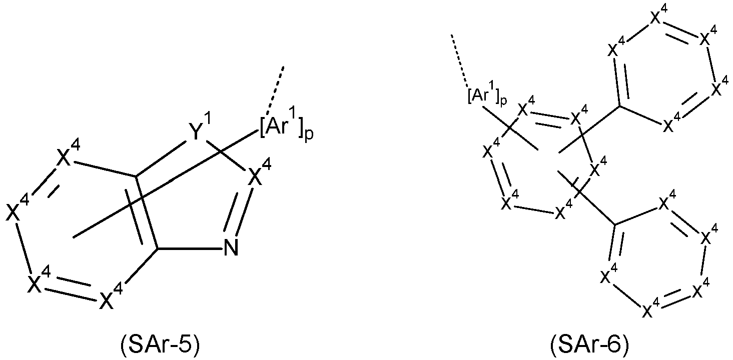 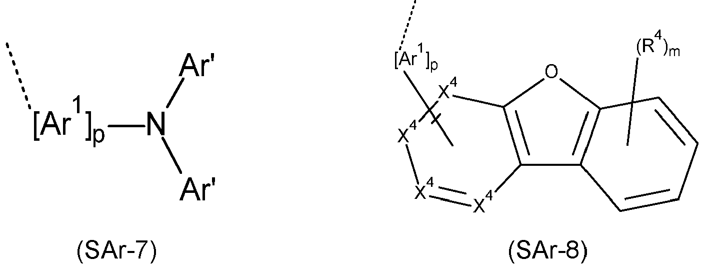 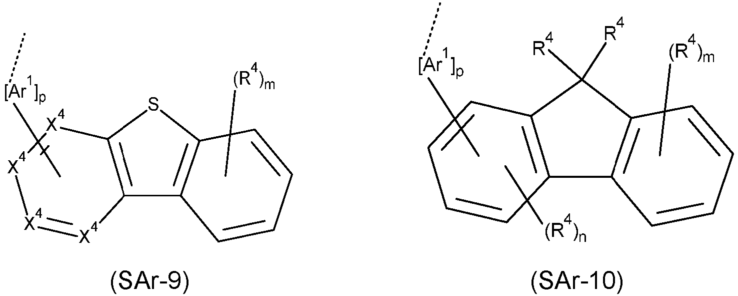 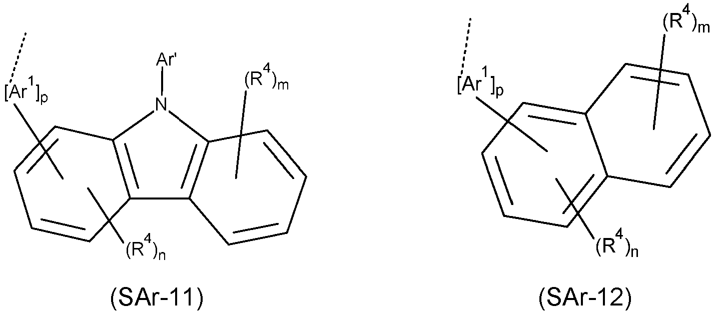 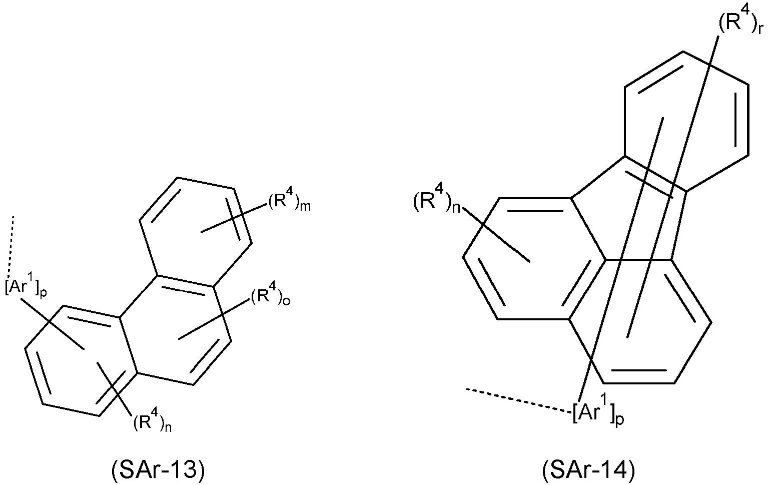 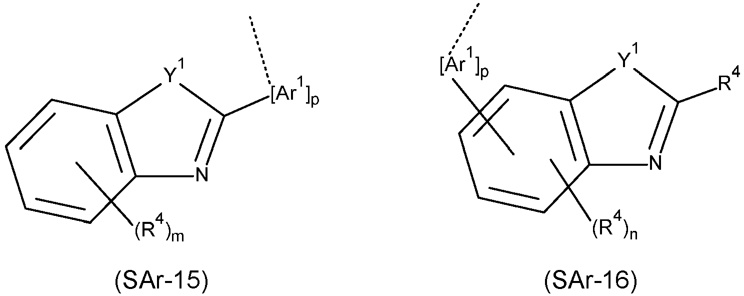 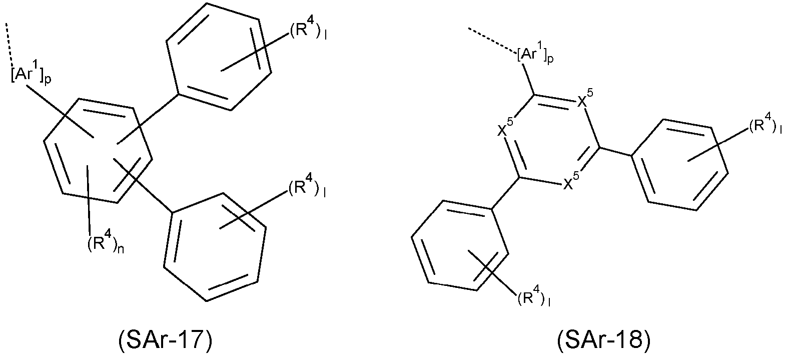 wobei R⁴ und Ar' die zuvor, insbesondere für Strukturelemente (A), (B) und (C) genannten Bedeutungen aufweisen, die gestrichelte Bindung die Bindung an die entsprechende Gruppe darstellt und für die weiteren Symbole und Indices gilt:
- X⁴: ist bei jedem Auftreten gleich oder verschieden CR⁴, N oder C, falls hieran die Gruppe [Ar¹]ₚ bindet, vorzugsweise CR⁴, wobei bevorzugt keine N-N-Bindungen vorhanden sind;
- X⁵: ist bei jedem Auftreten gleich oder verschieden CR⁴ oder N, vorzugsweise N;
- Ar¹: ist bei jedem Auftreten gleich oder verschieden ein bivalentes aromatisches oder heteroaromatisches Ringsystem mit 6 bis 18 aromatischen Ringatomen, welches jeweils Reste R⁴ aufweist;
- Y¹: ist bei jedem Auftreten gleich oder verschieden C(R⁴)₂, NR⁴, O, S oder N, falls hieran die Gruppe [Ar¹]ₚ bindet;
- p: ist 0 oder 1, wobei p = 0 bedeutet, dass die Gruppe Ar¹ nicht vorhanden ist und dass die entsprechende aromatische bzw. heteroaromatische Gruppe direkt an den entsprechenden Rest gebunden ist;
- n: ist 0, 1, 2 oder 3, vorzugsweise 0, 1 oder 2;
- m: ist 0, 1, 2, 3 oder 4, vorzugsweise 0, 1 oder 2;
- l: ist 0, 1, 2, 3, 4 oder 5, vorzugsweise 0, 1 oder 2;
- r: ist 0, 1, 2, 3, 4, 5 oder 6, vorzugsweise 0, 1 oder 2.

Hierbei sind Strukturen der Formeln (SAr-1), (SAr-4), (SAr-8), (SAr-10), (SAr-11), (SAr-14), (SAr-18) bevorzugt.

Ferner kann vorgesehen sein, dass R, R¹, R² und/oder R³ gleich oder verschieden bei jedem Auftreten ausgewählt ist aus der Gruppe bestehend aus H, D oder einem aromatischen oder heteroaromatischen Ringsystem ausgewählt aus den Gruppen der folgenden Formeln Ar-1 bis Ar-79, vorzugsweise mindestens eine Gruppe R, R¹, R² und/oder R³ gleich oder verschieden ausgewählt ist aus den Gruppen der folgenden Formeln Ar-1 bis Ar-79 und/oder die Gruppe Ar' gleich oder verschieden bei jedem Auftreten ausgewählt ist aus den Gruppen der folgenden Formeln Ar-1 bis Ar-79, 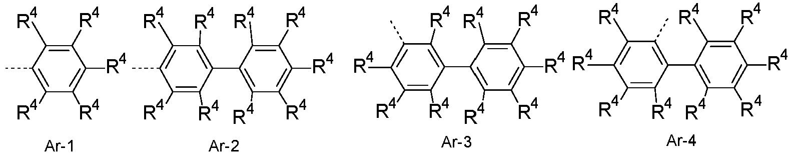 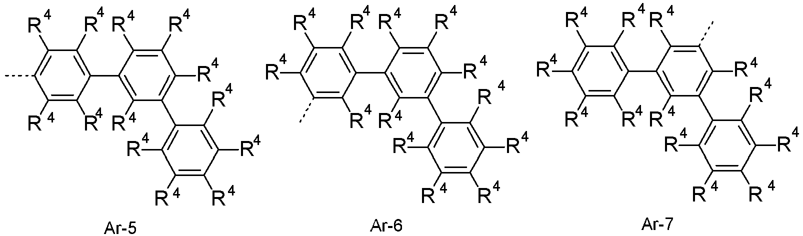 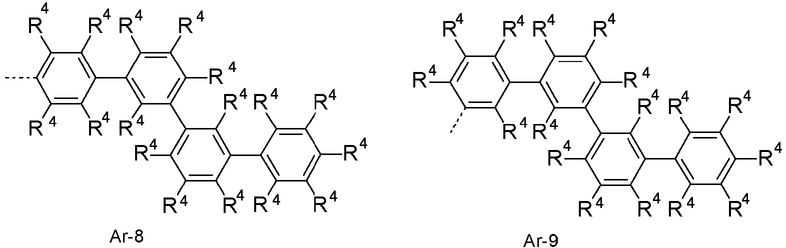 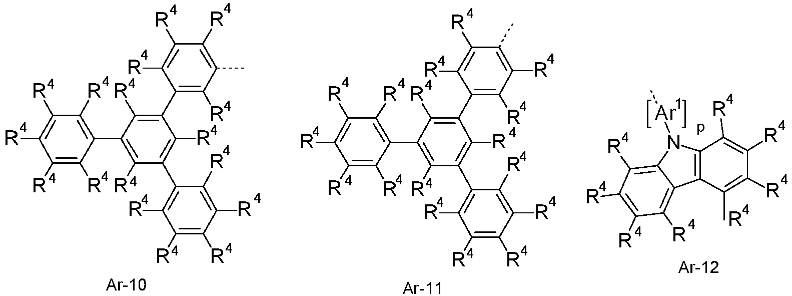 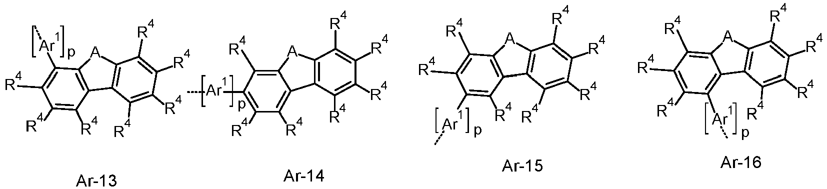 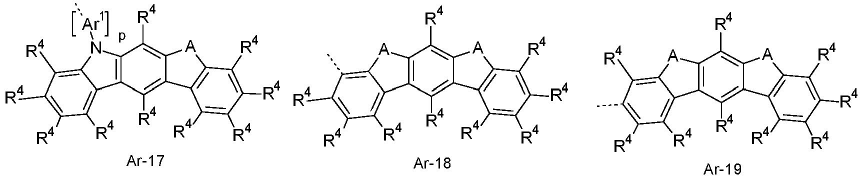 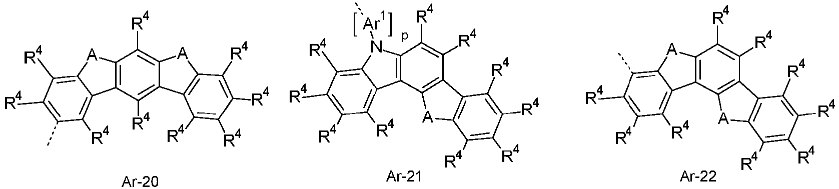 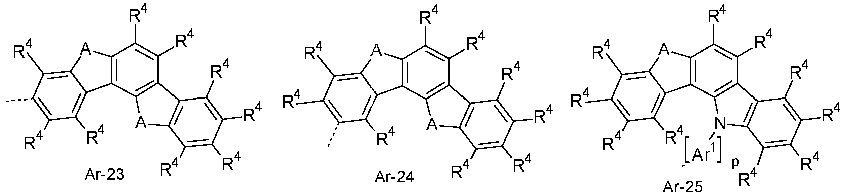 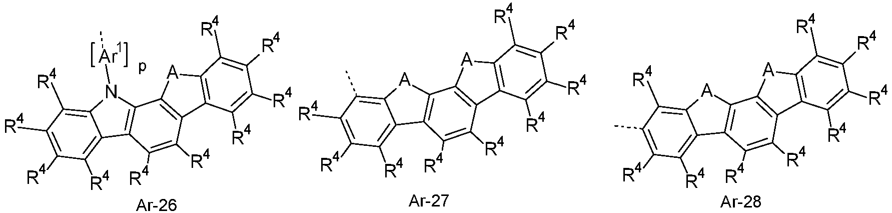 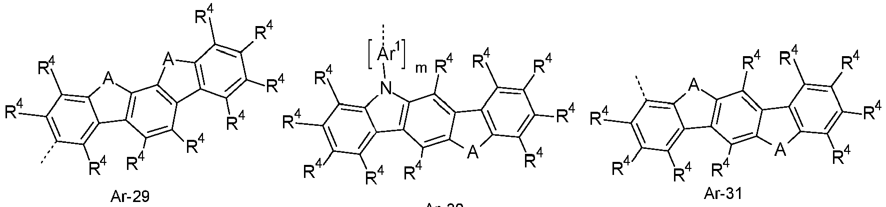 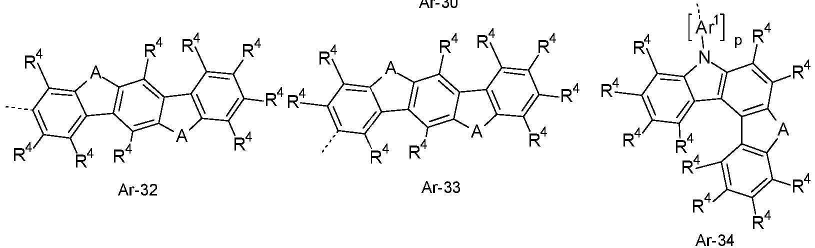 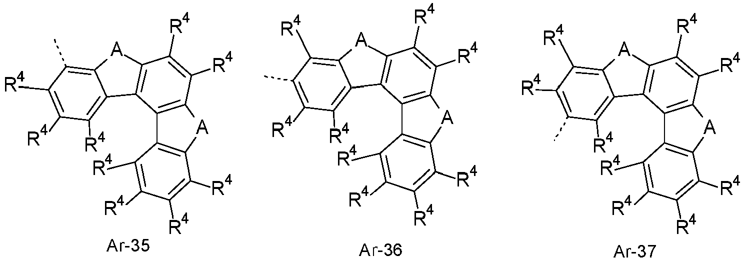 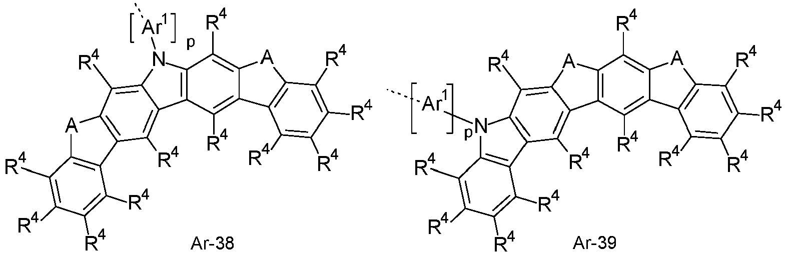 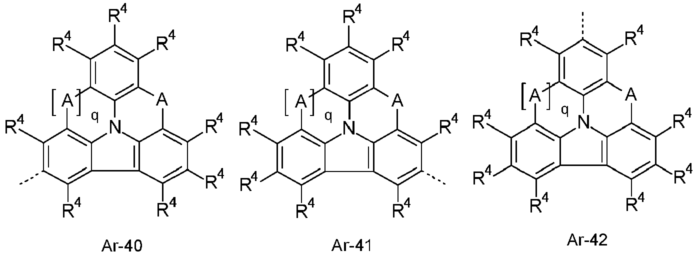 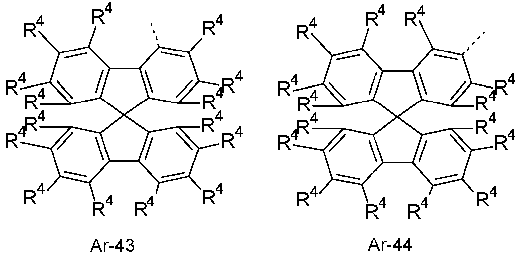 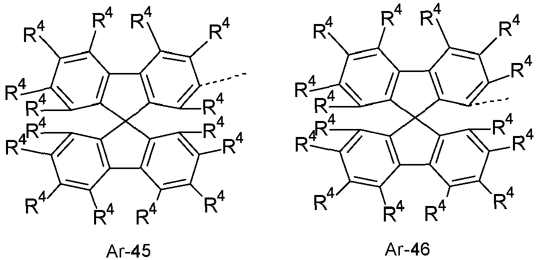 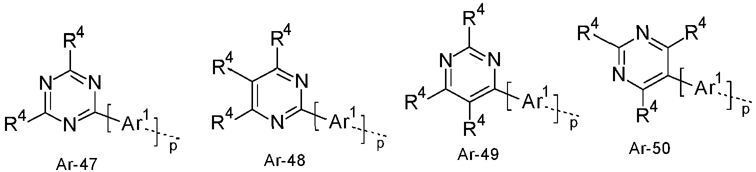 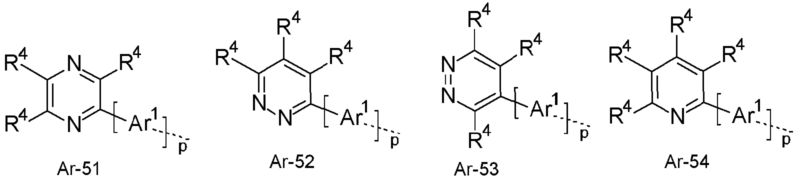 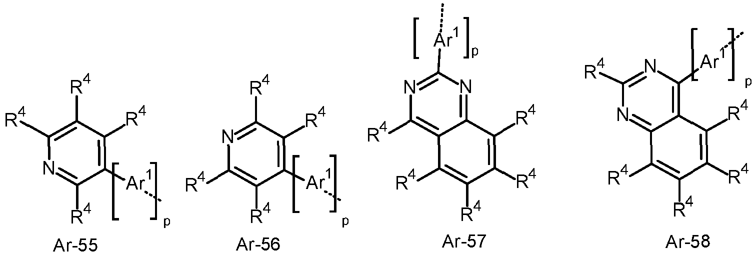 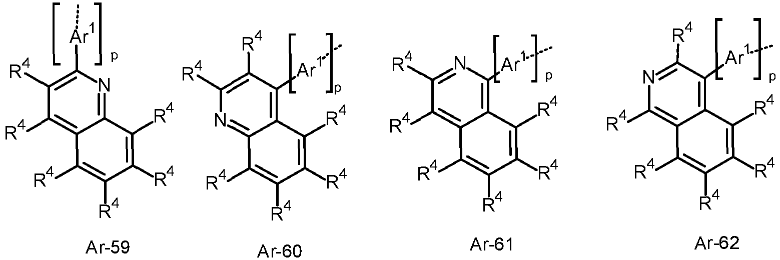 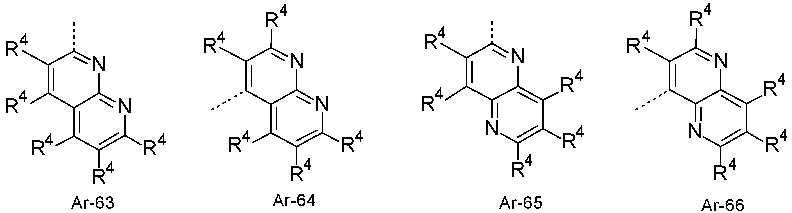 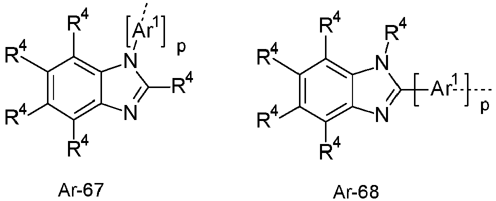 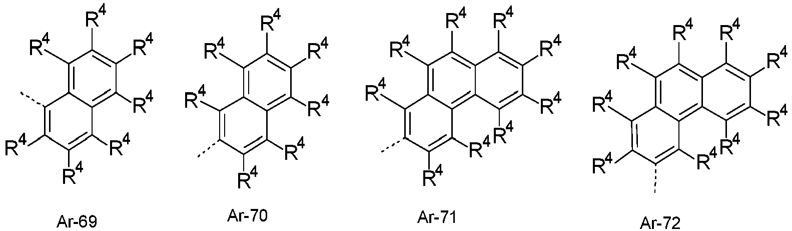 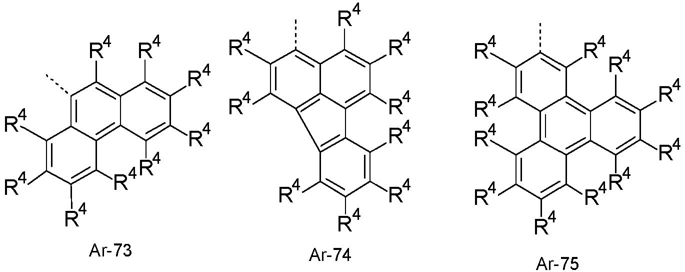 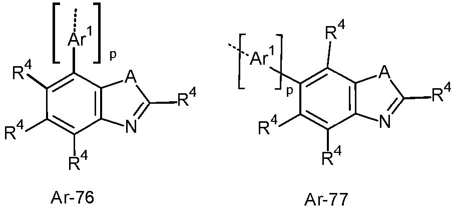 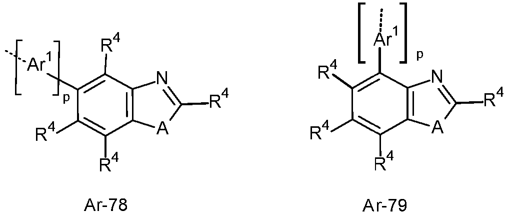 wobei R⁴ die oben genannten Bedeutungen aufweist, die gestrichelte Bindung die Bindung an die entsprechende Gruppe darstellt und weiterhin gilt:
- Ar¹: ist bei jedem Auftreten gleich oder verschieden ein bivalentes aromatisches oder heteroaromatisches Ringsystem mit 6 bis 18 aromatischen Ringatomen, welches jeweils Reste R⁴ aufweist;
- A: ist bei jedem Auftreten gleich oder verschieden C(R⁴)₂, NR⁴, O oder S;
- p: ist 0 oder 1, wobei p = 0 bedeutet, dass die Gruppe Ar¹ nicht vorhanden ist und dass die entsprechende aromatische bzw. heteroaromatische Gruppe direkt an den entsprechenden Rest gebunden ist;
- q: ist 0 oder 1, wobei q = 0 bedeutet, dass an dieser Position keine Gruppe A gebunden ist und an die entsprechenden Kohlenstoffatome statt dessen Reste R⁴ gebunden sind.

Hierbei sind Strukturen der Formeln (Ar-1), (Ar-2), (Ar-3), (Ar-12), (Ar-13), (Ar-14), (Ar-15), (Ar-16), (Ar-47), (Ar-70), (Ar-75), (Ar-76), (Ar-77), (Ar-78), (Ar-79), bevorzugt und Strukturen der Formeln (Ar-1), (Ar-2), (Ar-3), (Ar-12), (Ar-13), (Ar-14), (Ar-15), (Ar-16) besonders bevorzugt.

Wenn die oben genannten Gruppen für R, R¹, R², R³ mehrere Gruppen A aufweisen, so kommen hierfür alle Kombinationen aus der Definition von A in Frage. Bevorzugte Ausführungsformen sind dann solche, in denen eine Gruppe A für NR⁴ und die andere Gruppe A für C(R⁴)₂ steht oder in denen beide Gruppen A für NR⁴ stehen oder in denen beide Gruppen A für O stehen.

Wenn A für NR⁴ steht, steht der Substituent R⁴, der an das Stickstoffatom gebunden ist, bevorzugt für ein aromatisches oder heteroaromatisches Ringsystem mit 5 bis 24 aromatischen Ringatomen, welches auch durch einen oder mehrere Reste R⁵ substituiert sein kann. In einer besonders bevorzugten Ausführungsform steht dieser Substituent R⁴ gleich oder verschieden bei jedem Auftreten für ein aromatisches oder heteroaromatisches Ringsystem mit 6 bis 24 aromatischen Ringatomen, insbesondere mit 6 bis 18 aromatischen Ringatomen, welches keine kondensierten Arylgruppen aufweist und welches keine kondensierten Heteroarylgruppen, in denen zwei oder mehr aromatische bzw. heteroaromatische 6-RingGruppen direkt aneinander ankondensiert sind, aufweist, und welches jeweils auch durch einen oder mehrere Reste R⁵ substituiert sein kann. Bevorzugt sind Phenyl, Biphenyl, Terphenyl und Quaterphenyl mit Verknüpfungsmustern, wie vorne für Ar-1 bis Ar-11 aufgeführt, wobei diese Strukturen statt durch R⁴ durch einen oder mehrere Reste R⁵ substituiert sein können, bevorzugt aber unsubstituiert sind. Bevorzugt sind weiterhin Triazin, Pyrimidin und Chinazolin, wie vorne für Ar-47 bis Ar-50, Ar-57 und Ar-58 aufgeführt, wobei diese Strukturen statt durch R⁴ durch einen oder mehrere Reste R⁵ substituiert sein können.

Wenn A für C(R⁴)₂ steht, stehen die Substituenten R⁴, die an dieses Kohlenstoffatom gebunden sind, bevorzugt gleich oder verschieden bei jedem Auftreten für eine lineare Alkylgruppe mit 1 bis 10 C-Atomen oder für eine verzweigte oder cyclische Alkylgruppe mit 3 bis 10 C-Atomen oder für ein aromatisches oder heteroaromatisches Ringsystem mit 5 bis 24 aromatischen Ringatomen, welches auch durch einen oder mehrere Reste R⁵ substituiert sein kann. Ganz besonders bevorzugt steht R⁴ für eine Methylgruppe oder für eine Phenylgruppe. Dabei können die Reste R⁴ auch miteinander ein Ringsystem bilden, was zu einem Spirosystem führt.

Vorzugsweise kann vorgesehen sein, dass Ar gleich oder verschieden bei jedem Auftreten aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, insbesondere 1- oder 2-verknüpftem Naphthalin, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R substituiert sein können. Die Gruppe Ar ist insbesondere in Strukturelement (A) für den Fall enthalten, dass Z¹ für NAr steht. Ferner ist diese Gruppe explizit unter anderem in Formeln (I-1) bis (I-4) sowie bevorzugten Ausführungsformen dieser Formeln dargelegt. Besonders bevorzugte Gruppen Ar sind die oben aufgeführten Strukturen Ar-1 bis Ar-79, wobei Strukturen der Formeln (Ar-1), (Ar-2), (Ar-3), (Ar-12), (Ar-13), (Ar-14), (Ar-15), (Ar-16), (Ar-47), (Ar-70), (Ar-75), (Ar-76), (Ar-77), (Ar-78), (Ar-79), bevorzugt und Strukturen der Formeln (Ar-1), (Ar-2), (Ar-3), (Ar-12), (Ar-13), (Ar-14), (Ar-15), (Ar-16) besonders bevorzugt sind. In den zuvor dargelegten Strukturen Ar-1 bis Ar-79 sind in Bezug auf die Reste Ar die Substituenten R⁴ durch die entsprechenden Reste R zu ersetzen.

Im Folgenden werden bevorzugte Substituenten R, R¹, R² und R³ beschrieben.

In einer bevorzugten Ausführungsform der Erfindung ist R, R¹, R² und R³ gleich oder verschieden bei jedem Auftreten ausgewählt aus der Gruppe bestehend aus H, D, F, CN, NO₂, Si(R⁴)₃, B(OR⁴)₂, eine geradkettige Alkylgruppe mit 1 bis 20 C-Atomen oder eine verzweigte oder cyclische Alkylgruppe mit 3 bis 20 C-Atomen, wobei die Alkylgruppe jeweils mit einem oder mehreren Resten R⁴ substituiert sein kann, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R⁴ substituiert sein kann.

In einer weiterhin bevorzugten Ausführungsform der Erfindung ist R, R¹, R² und R³ gleich oder verschieden bei jedem Auftreten ausgewählt aus der Gruppe bestehend aus H, D, F, eine geradkettige Alkylgruppe mit 1 bis 20 C-Atomen oder eine verzweigte oder cyclische Alkylgruppe mit 3 bis 20 C-Atomen, wobei die Alkylgruppe jeweils mit einem oder mehreren Resten R⁴ substituiert sein kann, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R⁴ substituiert sein kann.

In einer weiterhin bevorzugten Ausführungsform der Erfindung ist R, R¹, R² und R³ gleich oder verschieden bei jedem Auftreten ausgewählt aus der Gruppe bestehend aus H, D, einem aromatischen oder heteroaromatischen Ringsystem mit 6 bis 30 aromatischen Ringatomen, welches mit einem oder mehreren Resten R⁴ substituiert sein kann, oder einer Gruppe N(Ar')₂. Besonders bevorzugt ist R, R¹, R² gleich oder verschieden bei jedem Auftreten ausgewählt aus der Gruppe bestehend aus H oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren Resten R⁴ substituiert sein kann.

Bevorzugte aromatische bzw. heteroaromatische Ringsysteme, für die die Reste R, R¹, R², R³ bzw. Ar' vorzugsweise stehen können, sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, insbesondere 1- oder 2-verknüpftem Naphthalin, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R⁴ substituiert sein können. Besonders bevorzugt sind die oben aufgeführten Strukturen Ar-1 bis Ar-79, wobei Strukturen der Formeln (Ar-1), (Ar-2), (Ar-3), (Ar-12), (Ar-13), (Ar-14), (Ar-15), (Ar-16), (Ar-47), (Ar-70), (Ar-75), (Ar-76), (Ar-77), (Ar-78), (Ar-79), bevorzugt und Strukturen der Formeln (Ar-1), (Ar-2), (Ar-3), (Ar-12), (Ar-13), (Ar-14), (Ar-15), (Ar-16) besonders bevorzugt sind.

Ferner kann vorgesehen sein, dass zwei benachbarte Gruppen X¹, X², X³, für CR¹, CR² oder CR³ stehen, vorzugsweise zwei benachbarte Gruppen X², X³ für CR² oder CR³ stehen, wobei die zwei benachbarten Substituenten R¹, R², R³ ein kondensiertes aromatisches oder heteroaromatisches Ringsystem mit 5 bis 40 aromatischen Ringatomen bilden, welches mit einem oder mehreren Resten R⁴ substituiert sein kann, wobei mit den Ringatomen, der durch die Gruppen R¹, R², R³ substituierten 6-Ringen ein kondensiertes Ringsystem mit mindestens zwei Ringen gebildet wird, welches vorzugsweise ausgewählt ist aus Naphthalin, Indol, Carbazol, Dibenzofuran, Dibenzothiophen, Indenocarbazol, Indolocarbazol, Chinolin, Isochinolin, Chinazolin, Chinoxalin, Phenanthren oder Triphenylen.

Weitere geeignete Gruppen R, R¹, R² und R³ sind Gruppen der Formel -Ar⁴-N(Ar²)(Ar³), wobei Ar², Ar³ und Ar⁴ gleich oder verschieden bei jedem Auftreten für ein aromatisches oder heteroaromatisches Ringsystem mit 5 bis 24 aromatischen Ringatomen stehen, welches jeweils mit einem oder mehreren Resten R⁴ substituiert sein kann. Dabei beträgt die Gesamtzahl der aromatischen Ringatome von Ar², Ar³ und Ar⁴ maximal 60 und bevorzugt maximal 40.

Dabei können Ar⁴ und Ar² miteinander und/oder Ar² und Ar³ miteinander auch durch eine Gruppe ausgewählt aus C(R⁴)₂, NR⁴, O oder S verbunden sein. Bevorzugt erfolgt die Verknüpfung von Ar⁴ und Ar² miteinander bzw. von Ar² und Ar³ miteinander jeweils ortho zur Position der Verknüpfung mit dem Stickstoffatom. In einer weiteren Ausführungsform der Erfindung sind keine der Gruppen Ar², Ar³ bzw. Ar⁴ miteinander verbunden.

Bevorzugt ist Ar⁴ ein aromatisches oder heteroaromatisches Ringsystem mit 6 bis 24 aromatischen Ringatomen, bevorzugt mit 6 bis 12 aromatischen Ringatomen, das jeweils mit einem oder mehreren Resten R⁴ substituiert sein kann. Besonders bevorzugt ist Ar⁴ ausgewählt aus der Gruppe bestehend aus ortho-, meta- oder para-Phenylen oder ortho-, meta- oder para-Biphenyl, welche jeweils durch einen oder mehrere Reste R⁴ substituiert sein können, bevorzugt aber unsubstituiert sind. Ganz besonders bevorzugt ist Ar⁴ eine unsubstituierte Phenylengruppe.

Bevorzugt sind Ar² und Ar³ gleich oder verschieden bei jedem Auftreten ein aromatisches oder heteroaromatisches Ringsystem mit 6 bis 24 aromatischen Ringatomen, das jeweils mit einem oder mehreren Resten R⁴ substituiert sein kann. Besonders bevorzugte Gruppen Ar² bzw. Ar³ sind gleich oder verschieden bei jedem Auftreten ausgewählt aus der Gruppe bestehend aus Benzol, ortho-, meta- oder para-Biphenyl, ortho-, meta-, para- oder verzweigtem Terphenyl, ortho-, meta-, para- oder verzweigtem Quaterphenyl, 1-, 2-, 3- oder 4-Fluorenyl, 1-, 2-, 3- oder 4-Spirobifluorenyl, 1- oder 2-Naphthyl, Indol, Benzofuran, Benzothiophen, 1-, 2-, 3- oder 4-Carbazol, 1-, 2-, 3- oder 4-Dibenzofuran, 1-, 2-, 3- oder 4-Dibenzothiophen, Indenocarbazol, Indolocarbazol, 2-, 3- oder 4-Pyridin, 2-, 4- oder 5-Pyrimidin, Pyrazin, Pyridazin, Triazin, Phenanthren oder Triphenylen, welche jeweils mit einem oder mehreren Resten R¹ substituiert sein können. Ganz besonders bevorzugt sind Ar² und Ar³ gleich oder verschieden bei jedem Auftreten ausgewählt aus der Gruppe bestehend aus Benzol, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, insbesondere 1-, 2-, 3- oder 4-Fluoren, oder Spirobifluoren, insbesondere 1-, 2-, 3- oder 4-Spirobifluoren.

In einer weiteren bevorzugten Ausführungsform der Erfindung ist R⁴ gleich oder verschieden bei jedem Auftreten ausgewählt aus der Gruppe bestehend aus H, D, F, CN, einer geradkettigen Alkylgruppe mit 1 bis 10 C-Atomen oder einer verzweigten oder cyclischen Alkylgruppe mit 3 bis 10 C-Atomen, wobei die Alkylgruppe jeweils mit einem oder mehreren Resten R² substituiert sein kann, oder einem aromatischen oder heteroaromatischen Ringsystem mit 6 bis 24 aromatischen Ringatomen, das jeweils durch einen oder mehrere Reste R⁵ substituiert sein kann. In einer besonders bevorzugten Ausführungsform der Erfindung ist R⁴ gleich oder verschieden bei jedem Auftreten ausgewählt aus der Gruppe bestehend aus H, einer geradkettigen Alkylgruppe mit 1 bis 6 C-Atomen, insbesondere mit 1, 2, 3 oder 4 C-Atomen, oder einer verzweigten oder cyclischen Alkylgruppe mit 3 bis 6 C-Atomen, wobei die Alkylgruppe mit einem oder mehreren Resten R⁵ substituiert sein kann, bevorzugt aber unsubstituiert ist, oder einem aromatischen oder heteroaromatischen Ringsystem mit 6 bis 13 aromatischen Ringatomen, das jeweils durch einen oder mehrere Reste R⁵ substituiert sein kann, bevorzugt aber unsubstituiert ist.

In einer weiteren bevorzugten Ausführungsform der Erfindung ist R⁵ gleich oder verschieden bei jedem Auftreten H, eine Alkylgruppe mit 1 bis 4 C-Atomen oder eine Arylgruppe mit 6 bis 10 C-Atomen, welche mit einer Alkylgruppe mit 1 bis 4 C-Atomen substituiert sein kann, bevorzugt aber unsubstituiert ist.

Dabei haben in erfindungsgemäßen Verbindungen, die durch Vakuumverdampfung verarbeitet werden, die Alkylgruppen bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgruppen, insbesondere verzweigten Alkylgruppen, mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

Ferner kann vorgesehen sein, dass die Verbindung genau zwei oder genau drei Strukturen gemäß Formeln (I-1) bis (I-7), (II-1) bis (II-30) und/oder (III-1) bis (III-10) umfasst, wobei vorzugsweise eines der aromatischen oder heteroaromatischen Ringsysteme, die durch die mindestens einer der Gruppen R, R¹, R², R³ darstellbar ist oder an das die Gruppen R, R¹, R², R³ binden, von beiden Strukturen geteilt wird. Weiterhin kann vorgesehen sein, dass die Verbindung eine Verbindungsgruppe umfasst, über die die genau zwei oder drei Strukturen gemäß Formeln (I-1) bis (I-7), (II-1) bis (II-30) und/oder (III-1) bis (III-10) miteinander verbunden sind. Diese Verbindungsgruppen sind vorzugsweise von Gruppen abgeleitet, die für die Gruppen R, R¹, R², R³ definiert sind, wobei jedoch ein oder zwei Wasserstoffatome durch Bindungsstellen zu ersetzen sind. Gemäß einer weiteren Ausgestaltung kann eine erfindungsgemäße Verbindung umfassend Strukturen gemäß Formeln (I-1) bis (I-7), (II-1) bis (II-30) und/oder (III-1) bis (III-10) als Oligomer, Polymer oder Dendrimer ausgestaltet sein, wobei statt eines Wasserstoffatoms oder eines Substituenten eine oder mehrere Bindungen der Verbindungen zum Polymer, Oligomer oder Dendrimer vorhanden sind.

Wenn die Verbindungen Strukturelemente (A), (B) und (C) und/oder Verbindung umfassend Strukturen gemäß Formeln (I-1) bis (I-7) bzw. die bevorzugten Ausführungsformen als Matrixmaterial für einen phosphoreszierenden Emitter oder in einer Schicht, die direkt an eine phosphoreszierende Schicht angrenzt, verwendet werden, ist es weiterhin bevorzugt, wenn die Verbindung keine kondensierten Aryl- bzw. Heteroarylgruppen enthält, in denen mehr als zwei Sechsringe direkt aneinander ankondensiert sind. Eine Ausnahme hiervon bilden Phenanthren und Triphenylen, die aufgrund ihrer hohen Triplettenergie trotz der Anwesenheit kondensierter aromatischer Sechsringe bevorzugt sein können.

Weiterhin zeichnen sich bevorzugte erfindungsgemäße Verbindungen dadurch aus, dass diese sublimierbar sind. Diese Verbindungen weisen im Allgemeinen eine Molmasse von weniger als ca. 1200 g/mol auf.

Die oben genannten bevorzugten Ausführungsformen können beliebig innerhalb der in Anspruch 1 definierten Einschränkungen miteinander kombiniert werden. In einer besonders bevorzugten Ausführungsform der Erfindung treten die oben genannten Bevorzugungen gleichzeitig auf.

Beispiele für bevorzugte Verbindungen gemäß den oben aufgeführten Ausführungsformen sind die in der folgenden Tabelle aufgeführten Verbindungen.

| | | |
|---|---|---|
| 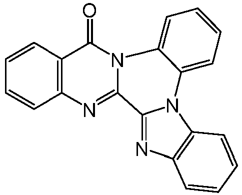 | 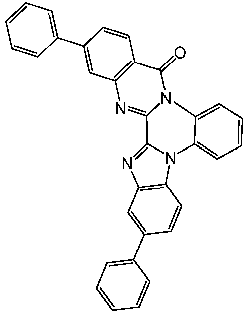 | 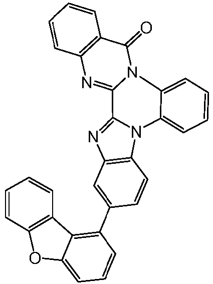 |
| 1 | 2 | 3 |
| 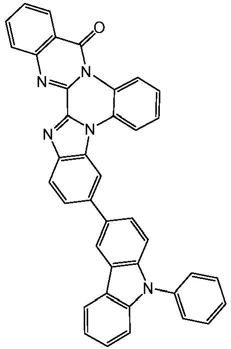 | 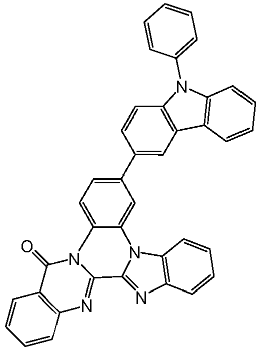 | 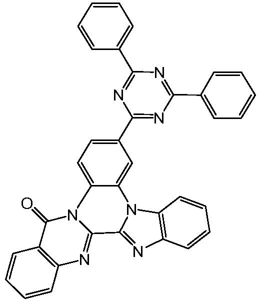 |
| 4 | 5 | 6 |
| 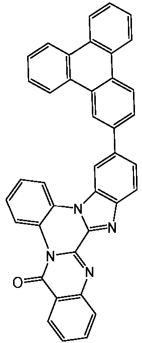 | 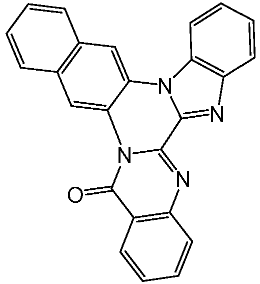 | 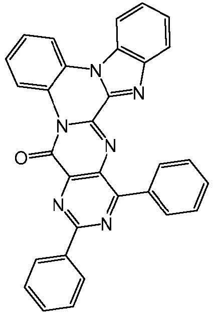 |
| 7 | 8 | 9 |
| 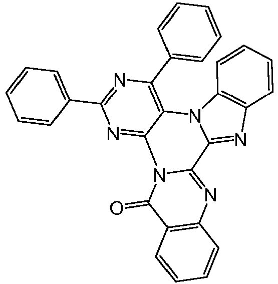 | 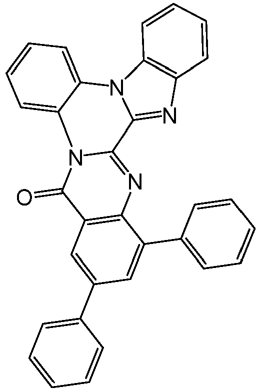 | 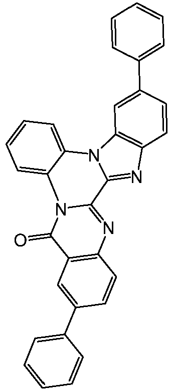 |
| 10 | 11 | 12 |
| 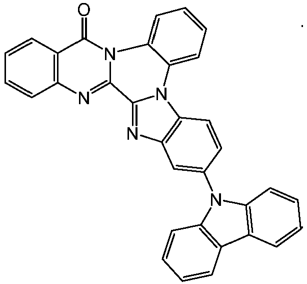 | 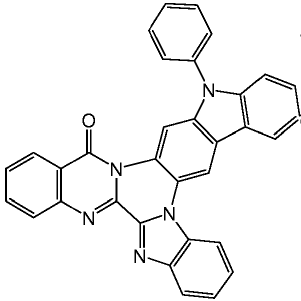 | 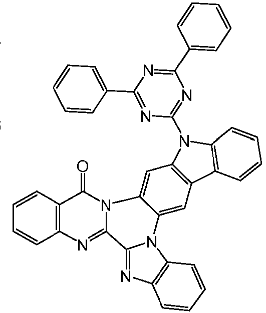 |
| 13 | 14 | 15 |
| 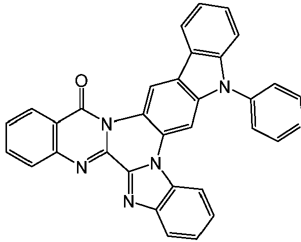 | 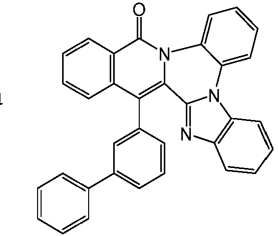 | 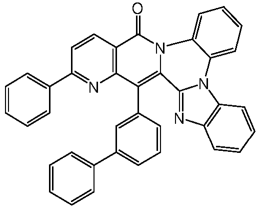 |
| 16 | 17 | 18 |
| 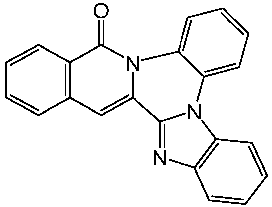 | 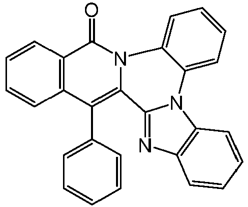 | 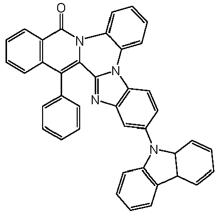 |
| 19 | 20 | 21 |
| 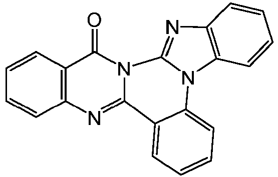 | 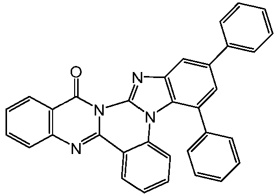 | 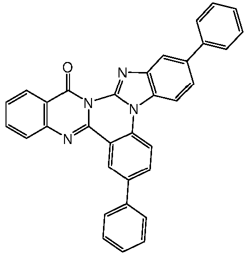 |
| 22 | 23 | 24 |
| 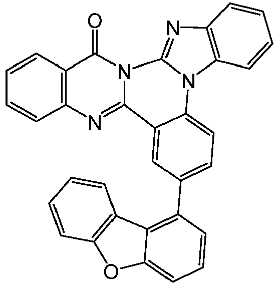 | 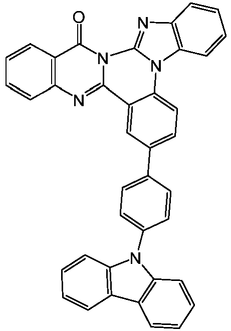 | 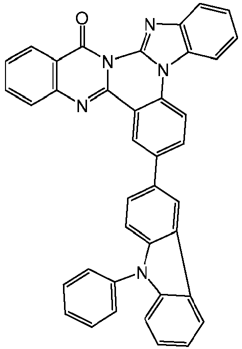 |
| 25 | 26 | 27 |
| 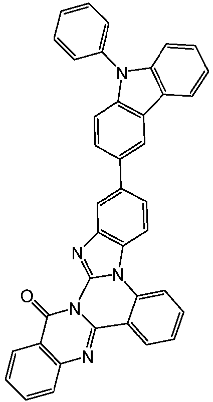 | 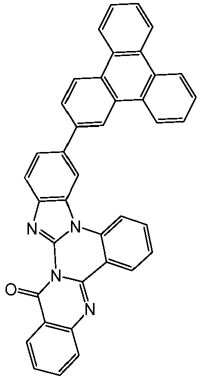 | 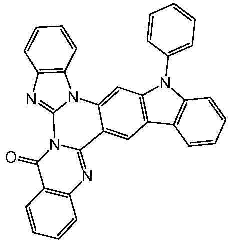 |
| 28 | 29 | 30 |
| 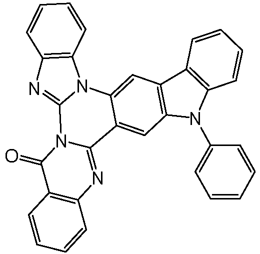 | 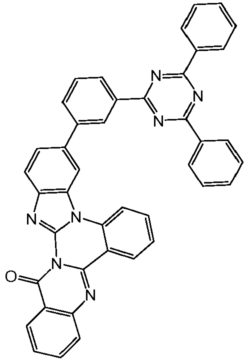 | 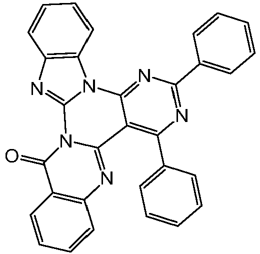 |
| 31 | 32 | 33 |
| 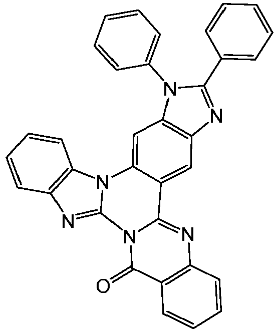 | 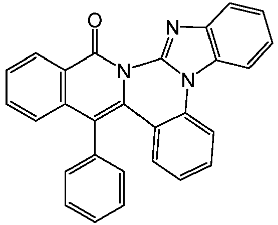 | 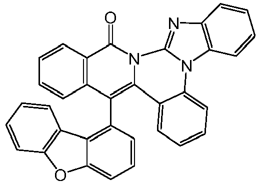 |
| 34 | 35 | 36 |
| 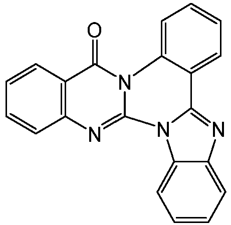 | 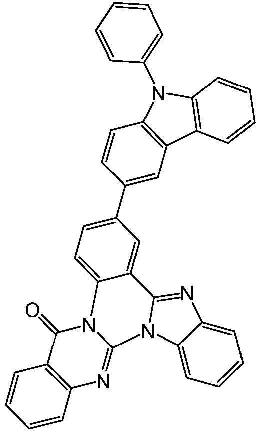 | 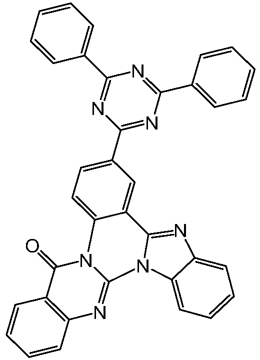 |
| 37 | 38 | 39 |
| 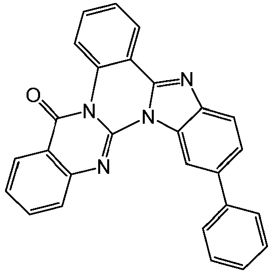 | 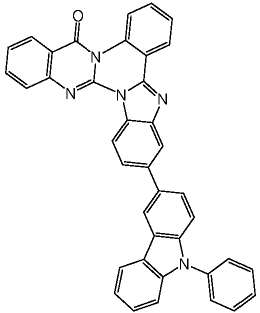 | 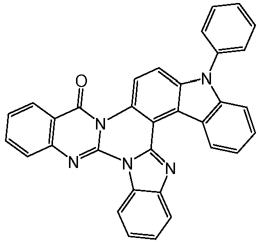 |
| 40 | 41 | 42 |
| 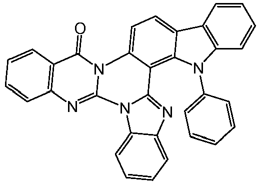 | 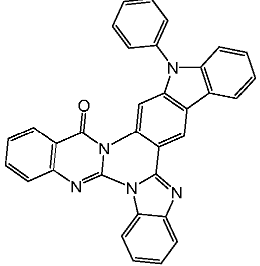 | 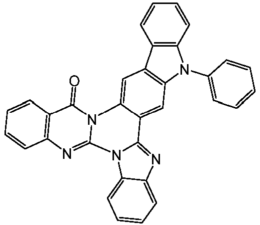 |
| 43 | 44 | 45 |
| 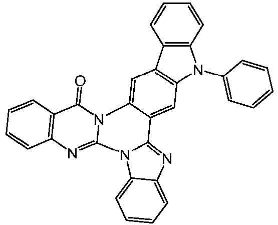 | 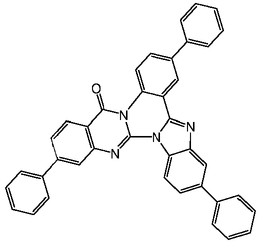 | 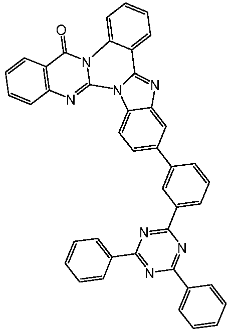 |
| 46 | 47 | 48 |
| 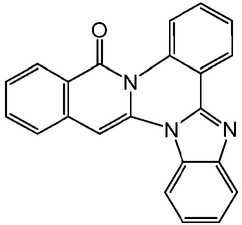 | 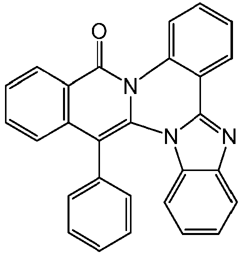 | 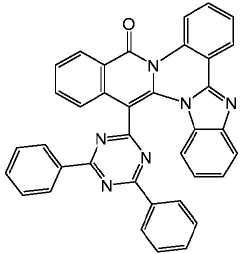 |
| 49 | 50 | 51 |
| 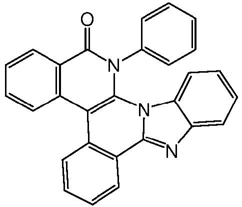 | 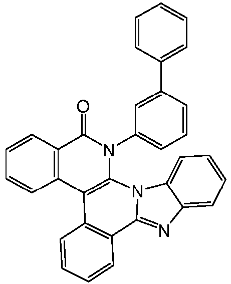 | 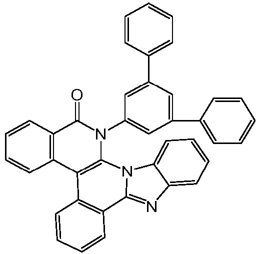 |
| 52 | 53 | 54 |
| 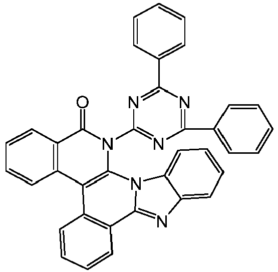 | 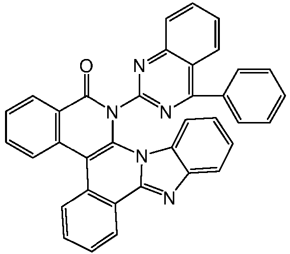 | 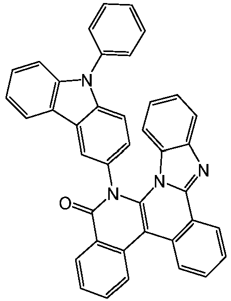 |
| 55 | 56 | 57 |
| 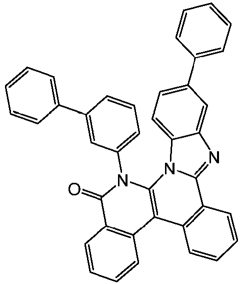 | 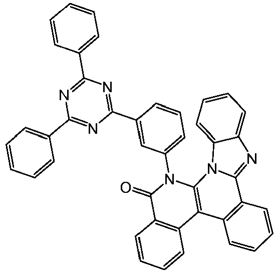 | 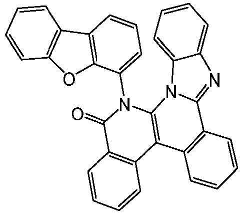 |
| 58 | 59 | 60 |
| 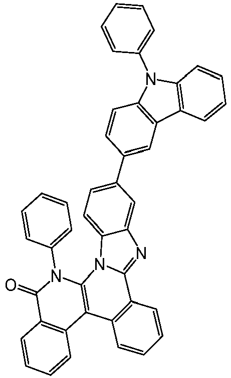 | 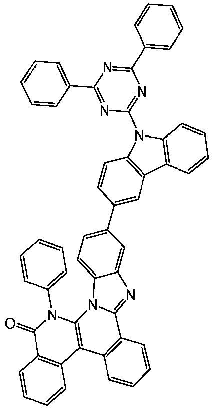 | 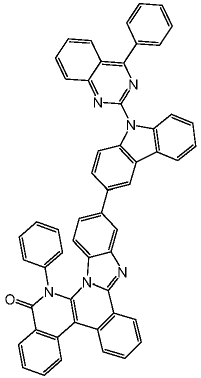 |
| 61 | 62 | 63 |
| 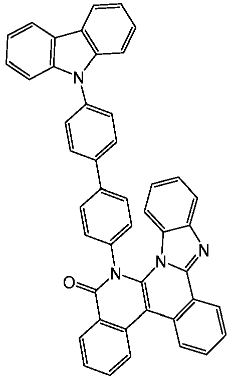 | 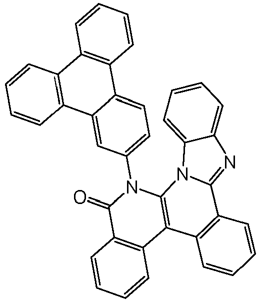 | 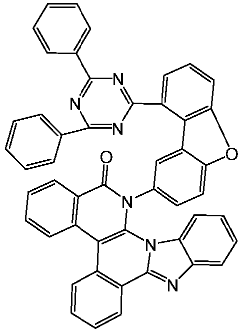 |
| 64 | 65 | 66 |
| 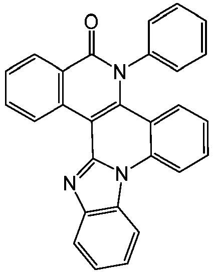 | 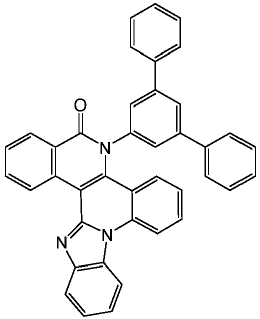 | 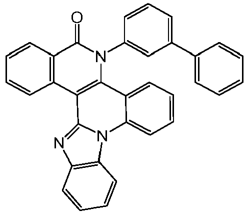 |
| 67 | 68 | 69 |
| 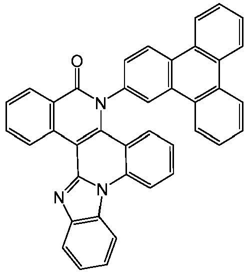 | 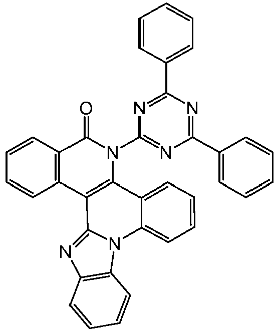 | 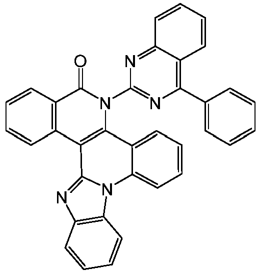 |
| 70 | 71 | 72 |
| 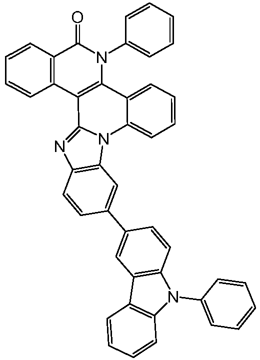 | 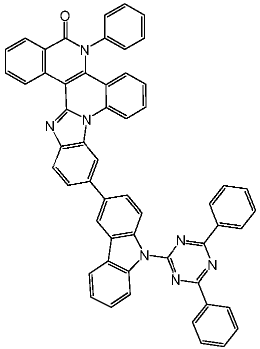 | 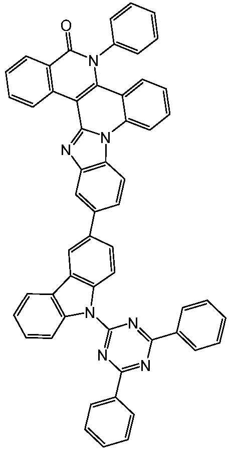 |
| 73 | 74 | 75 |
| 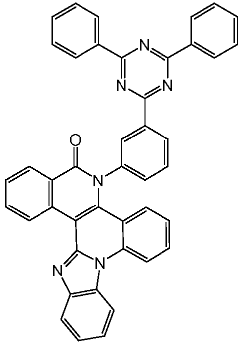 | 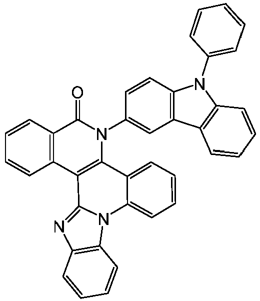 | 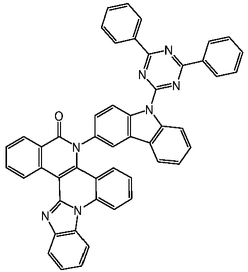 |
| 76 | 77 | 78 |
| 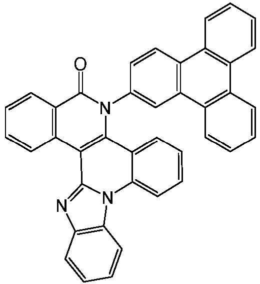 | 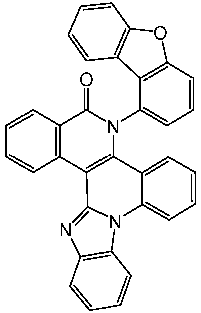 | 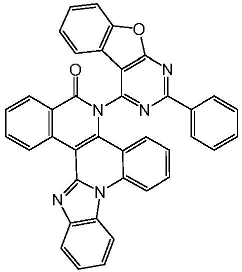 |
| 79 | 80 | 81 |
| 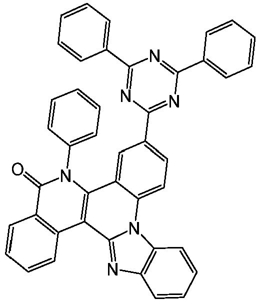 | 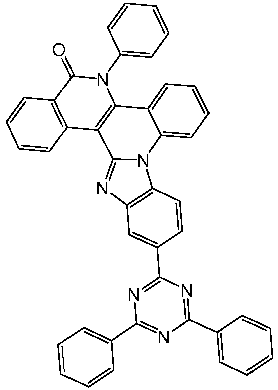 | 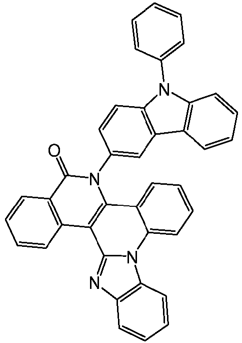 |
| 82 | 83 | 84 |
| 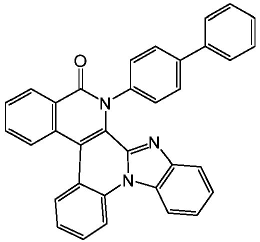 | 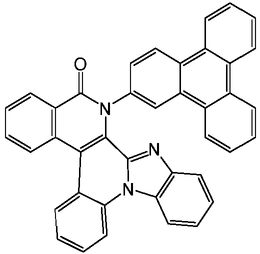 | 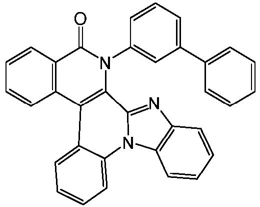 |
| 85 | 86 | 87 |
| 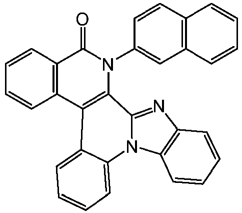 | 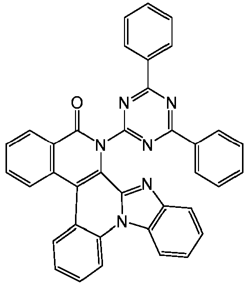 | 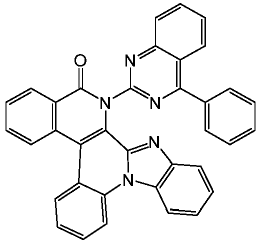 |
| 88 | 89 | 90 |
| 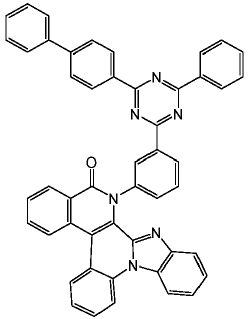 | 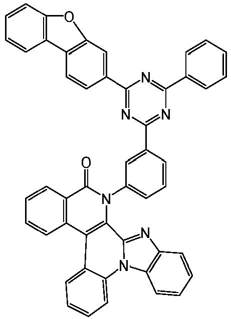 | 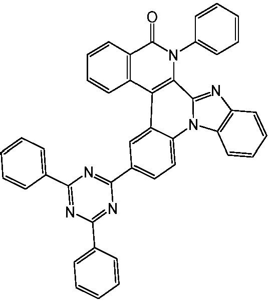 |
| 91 | 92 | 93 |
| 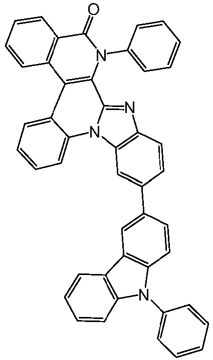 | 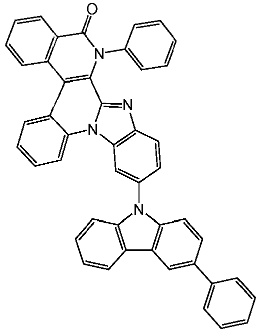 | 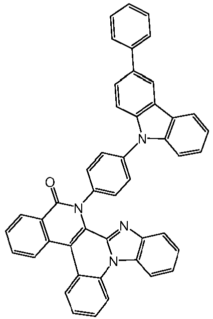 |
| 94 | 95 | 96 |
| 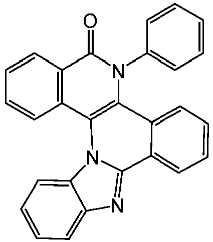 | 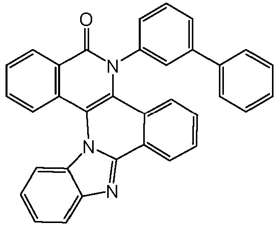 | 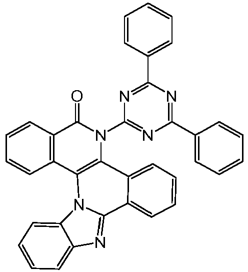 |
| 97 | 98 | 99 |
| 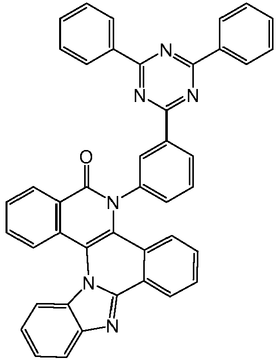 | 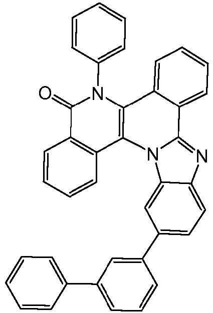 | 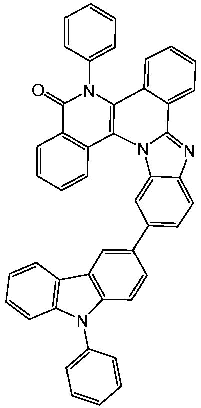 |
| 100 | 101 | 102 |
| 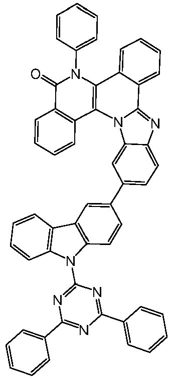 | 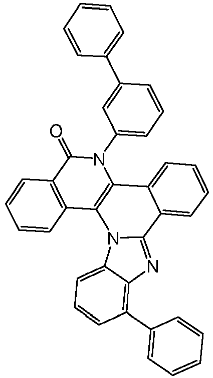 | 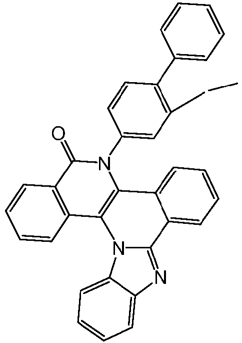 |
| 103 | 104 | 105 |
| 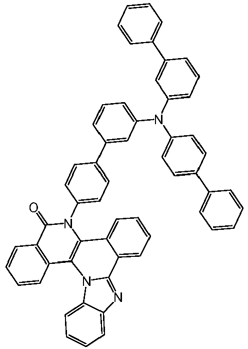 | 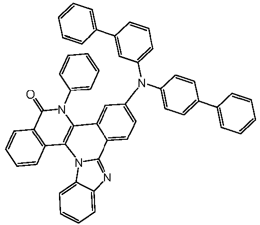 | 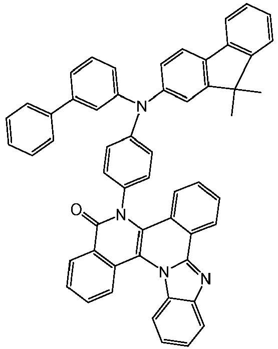 |
| 106 | 107 | 108 |
| 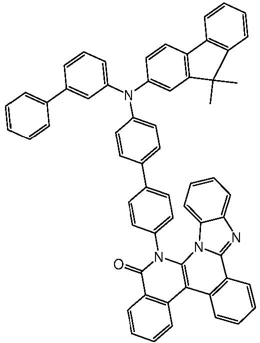 | 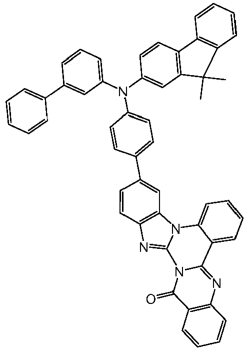 | 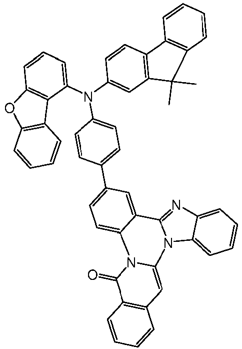 |
| 109 | 110 | 111 |
| 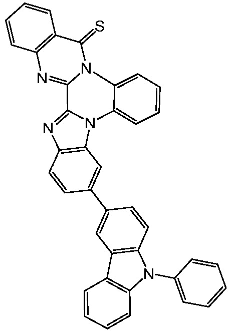 | 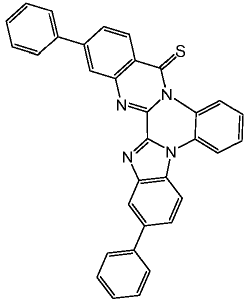 | 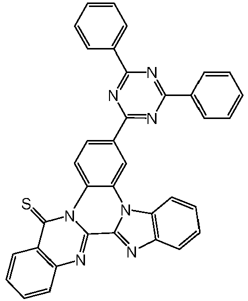 |
| 112 | 113 | 114 |
| 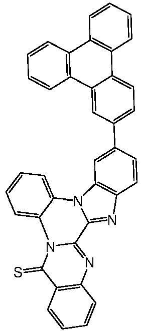 | 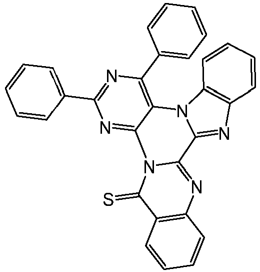 | 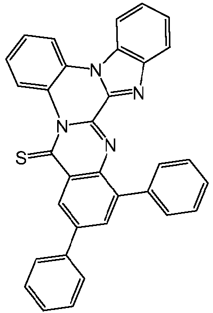 |
| 115 | 116 | 117 |
| 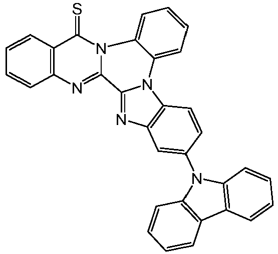 | 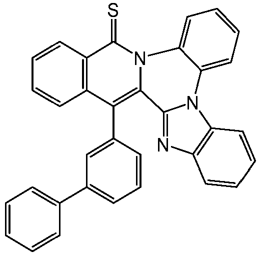 | 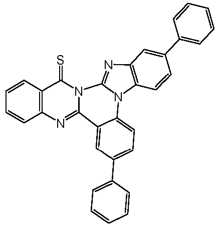 |
| 118 | 119 | 120 |
| 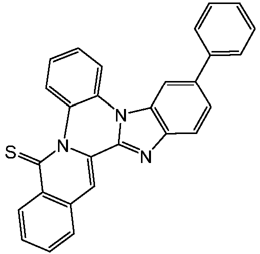 | 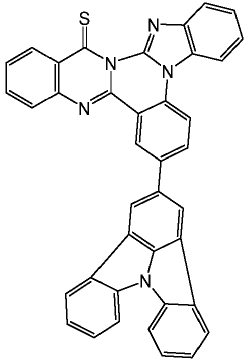 | 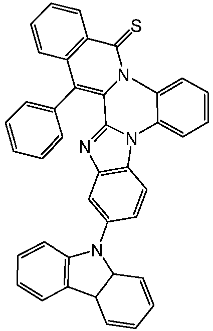 |
| 121 | 122 | 123 |
| 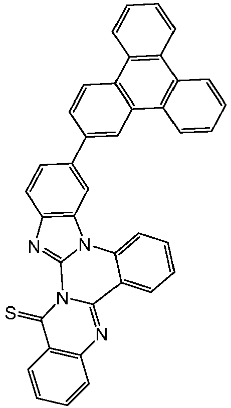 | 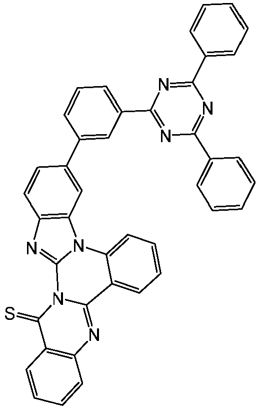 | 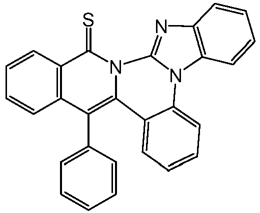 |
| 124 | 125 | 126 |
| 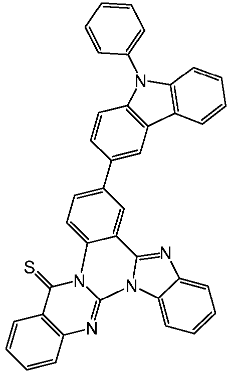 | 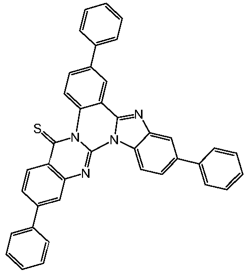 | 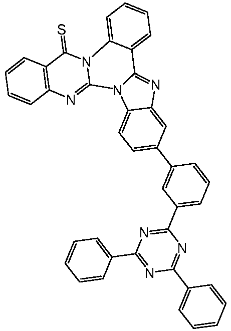 |
| 127 | 128 | 129 |
| 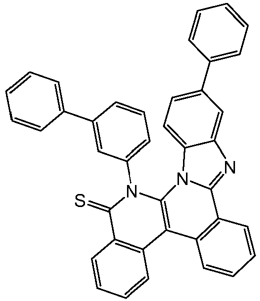 | 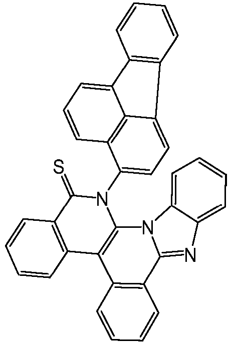 | 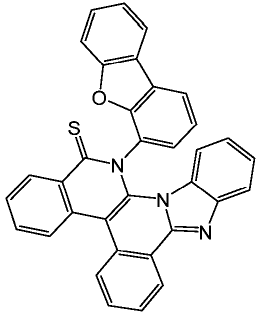 |
| 130 | 131 | 132 |
| 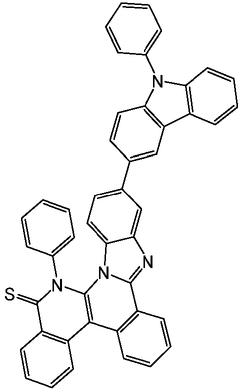 | 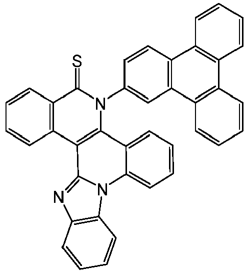 | 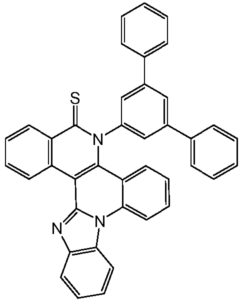 |
| 133 | 134 | 135 |
| 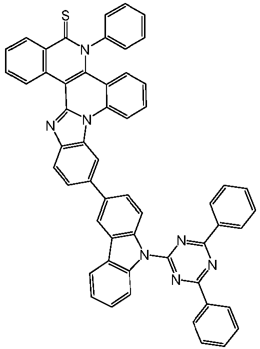 | 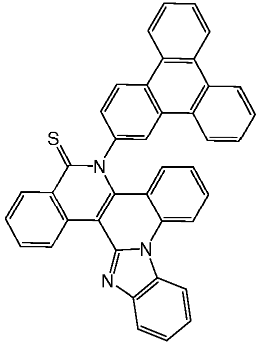 | 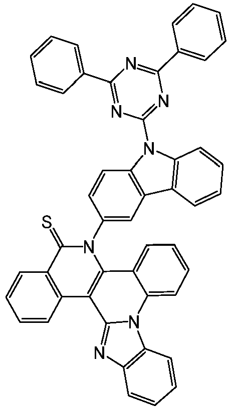 |
| 136 | 137 | 138 |
| 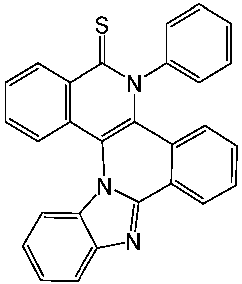 | 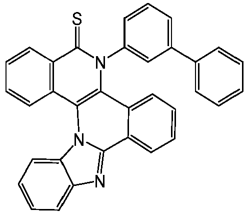 | 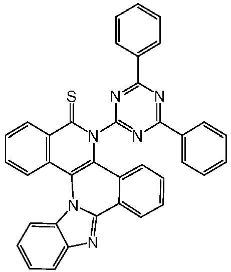 |
| 139 | 140 | 141 |
| 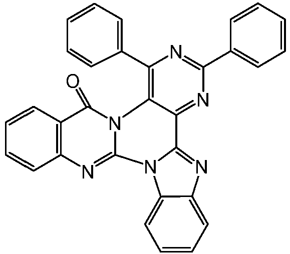 | 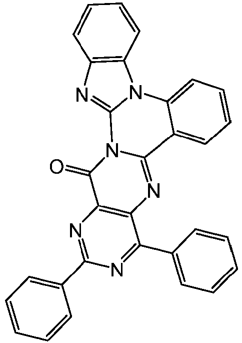 | 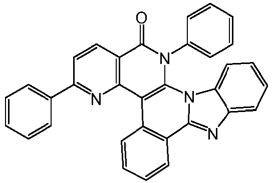 |
| 142 | 143 | 144 |
| 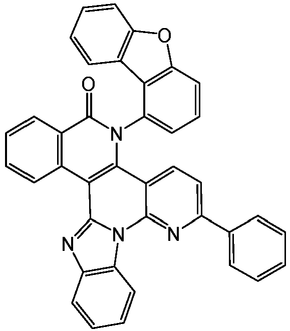 | 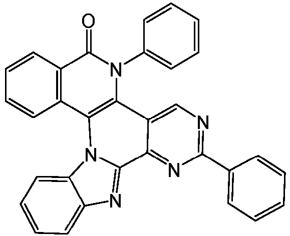 | 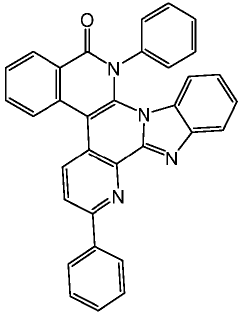 |
| 145 | 146 | 147 |
| 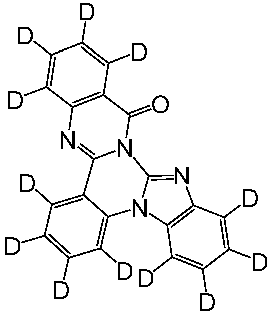 | 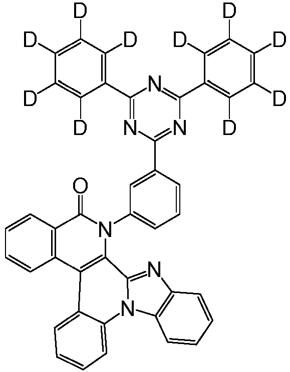 | 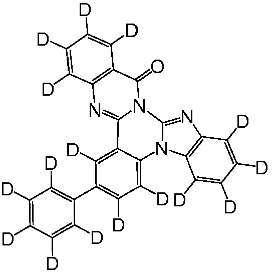 |
| 148 | 149 | 150 |
| 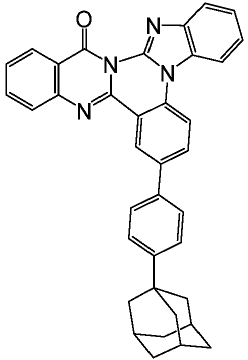 | 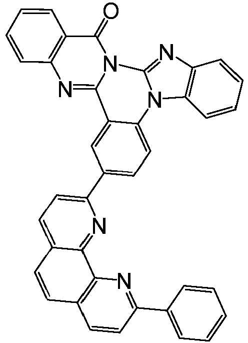 | 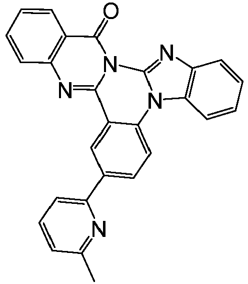 |
| 151 | 152 | 153 |
| 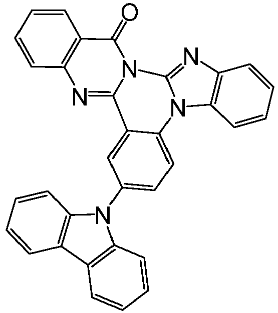 | 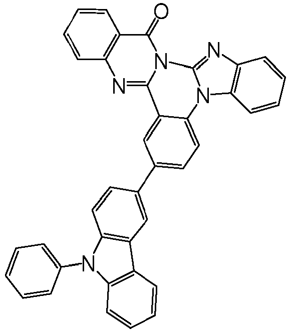 | 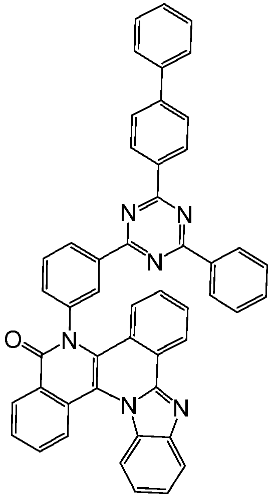 |
| 154 | 155 | 156 |

Die Grundstruktur der erfindungsgemäßen Verbindungen kann nach den in den nachfolgenden Schemata skizzierten Wegen dargestellt werden. Dabei sind die einzelnen Syntheseschritte, wie beispielsweise C-C-Kupplungsreaktionen gemäß Suzuki, C-N-Kupplungsreaktionen gemäß Buchwald beziehungsweise Hartwig-Buchwald oder Cyclisierungsreaktionen, dem Fachmann prinzipiell bekannt. Weitere Informationen zur Synthese der erfindungsgemäßen Verbindungen können den Synthesebeispielen entnommen werden. Mögliche Synthesen der Grundstruktur sind in Schemata 1 bis 7 dargestellt. Diese können gemäß bekannten Reaktionen erfolgen, wie diese beispielhaft in CN 109535200, WO 2009/086264, WO 2010/059773, WO 2013/132253, KR 2018041482, WO 2018/070836 und in den Veröffentlichungen ChemMedChem (2009), 4(5), 866-876; European Journal of Medicinal Chemistry, 157, 1361-1375; 2018; European Journal of Medicinal Chemistry, 162, 586-601; 2019; Journal of Heterocyclic Chemistry, 56(7), 2046-2051; 2019; Journal of Catalysis, 373, 93-102; 2019; Journal of Organic Chemistry (2010), 75(7), 2302-2308; Journal of the Chemical Society [Section] C: Organic (1971), (7), 1227-31; Journal of the Serbian Chemical Society (1987), 52(11), 633-9; Journal of the Serbian Chemical Society (1989), 54(4), 179-87; Justus Liebigs Annalen der Chemie, 729, 97-105; 1969; Letters in Organic Chemistry, 16(11), 898-905; 2019; Medicinal Chemistry Research, 25(6), 1125-1139; 2016; Organic & Biomolecular Chemistry, 17(18), 4465-4469; 2019; Organic & Biomolecular chemistry11(45), 7966-7977; 2013; Pharmazie, 35(5-6), 293-6; 1980; Synthesis, (8), 1343-1350; 2006; Synthesis, (16), 2794-2798; 2010; Tetrahedron Letters, 55(16), 2742-2744; 2014; Tetrahedron Letters, 41(31), 5857-5860; 2000 und Tetrahedron (2012), 68(1), 250-261 dargelegt sind. In Schema 8 werden verschiedene weitere Möglichkeiten zur Derivatisierung der Grundstruktur dargestellt.

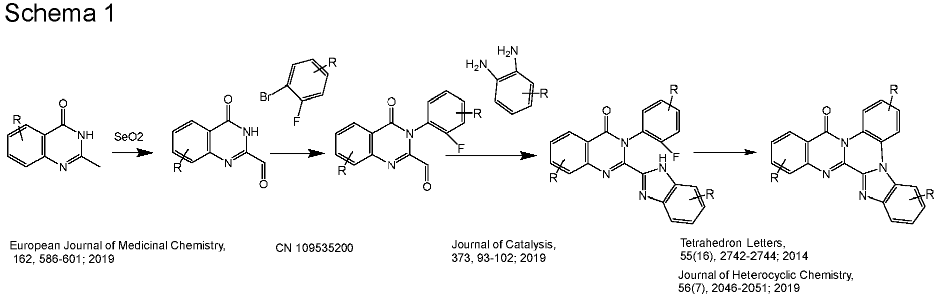

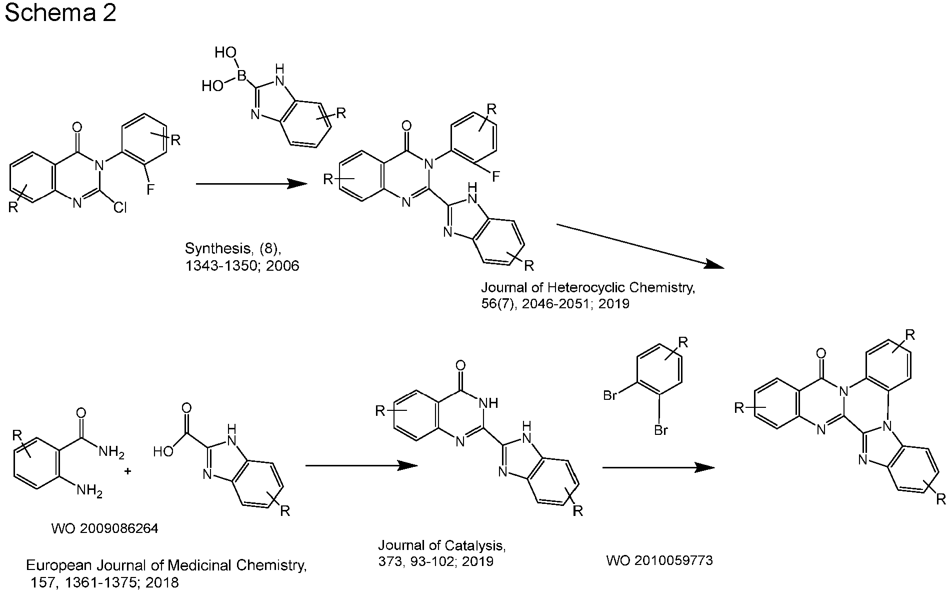

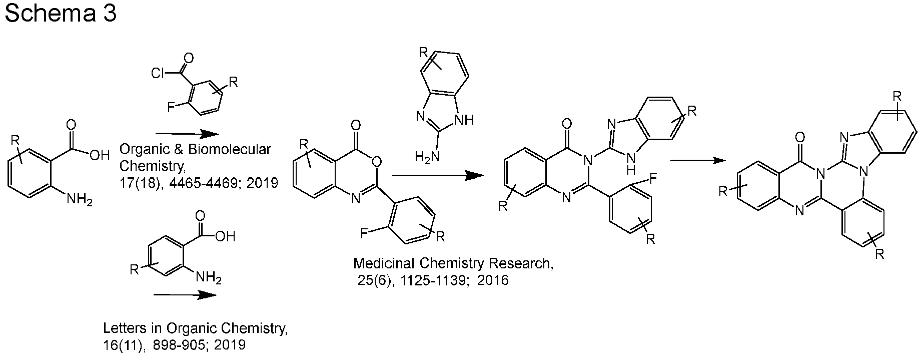

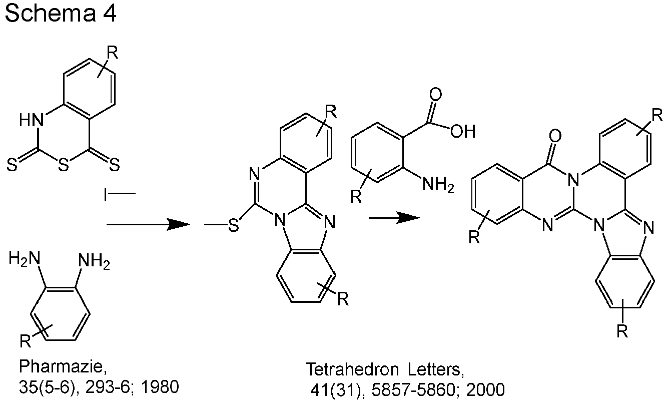

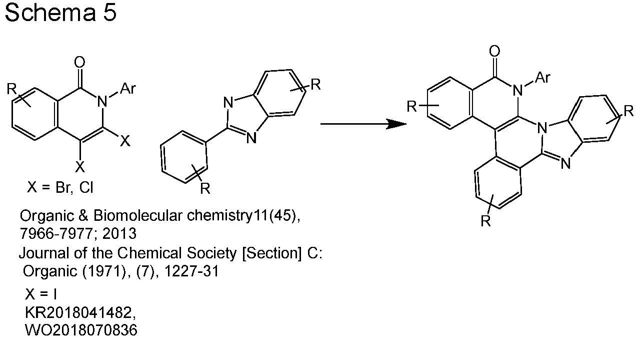

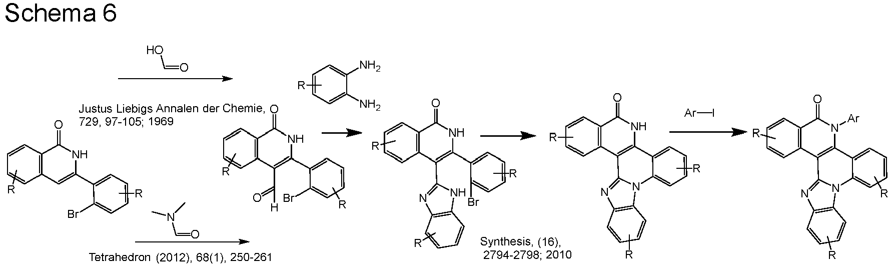

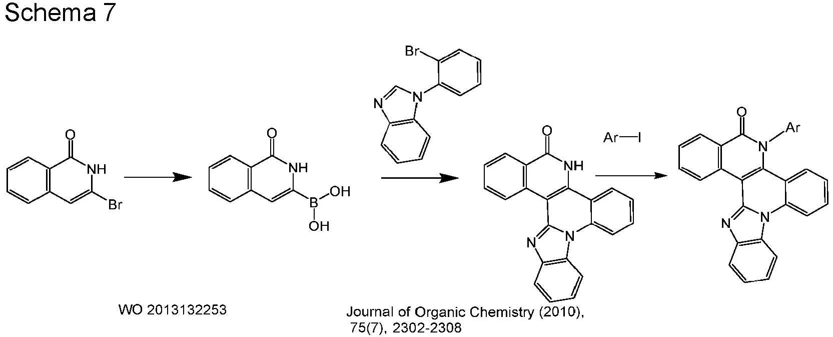

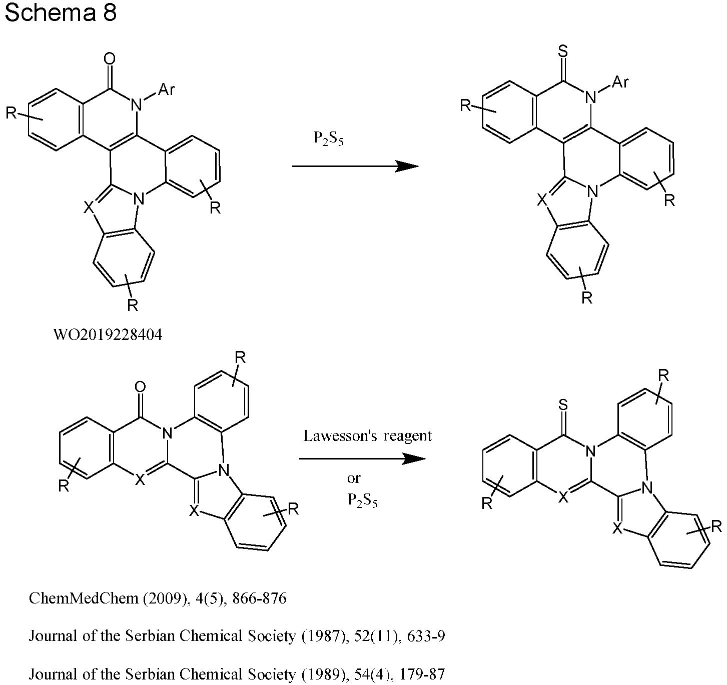

Die Bedeutung der in Schemata 1 bis 8 verwendeten Symbole entspricht im Wesentlichen denen, die für Strukturelemente (A), (B) und (C) definiert wurde, wobei aus Gründen der Übersichtlichkeit auf eine Nummerierung sowie auf eine vollständige Darstellung aller Symbole verzichtet wurde. Darüber hinaus wurde aus Gründen der Übersichtlichkeit vielfach auf die Verwendung des Symbols X zur Darstellung möglicher Stickstoffatome in den heteroaromatischen Ringen verzichtet, wie diese insbesondere für Strukturelemente (A), (B) und (C) beziehungsweise in Formeln (I-1) bis (I-7) und/oder (II-1) bis (II-30) durch die Symbole X¹, X² und X³ dargelegt sind. Diese Angaben sind daher beispielhaft zu verstehen, wobei der Fachmann in der Lage ist die zuvor und nachfolgend, insbesondere in den Beispielen dargelegten Synthesen auf Verbindungen zu übertragen, bei denen ein oder mehrere der Symbole X¹, X² und X³ für Stickstoff stehen.

Ein weiterer Gegenstand der vorliegenden Erfindung ist daher ein Verfahren zur Herstellung einer erfindungsgemäßen Verbindung, wobei eine stickstoffhaltige aromatische oder heteroaromatische Verbindung in einer Ringbildungsreaktion umgesetzt wird.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, 2-Methylbiphenyl, 3-Methylbiphenyl, 1-Methylnaphthalin, 1-Ethylnaphthalin, Ethyloctanoat, Sebacinsäure-diethylester, Octyloctanoat, Heptylbenzol, Menthyl-isovalerat, Cyclohexylhexanoat oder Mischungen dieser Lösemittel.

Ein weiterer Gegenstand der vorliegenden Erfindung ist daher eine Formulierung bzw. eine Zusammensetzung, enthaltend mindestens eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Falls die weitere Verbindung ein Lösungsmittel umfasst, so wird diese Mischung hierin als Formulierung bezeichnet. Die weitere Verbindung kann aber auch mindestens eine weitere organische oder anorganische Verbindung sein, die ebenfalls in der elektronischen Vorrichtung eingesetzt wird, beispielsweise eine emittierende Verbindung und/oder ein weiteres Matrixmaterial. Geeignete emittierende Verbindungen und weitere Matrixmaterialien sind hinten im Zusammenhang mit der organischen Elektrolumineszenzvorrichtung aufgeführt. Die weitere Verbindung kann auch polymer sein.

Ein weiterer Gegenstand der vorliegenden Erfindung ist die Verwendung einer Verbindung, enthaltend mindestens eine Verbindung umfassend mindestens eine Struktur mit mindestens drei miteinander kondensierten Strukturelementen gemäß den Formeln (A'), (B') und (C'), vorzugsweise Verbindung bestehend aus drei miteinander kondensierten Strukturelementen gemäß den Formeln (A'), (B') und (C') 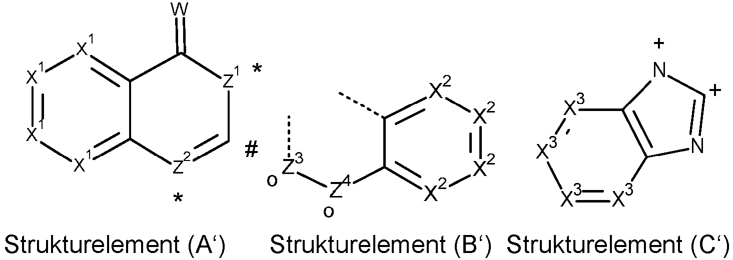 wobei das Strukturelement (A') mit Strukturelement (B') kondensiert ist und das Strukturelement (B') mit Strukturelement (C') kondensiert ist; wobei das Strukturelement (B') über die gestrichelt dargestellten Bindungen an das Strukturelement (A') bindet, wobei eine Bindung über die mit # markierte Bindungsstelle erfolgt und eine Bindung über eine mit * markierte Bindungsstelle erfolgt; wobei das Strukturelement (B') mit dem Strukturelement (C') über die mit o und + markierten Atome miteinander kondensiert ist und die jeweils markierten Atome von den Strukturelementen (B') und (C') geteilt sind, und
für die verwendeten Symbole und Indizes gilt:
   - W: steht für O oder S, vorzugsweise für O;
   - Z¹: steht für NAr oder N, für den Fall, dass das Strukturelement (A') mit dem Strukturelement (B') über Z¹ kondensiert ist;
   - Z²: steht für X oder C, für den Fall, dass das Strukturelement (A') mit dem Strukturelement (B') über Z² kondensiert ist;
   - Z³, Z⁴: steht für N oder C, wobei einer der Reste Z³, Z⁴ für N und einer der Reste Z³, Z⁴ für C steht;
   - X: steht für N oder CR, vorzugsweise für N;
   - X¹: steht bei jedem Auftreten gleich oder verschieden für N oder CR¹, vorzugsweise für CR¹, mit der Maßgabe, dass nicht mehr als zwei der Gruppen X¹ in einem Cyclus für N stehen;
   - X²: steht bei jedem Auftreten gleich oder verschieden für N oder CR², mit der Maßgabe, dass nicht mehr als zwei der Gruppen X² in einem Cyclus für N stehen;
   - X³: steht bei jedem Auftreten gleich oder verschieden für N oder CR³, vorzugsweise für CR³, mit der Maßgabe, dass nicht mehr als zwei der Gruppen X³ in einem Cyclus für N stehen;
   - Ar: ist bei jedem Auftreten gleich oder verschieden ein aromatisches oder heteroaromatisches Ringsystem mit 5 bis 40 aromatischen Ringatomen, welches mit einem oder mehreren Resten R substituiert sein kann, vorzugsweise eine Arylgruppe mit 6 bis 30 aromatischen Ringatomen oder eine Heteroarylgruppe mit 5 bis 14 aromatischen Ringatomen, welche mit einem oder mehreren Resten R substituiert sein kann;
   - R: ist bei jedem Auftreten gleich oder verschieden H, D, F, Cl, Br, I, R⁴C=C(R⁴)₂, N(R⁴)₂, N(Ar')₂, CN, NO₂, OR⁴, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, eine geradkettige Alkyl-, Alkoxy- oder Thioalkylgruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkylgruppe mit 3 bis 20 C-Atomen, wobei die Alkyl-, Alkoxy- oder Thioalkyl-, Alkenyl- oder Alkinylgruppe jeweils Reste R⁴ aufweist und wobei eine oder mehrere nicht benachbarte CH₂-Gruppen durch R⁴C=CR⁴, C≡C, Si(R⁴)₂, C=O, C=S, C=Se, C=NR⁴, -C(=O)O-, -C(=O)NR⁴-, NR⁴, P(=O)( R⁴), -O-, -S-, SO oder SO₂ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils Reste R⁴ aufweist, oder eine Aryloxy- oder Heteroaryloxygruppe mit 5 bis 40 aromatischen Ringatomen, die jeweils Reste R⁴ aufweist, oder eine Diarylamino-, Arylheteroarylamino-, Diheteroarylaminogruppe mit 5 bis 60 aromatischen Ringatomen im jeweiligen aromatischen oder heteroaromatischen Rest, wobei die Diarylamino-, Arylheteroarylamino-, Diheteroarylaminogruppe Reste R⁴ aufweist; dabei kann ein Rest R mit einem weiteren Rest, vorzugsweise einer Gruppe R¹, R², R³ ein aliphatisches, heteroaliphatisches, aromatisches oder heteroaromatisches Ringsystem, vorzugsweise ein aliphatisches, heteroaliphatisches oder heteroaromatisches Ringsystem bilden, besonders bevorzugt bildet der Rest R kein solches Ringsystem;
   - R¹: ist bei jedem Auftreten gleich oder verschieden H, D, F, Cl, Br, I, R⁴C=C(R⁴)₂, N(R⁴)₂, N(Ar')₂, CN, NO₂, OR₄, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, eine geradkettige Alkyl-, Alkoxy- oder Thioalkylgruppe mit 1 bis 20 C-Atomen oder eine Alkenyl- oder Alkinyl- gruppe mit 2 bis 20 C-Atomen oder eine verzweigte oder cyclische Alkyl-, Alkoxy- oder Thioalkylgruppe mit 3 bis 20 C-Atomen, wobei die Alkyl-, Alkoxy- oder Thioalkyl-, Alkenyl- oder Alkinylgruppe jeweils Reste R⁴ aufweist und wobei eine oder mehrere nicht benachbarte CH₂-Gruppen durch R⁴C=CR⁴, C≡C, Si(R⁴)₂, C=O, C=S, C=Se, C=NR⁴, -C(=O)O-, -C(=O)NR⁴-, NR⁴, P(=O)( R⁴), -O-, -S-, SO oder SO₂ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils Reste R⁴ aufweist, oder eine Aryloxy- oder Heteroaryloxygruppe mit 5 bis 40 aromatischen Ringatomen, die jeweils Reste R⁴ aufweist, oder eine Diarylamino-, Arylheteroarylamino-, Diheteroarylaminogruppe mit 5 bis 60 aromatischen Ringatomen im jeweiligen aromatischen oder heteroaromatischen Rest, wobei die Diarylamino-, Arylheteroarylamino-, Diheteroarylaminogruppe Reste R⁴ aufweist; dabei können zwei Reste R¹ oder ein Rest R¹ mit einem weiteren Rest, vorzugsweise einer Gruppe R, R², R³ auch miteinander ein aromatisches, heteroaromatisches, aliphatisches oder heteroaliphatisches Ringsystem, vorzugsweise ein aliphatisches, heteroaliphatisches oder heteroaromatisches Ringsystem bilden, besonders bevorzugt bilden die Reste R¹ kein solches Ringsystem;
   - R²: ist bei jedem Auftreten gleich oder verschieden H, D, F, Cl, Br, I, R⁴C=C(R⁴)₂, N(R⁴)₂, N(Ar')₂, CN, NO₂, OR₄, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, eine geradkettige Alkyl-, Alkoxy- oder Thioalkylgruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkylgruppe mit 3 bis 20 C-Atomen, wobei die Alkyl-, Alkoxy- oder Thioalkyl-, Alkenyl- oder Alkinylgruppe jeweils Reste R⁴ aufweist und wobei eine oder mehrere nicht benachbarte CH₂-Gruppen durch R⁴C=CR⁴, C≡C, Si(R⁴)₂, C=O, C=S, C=Se, C=NR⁴, -C(=O)O-, -C(=O)NR⁴-, NR⁴, P(=O)( R⁴), -O-, -S-, SO oder SO₂ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils Reste R⁴ aufweist, oder eine Aryloxy- oder Heteroaryloxygruppe mit 5 bis 40 aromatischen Ringatomen, die jeweils Reste R⁴ aufweist, oder eine Diarylamino-, Arylheteroarylamino-, Diheteroarylaminogruppe mit 5 bis 60 aromatischen Ringatomen im jeweiligen aromatischen oder heteroaromatischen Rest, wobei die Diarylamino-, Arylheteroarylamino-, Diheteroarylaminogruppe Reste R⁴ aufweist; dabei können zwei Reste R² oder ein Rest R² mit einem weiteren Rest, vorzugsweise einer Gruppe R, R¹, R³ auch miteinander ein aromatisches, heteroaromatisches, aliphatisches oder heteroaliphatisches Ringsystem, vorzugsweise ein aliphatisches, heteroaliphatisches oder heteroaromatisches Ringsystem bilden, besonders bevorzugt bilden die Reste R² kein solches Ringsystem;
   - R³: ist bei jedem Auftreten gleich oder verschieden H, D, F, Cl, Br, I, R⁴C=C(R⁴)₂, N(R⁴)₂, N(Ar')₂, CN, NO₂, OR₄, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, eine geradkettige Alkyl-, Alkoxy- oder Thioalkylgruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkylgruppe mit 3 bis 20 C-Atomen, wobei die Alkyl-, Alkoxy- oder Thioalkyl-, Alkenyl- oder Alkinylgruppe jeweils Reste R⁴ aufweist und wobei eine oder mehrere nicht benachbarte CH₂-Gruppen durch R⁴C=CR⁴, C≡C, Si(R⁴)₂, C=O, C=S, C=Se, C=NR⁴, -C(=O)O-, -C(=O)NR⁴-, NR⁴, P(=O)( R⁴), -O-, -S-, SO oder SO₂ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils Reste R⁴ aufweist, oder eine Aryloxy- oder Heteroaryloxygruppe mit 5 bis 40 aromatischen Ringatomen, die jeweils Reste R⁴ aufweist, oder eine Diarylamino-, Arylheteroarylamino-, Diheteroarylaminogruppe mit 5 bis 60 aromatischen Ringatomen im jeweiligen aromatischen oder heteroaromatischen Rest, wobei die Diarylamino-, Arylheteroarylamino-, Diheteroarylaminogruppe Reste R⁴ aufweist; dabei können zwei Reste R³ oder ein Rest R³ mit einem weiteren Rest, vorzugsweise einer Gruppe R, R¹, R² auch miteinander ein aromatisches, heteroaromatisches, aliphatisches oder heteroaliphatisches Ringsystem, vorzugsweise ein aliphatisches, heteroaliphatisches oder heteroaromatisches Ringsystem bilden, besonders bevorzugt bilden die Reste R³ kein solches Ringsystem;
   - Ar': ist bei jedem Auftreten gleich oder verschieden ein aromatisches oder heteroaromatisches Ringsystem mit 5 bis 40 aromatischen Ringatomen, welches Reste R⁴ aufweist, vorzugsweise eine Arylgruppe mit 6 bis 30 aromatischen Ringatomen oder eine Heteroarylgruppe mit 5 bis 14 aromatischen Ringatomen, welche Reste R⁴ aufweist;
   - R⁴: ist bei jedem Auftreten gleich oder verschieden H, D, F, Cl, Br, I, R⁵C=C(R⁵)₂, N(R⁵)₂, CN, NO₂, OR⁵, SR⁵, Si(R⁵)₃, B(OR⁵)₂, C(=O)R⁵, P(=O)(R⁵)₂, S(=O)R⁵, S(=O)₂R⁵, OSO₂R⁵, eine geradkettige Alkyl-, Alkoxy- oder Thioalkylgruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkylgruppe mit 3 bis 20 C-Atomen, wobei die Alkyl-, Alkoxy- oder Thioalkyl-, Alkenyl- oder Alkinylgruppe jeweils Reste R⁵ aufweist, wobei eine oder mehrere nicht benachbarte CH₂-Gruppen durch R⁵C=CR⁵, C=C, Si(R⁵)₂, C=O, C=S, C=Se, C=NR⁵, -C(=O)O-, -C(=O)NR⁵-, NR⁵, P(=O)( R⁵), -O-, -S-, SO oder SO₂ ersetzt sein können, oder ein aromatisches oder heteroaromatisches Ringsystem mit 5 bis 40 aromatischen Ringatomen, das jeweils Reste R⁵ aufweist, oder eine Aryloxy- oder Heteroaryloxygruppe mit 5 bis 40 aromatischen Ringatomen, die jeweils Reste R⁵ aufweist, oder eine Diarylamino-, Arylheteroarylamino-, Diheteroarylaminogruppe mit 5 bis 60 aromatischen Ringatomen im jeweiligen aromatischen oder heteroaromatischen Rest, wobei die Diarylamino-, Arylheteroarylamino-, Diheteroarylaminogruppe Reste R⁵ aufweist; dabei können zwei oder mehrere Reste R⁴ miteinander ein aromatisches, heteroaromatisches, aliphatisches oder heteroaliphatisches Ringsystem bilden, vorzugsweise ein aliphatisches Ringsystem, besonders bevorzugt bilden die Reste R⁴ kein solches Ringsystem;
   - R⁵: ist bei jedem Auftreten gleich oder verschieden H, D, F oder ein aliphatischer, aromatischer oder heteroaromatischer organischer Rest, insbesondere ein Kohlenwasserstoffrest, mit 1 bis 20 C-Atomen, in dem auch ein oder mehrere H-Atome durch F ersetzt sein können, dabei können zwei oder mehrere Reste R⁵ miteinander ein Ringsystem bilden;
in einer elektronischen Vorrichtung, insbesondere in einer organischen Elektrolumineszenzvorrichtung.

Die für Strukturelemente (A'), (B') und (C') definierten Gruppen entsprechen vielfach den zuvor für Strukturelemente (A), (B) und (C) definierten Resten, so dass die zuvor dargelegten Ausführungen, Definitionen und/oder Bevorzugungen auch für die Strukturelemente (A'), (B') und (C') gelten. Weiterhin stellen Strukturen/Verbindungen umfassend mindestens drei miteinander kondensierte Strukturelemente gemäß den Formeln (A'), (B') und (C'), sowie bevorzugte Ausführungsformen derselben bevorzugte Strukturen/Verbindungen umfassend mindestens drei miteinander kondensierte Strukturelemente gemäß den Formeln (A), (B) und (C) dar, so dass die zuvor und nachfolgenden Ausführungen hierzu auch entsprechend für Strukturen/Verbindungen umfassend mindestens drei miteinander kondensierte Strukturelemente gemäß den Formeln (A'), (B') und (C') gelten.

Ein nochmals weiterer Gegenstand der vorliegenden Erfindung ist eine elektronische Vorrichtung enthaltend mindestens eine Verbindung umfassend mindestens eine Struktur mit mindestens drei miteinander kondensierten Strukturelementen gemäß den Formeln (A'), (B') und (C'), vorzugsweise Verbindung bestehend aus drei miteinander kondensierten Strukturelementen gemäß den Formeln (A'), (B') und (C') 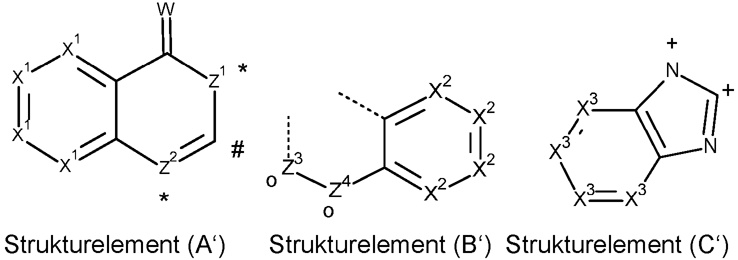
wobei das Strukturelement (A') mit Strukturelement (B') kondensiert ist und das Strukturelement (B') mit Strukturelement (C') kondensiert ist;
wobei das Strukturelement (B') über die gestrichelt dargestellten Bindungen an das Strukturelement (A') bindet, wobei eine Bindung über die mit # markierte Bindungsstelle erfolgt und eine Bindung über eine mit * markierte Bindungsstelle erfolgt; wobei das Strukturelement (B') mit dem Strukturelement (C') über die mit o und + markierten Atome miteinander kondensiert ist und die jeweils markierten Atome von den Strukturelementen (B') und (C') geteilt sind;
wobei die verwendeten Symbole und Indizes die zuvor, insbesondere für Strukturelemente (A'), (B') und (C') genannten Bedeutungen aufweisen;
wobei es sich bei der elektronischen Vorrichtung vorzugsweise um eine Elektrolumineszenzvorrichtung handelt.

Eine elektronische Vorrichtung im Sinne der vorliegenden Erfindung ist eine Vorrichtung, welche mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind.

Besonders bevorzugt ist elektronische Vorrichtung ausgewählt aus der Gruppe bestehend aus organischen Elektrolumineszenzvorrichtungen (OLEDs, sOLED, PLEDs, LECs, etc.), vorzugsweise organische lichtemittierenden Dioden (OLEDs), organische lichtemittierenden Dioden auf Basis von kleinen Molekülen (sOLEDs), organische lichtemittierenden Dioden auf Basis von Polymeren (PLEDs), lichtemittierenden elektrochemischen Zellen (LECs), organischen Laserdioden (O-Laser), "organic plasmon emitting devices" (D. M. Koller et al., Nature Photonics 2008, 1-4); organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs) und organischen elektrischen Sensoren, bevorzugt organischen Elektrolumineszenzvorrichtungen (OLEDs, sOLED, PLEDs, LECs, etc.), besonders bevorzugt organische lichtemittierenden Dioden (OLEDs), organische lichtemittierenden Dioden auf Basis von kleiner Moleküle (sOLEDs), organische lichtemittierenden Dioden auf Basis von Polymeren (PLEDs), insbesondere phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen. Es kann sich bei der erfindungsgemäßen organischen Elektrolumineszenzvorrichtung auch um eine Tandem- Elektrolumineszenzvorrichtung handeln, insbesondere für weiß emittierende OLEDs.

Die erfindungsgemäße Verbindung kann dabei in unterschiedlichen Schichten eingesetzt werden, je nach genauer Struktur. Bevorzugt ist eine organische Elektrolumineszenzvorrichtung, enthaltend eine Verbindung, umfassend mindestens eine Struktur mit mindestens drei miteinander kondensierten Strukturelementen gemäß den Formeln (A'), (B') und (C'), vorzugsweise Verbindung bestehend aus drei miteinander kondensierten Strukturelementen gemäß den Formeln (A'), (B') und (C'), als Matrixmaterial für phosphoreszierende Emitter oder für Emitter, die TADF (thermally activated delayed fluorescence) zeigen, insbesondere für phosphoreszierende Emitter. Weiterhin kann die erfindungsgemäße Verbindung auch in einer Elektronentransportschicht und/ oder in einer Lochblockierschicht in einer Elektronenblockierschicht, vorzugsweise in einer Elektronentransportschicht eingesetzt werden. Besonders bevorzugt wird die erfindungsgemäße Verbindung als Matrixmaterial für phosphoreszierende Emitter, insbesondere für rot, orange, grün oder gelb, bevorzugt rot oder grün phosphoreszierende Emitter, in einer emittierenden Schicht oder als Elektronentransportmaterial in einer Elektronentransportschicht eingesetzt, besonders bevorzugt als Matrixmaterial in einer emittierenden Schicht.

Wenn die erfindungsgemäße Verbindung als Matrixmaterial für eine phosphoreszierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Die Mischung aus der erfindungsgemäßen Verbindung und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der erfindungsgemäßen Verbindung bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

In einer Ausführungsform der Erfindung wird die erfindungsgemäße Verbindung dabei als einziges Matrixmaterial ("single host") für den phosphoreszierenden Emitter eingesetzt.

Eine weitere Ausführungsform der vorliegenden Erfindung ist der Einsatz einer Verbindung umfassend mindestens eine Struktur mit mindestens drei miteinander kondensierten Strukturelementen gemäß den Formeln (A'), (B') und (C'), beziehungsweise den Formeln (A), (B) und (C), oder einer bevorzugten Ausführungsform derselben als Matrixmaterial für einen phosphoreszierenden Emitter in Kombination mit einem weiteren Matrixmaterial. Ein weiteres Matrixmaterial, welches zusätzlich zu einer Verbindung, umfassend mindestens eine Struktur mit mindestens drei miteinander kondensierten Strukturelementen gemäß den Formeln (A'), (B') und (C'), beziehungsweise den Formeln (A), (B) und (C), oder einer bevorzugten Ausführungsform eingesetzt wird, wird nachfolgend zumindest teilweise als Co-Host bezeichnet. Geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Bis-carbazolylbiphenyl) oder die in WO 2005/039246, US 2005/0069729, JP 2004/288381, EP 1205527, WO 2008/086851 oder WO 2013/041176, Indolocarbazolderivate, z. B. gemäß WO 2007/063754 oder WO 2008/056746, Indenocarbazolderivate, z. B. gemäß WO 2010/136109, WO 2011/000455, WO 2013/041176 oder WO 2013/056776, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte Carbazol-Derivate, z. B. gemäß WO 2011/042107, WO 2011/060867, WO 2011/088877 und WO 2012/143080, Triphenylenderivate, z. B. gemäß WO 2012/048781, Dibenzofuranderivate, z. B. gemäß WO 2015/169412, WO 2016/015810, WO 2016/023608, WO 2017/148564 oder WO 2017/148565 oder Biscarbazole, z. B. gemäß JP 3139321 B2.

Ebenso kann ein weiterer phosphoreszierender Emitter, welcher kürzerwellig als der eigentliche Emitter emittiert, als Co-Host in der Mischung vorhanden sein. Besonders gute Ergebnisse werden erzielt, wenn als Emitter ein rot phoshoreszierender Emitter eingesetzt wird und als Co-Host in Kombination mit der erfindungsgemäßen Verbindung ein gelb phosphoreszierender Emitter verwendet wird.

Weiterhin kann als Co-Host eine Verbindung verwendet werden, die nicht oder nicht in wesentlichem Umfang am Ladungstransport teilnimmt, wie beispielsweise in WO 2010/108579 beschrieben. Insbesondere eignen sich in Kombination mit der erfindungsgemäßen Verbindung als Co-Matrix-Material Verbindungen, welche eine große Bandlücke aufweisen und selber nicht oder zumindest nicht in wesentlichem Maße am Ladungstransport der emittierenden Schicht teilnehmen. Es handelt sich bei solchen Materialien bevorzugt um reine Kohlenwasserstoffe. Beispiele für solche Materialien finden sich beispielsweise in der WO 2009/124627 oder in der WO 2010/006680.

Besonders bevorzugte Co-Host-Materialien, welche in Kombination mit einer Verbindung, umfassend mindestens eine Struktur mit mindestens drei miteinander kondensierten Strukturelementen gemäß den Formeln (A'), (B') und (C') oder einer bevorzugten Ausführungsform derselben, eingesetzt werden können, sind Verbindungen gemäß einer der Formeln (H-1), (H-2), (H-3), (H-4) oder (H-5), 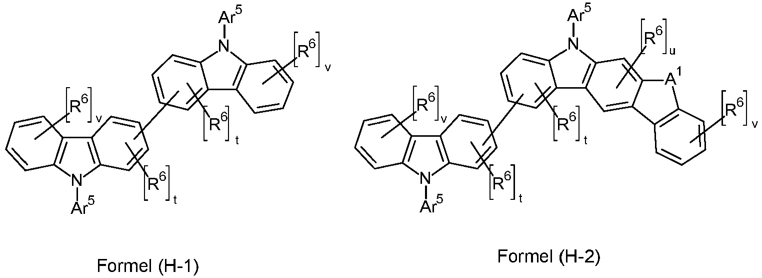 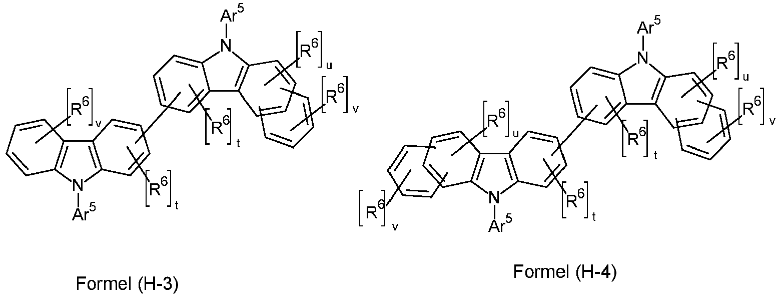 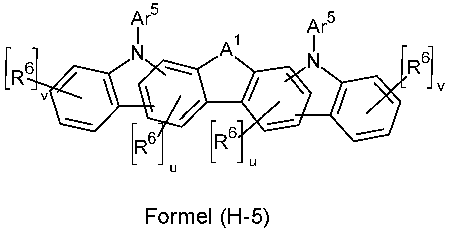 wobei für die verwendeten Symbole und Indizes gilt:
- R⁶: ist bei jedem Auftreten gleich oder verschieden H, D, F, Cl, Br, I, N(R⁷)₂, N(Ar")₂, CN, NO₂, OR⁷, SR⁷, COOR⁷, C(=O)N(R⁷)₂, Si(R⁷)₃, B(OR⁷)₂, C(=O)R⁷, P(=O)(R⁷)₂, S(=O)R⁷, S(=O)₂R⁷, OSO₂R⁷, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils Reste R⁷ aufweisen und wobei eine oder mehrere nicht benachbarte CH₂-Gruppen durch Si(R⁷)₂, C=O, NR⁷, O, S oder CONR⁷ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils Reste R⁷ aufweist; dabei können zwei Reste R⁶ auch miteinander ein aromatisches, heteroaromatisches, aliphatisches oder heteroaliphatisches Ringsystem bilden, vorzugsweise bilden die Reste R⁶ kein solches Ringsystem;
- Ar": ist bei jedem Auftreten gleich oder verschieden ein aromatisches oder heteroaromatisches Ringsystem mit 5 bis 40 aromatischen Ringatomen, welches Reste R⁷ aufweist;
- A¹: ist C(R⁷)₂, NR⁷, O oder S;
- Ar⁵: steht gleich oder verschieden bei jedem Auftreten für ein aromatisches oder heteroaromatisches Ringsystem mit 5 bis 40 aromatischen Ringatomen steht, welches Reste R⁷ aufweist;
- R⁷: ist bei jedem Auftreten gleich oder verschieden H, D, F, Cl, Br, I, N(R⁸)₂, CN, NO₂, OR⁸, SR⁸, Si(R⁸)₃, B(OR⁸)₂, C(=O)R⁸, P(=O)(R⁸)₂, S(=O)R⁸, S(=O)₂R⁸, OSO₂R⁸, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils Reste R⁸ aufweisen, wobei eine oder mehrere nicht benachbarte CH₂-Gruppen durch Si(R⁸)₂, C=O, NR⁸, O, S oder CONR⁸ ersetzt sein können, oder ein aromatisches oder heteroaromatisches Ringsystem mit 5 bis 40 aromatischen Ringatomen, das jeweils Reste R⁸ aufweist; dabei können zwei oder mehrere Reste R⁷ miteinander ein aromatisches, heteroaromatisches, aliphatisches oder heteroaliphatisches Ringsystem bilden, vorzugsweise bilden die Reste R⁷ kein solches Ringsystem;
- R⁸: ist bei jedem Auftreten gleich oder verschieden H, D, F oder ein aliphatischer, aromatischer oder heteroaromatischer organischer Rest, insbesondere ein Kohlenwasserstoffrest, mit 1 bis 20 C-Atomen, in dem auch ein oder mehrere H-Atome durch F ersetzt sein können;
- v: ist bei jedem Auftreten gleich oder verschieden 0, 1, 2, 3 oder 4, vorzugsweise 0 oder 1 und ganz bevorzugt 0;
- t: ist bei jedem Auftreten gleich oder verschieden 0, 1, 2, oder 3, vorzugsweise 0 oder 1 und ganz bevorzugt 0;
- u: ist bei jedem Auftreten gleich oder verschieden 0, 1 oder 2, vorzugsweise 0 oder 1 und ganz bevorzugt 0.

Die Summe der Indices v, t und u in Verbindungen der Formeln (H-1), (H-2), (H-3), (H-4) oder (H-5) beträgt vorzugsweise höchstens 6, besonders bevorzugt höchstens 4 und speziell bevorzugt höchstens 2.

In einer bevorzugten Ausführungsform der Erfindung ist R⁶ gleich oder verschieden bei jedem Auftreten ausgewählt aus der Gruppe bestehend aus H, D, F, CN, NO₂, Si(R⁷)₃, B(OR⁷)₂, eine geradkettige Alkylgruppe mit 1 bis 20 C-Atomen oder eine verzweigte oder cyclische Alkylgruppe mit 3 bis 20 C-Atomen, wobei die Alkylgruppe jeweils mit einem oder mehreren Resten R⁷ substituiert sein kann, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R⁷ substituiert sein kann.

In einer weiterhin bevorzugten Ausführungsform der Erfindung ist R⁶ gleich oder verschieden bei jedem Auftreten ausgewählt aus der Gruppe bestehend aus H, D, F, eine geradkettige Alkylgruppe mit 1 bis 20 C-Atomen oder eine verzweigte oder cyclische Alkylgruppe mit 3 bis 20 C-Atomen, wobei die Alkylgruppe jeweils mit einem oder mehreren Resten R⁷ substituiert sein kann, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R⁷ substituiert sein kann.

In einer weiterhin bevorzugten Ausführungsform der Erfindung ist R⁶ gleich oder verschieden bei jedem Auftreten ausgewählt aus der Gruppe bestehend aus H, D, einem aromatischen oder heteroaromatischen Ringsystem mit 6 bis 30 aromatischen Ringatomen, welches mit einem oder mehreren Resten R⁷ substituiert sein kann, oder einer Gruppe N(Ar")₂. Besonders bevorzugt ist R⁶ gleich oder verschieden bei jedem Auftreten ausgewählt aus der Gruppe bestehend aus H oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren Resten R⁷ substituiert sein kann.

Bevorzugte aromatische bzw. heteroaromatische Ringsysteme, für die die Gruppen R⁶ bzw. Ar" stehen, sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, insbesondere 1- oder 2-verknüpftem Naphthalin, Indol, Benzofuran, Benzothiophen, Carbazol, welches über die 1-, 2-, 3- oder 4-Position verknüpft sein kann, Dibenzofuran, welches über die 1-, 2-, 3-, oder 4-Position verknüpft sein kann, Dibenzothiophen, welches über die 1-2-, 3- oder 4-Position verknüpft sein kann, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R⁷ substituiert sein können. Besonders bevorzugt sind die oben aufgeführten Strukturen Ar-1 bis Ar-79, wobei Strukturen der Formeln (Ar-1), (Ar-2), (Ar-3), (Ar-12), (Ar-13), (Ar-14), (Ar-15), (Ar-16), (Ar-69), (Ar-70), (Ar-75) bevorzugt und Strukturen der Formeln (Ar-1), (Ar-2), (Ar-3), (Ar-12), (Ar-13), (Ar-14), (Ar-15), (Ar-16) besonders bevorzugt sind. In den zuvor dargelegten Strukturen Ar-1 bis Ar-79 sind in Bezug auf die Reste R⁶ und Ar" die Substituenten R⁴ durch die entsprechenden Reste R⁷ zu ersetzen. Die zuvor für die Gruppen R¹, R² und R³ dargelegten Bevorzugungen gelten entsprechend für die Gruppe R⁶.

Weitere geeignete Gruppen R⁶ sind Gruppen der Formel -Ar⁴-N(Ar²)(Ar³), wobei Ar², Ar³ und Ar⁴ gleich oder verschieden bei jedem Auftreten für ein aromatisches oder heteroaromatisches Ringsystem mit 5 bis 24 aromatischen Ringatomen stehen, welches jeweils mit einem oder mehreren Resten R⁴ substituiert sein kann. Dabei beträgt die Gesamtzahl der aromatischen Ringatome von Ar², Ar³ und Ar⁴ maximal 60 und bevorzugt maximal 40. Weitere Bevorzugungen der Gruppen Ar², Ar³ und Ar⁴ wurden zuvor dargelegt und gelten entsprechend.

Weiterhin kann vorgesehen sein, dass die Substituenten R⁶ gemäß obigen Formeln mit den Ringatomen des Ringsystems kein kondensiertes aromatisches oder heteroaromatisches Ringsystem, vorzugsweise kein kondensiertes Ringsystem bilden. Dies schließt die Bildung eines kondensierten Ringsystems mit möglichen Substituenten R⁷, R⁸ ein, die an die Reste R⁶ gebunden sein können.

Wenn A¹ für NR⁷ steht, steht der Substituent R⁷, der an das Stickstoffatom gebunden ist, bevorzugt für ein aromatisches oder heteroaromatisches Ringsystem mit 5 bis 24 aromatischen Ringatomen, welches auch durch einen oder mehrere Reste R⁸ substituiert sein kann. In einer besonders bevorzugten Ausführungsform steht dieser Substituent R⁷ gleich oder verschieden bei jedem Auftreten für ein aromatisches oder heteroaromatisches Ringsystem mit 6 bis 24 aromatischen Ringatomen, insbesondere mit 6 bis 18 aromatischen Ringatomen, welches keine kondensierten Arylgruppen aufweist und welches keine kondensierten Heteroarylgruppen, in denen zwei oder mehr aromatische bzw. heteroaromatische 6-RingGruppen direkt aneinander ankondensiert sind, aufweist, und welches jeweils auch durch einen oder mehrere Reste R⁸ substituiert sein kann. Bevorzugt sind Phenyl, Biphenyl, Terphenyl und Quaterphenyl mit Verknüpfungsmustern, wie vorne für Ar-1 bis Ar-11 aufgeführt, wobei diese Strukturen statt durch R⁴ durch einen oder mehrere Reste R⁸ substituiert sein können, bevorzugt aber unsubstituiert sind. Bevorzugt sind weiterhin Triazin, Pyrimidin und Chinazolin, wie vorne für Ar-47 bis Ar-50, Ar-57 und Ar-58 aufgeführt, wobei diese Strukturen statt durch R⁴ durch einen oder mehrere Reste R⁸ substituiert sein können.

Wenn A¹ für C(R⁷)₂ steht, stehen die Substituenten R⁷, die an dieses Kohlenstoffatom gebunden sind, bevorzugt gleich oder verschieden bei jedem Auftreten für eine lineare Alkylgruppe mit 1 bis 10 C-Atomen oder für eine verzweigte oder cyclische Alkylgruppe mit 3 bis 10 C-Atomen oder für ein aromatisches oder heteroaromatisches Ringsystem mit 5 bis 24 aromatischen Ringatomen, welches auch durch einen oder mehrere Reste R⁸ substituiert sein kann. Ganz besonders bevorzugt steht R⁷ für eine Methylgruppe oder für eine Phenylgruppe. Dabei können die Reste R⁷ auch miteinander ein Ringsystem bilden, was zu einem Spirosystem führt.

Bevorzugte aromatische bzw. heteroaromatische Ringsysteme Ar⁵ sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, insbesondere 1- oder 2-verknüpftem Naphthalin, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R⁷ substituiert sein können.

Dabei sind die Gruppen Ar⁵ unabhängig voneinander besonders bevorzugt gewählt aus den Gruppen der zuvor dargelegten Formeln Ar-1 bis Ar-79, wobei Strukturen der Formeln (Ar-1), (Ar-2), (Ar-3), (Ar-12), (Ar-13), (Ar-14), (Ar-15), (Ar-16), (Ar-69), (Ar-70), (Ar-75) bevorzugt und Strukturen der Formeln (Ar-1), (Ar-2), (Ar-3), (Ar-12), (Ar-13), (Ar-14), (Ar-15), (Ar-16) besonders bevorzugt sind. In den zuvor dargelegten Strukturen Ar-1 bis Ar-79 sind in Bezug auf die Reste Ar⁵ die Substituenten R⁴ durch die entsprechenden Reste R⁷.

In einer weiteren bevorzugten Ausführungsform der Erfindung ist R⁷ gleich oder verschieden bei jedem Auftreten ausgewählt aus der Gruppe bestehend aus H, D, F, CN, einer geradkettigen Alkylgruppe mit 1 bis 10 C-Atomen oder einer verzweigten oder cyclischen Alkylgruppe mit 3 bis 10 C-Atomen, wobei die Alkylgruppe jeweils mit einem oder mehreren Resten R⁸ substituiert sein kann, oder einem aromatischen oder heteroaromatischen Ringsystem mit 6 bis 24 aromatischen Ringatomen, das jeweils durch einen oder mehrere Reste R⁸ substituiert sein kann. In einer besonders bevorzugten Ausführungsform der Erfindung ist R⁷ gleich oder verschieden bei jedem Auftreten ausgewählt aus der Gruppe bestehend aus H, einer geradkettigen Alkylgruppe mit 1 bis 6 C-Atomen, insbesondere mit 1, 2, 3 oder 4 C-Atomen, oder einer verzweigten oder cyclischen Alkylgruppe mit 3 bis 6 C-Atomen, wobei die Alkylgruppe mit einem oder mehreren Resten R⁸ substituiert sein kann, bevorzugt aber unsubstituiert ist, oder einem aromatischen oder heteroaromatischen Ringsystem mit 6 bis 13 aromatischen Ringatomen, das jeweils durch einen oder mehrere Reste R⁸ substituiert sein kann, bevorzugt aber unsubstituiert ist.

In einer weiteren bevorzugten Ausführungsform der Erfindung ist R⁸ gleich oder verschieden bei jedem Auftreten H, eine Alkylgruppe mit 1 bis 4 C-Atomen oder eine Arylgruppe mit 6 bis 10 C-Atomen, welche mit einer Alkylgruppe mit 1 bis 4 C-Atomen substituiert sein kann, bevorzugt aber unsubstituiert ist.

Bevorzugte Ausführungsformen der Verbindungen der Formeln (H-1) bzw. (H-2) sind die Verbindungen der folgenden Formeln (H-1a) bzw. (H-2a), 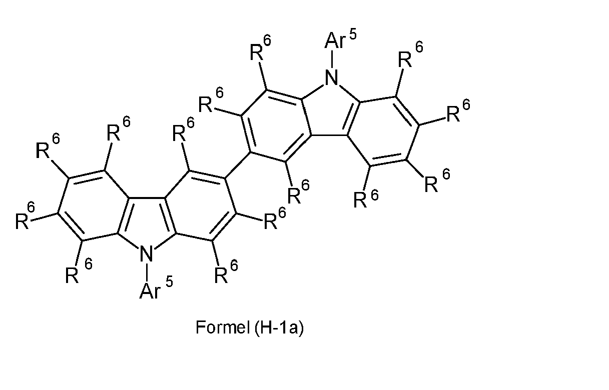 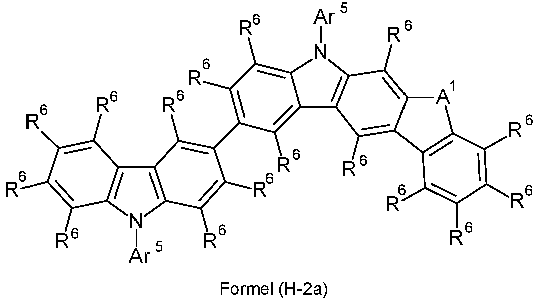 wobei R⁶, Ar⁵ und A¹ die zuvor, insbesondere für Formel (H-1) oder (H-2) genannten Bedeutungen aufweisen. In einer bevorzugten Ausführungsform der Erfindung steht A¹ in Formel (H-2a) für C(R⁷)₂.

Bevorzugte Ausführungsformen der Verbindungen der Formeln (H-1a) bzw. (H-2a) sind die Verbindungen der folgenden Formeln (H-1b) bzw. (H-2b), 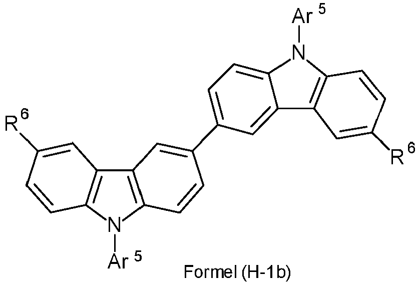 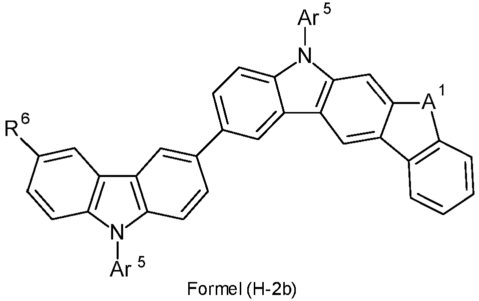 wobei R⁶, Ar⁵ und A¹ die zuvor, insbesondere für Formel (H-1) oder (H-2) genannten Bedeutungen aufweisen. In einer bevorzugten Ausführungsform der Erfindung steht A¹ in Formel (H-2b) für C(R⁷)₂.

Beispiele für geeignete Verbindungen gemäß Formel (H-1), (H-2), (H-3), (H-4) oder (H-5) sind Verbindungen, die in der Druckschrift WO2021/043755 auf Seiten 69 bis 73 als Beispiele für Verbindungen gemäß Formel (6), (7), (8), (9) oder (10) abgebildet sind. Diese Verbindungen werden durch Referenz hierauf zu Offenbarungszwecken in die vorliegende Anmeldung eingefügt.

Durch die Kombination mindestens einer Verbindung, umfassend mindestens eine Struktur mit mindestens drei miteinander kondensierten Strukturelementen gemäß den Formeln (A), (B) und (C), oder deren zuvor dargelegten bevorzugten Ausführungsformen mit einer Verbindung gemäß einer der Formeln (H-1), (H-2), (H-3), (H-4) oder (H-5) können überraschende Vorteile erzielt werden. Ein weiterer Gegenstand der vorliegenden Erfindung ist daher eine Zusammensetzung, enthaltend mindestens eine Verbindung, umfassend mindestens eine Struktur mit mindestens drei miteinander kondensierten Strukturelementen gemäß den Formeln (A'), (B') und (C') oder deren zuvor dargelegten bevorzugten Ausführungsformen, insbesondere Verbindungen umfassend mindestens eine Struktur mit mindestens drei miteinander kondensierten Strukturelementen gemäß den Formeln (A), (B) und (C), und mindestens ein weiteres Matrixmaterial, wobei das weitere Matrixmaterial ausgewählt ist aus Verbindungen gemäß einer der Formeln (H-1), (H-2), (H-3), (H-4) oder (H-5).

In einer bevorzugten Ausgestaltung kann vorgesehen sein, dass die erfindungsgemäße Verbindung, umfassend mindestens eine Struktur mit mindestens drei miteinander kondensierten Strukturelementen gemäß den Formeln (A'), (B') und (C') als Matrixmaterial für phosphoreszierende Emitter eingesetzt wird in Kombination mit einem weiteren Matrixmaterial, welches ausgewählt ist aus Verbindungen gemäß einer der Formeln (H-1), (H-2), (H-3), (H-4) oder (H-5). Demgemäß sind elektronische Vorrichtungen bevorzugt, bei denen die Verbindung, umfassend mindestens eine Struktur mit mindestens drei miteinander kondensierten Strukturelementen gemäß den Formeln (A'), (B') und (C') als Matrixmaterial für phosphoreszierende Emitter eingesetzt wird in Kombination mit einem weiteren Matrixmaterial, welches ausgewählt ist aus Verbindungen gemäß einer der Formeln (H-1), (H-2), (H-3), (H-4) oder (H-5).

Vorzugsweise kann vorgesehen sein, dass die Zusammensetzung aus mindestens einer Verbindung, umfassend mindestens eine Struktur mit mindestens drei miteinander kondensierten Strukturelementen gemäß den Formeln (A'), (B') und (C') oder deren zuvor dargelegten bevorzugten Ausführungsformen, insbesondere Verbindungen umfassend mindestens eine Struktur mit mindestens drei miteinander kondensierten Strukturelementen gemäß den Formeln (A), (B) und (C), und mindestens einer Verbindungen gemäß einer der Formeln (H-1), (H-2), (H-3), (H-4) oder (H-5) besteht. Diese Zusammensetzungen eignen sich insbesondere als sogenannte Premix-Mischungen, die gemeinsam verdampft werden können.

Hierbei können die Verbindungen gemäß einer der Formeln (H-1), (H-2), (H-3), (H-4) oder (H-5) jeweils einzeln oder als Mischung von zwei, drei oder mehr Verbindungen der jeweiligen Strukturen eingesetzt werden.

Weiterhin können die Verbindungen gemäß der Formeln (H-1), (H-2), (H-3), (H-4) oder (H-5) einzeln oder als Mischung von zwei, drei oder mehr Verbindungen unterschiedlicher Strukturen eingesetzt werden.

Die Verbindung, enthaltend mindestens eine Verbindung umfassend mindestens eine Struktur mit mindestens drei miteinander kondensierten Strukturelementen gemäß den Formeln (A'), (B') und (C') oder deren zuvor dargelegten bevorzugten Ausführungsformen, insbesondere Verbindungen umfassend mindestens eine Struktur mit mindestens drei miteinander kondensierten Strukturelementen gemäß den Formeln (A), (B) und (C), weist in der Zusammensetzung vorzugsweise einen Massenanteil im Bereich von 10 Gew.-% bis 95 Gew.-%, bevorzugt im Bereich von 15 Gew.-% bis 90 Gew.-%, und ganz bevorzugt im Bereich von 40 Gew.-% bis 70 Gew.-% auf, bezogen auf die Gesamtmasse der Zusammensetzung. Verbindungen, umfassend mindestens eine Struktur mit mindestens drei miteinander kondensierten Strukturelementen gemäß den Formeln (A'), (B') und (C'), können einzeln oder als Mischung von zwei, drei oder mehr Verbindungen eingesetzt werden.

Ferner kann vorgesehen sein, dass die Verbindungen gemäß einer der Formeln (H-1), (H-2), (H-3), (H-4) oder (H-5) in der Zusammensetzung einen Massenanteil im Bereich von 5 Gew.-% bis 90 Gew.-%, bevorzugt im Bereich von 10 Gew.-% bis 85 Gew.-%, mehr bevorzugt im Bereich von 20 Gew.-% bis 85 Gew.-%, noch mehr bevorzugt im Bereich von 30 Gew.-% bis 80 Gew.-%, ganz besonders bevorzugt im Bereich von 20 Gew.-% bis 60 Gew.-% und am meisten bevorzugt im Bereich von 30 Gew.-% bis 50 Gew.-% aufweist, bezogen auf die gesamte Zusammensetzung.

Darüber hinaus kann vorgesehen sein, dass die Zusammensetzung ausschließlich aus Verbindungen, umfassend mindestens eine Struktur mit mindestens drei miteinander kondensierten Strukturelementen gemäß den Formeln (A'), (B') und (C'), oder deren zuvor dargelegten bevorzugten Ausführungsformen und einem der genannten weiteren Matrixmaterialien, vorzugsweise Verbindungen nach mindestens einer der Formeln (H-1), (H-2), (H-3), (H-4) oder (H-5) besteht.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2017/032439 und WO 2018/011186 entnommen werden. Generell eignen sich alle phosphoreszierenden Komplexe, wie sie gemäß dem Stand der Technik für phosphoreszierende Elektrolumineszenzvorrichtungen verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Beispiele für phosphoreszierende Dotanden sind in der folgenden Tabelle aufgeführt.

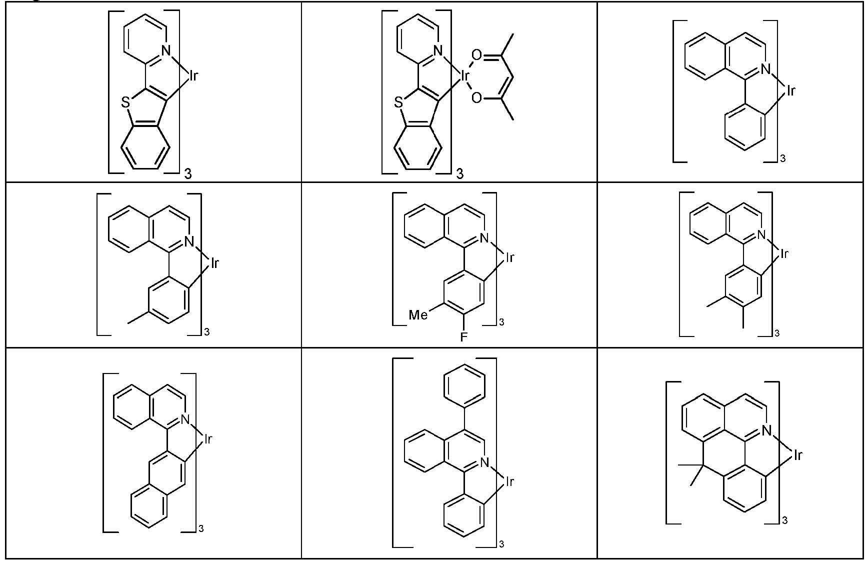

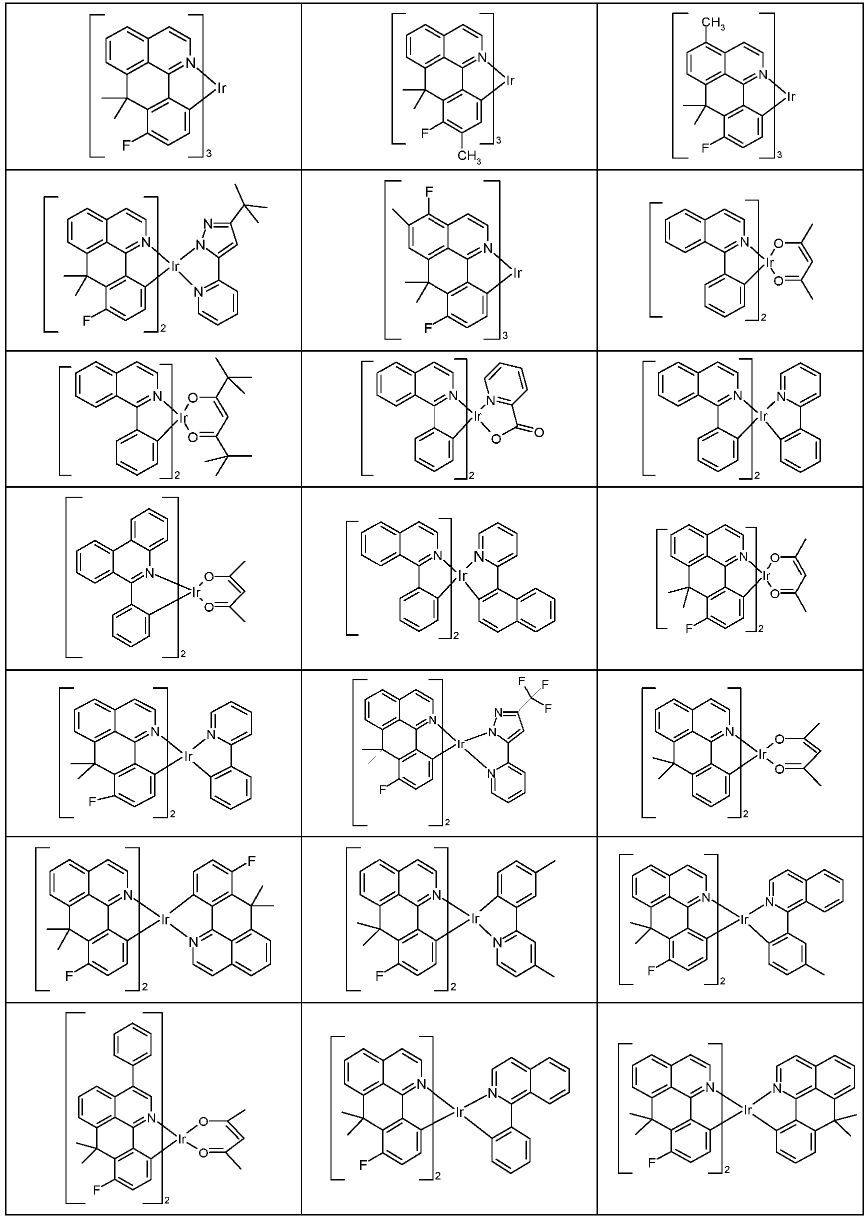

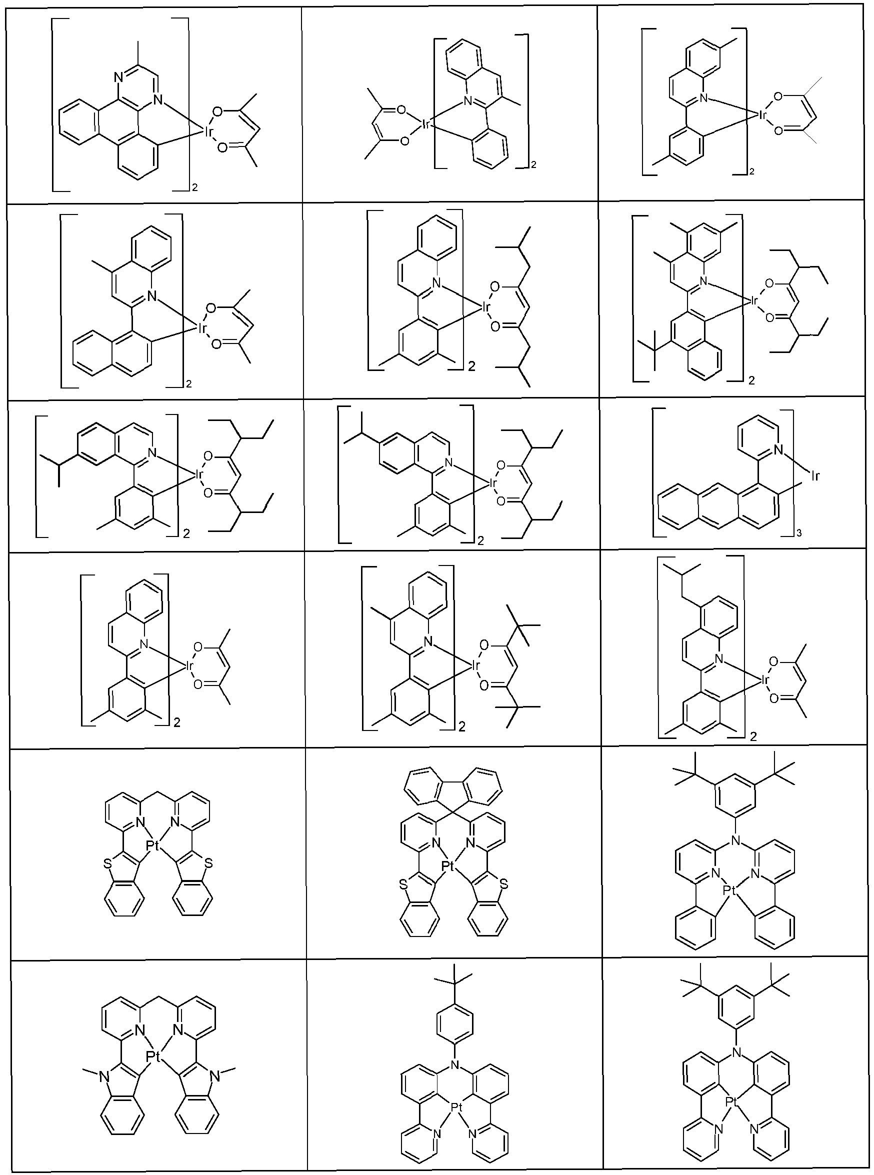

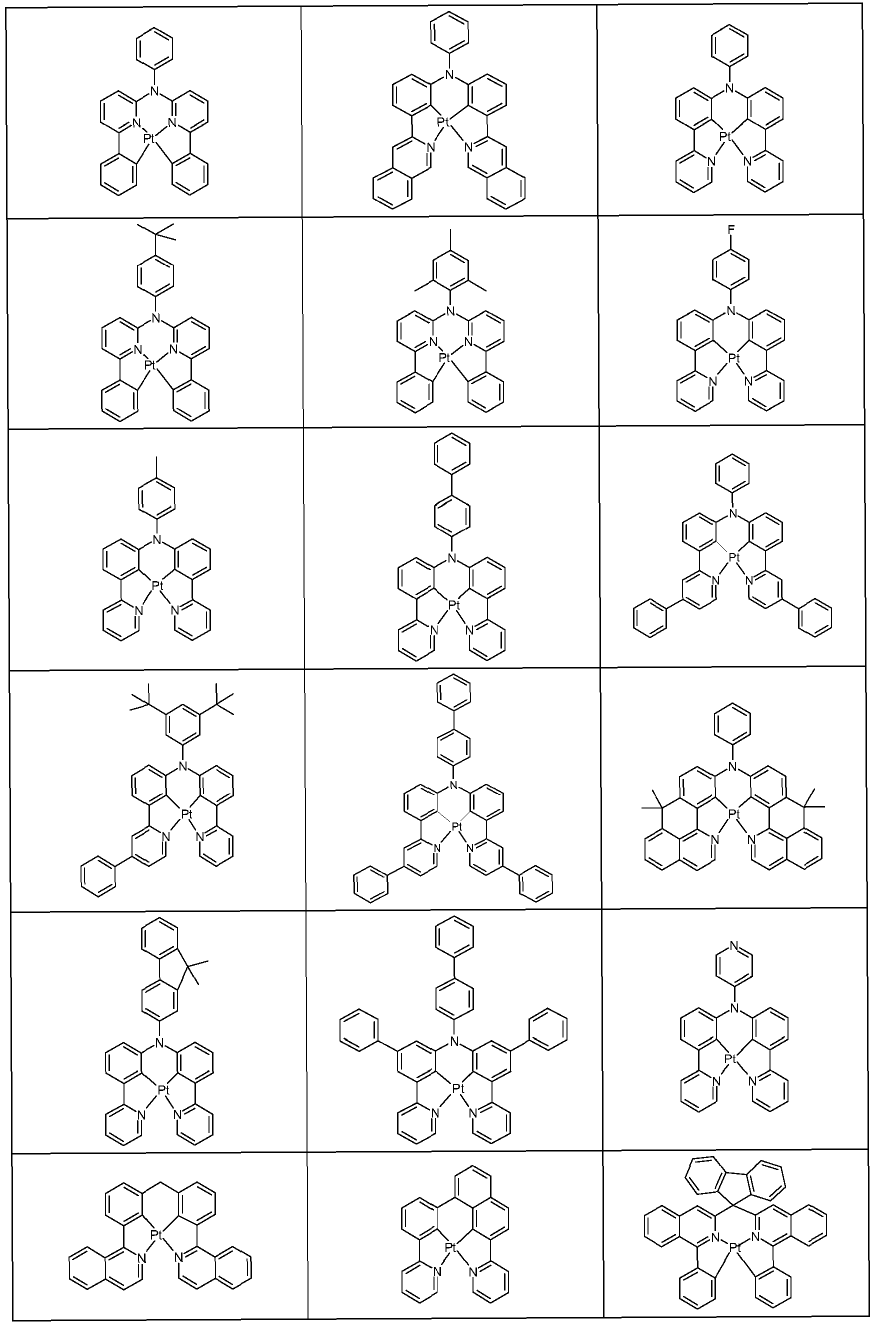

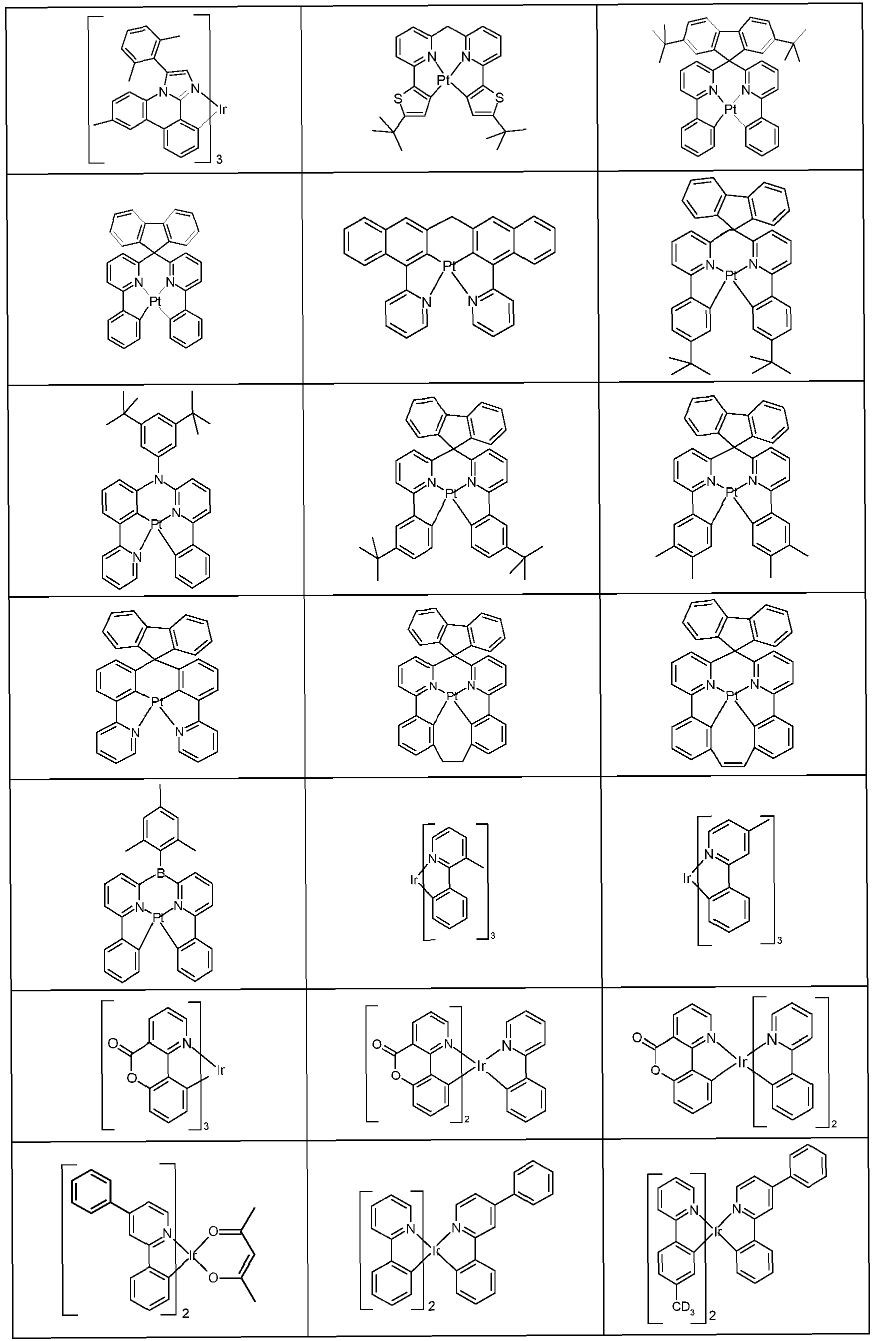

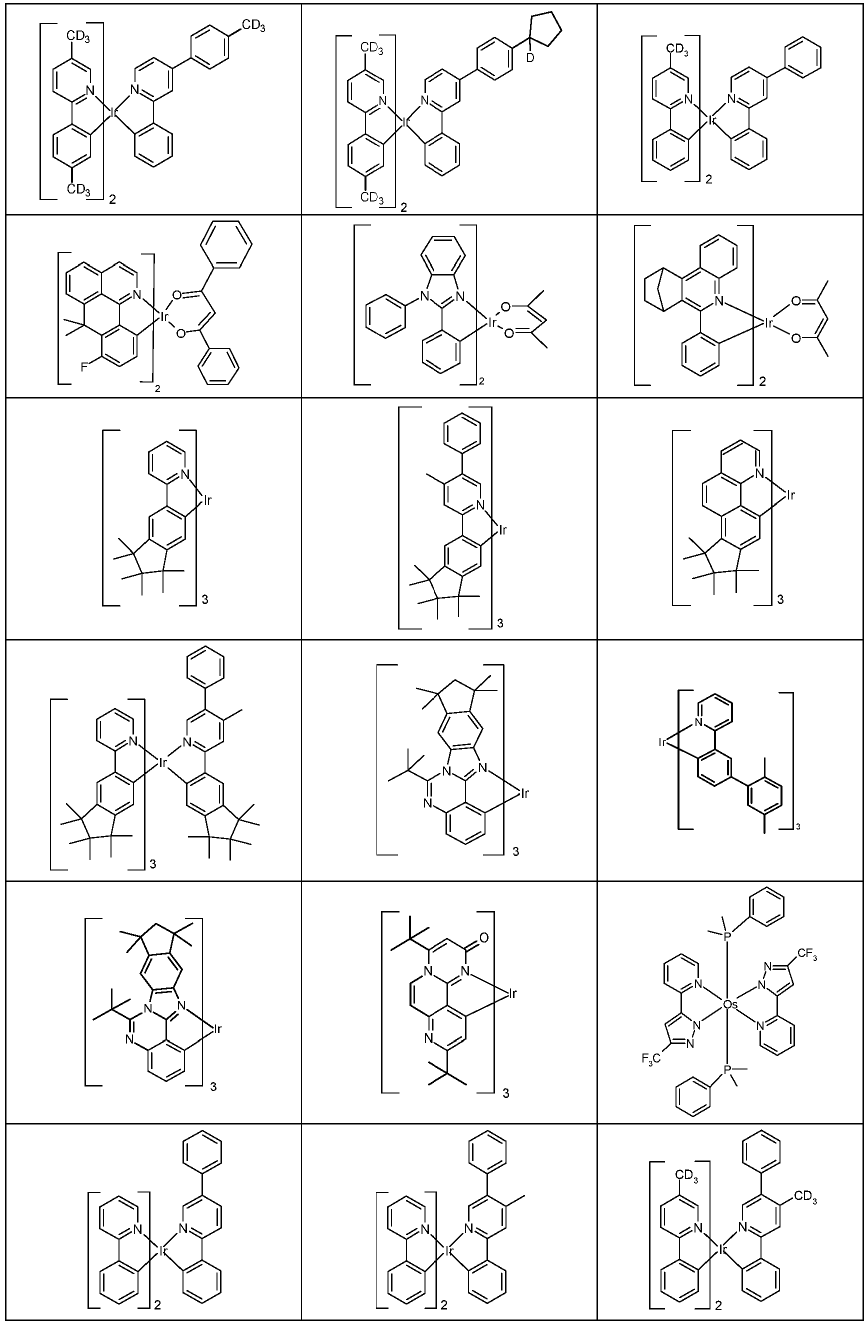

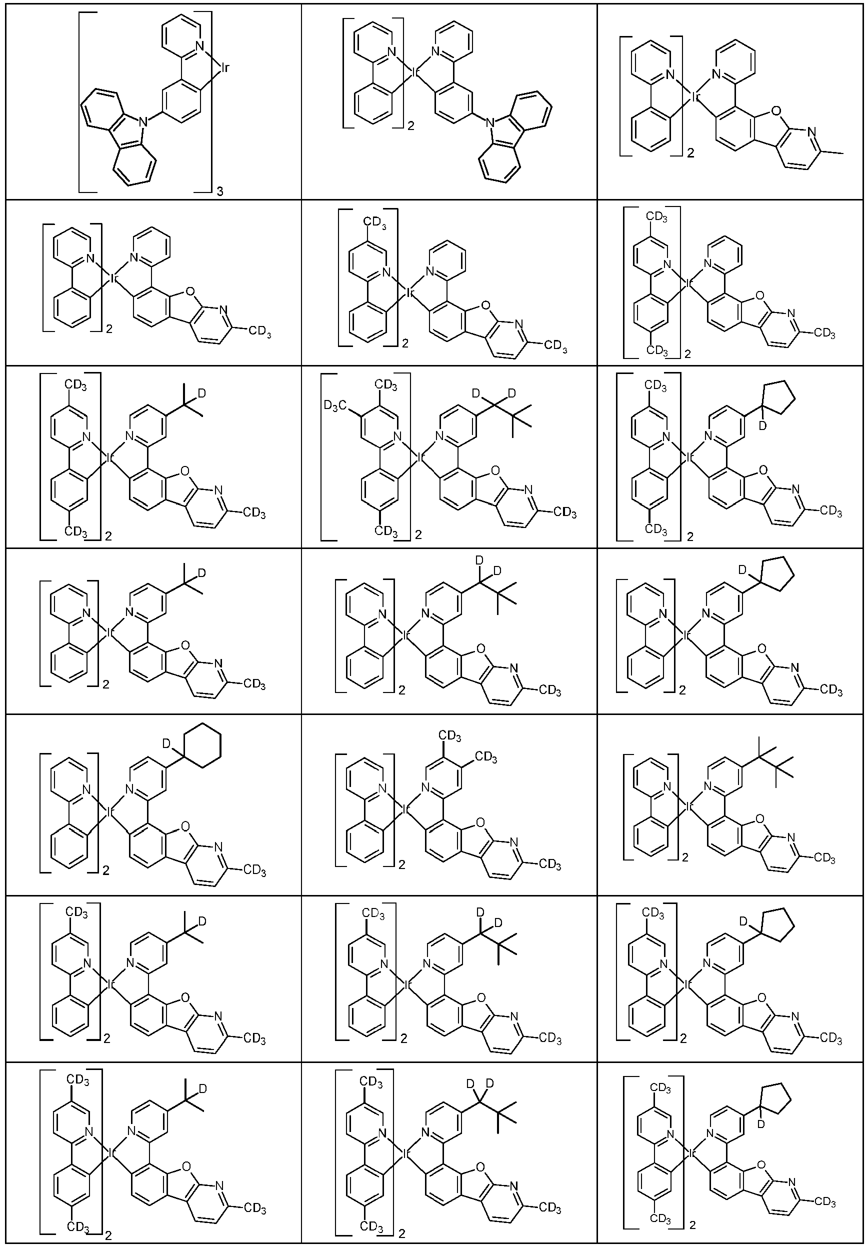

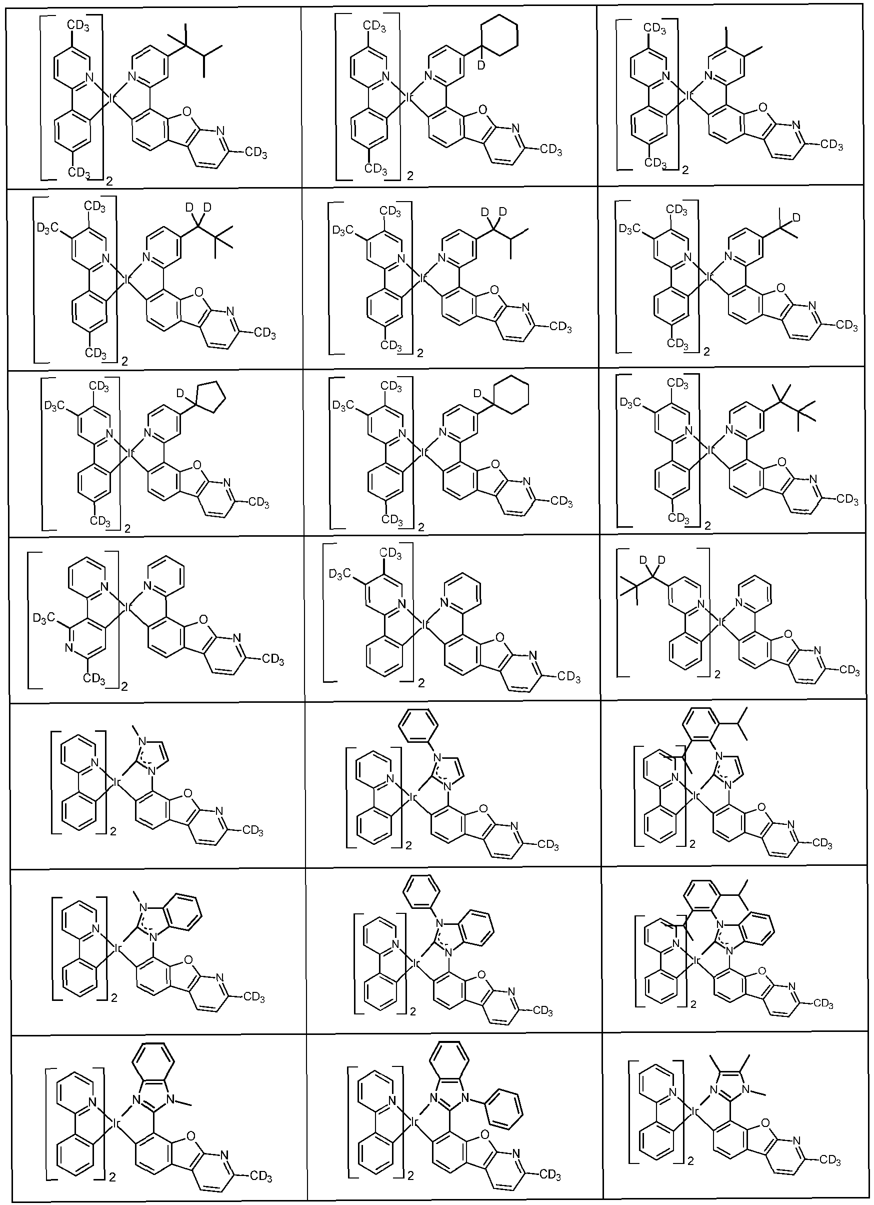

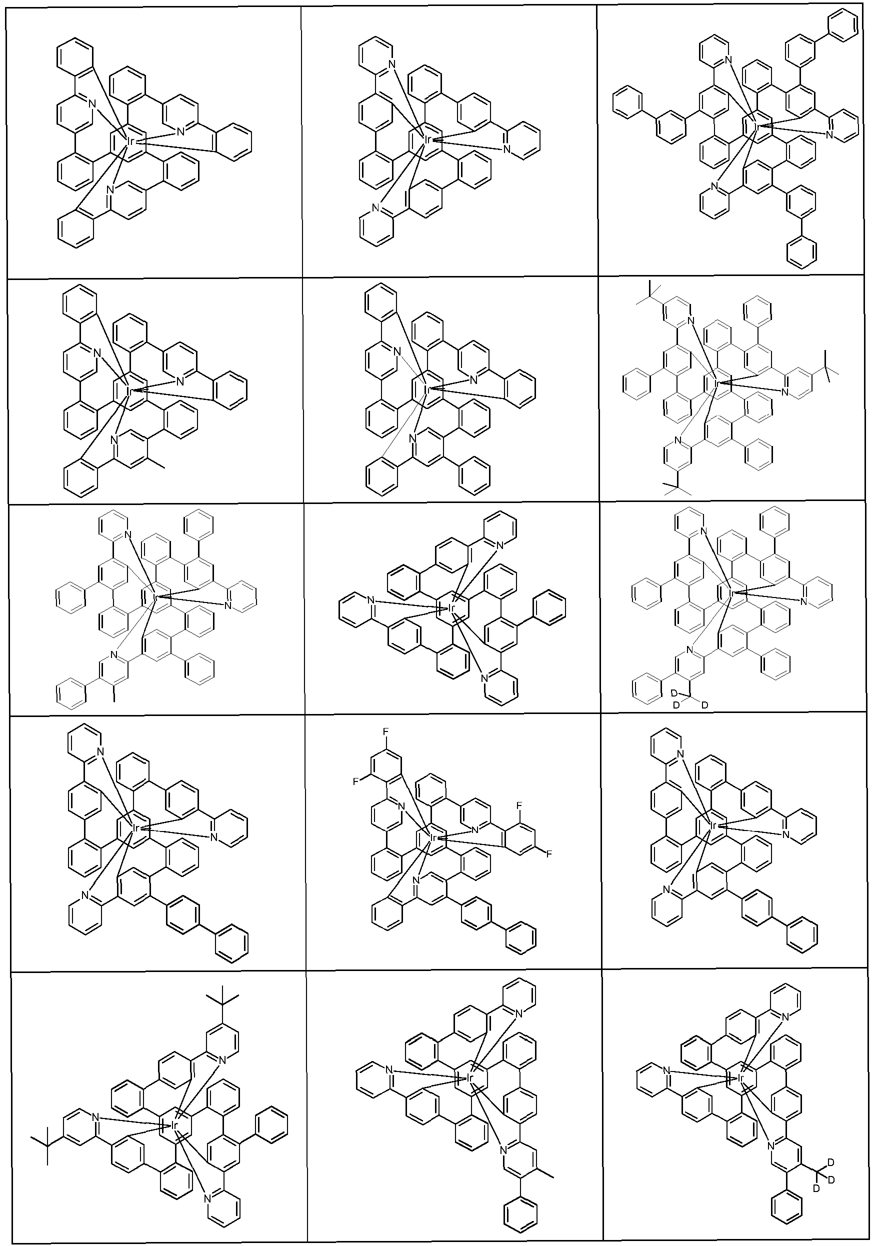

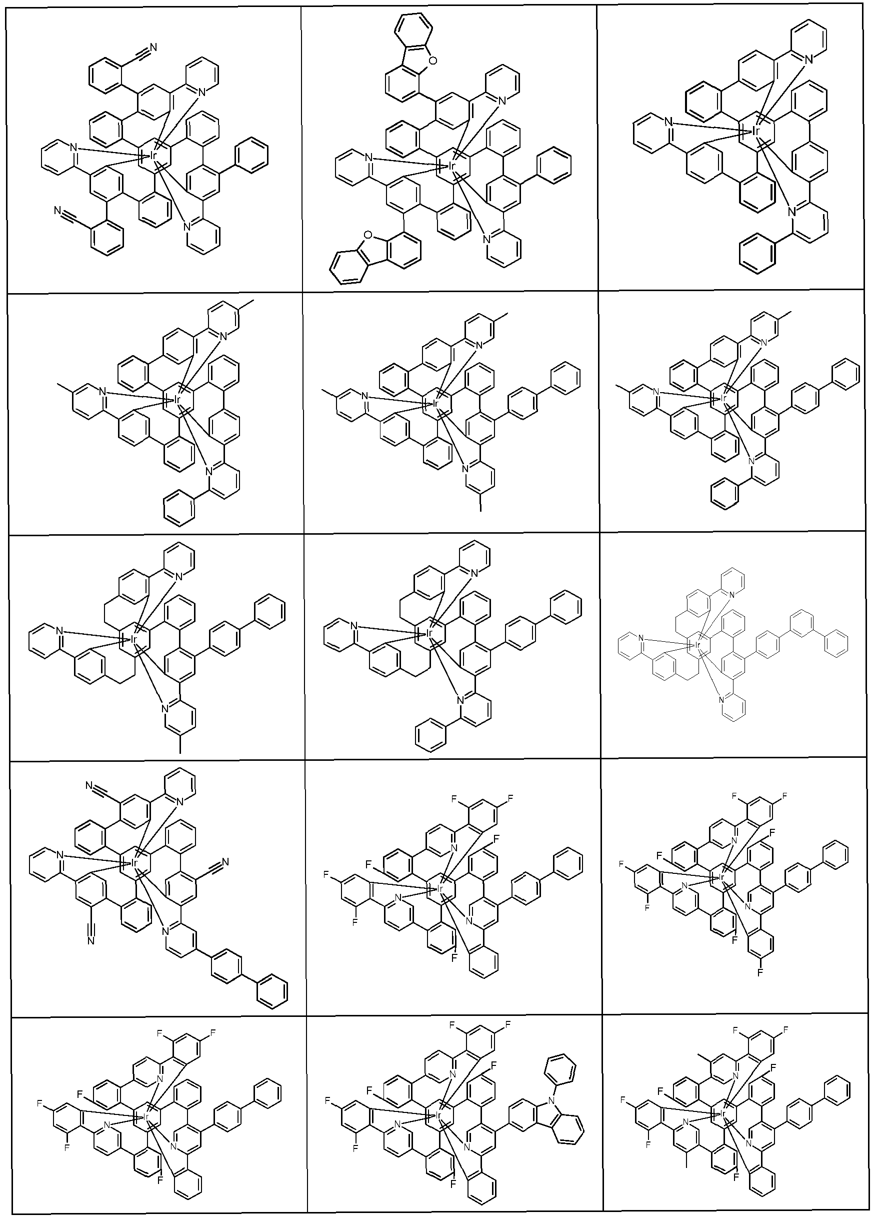

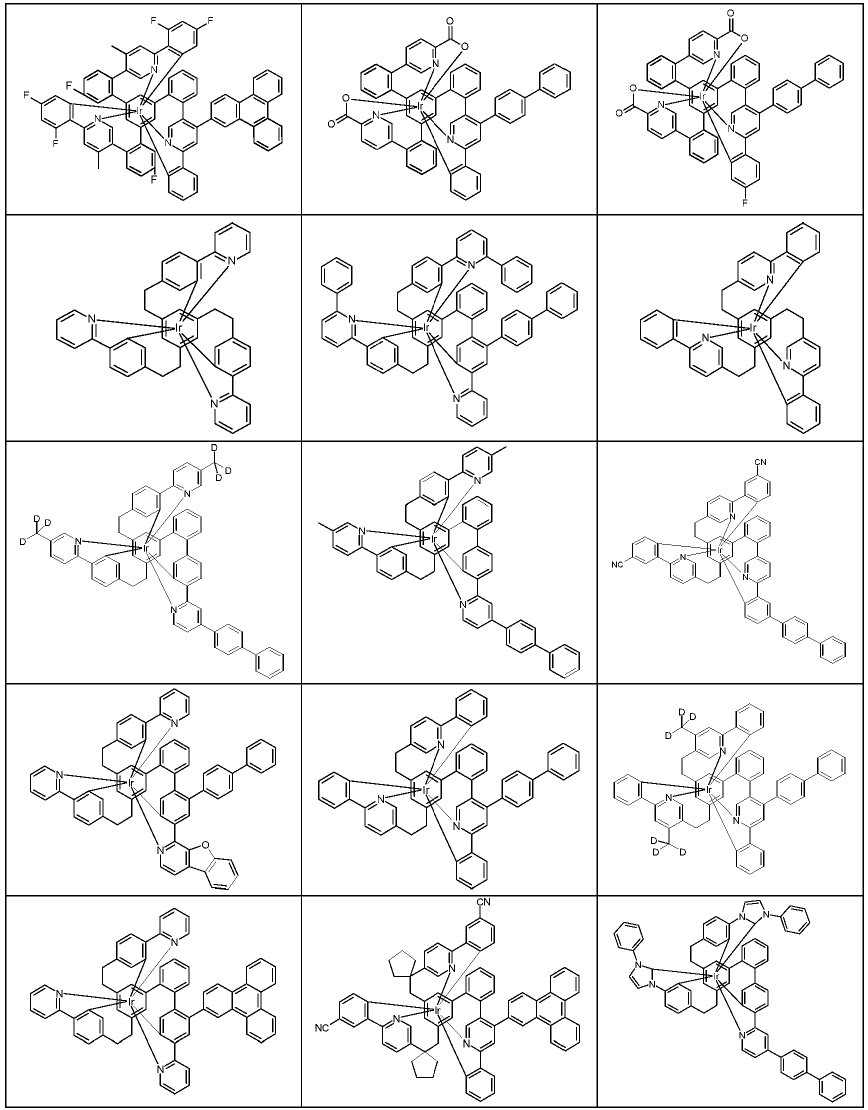

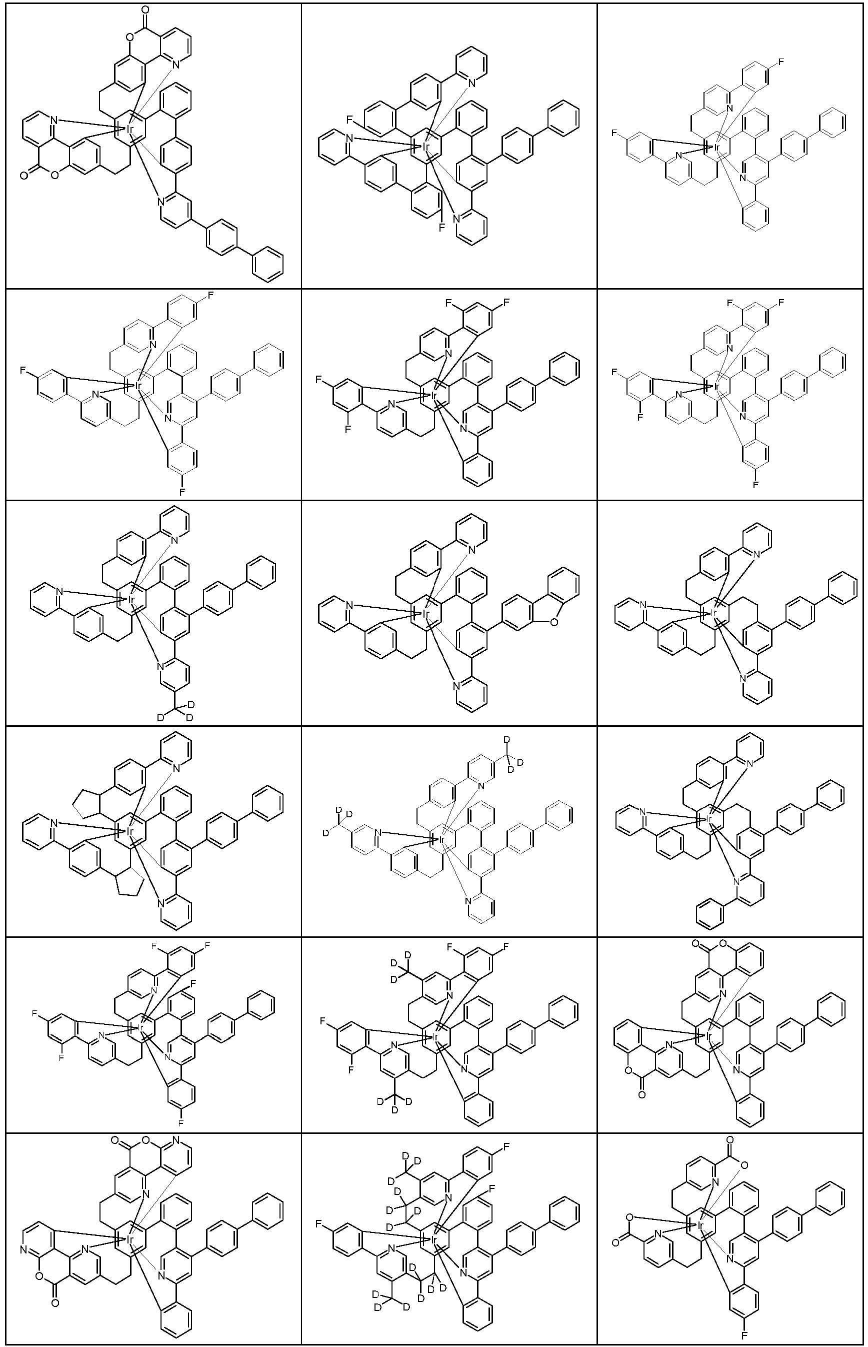

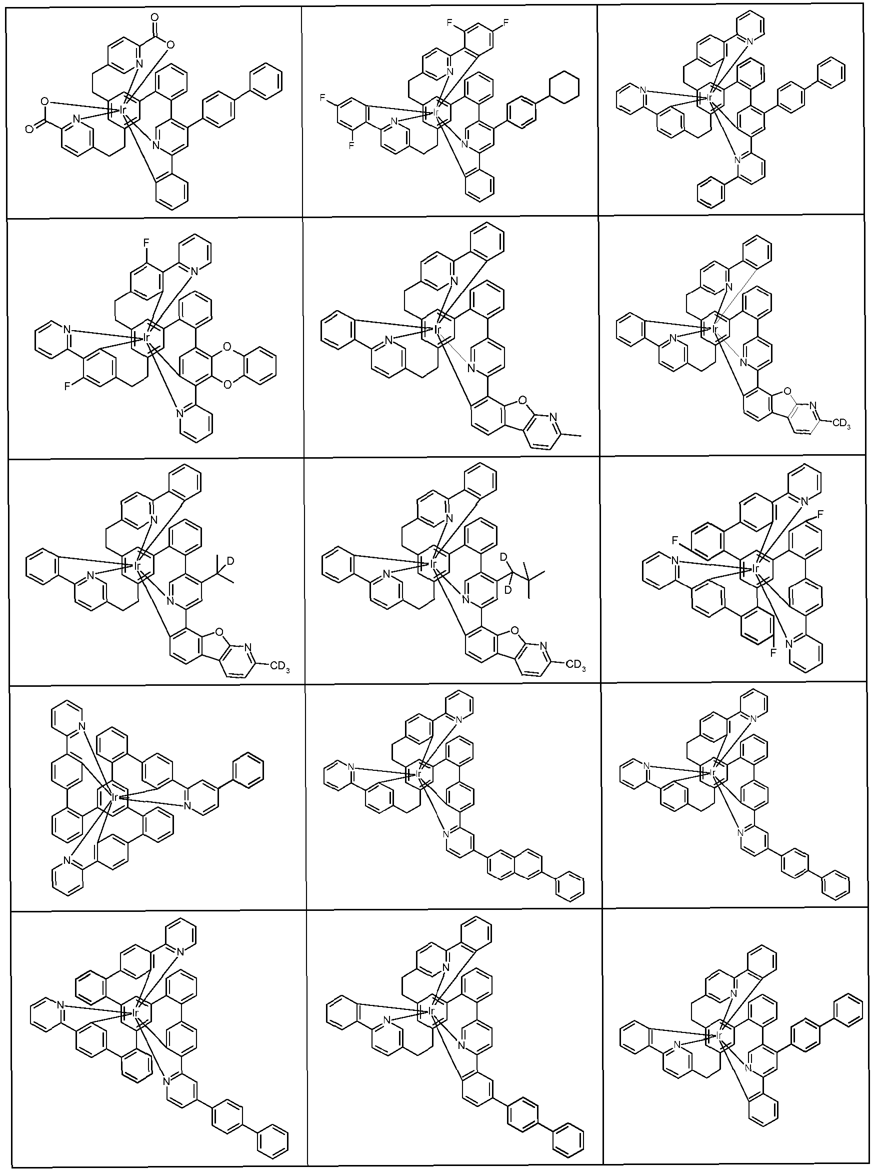

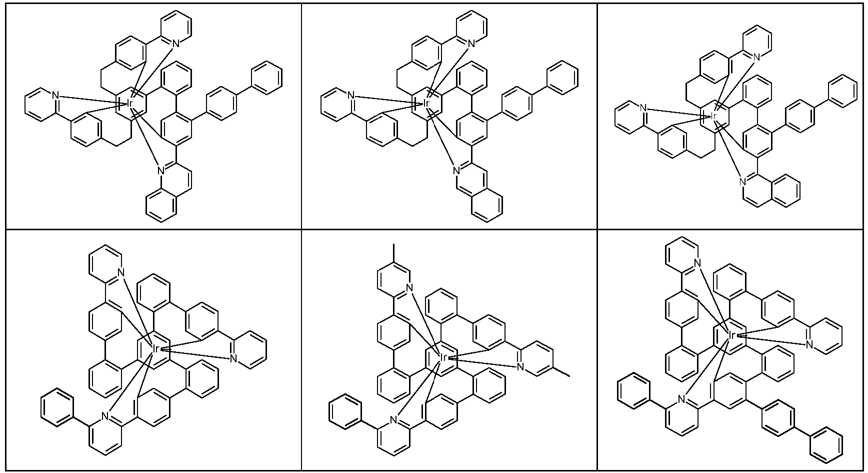

Die erfindungsgemäßen Verbindungen sind insbesondere auch geeignet als Matrixmaterialien für phosphoreszierende Emitter in organischen Elektrolumineszenzvorrichtungen, wie sie z. B. in WO 98/24271, US 2011/0248247 und US 2012/0223633 beschrieben sind. In diesen mehrfarbigen Display-Bauteilen wird eine zusätzliche blaue Emissionsschicht vollflächig auf alle Pixel, auch diejenigen mit einer von Blau verschiedenen Farbe, aufgedampft.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen Verbindung, umfassend mindestens eine Struktur mit mindestens drei miteinander kondensierten Strukturelementen gemäß den Formeln (A'), (B') und (C') bzw. den oben ausgeführten bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden.

Formulierungen zum Auftragen einer Verbindung umfassend mindestens eine Struktur mit mindestens drei miteinander kondensierten Strukturelementen gemäß den Formeln (A), (B) und (C), oder deren oder deren zuvor dargelegten bevorzugten Ausführungsformen sind neu. Ein weiterer Gegenstand der vorliegenden Erfindung ist daher eine Formulierung, enthaltend mindestens ein Lösungsmittel und eine Verbindung, umfassend mindestens eine Struktur mit mindestens drei miteinander kondensierten Strukturelementen gemäß den Formeln (A), (B) und (C) oder deren zuvor dargelegten bevorzugten Ausführungsformen. Weiterhin ist eine Formulierung, enthaltend mindestens ein Lösungsmittel und eine Verbindung, umfassend mindestens eine Struktur mit mindestens drei miteinander kondensierten Strukturelementen gemäß den Formeln (A'), (B') und (C'), oder deren zuvor dargelegten bevorzugten Ausführungsformen sowie eine Verbindung nach mindestens einer der Formeln (H-1), (H-2), (H-3), (H-4) oder (H-5) Gegenstand der vorliegenden Erfindung.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen und die erfindungsgemäßen organischen Elektrolumineszenzvorrichtungen zeichnen sich gegenüber dem Stand der Technik insbesondere durch eine verbesserte Lebensdauer aus. Dabei bleiben die weiteren elektronischen Eigenschaften der Elektrolumineszenzvorrichtungen, wie Effizienz oder Betriebsspannung, mindestens gleich gut. In einer weiteren Varianten zeichnen sich die erfindungsgemäßen Verbindungen und die erfindungsgemäßen organischen Elektrolumineszenzvorrichtungen gegenüber dem Stand der Technik insbesondere durch eine verbesserte Effizienz und/oder Betriebsspannung und höhere Lebensdauer aus.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:
1. Elektronische Vorrichtungen, insbesondere organische Elektrolumineszenzvorrichtungen enthaltend Verbindungen, umfassend mindestens eine Struktur mit mindestens drei miteinander kondensierten Strukturelementen gemäß den Formeln (A'), (B') und (C'), bzw. die zuvor und nachfolgend ausgeführten bevorzugten Ausführungsformen, insbesondere als Matrixmaterial oder als elektronenleitende Materialien, weisen eine sehr gute Lebensdauer auf. Hierbei bewirken diese Verbindungen insbesondere einen geringen Roll-off, d.h. einen geringen Abfall der Leistungseffizienz der Vorrichtung bei hohen Leuchtdichten.
2. Elektronische Vorrichtungen, insbesondere organische Elektrolumineszenzvorrichtungen enthaltend Verbindungen, umfassend mindestens eine Struktur mit mindestens drei miteinander kondensierten Strukturelementen gemäß den Formeln (A'), (B') und (C'), bzw. die zuvor und nachfolgend ausgeführten bevorzugten Ausführungsformen als elektronenleitende Materialien, und/oder Matrixmaterialien weisen eine hervorragende Effizienz auf. Hierbei bewirken erfindungsgemäße Verbindungen, umfassend mindestens eine Struktur mit mindestens drei miteinander kondensierten Strukturelementen gemäß den Formeln (A), (B) und (C), bzw. die zuvor und nachfolgend ausgeführten bevorzugten Ausführungsformen eine geringe Betriebsspannung bei Verwendung in elektronischen Vorrichtungen.
3. Die erfindungsgemäßen Verbindungen, umfassend mindestens eine Struktur mit mindestens drei miteinander kondensierten Strukturelementen gemäß den Formeln (A), (B) und (C), bzw. die zuvor und nachfolgend ausgeführten bevorzugten Ausführungsformen zeigen eine sehr hohe Stabilität und Lebensdauer.
4. Mit Verbindungen, umfassend mindestens eine Struktur mit mindestens drei miteinander kondensierten Strukturelementen gemäß den Formeln (A), (B) und (C), bzw. den zuvor und nachfolgend ausgeführten bevorzugten Ausführungsformen kann in elektronischen Vorrichtungen, insbesondere organische Elektrolumineszenzvorrichtungen die Bildung von optischen Verlustkanäle vermieden werden. Hierdurch zeichnen sich diese Vorrichtungen durch eine hohe PL- und damit hohe EL-Effizienz von Emittern bzw. eine ausgezeichnete Energieübertragung der Matrices auf Dotanden aus.
5. Verbindungen, umfassend mindestens eine Struktur mit mindestens drei miteinander kondensierten Strukturelementen gemäß den Formeln (A), (B) und (C), bzw. die zuvor und nachfolgend ausgeführten bevorzugten Ausführungsformen weisen eine ausgezeichnete Glasfilmbildung auf.
6. Verbindungen, umfassend mindestens eine Struktur mit mindestens drei miteinander kondensierten Strukturelementen gemäß den Formeln (A), (B) und (C), bzw. die zuvor und nachfolgend ausgeführten bevorzugten Ausführungsformen bilden aus Lösungen sehr gute Filme.
7. Elektronische Vorrichtungen, insbesondere organische Elektrolumineszenzvorrichtungen enthaltend Verbindungen, umfassend mindestens eine Struktur mit mindestens drei miteinander kondensierten Strukturelementen gemäß den Formeln (A'), (B') und (C'), bzw. die zuvor und nachfolgend ausgeführten bevorzugten Ausführungsformen in Kombination mit Hostmaterialien gemäß einer oder mehrerer der Formeln (H-1) bis (H-5), insbesondere als Matrixmaterial, weisen eine verbesserte Lebensdauer und eine höhere Effizienz auf.
8. Die Verbindungen, umfassend mindestens eine Struktur mit mindestens drei miteinander kondensierten Strukturelementen gemäß den Formeln (A), (B) und (C), bzw. die zuvor und nachfolgend ausgeführten bevorzugten Ausführungsformen weisen ein tiefes Triplett-Niveau T₁ auf, welches beispielsweise im Bereich von -2,22 eV bis -2.9 eV liegen kann.

Diese oben genannten Vorteile gehen nicht mit einer unmäßig hohen Verschlechterung der weiteren elektronischen Eigenschaften einher.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre in getrockneten Lösungsmitteln durchgeführt. Die Lösungsmittel und Reagenzien können z. B. von Sigma-ALDRICH bzw. ABCR bezogen werden. Zu den literaturbekannten Verbindungen sind jeweils auch die entsprechenden CAS-Nummern angegeben.

### Synthesebeispiele

### a) 2-(2-Fluorophenyl)-3-methyl-isoquinolin-1-on

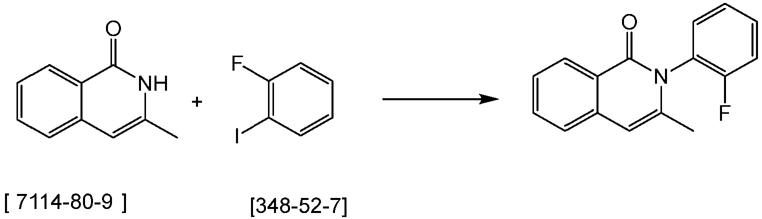

6,3 g (40mmol) 3-Methyl-1(2H)-isoquinolinon und 9,9 g (45 mmol) 1-Fluoro-2- iodbenzol und 44,7 g (320 mmol) Kaliumcarbonat, 3 g (16 mmol) Kupfer(I])-iodid und 3,6 g (16 mmol) 1,3-Di-(pyridin-2-yl)-propan-1,3-dion werden in 100 ml DMF bei 150°C für 30 h gerührt. Die Lösung wird mit Wasser verdünnt und mit Essigester zweimal extrahiert, die vereinigten organischen Phasen über Na₂SO₄ getrocknet und eingeengt. Der Rückstand wird chromatographisch (EtOAc/Hexan: 2/3) gereinigt, aus Toluol umkristallisiert und abschließend im Hochvakuum (p = 5 x 10-5 mbar) gereinigt. Die Reinheit beträgt 99.9%. Die Ausbeute beträgt 8,1 g (30 mmol), 77% der Theorie.

Analog dazu werden die folgenden Verbindungen hergestellt:

| **Bsp.** | **Edukt 1** | **Edukt 2** | **Produkt** | **Ausbeute** |
|---|---|---|---|---|
| 1a | 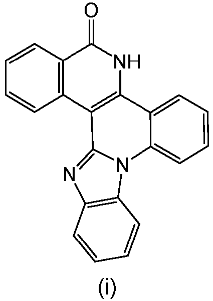 | 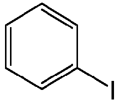 | 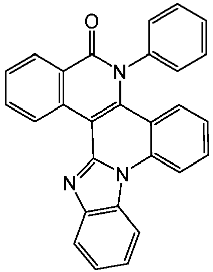 | 75% |
| 2a | 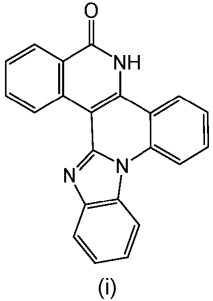 | 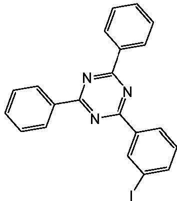 [1476799-05-9 | 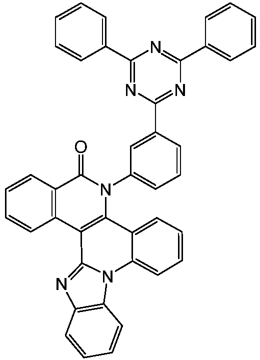 | 68% |
| 3a | 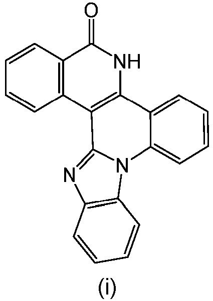 | 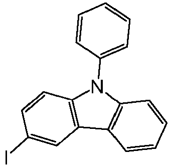 [502161-03-7] | 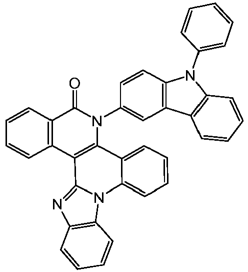 | 74% |
| 4a | 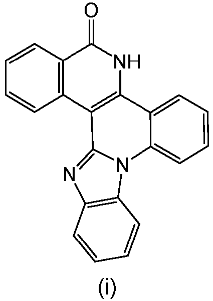 | 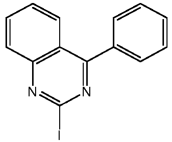 [1628067-38-8 | 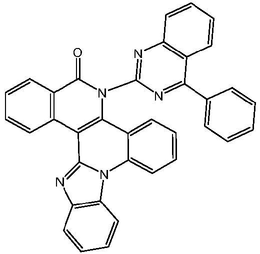 | 61% |
| 5a | 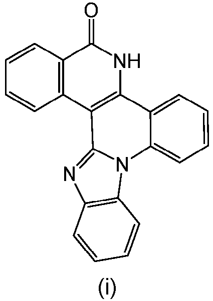 | 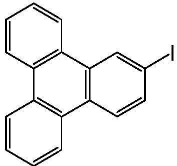 [1228778-59-3] | 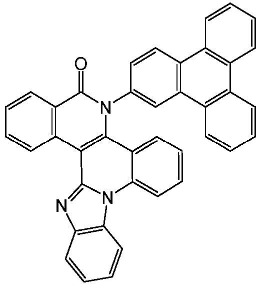 | 79% |
| 6a | 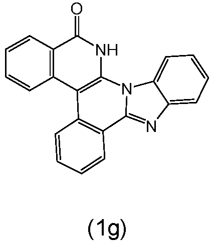 | 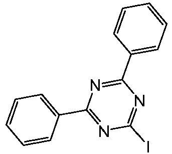 [83819-97-0 | 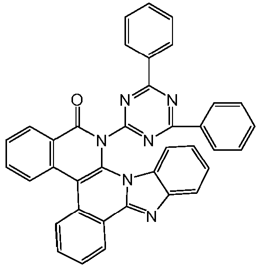 | 70% |
| 7a | 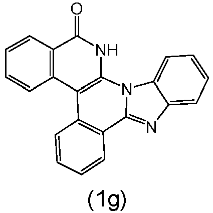 | 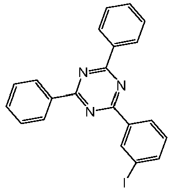 [1476799-05-9 | 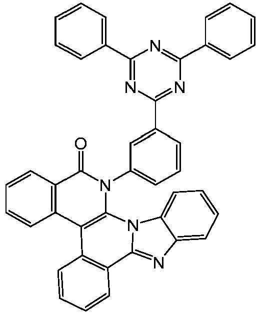 | 66% |
| 8a | 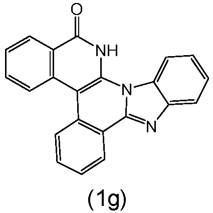 | 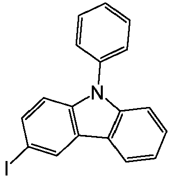 [502161-03-7] | 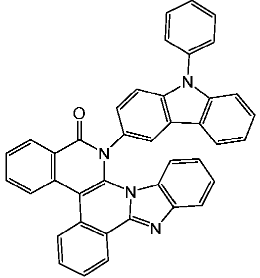 | 71% |

### b) 3-(2-Fluorophenyl)-4-oxo-quinazoline-2-carbaldehyd

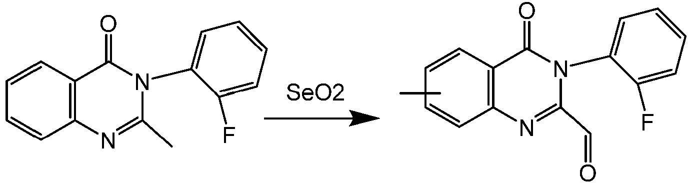

25,4 g (100 mmol) 2-(2-Fluorophenyl)-3-methyl-isoquinolin-1-on wird in 500 ml heißem Dioxan gelöst, und dann wird 12 g pulverisiertes Selendioxid (200 mmol) Portionsweise unter Rühren zugeben. Nach vollständiger Zugabe wird das Reaktionsgemisch 8 h unter Rückfluß erhitzt. Dann wird das Reaktionsgemisch abfiltriert und die Losung mit eiskaltem Wasser versetzt und das Produkt abfiltriert und in Toluol umkristallisiert.

Die Ausbeute beträgt 18 g (73 mmol), entsprechend 70 % der Theorie.

Analog dazu werden die folgenden Verbindungen hergestellt:

| **Bsp.** | **Edukt 1** | **Produkt** | **Ausbeute** |
|---|---|---|---|
| 1b | 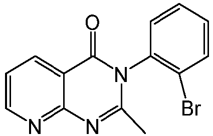 [773883-03-7 ] | 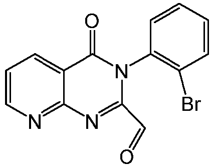 | 65% |

### c) 2,6-Diphenyl-8-(2-phenylphenyl)-9H-purin

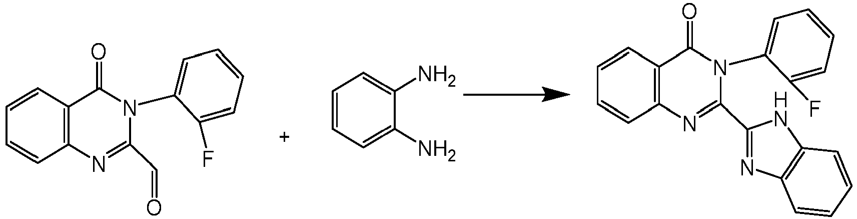

Eine Lösung von 7,5 g (28 mmol) 3-(2-Fluorophenyl)-4-oxo-quinazoline-2-carbaldehyd und 3 g (28 mmol) Phenylen-1,2-diamin wird in 50 ml DMF auf 80 ° C erhitzt. Das Reaktionsgemisch wird 8 h rühren gelassen und die entstandene Lösung wird auf Raumtemperatur gebracht und dann mit Ethylacetat (EtOAc) extrahiert. Die organische Schicht wird mit Salzlösung gewaschen, über wasserfreiem Na₂SO₄ getrocknet und unter vermindertem Druck eingeengt. Das Rohprodukt wird durch Kieselgel-Säulenchromatographie gereinigt mit n-Hexan-EtOAc. Ausbeute: 7,7 (21 mmol), 78 % d. Th.

Analog können folgende Verbindungen erhalten werden:

| **Bsp.** | **Edukt 1** | **Edukt 2** | **Produkt** | **Ausbeute** |
|---|---|---|---|---|
| 1c | 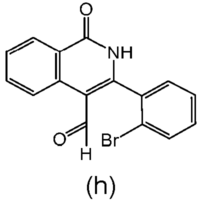 | 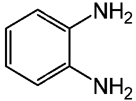 | 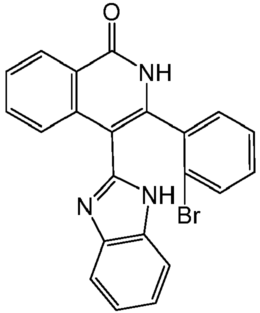 | 73% |
| 2c | 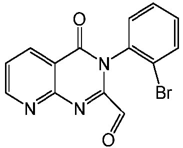 | 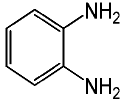 | 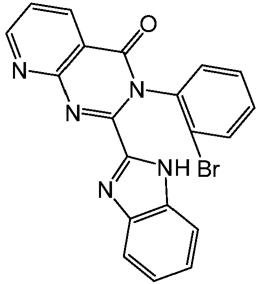 | 60% |

### d) 3-(1H-Benzimidazol-2-yl)-2-(2-fluorophenyl)-chinazolin-4-on

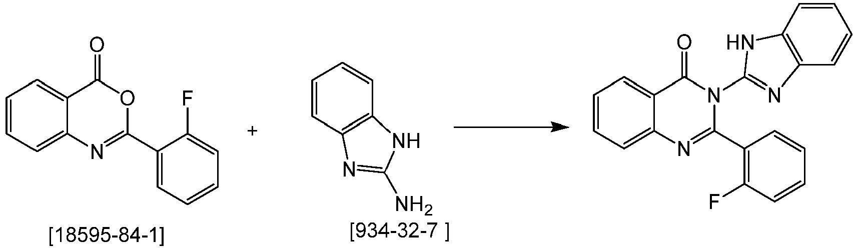

Eine Mischung aus 4,8 g (20 mmol) 2-(2-Fluorophenyl)-3,1-benzoxazin-4-on und 2,66 g (20 mmol) 2-Aminobenzimidazol wird 30 Minuten bei 200-250°C in einem Ölbad miteinander verschmolzen, so dass das Reaktionsgemisch wegen hoher Heizung nicht abgedampft werden sollte. Die Mischung wird abgekühlt und mit 20 ml Ethanol versetzt und abfiltriert. Der abgetrennte Feststoff wird mit Ethanol gewaschen, getrocknet und mehrmals mit Ethanol umkristallisiert, um das reine Produkt zu erhalten. Ausbeute: 4,6 (13 mmol), 65 % d. Th.

Analog können folgende Verbindungen erhalten werden:

| **Bsp.** | **Edukt 1** | **Edukt 2** | **Produkt** | **Ausbeute** |
|---|---|---|---|---|
| 1d | 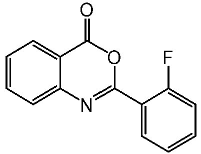 [18595-84-1] | 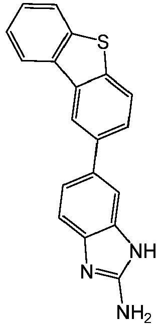 [2179082-00-7 ] | 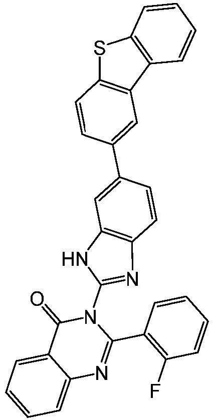 | 57% |
| 2d | 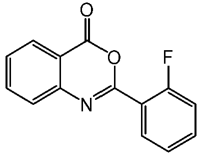 [18595-84-1] | 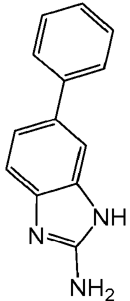 [445010-80-0] | 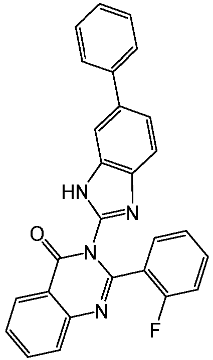 | 61% |
| 3d | 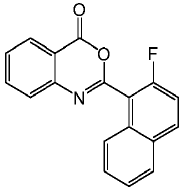 [2272167-51-6 ] | 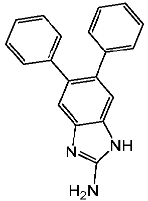 [2127149-53-3 ] | 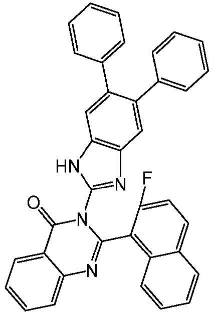 | 60% |
| 4d | 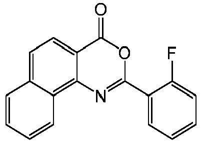 [775356-23-5 ] | 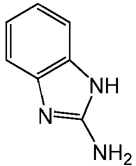 [934-32-7 ] | 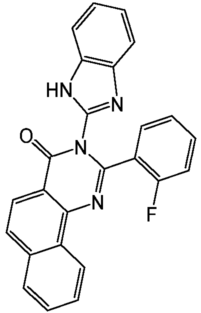 | 53% |

### e) Cyclisierung

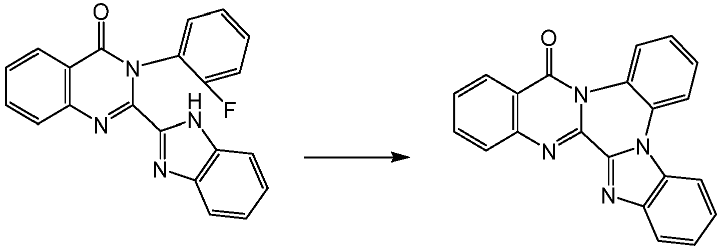

Zu einer Lösung von 1,78 g ( 5 mmol) 2,6-Diphenyl-8-(2-phenylphenyl)-9H-purin in 50 ml DMF wird 0,32 g (1 mmol) Cs₂CO₃ zugegeben und das Reaktionsgemisch wird 10 min. bei 150°C in einer Mikrowelle erhitzt. Nachdem das Mikrowellenfläschchen auf Raumtemperatur abgekühlt ist, wird das Lösungsmittel mit EtOAc (150 ml) verdünnt und mit gesättigten NaCl-Lösung gewaschen (2 X100 ml). Die organische Phase wird getrennt und über Mg₂SO₄ getrocknet. Der Rückstand wird durch Säulenchromatographie unter Verwendung eines Gradienten Ethylacetat / Hexan (0-60%) gereinigt. Der Rückstand wird aus Toluol umkristallisiert und abschließend im Hochvakuum (p = 5 x 10-5 mbar). Die Reinheit beträgt 99.9%.

Ausbeute: 1,3 (3,9 mmol), 78 % d. Th.

Analog können folgende Verbindungen erhalten werden:

| **Bsp.** | **Edukt 1** | **Produkt** | **Ausbeute** |
|---|---|---|---|
| 1e | 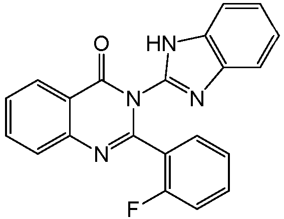 | 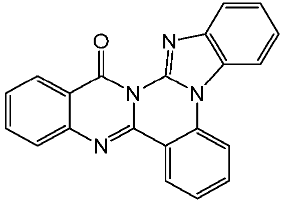 | 69% |
| 2e | 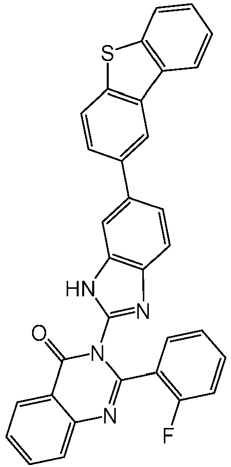 | 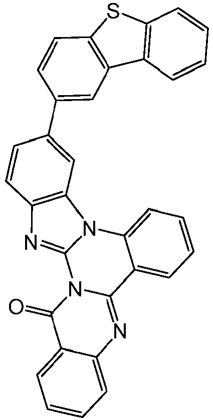 | 74% |
| 3e | 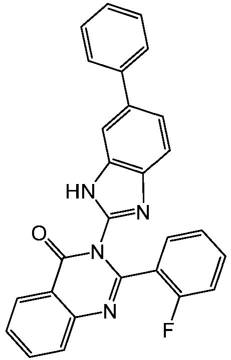 | 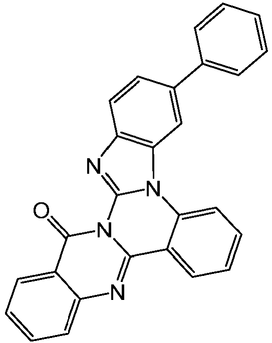 | 71% |
| 4e | 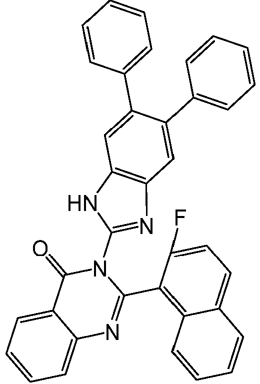 | 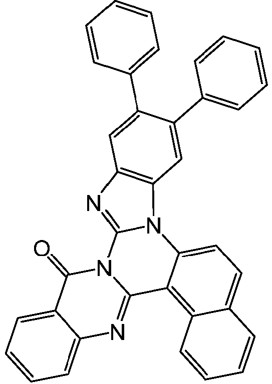 | 68% |

### f) Cyclisierung

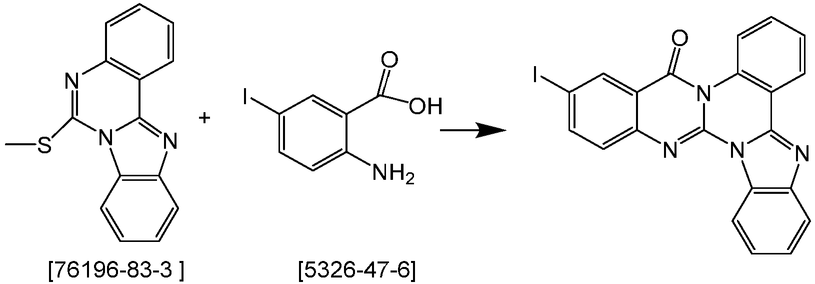

Eine Mischung aus 2 g, (7,5 mmol) 6-(Methylthio)benzimidazo[1,2-c]chinazolin, 3,74 g ( 43 mmol) Anthranilsäure und 20 g Graphit werden in einem 70 ml Quarzfläschchen in den Mikrowellenofen gegeben. Die Bestrahlung wird 90 min. bei 105 W programmiert. Nach einem Zeitraum von 2 ± 3 min. wird 170 °C erreicht und bleibt bei dieser Temperatur konstant. Nach dem Abkühlen wird das Graphitpulver filtriert, mit 10 ml Dichlormethan umgesetzt, filtriert und mit 5 ml Dichlormethan nachgewaschen. Die organische Lösung wird mit einer gesättigten Natriumbicarbonatlösung gewaschen und das Rohprodukt in Ethanol um kristallisiert.

Ausbeute: 3,0g (6,64 mmol), 88 % d. Th.

Analog können folgende Verbindungen erhalten werden:

| **Bsp.** | **Edukt 1** | **Edukt 2** | **Produkt** | **Ausbeute** |
|---|---|---|---|---|
| 1f | 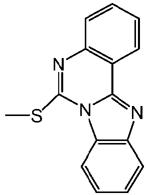 [76196-83-3 ] | 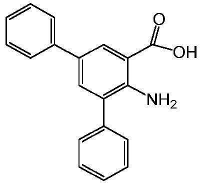 [676168-62-0] | 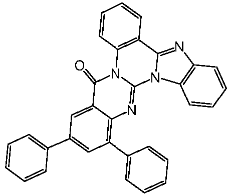 | 89% |
| 2f | 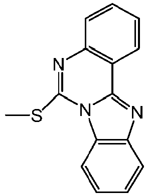 [76196-83-3 ] | 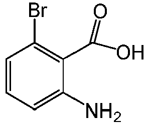 [20776-52-7 ] | 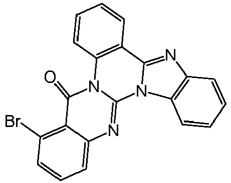 | 87% |
| 3f | 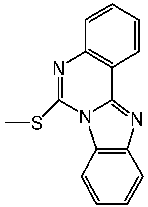 [76196-83-3 ] | 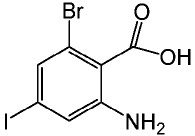 [2166641-39-8 ] | 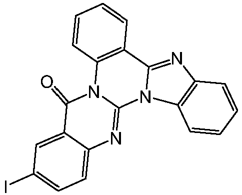 | 64% |
| 4f | 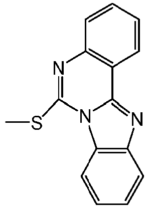 [76196-83-3 ] | 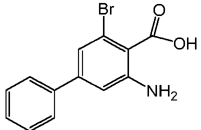 [4445-43-6 ] | 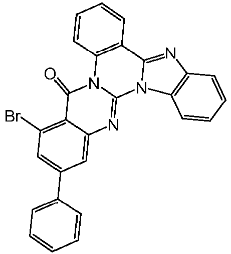 | 61% |
| 5f | 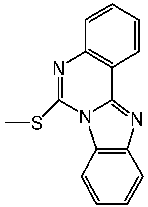 [76196-83-3 ] | 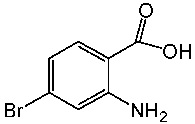 [20776-50-5 ] | 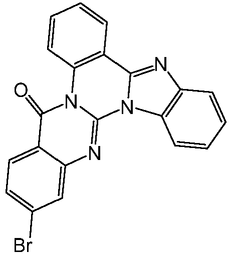 | 72% |
| 6f | 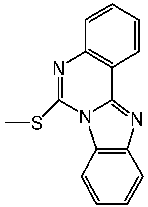 [76196-83-3 ] | 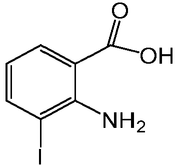 [20776-55-0 ] | 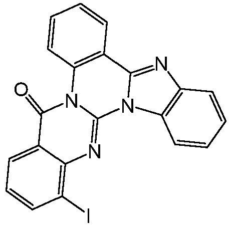 | 70% |
| 7f | 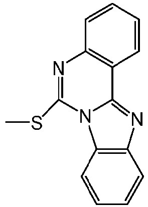 [76196-83-3 ] | 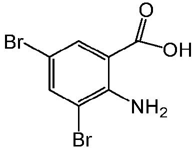 [609-85-8] | 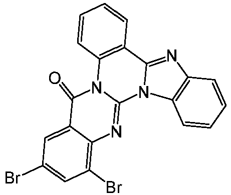 | 58% |

### g) Cyclisierung

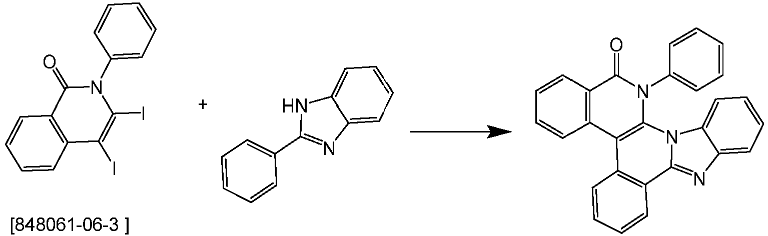

Unter Schutzgas wird 3,9 g (20 mmol, 1,0 eq.)2-Phenylbenzimidazol, 14,1 g (30 mmol, 1,5 eq.) 3,4-Diiodo-2-phenyl-1(2H)-isoquinolinon, 8,2 g (60 mmol, 3,0 eq.) K₂CO₃, 0,5 (2 mmol) Pd(OAc)₂ und schließlich 1,9 g (4 mmol) Xphos in 200 ml DMF zugegeben. Die Mischung wird 80 h bei 160°C erhitzt und kommt dann auf Raumtemperatur. Anschließend wird 5 min mit Wasser gequencht und mit 8 ml Ethylacetat verdünnt. Die organische Phase wird getrennt und im Vakuum eingeengt. Das Rohprodukt wird dann durch Flash-Chromatographie an Kieselgel getrennt (3-10% Ethylacetat / Petrolether).

Ausbeute: 2,9 g (18,9 mmol), 63 % d. Th.

Analog können folgende Verbindungen erhalten werden:

| **Bsp.** | **Edukt 1** | **Edukt 2** | **Produkt** | **Ausbeute** |
|---|---|---|---|---|
| 1g | 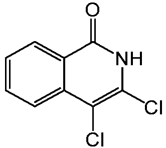 [31728-18-4 | 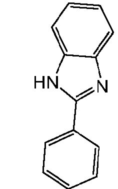 | 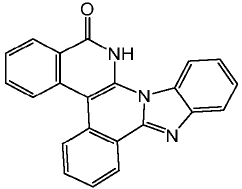 | 32% |
| 2g | 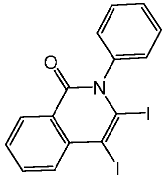 [848061-06-3 ] | 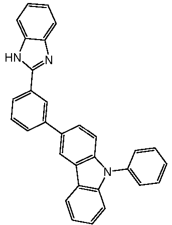 [2300968-77-6] | 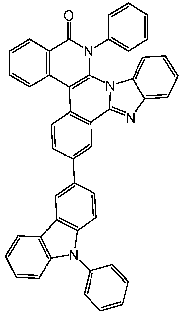 | 37% |
| 3g | 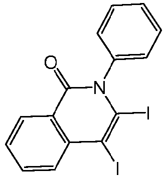 [848061-06-3 ] | 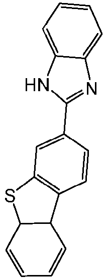 [2252356-12-8 ] | 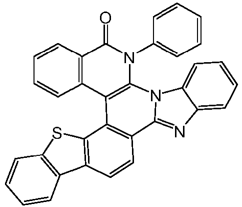 | 41% |
| 4g | 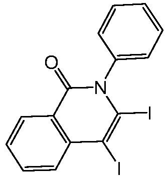 [848061-06-3 ] | 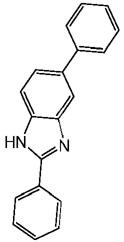 [6528-80-9 ] | 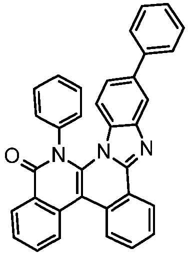 | 55% |

### h) 3-(2-Bromophenyl)-1-oxo-2H-isochinolin-4-carbaldehyd

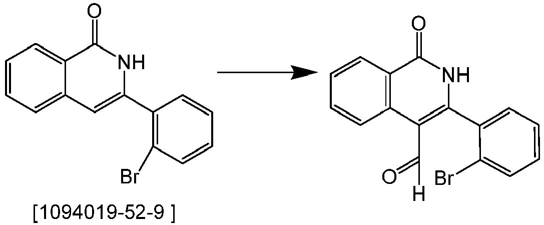

9,7 g 63,6 mmol) Phosphoroxychlorid wird zu 40 ml Dimethylformamid gegeben, und in einem Eisbad gekühlt. Der Kolben wird 4 Stunden gerührt. Die resultierende Mischung wird 4 h bei der gleichen Temperatur gerührt. Eine eiskalte Lösung von 3,2 g (12,7 mmol) 3-(2-Bromophenyl)-2H-isoquinolin-1-on in DMF (10 ml) wird tropfenweise zu der Mischung gegeben und nach Beendigung der Zugabe langsam auf 60°C erwärmt und 20 Stunden gerührt. Das Reaktionsgemisch wird in einem Eisbad gekühlt, vorsichtig mit 50 ml eiskaltem Wasser versetzt und mit 35% iger NaOH-Lösung basisch gemacht. Der Feststoff wird abfiltriert. Das Filtrat wird mit Ethylacetat extrahiert. Die vereinigten Ethylacetatextrakte werden mit Wasser und Salzlösung gewaschen, über wasserfreiem Natriumsulfat getrocknet und dann eingeengt. Der Rückstand wird aus Methanol um kristallisiert.

Ausbeute: 2,8 g (8,5 mmol), 80 % d. Th.

### i) Cyclisierung:

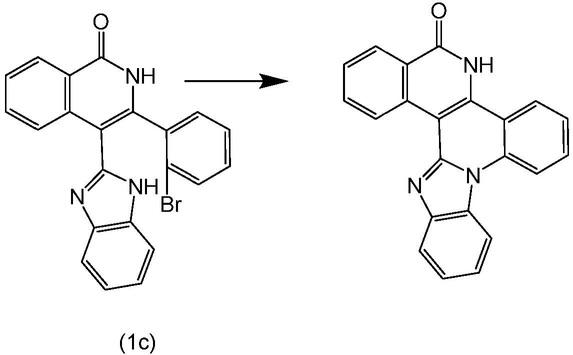

Unter Schutzgas wird 16,6 g (40 mmol, 1,0 eq.) Verbindung 1c, 16,4 g (120 mmol, 3,0 eq.) K₂CO₃, 1 g (4 mmol) Pd(OAc)₂ und schließlich 3,8 g (8 mmol) Xphos in 400 ml DMF zugegeben. Die Mischung wird 80 h bei 160°C erhitzt und kommt dann auf Raumtemperatur. Anschließend wird mit 20 ml Wasser gequencht und mit 20 ml Ethylacetat verdünnt. Die organische Phase wird getrennt und im Vakuum eingeengt. Das Rohprodukt wird dann durch Flash-Chromatographie an Kieselgel getrennt (3-10% Ethylacetat / Petrolether).

Ausbeute: 8 g (22 mmol), 75 % d. Th.

Analog können folgende Verbindungen erhalten werden:

| **Bsp.** | **Edukt 1** | **Produkt** | **Ausbeute** |
|---|---|---|---|
| 1i | 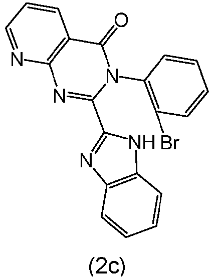 | 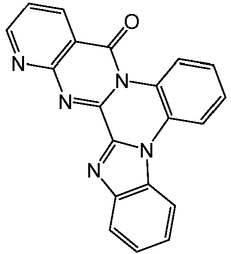 | 65% |

### j) Suzuki-Reaktion

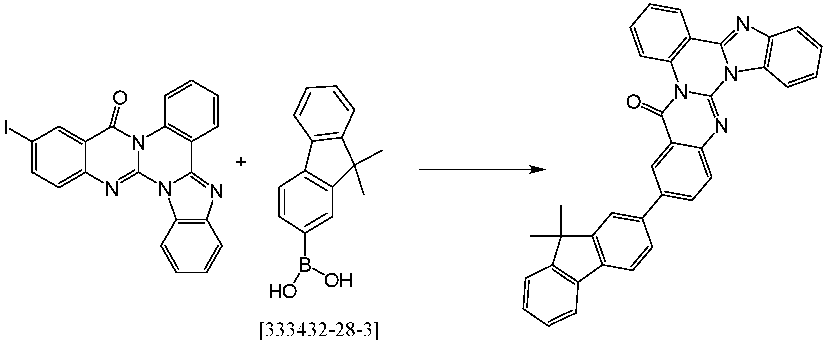

53 g (155 mmol) Verbindung f, 41 g (172 mmol) 9,9-Dimethyl-9H-fluoren-2-boronsäure und 36 g (340 mmol) Natriumcarbonat werden in 1000 mL Ethylenglycoldiaminether und 280 mL Wasser suspendiert. Zu dieser Suspension werden 1,8 g (1,5 mmol) Tetrakies(triphenylphosphin)-palladium(0) zugegeben, und die Reaktionsmischung wird 16 h unter Rückfluss erhitzt. Nach Erkalten wird die organische Phase abgetrennt, über Kieselgel filtriert und anschließend zur Trockene eingeengt. Das Produkt wird via Säulenchromatographie an Kieselgel mit Toluol/Heptan (1:2) gereinigt und abschließend im Hochvakuum (p = 5 x 10⁻⁷ mbar) sublimiert (Reinheit 99,9%). Die Ausbeute beträgt 42 g (80 mmol), entsprechend 70 % der Theorie.

Analog dazu werden die folgenden Verbindungen hergestellt:

| **Bsp.** | **Edukt 1** | **Edukt 2** | **Produkt** | **Ausbeute** |
|---|---|---|---|---|
| **1j** | 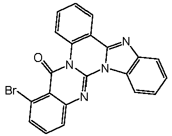 | 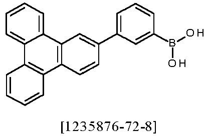 | 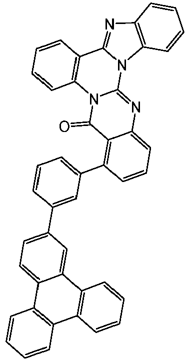 | 73% |
| **2j** | 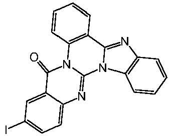 | 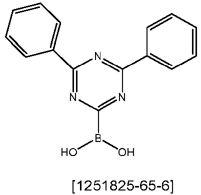 | 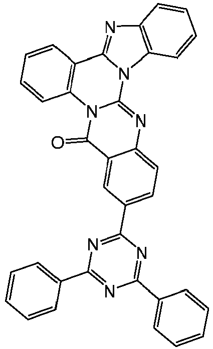 | 84% |
| **3j** | 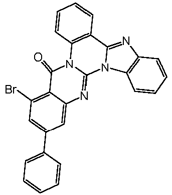 | 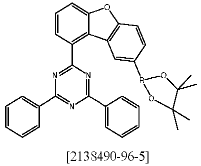 | 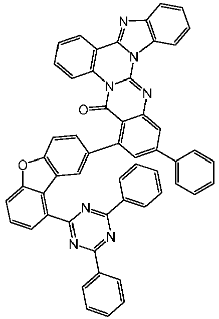 | 72% |
| **4j** | 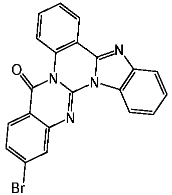 | 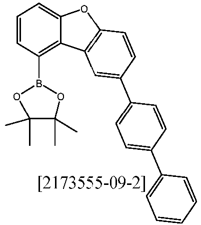 | 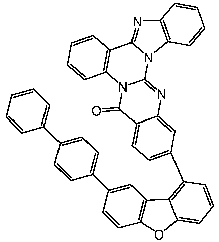 | 79% |
| **5j** | 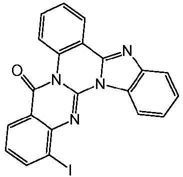 | 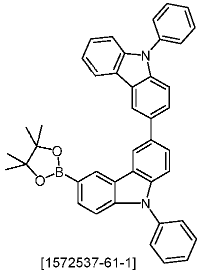 | 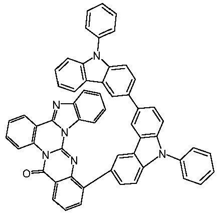 | 71% |
| **6j** | 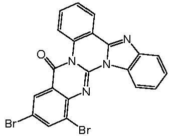 | 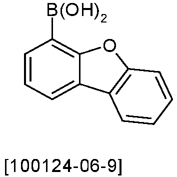 | 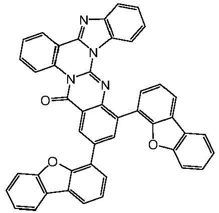 | 69% |
| **7j** | 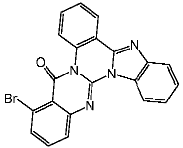 | 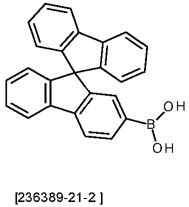 | 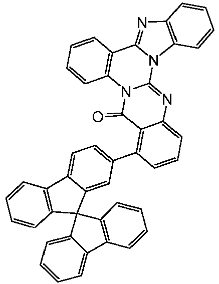 | 71% |
| **8j** | 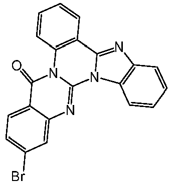 | 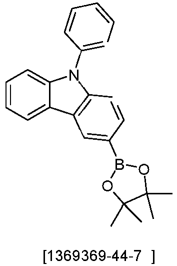 | 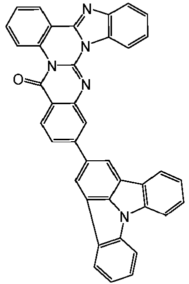 | 80% |

### Herstellung der Elektrolumineszenzvorrichtungen

In den folgenden Beispielen E1 bis E16 (siehe Tabelle 1) wird der Einsatz der erfindungsgemäßen Materialien in OLEDs vorgestellt.

**Vorbehandlung für die Beispiele E1 bis E16:** Glasplättchen, die mit strukturiertem ITO (Indium Zinn Oxid) der Dicke 50 nm beschichtet sind, werden vor der Beschichtung mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 2 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie EG1: IC2:TEG1 (49%:44%:7%) bedeutet hierbei, dass das Material EG1 in einem Volumenanteil von 49%, IC2 in einem Anteil von 44% und TEG1 in einem Anteil von 7% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (SE, gemessen in cd/A) und die externe Quanteneffizienz (EQE, gemessen in %) in Abhängigkeit der Leuchtdichte, berechnet aus Strom-Spannungs-Leuchtdichte-Kennlinien unter Annahme einer lambertschen Abstrahlcharakteristik bestimmt. Die Angabe U1000 bezeichnet die Spannung, die für eine Leuchtdichte von 1000 cd/m² benötigt wird. SE1000 und EQE1000 bezeichnen die Stromeffizienz bzw. die externe Quanteneffizienz, die bei 1000cd/m² erreicht werden. Die Elektrolumineszenzspektren werden bei einer Leuchtdichte von 1000 cd/m² bestimmt und daraus die CIE 1931 x und y Farbkoordinaten berechnet. Die so erhaltenen Ergebnisse sind Tabelle 3 zu entnehmen.

### Verwendung der erfindungsgemäßen Materialien in OLEDs

Die erfindungsgemäße Verbindung EG1 wird in Beispiel E1 als Matrixmaterial in der Emissionsschicht von rot phosphoreszierenden OLEDs eingesetzt. Verbindungen EG2 bis EG13 werden in den Beispielen E2 bis E13 als Matrixmaterial in der Emissionsschicht von grün phosphoreszierenden OLEDs eingesetzt. Die erfindungsgemäßen Verbindungen EG3, EG7 und EG9 werden in den Beispielen E14 bis E16 als Elektrontransporter in der Elektronentransportschicht (ETL) von grün phosphoreszierenden OLEDs eingesetzt.

Dabei werden in allen Fällen gute Leistungsdaten der OLEDs erzielt (Tabelle 3).

**Tabelle 1: Aufbau der Elektrolumineszenzvorrichtungen**

| Bsp | HIL Dicke | HTL Dicke | EBL Dicke | EML Dicke | HBL Dicke | ETL Dicke | EIL Dicke |
|---|---|---|---|---|---|---|---|
| E1 | HATCN 5nm | SpMA1 230nm | SpMA2 20nm | EG1:IC2:TER5 (57%:40%:7%) 35nm | ST2 5nm | ST2:LiQ (50%:50%) 30nm | LiQ 1nm |
| E2 | HATCN 5nm | SpMA1 230nm | SpMA2 20nm | EG2:IC2:TEG1 (49%:44%:7%) 40nm | ST2 5nm | ST2:LiQ (50%:50%) 30nm | LiQ 1nm |
| E3 | HATCN 5nm | SpMA1 230nm | SpMA2 20nm | EG3:IC2:TEG1 (49%:44%:7%) 40nm | ST2 5nm | ST2:LiQ (50%:50%) 30nm | LiQ 1nm |
| E4 | HATCN 5nm | SpMA1 230nm | SpMA2 20nm | EG4:IC2:TEG1 (49%:44%:7%) 40nm | ST2 5nm | ST2:LiQ (50%:50%) 30nm | LiQ 1nm |
| E5 | HATCN 5nm | SpMA1 230nm | SpMA2 20nm | EG5:IC3:TEG1 (45%:45%:10%) 30nm | ST2 10nm | ST2:LiQ (50%:50%) 30nm | LiQ 1nm |
| E6 | HATCN 5nm | SpMA1 230nm | SpMA2 20nm | EG6: IC3:TEG1 (44%:44%:12%) 30nm | ST2 10nm | ST2:LiQ (50%:50%) 30nm | LiQ 1nm |
| E7 | HATCN 5nm | SpMA1 230nm | SpMA2 20nm | EG7: IC3:TEG1 (44%:44%:12%) 30nm | ST2 10nm | ST2:LiQ (50%:50%) 30nm | LiQ 1nm |
| E8 | HATCN 5nm | SpMA1 230nm | SpMA2 20nm | EG8: IC3:TEG1 (46%:47%:7%) 30nm | ST2 10nm | ST2:LiQ (50%:50%) 30nm | LiQ 1nm |
| E9 | HATCN 5nm | SpMA1 230nm | SpMA2 20nm | EG9: IC3:TEG1 (46%:47%:7%) 30nm | ST2 10nm | ST2:LiQ (50%:50%) 30nm | LiQ 1nm |
| E10 | HATCN 5nm | SpMA1 230nm | SpMA2 20nm | EG10: IC3:TEG1 (46%:47%:7%) 30nm | ST2 10nm | ST2:LiQ (50%:50%) 30nm | LiQ 1nm |
| E11 | HATCN 5nm | SpMA1 230nm | SpMA2 20nm | EG11: IC3:TEG1 (46%:47%:7%) 30nm | ST2 10nm | ST2:LiQ (50%:50%) 30nm | LiQ 1nm |
| E12 | HATCN 5nm | SpMA1 230nm | SpMA2 20nm | EG12: IC3:TEG1 (46%:47%:7%) 30nm | ST2 10nm | ST2:LiQ (50%:50%) 30nm | LiQ 1nm |
| E13 | HATCN 5nm | SpMA1 230nm | SpMA2 20nm | EG13: IC1:TEG1 (46%:47%:7%) 30nm | ST2 10nm | ST2:LiQ (50%:50%) 30nm | LiQ 1nm |
| E14 | HATCN 5nm | SpMA1 70nm | SpMA2 15nm | IC1:TEG1 (90%:10%) 25nm | --- | EG3 45nm | LiQ 3nm |
| E15 | HATCN 5nm | SpMA1 70nm | SpMA2 15nm | IC1:TEG1 (90%:10%) 25nm | --- | EG7 45nm | LiQ 3nm |
| E16 | HATCN 5nm | SpMA1 70nm | SpMA2 15nm | IC1:TEG1 (90%:10%) 25nm | --- | EG9 45nm | LiQ 3nm |

**Tabelle 2: Strukturformeln der Materialien für die Elektrolumineszenzvorrichtungen**

| | |
|---|---|
| 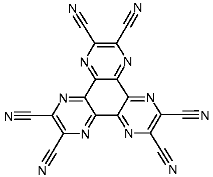 | 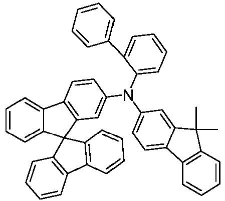 |
| HATCN | SpMA1 |
| 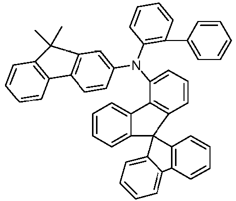 | 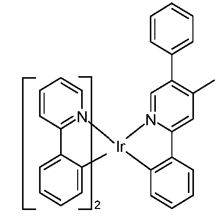 |
| SpMA3 | TEG1 |
| 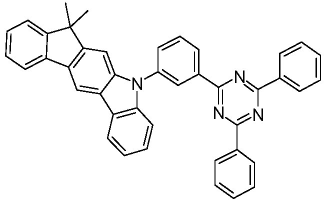 | 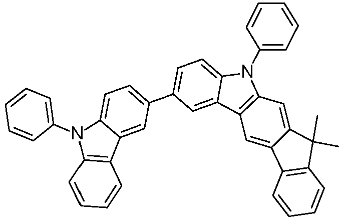 |
| IC1 | IC2 |
| 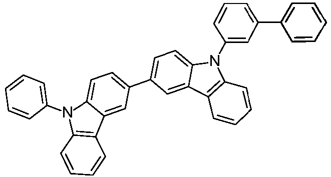 | 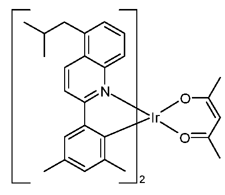 |
| IC3 | TER5 |
| 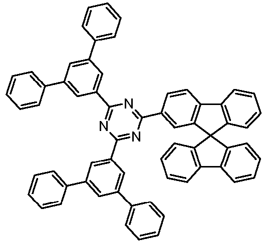 | 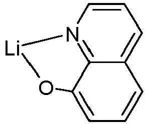 |
| ST2 | LiQ |
| 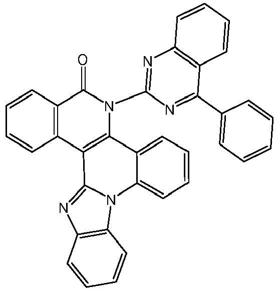 | 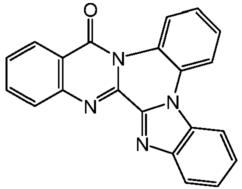 |
| EG1 (4a) | **EG2** (e) |
| 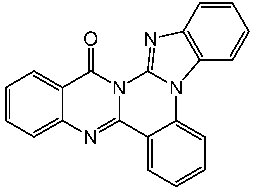 | 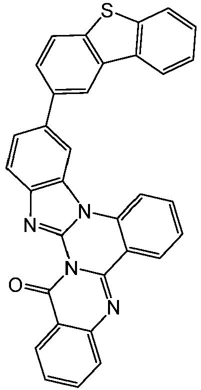 |
| EG3 (1e) | EG4 (2e) |
| 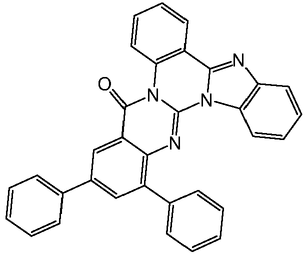 | 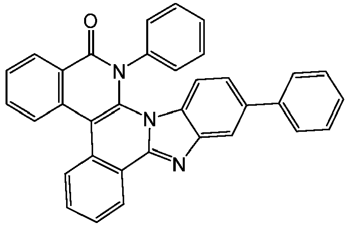 |
| EG5 (1f) | EG6 (4g) |
| 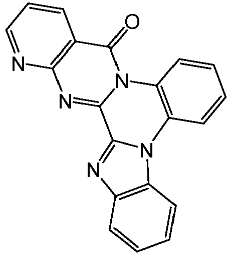 | 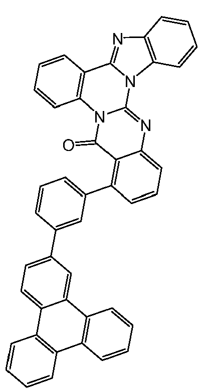 |
| EG7 (1i) | EG8 (1j) |
| 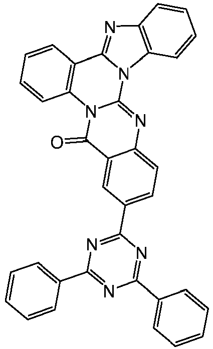 | 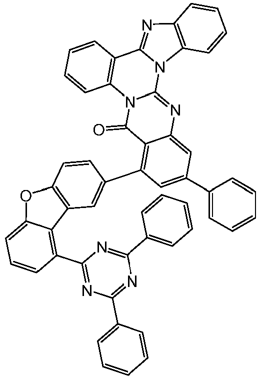 |
| EG9 (2j) | EG10(3j) |
| 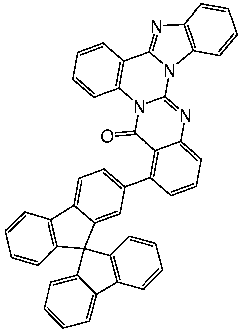 | 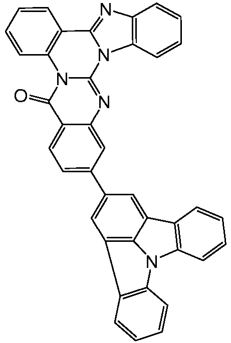 |
| EG11 (7j) | EG12 (8j) |
| 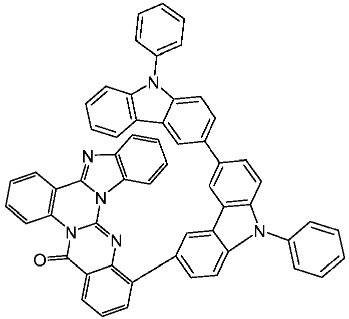 | |
| EG13 (5j) | |

Die in Tabelle 2 in den Klammern zu den jeweiligen Verbindung dargelegte Angabe bezieht sich auf das Synthesebeispiel.

**Tabelle 3: Leistungsdaten der Elektrolumineszenzvorrichtungen**

| Bsp. | U1000 (V) | SE1000 (cd/A) | EQE 1000 (%) | CIE x/y bei 1000 cd/m² |
|---|---|---|---|---|
| E1 | 3.7 | 23 | 21 | 0.66/0.34 |
| E2 | 4.1 | 67 | 16 | 0.33/0.62 |
| E3 | 3.9 | 65 | 17 | 0.35/0.61 |
| E4 | 3.7 | 70 | 16.7 | 0.35/0.62 |
| E5 | 4.2 | 74 | 16.8 | 0.33/0.63 |
| E6 | 4.4 | 63 | 17.0 | 0.33/0.62 |
| E7 | 3.8 | 70 | 16.3 | 0.32/0.64 |
| E8 | 3.6 | 79 | 17.3 | 0.32/0.63 |
| E9 | 3.7 | 73 | 17.8 | 0.33/0.62 |
| E10 | 3.3 | 75 | 18.8 | 0.33/0.63 |
| E11 | 3.2 | 76 | 19.7 | 0.33/0.63 |
| E12 | 3.2 | 73 | 19.9 | 0.33/0.63 |
| E13 | 3.1 | 67 | 19.5 | 0.33/0.63 |
| E14 | 3.4 | 64 | 18.8 | 0.33/0.62 |
| E15 | 3.5 | 64 | 17.9 | 0.33/0.63 |
| E16 | 3.3 | 66 | 18.2 | 0.33/0.62 |

## Patentansprüche

1. Verbindung umfassend mindestens eine Struktur mit mindestens drei miteinander kondensierten Strukturelementen gemäß den Formeln (A), (B) und (C), vorzugsweise Verbindung bestehend aus drei miteinander kondensierten Strukturelementen gemäß den Formeln (A), (B) und (C), 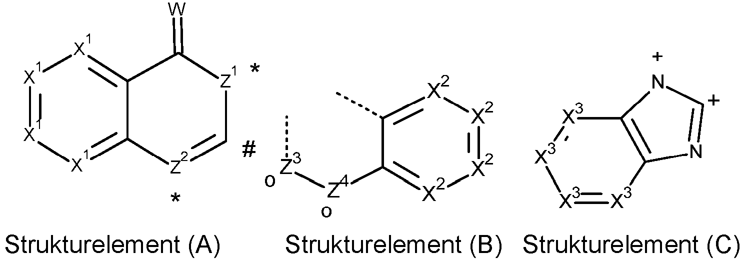
wobei das Strukturelement (A) mit Strukturelement (B) kondensiert ist und das Strukturelement (B) mit Strukturelement (C) kondensiert ist; wobei das Strukturelement (B) über die gestrichelt dargestellten Bindungen an das Strukturelement (A) bindet, wobei eine Bindung über die mit # markierte Bindungsstelle erfolgt und eine Bindung über eine mit * markierte Bindungsstelle erfolgt; wobei das Strukturelement (B) mit dem Strukturelement (C) über die mit o und + markierten Atome miteinander kondensiert ist und die jeweils markierten Atome von den Strukturelementen (B) und (C) geteilt sind, und
für die verwendeten Symbole und Indizes gilt:
W steht für O oder S, vorzugsweise für O;
Z¹ steht für NAr oder N, für den Fall, dass das Strukturelement (A) mit dem Strukturelement (B) über Z¹ kondensiert ist;
Z² steht für X oder C, für den Fall, dass das Strukturelement (A) mit dem Strukturelement (B) über Z² kondensiert ist;
Z³, Z⁴ steht für N oder C, wobei einer der Reste Z³, Z⁴ für N und einer der Reste Z³, Z⁴ für C steht;
X steht für N oder CR, vorzugsweise für N;
X¹ steht bei jedem Auftreten gleich oder verschieden für N oder CR¹, vorzugsweise für CR¹, mit der Maßgabe, dass nicht mehr als zwei der Gruppen X¹ in einem Cyclus für N stehen;
X² steht bei jedem Auftreten gleich oder verschieden für N oder CR², mit der Maßgabe, dass nicht mehr als zwei der Gruppen X² in einem Cyclus für N stehen;
X³ steht bei jedem Auftreten gleich oder verschieden für N oder CR³, vorzugsweise für CR³, mit der Maßgabe, dass nicht mehr als zwei der Gruppen X³ in einem Cyclus für N stehen;
Ar ist bei jedem Auftreten gleich oder verschieden ein aromatisches oder heteroaromatisches Ringsystem mit 5 bis 40 aromatischen Ringatomen, welches Reste R aufweist, vorzugsweise eine Arylgruppe mit 6 bis 30 aromatischen Ringatomen oder eine Heteroarylgruppe mit 5 bis 14 aromatischen Ringatomen, welche Reste R aufweist;
R ist bei jedem Auftreten gleich oder verschieden H, D, F, Cl, Br, I, R⁴C=C(R⁴)₂, N(R⁴)₂, N(Ar')₂, CN, NO₂, OR⁴, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, eine geradkettige Alkyl-, Alkoxy- oder Thioalkylgruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkylgruppe mit 3 bis 20 C-Atomen, wobei die Alkyl-, Alkoxy- oder Thioalkyl-, Alkenyl- oder Alkinylgruppe jeweils Reste R⁴ aufweist und wobei eine oder mehrere nicht benachbarte CH₂-Gruppen durch R⁴C=CR⁴, C=C, Si(R⁴)₂, C=O, C=S, C=Se, C=NR⁴, -C(=O)O-, -C(=O)NR⁴-, NR⁴, P(=O)( R⁴), -O-, -S-, SO oder SO₂ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils Reste R⁴ aufweist, oder eine Aryloxy- oder Heteroaryloxygruppe mit 5 bis 40 aromatischen Ringatomen, die jeweils Reste R⁴ aufweist, oder eine Diarylamino-, Arylheteroarylamino-, Diheteroarylaminogruppe mit 5 bis 60 aromatischen Ringatomen im jeweiligen aromatischen oder heteroaromatischen Rest, wobei die Diarylamino-, Arylheteroarylamino-, Diheteroarylaminogruppe Reste R⁴ aufweist; dabei können zwei Reste R oder ein Rest R mit einem weiteren Rest, vorzugsweise einer Gruppe R¹, R², R³ ein aliphatisches, heteroaliphatisches, aromatisches oder heteroaromatisches Ringsystem, vorzugsweise ein aliphatisches, heteroaliphatisches oder heteroaromatisches Ringsystem bilden, besonders bevorzugt bildet der Rest R kein solches Ringsystem;
R¹ ist bei jedem Auftreten gleich oder verschieden H, D, F, Cl, Br, I, R⁴C=C(R⁴)₂, N(R⁴)₂, N(Ar')₂, CN, NO₂, OR⁴, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, eine geradkettige Alkyl-, Alkoxy- oder Thioalkylgruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkylgruppe mit 3 bis 20 C-Atomen, wobei die Alkyl-, Alkoxy- oder Thioalkyl-, Alkenyl- oder Alkinylgruppe jeweils Reste R⁴ aufweist und wobei eine oder mehrere nicht benachbarte CH₂-Gruppen durch R⁴C=CR⁴, C=C, Si(R⁴)₂, C=O, C=S, C=Se, C=NR⁴, -C(=O)O-, -C(=O)NR⁴-, NR⁴, P(=O)( R⁴), -O-, -S-, SO oder SO₂ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils Reste R⁴ aufweist, oder eine Aryloxy- oder Heteroaryloxygruppe mit 5 bis 40 aromatischen Ringatomen, die jeweils Reste R⁴ aufweist, oder eine Diarylamino-, Arylheteroarylamino-, Diheteroarylaminogruppe mit 5 bis 60 aromatischen Ringatomen im jeweiligen aromatischen oder heteroaromatischen Rest, wobei die Diarylamino-, Arylheteroarylamino-, Diheteroarylaminogruppe Reste R⁴ aufweist; dabei können zwei Reste R¹ oder ein Rest R¹ mit einem weiteren Rest, vorzugsweise einer Gruppe R, R², R³ auch miteinander ein aromatisches, heteroaromatisches, aliphatisches oder heteroaliphatisches Ringsystem, vorzugsweise ein aliphatisches, heteroaliphatisches oder heteroaromatisches Ringsystem bilden, besonders bevorzugt bilden die Reste R¹ kein solches Ringsystem;
R² ist bei jedem Auftreten gleich oder verschieden H, D, F, Cl, Br, I, R⁴C=C(R⁴)₂, N(R⁴)₂, N(Ar')₂^{,} CN, NO₂, OR⁴, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, eine geradkettige Alkyl-, Alkoxy- oder Thioalkylgruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkylgruppe mit 3 bis 20 C- Atomen, wobei die Alkyl-, Alkoxy- oder Thioalkyl-, Alkenyl- oder Alkinylgruppe jeweils Reste R⁴ aufweist und wobei eine oder mehrere nicht benachbarte CH₂-Gruppen durch R⁴C=CR⁴, C=C, Si(R⁴)₂, C=O, C=S, C=Se, C=NR⁴, -C(=O)O-, -C(=O)NR⁴-, NR⁴, P(=O)( R⁴), -O-, -S-, SO oder SO₂ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils Reste R⁴ aufweist, oder eine Aryloxy- oder Heteroaryloxygruppe mit 5 bis 40 aromatischen Ringatomen, die jeweils Reste R⁴ aufweist, oder eine Diarylamino-, Arylheteroarylamino-, Diheteroarylaminogruppe mit 5 bis 60 aromatischen Ringatomen im jeweiligen aromatischen oder heteroaromatischen Rest, wobei die Diarylamino-, Arylheteroarylamino-, Diheteroarylaminogruppe Reste R⁴ aufweist; dabei können zwei Reste R² oder ein Rest R² mit einem weiteren Rest, vorzugsweise einer Gruppe R, R¹, R³ auch miteinander ein aromatisches, heteroaromatisches, aliphatisches oder heteroaliphatisches Ringsystem, vorzugsweise ein aliphatisches, heteroaliphatisches oder heteroaromatisches Ringsystem bilden, besonders bevorzugt bilden die Reste R² kein solches Ringsystem;
R³ ist bei jedem Auftreten gleich oder verschieden H, D, F, Cl, Br, I, R⁴C=C(R⁴)₂, N(R⁴)₂, N(Ar')₂^{,} CN, NO₂, OR⁴, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, eine geradkettige Alkyl-, Alkoxy- oder Thioalkylgruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkylgruppe mit 3 bis 20 C-Atomen, wobei die Alkyl-, Alkoxy- oder Thioalkyl-, Alkenyl- oder Alkinylgruppe jeweils Reste R⁴ aufweist und wobei eine oder mehrere nicht benachbarte CH₂-Gruppen durch R⁴C=CR⁴, C=C, Si(R⁴)₂, C=O, C=S, C=Se, C=NR⁴, -C(=O)O-, -C(=O)NR⁴-, NR⁴, P(=O)( R⁴), -O-, -S-, SO oder SO₂ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils Reste R⁴ aufweist, oder eine Aryloxy- oder Heteroaryloxygruppe mit 5 bis 40 aromatischen Ringatomen, die jeweils Reste R⁴ aufweist, oder eine Diarylamino-, Arylheteroarylamino-, Diheteroarylaminogruppe mit 5 bis 60 aromatischen Ringatomen im jeweiligen aromatischen oder heteroaromatischen Rest, wobei die Diarylamino-, Arylheteroarylamino-, Diheteroarylaminogruppe Reste R⁴ aufweist; dabei können zwei Reste R³ oder ein Rest R³ mit einem weiteren Rest, vorzugsweise einer Gruppe R, R¹, R² auch miteinander ein aromatisches, heteroaromatisches, aliphatisches oder heteroaliphatisches Ringsystem, vorzugsweise ein aliphatisches, heteroaliphatisches oder heteroaromatisches Ringsystem bilden, besonders bevorzugt bilden die Reste R³ kein solches Ringsystem;
Ar' ist bei jedem Auftreten gleich oder verschieden ein aromatisches oder heteroaromatisches Ringsystem mit 5 bis 40 aromatischen Ringatomen, welches Reste R⁴ aufweist, vorzugsweise eine Arylgruppe mit 6 bis 30 aromatischen Ringatomen oder eine Heteroarylgruppe mit 5 bis 14 aromatischen Ringatomen, welche Reste R⁴ aufweist;
R⁴ ist bei jedem Auftreten gleich oder verschieden H, D, F, Cl, Br, I, R⁵C=C(R⁵)₂, N(R⁵)₂, CN, NO₂, OR⁵, SR⁵, Si(R⁵)₃, B(OR⁵)₂, C(=O)R⁵, P(=O)(R⁵)₂, S(=O)R⁵, S(=O)₂R⁵, OSO₂R⁵, eine gerad-kettige Alkyl-, Alkoxy- oder Thioalkylgruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkylgruppe mit 3 bis 20 C-Atomen, wobei die Alkyl-, Alkoxy- oder Thioalkyl-, Alkenyl- oder Alkinylgruppe jeweils Reste R⁵ aufweist, wobei eine oder mehrere nicht benachbarte CH₂-Gruppen durch R⁵C=CR⁵, C=C, Si(R⁵)₂, C=O, C=S, C=Se, C=NR⁵, -C(=O)O-, -C(=O)NR⁵-, NR⁵, P(=O)(R⁵), -O-, -S-, SO oder SO₂ ersetzt sein können, oder ein aromatisches oder heteroaromatisches Ringsystem mit 5 bis 40 aromatischen Ringatomen, das jeweils Reste R⁵ aufweist, oder eine Aryloxy- oder Heteroaryloxygruppe mit 5 bis 40 aromatischen Ringatomen, die jeweils Reste R⁵ aufweist, oder eine Diarylamino-, Arylheteroarylamino-, Diheteroarylaminogruppe mit 5 bis 60 aromatischen Ringatomen im jeweiligen aromatischen oder heteroaromatischen Rest, wobei die Diarylamino-, Arylheteroarylamino-, Diheteroarylaminogruppe Reste R⁵ aufweist; dabei können zwei oder mehrere Reste R⁴ miteinander ein aromatisches, heteroaromatisches, aliphatisches oder heteroaliphatisches Ringsystem bilden, vorzugsweise ein aliphatisches Ringsystem, besonders bevorzugt bilden die Reste R⁴ kein solches Ringsystem;
R⁵ ist bei jedem Auftreten gleich oder verschieden H, D, F oder ein aliphatischer, aromatischer oder heteroaromatischer organischer Rest, insbesondere ein Kohlenwasserstoffrest, mit 1 bis 20 C-Atomen, in dem auch ein oder mehrere H-Atome durch F ersetzt sein können, dabei können zwei oder mehrere Reste R⁵ miteinander ein Ringsystem bilden;
wobei mindestens einer der Reste R, R¹, R², R³ ausgewählt ist aus der Gruppe bestehend aus einem heteroaromatischen Ringsystem mit 6 bis 60 aromatischen Ringatomen, das Reste R⁴ aufweist, einem aromatischen Ringsystem mit 10 bis 60 aromatischen Ringatomen, das Reste R⁴ aufweist, einer Aryloxygruppe mit 10 bis 60 aromatischen Ringatomen oder Heteroaryloxygruppe mit 6 bis 40 aromatischen Ringatomen, die jeweils Reste R⁴ aufweist, einer Diarylaminogruppe mit 6 bis 60 aromatischen Ringatomen im jeweiligen aromatischen Rest, einer Arylheteroarylaminogruppe mit 6 bis 60 aromatischen Ringatomen im jeweiligen aromatischen oder heteroaromatischen Rest und einer Diheteroarylaminogruppe mit 6 bis 60 aromatischen Ringatomen mit 6 bis 60 aromatischen Ringatomen im jeweiligen heteroaromatischen Rest, wobei die Diarylamino-, Arylheteroarylamino-, Diheteroarylaminogruppe Reste R⁴ aufweist.

2. Verbindung nach Anspruch 1, umfassend mindestens eine Struktur der Formeln (I-1) bis (I-7), vorzugsweise ausgewählt aus den Verbindungen der Formeln (I-1) bis (I-7), 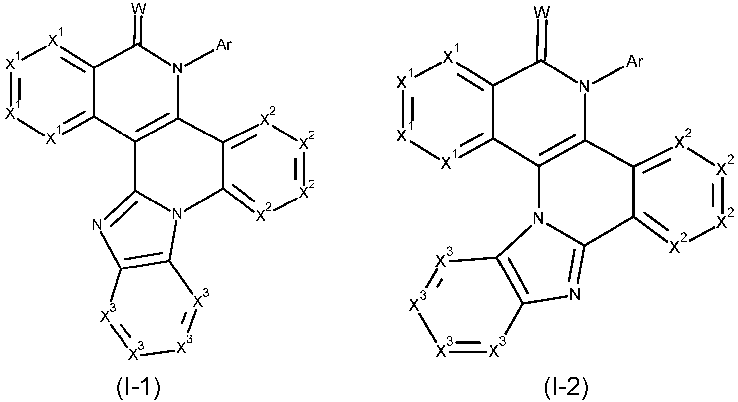 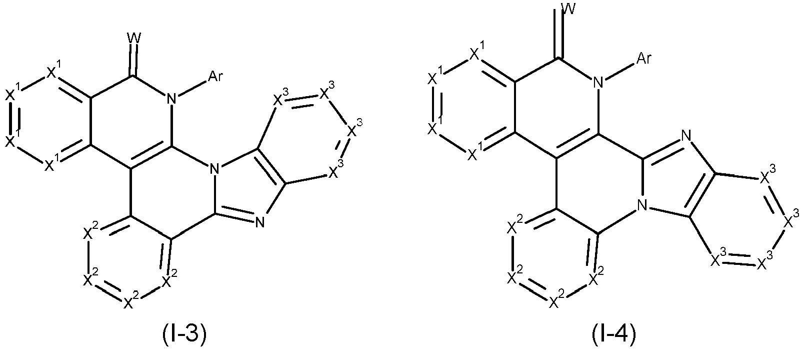 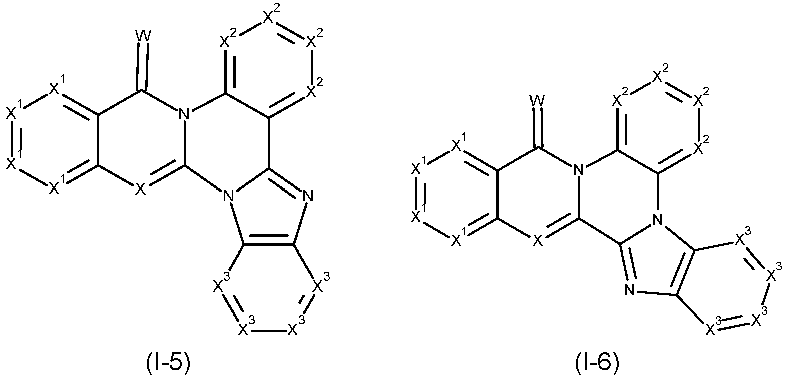 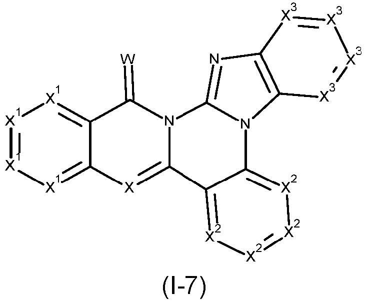 wobei Symbole W, X, X¹, X², X³ und Ar, die in Anspruch 1 genannten Bedeutungen aufweisen.

3. Verbindung nach Anspruch 1 oder 2, umfassend mindestens eine Struktur der Formeln (II-1) bis (II-30), vorzugsweise ausgewählt aus den Verbindungen der Formeln (II-1) bis (II-30), 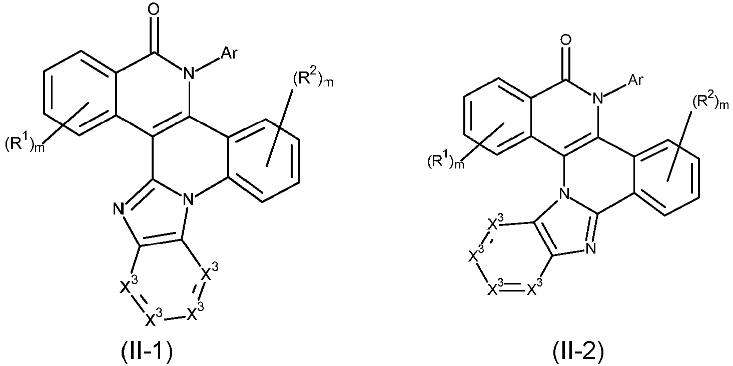 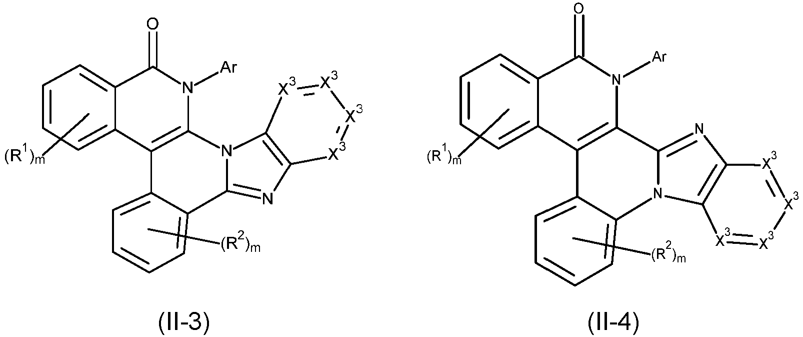 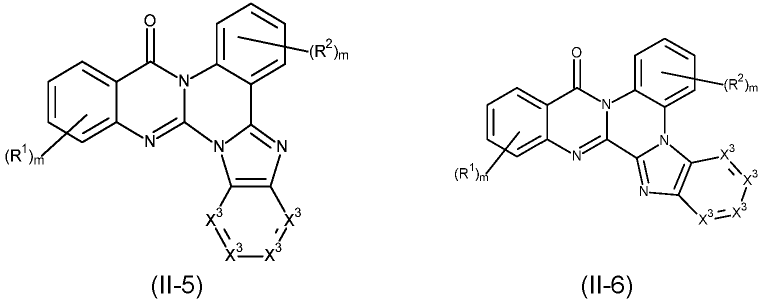 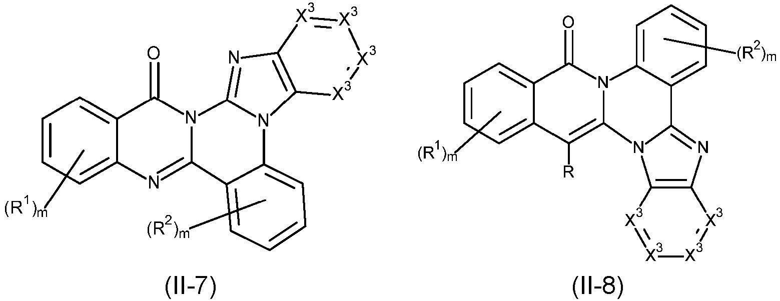 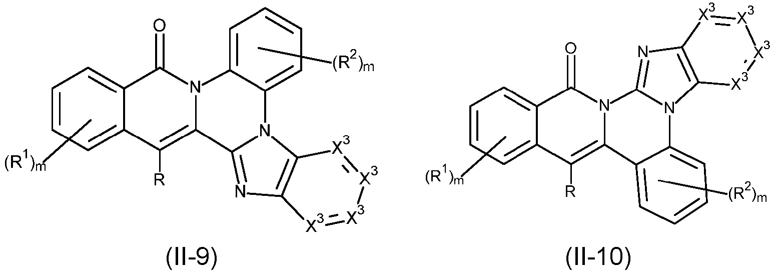 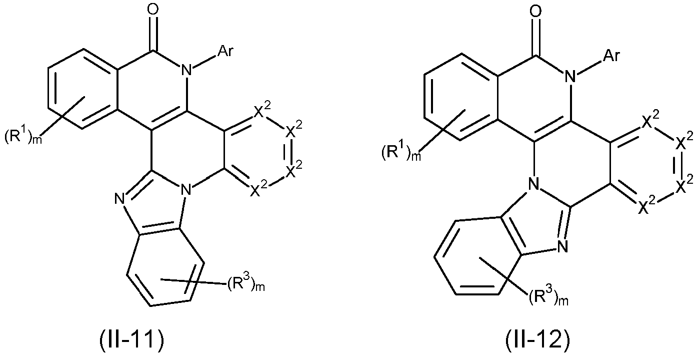 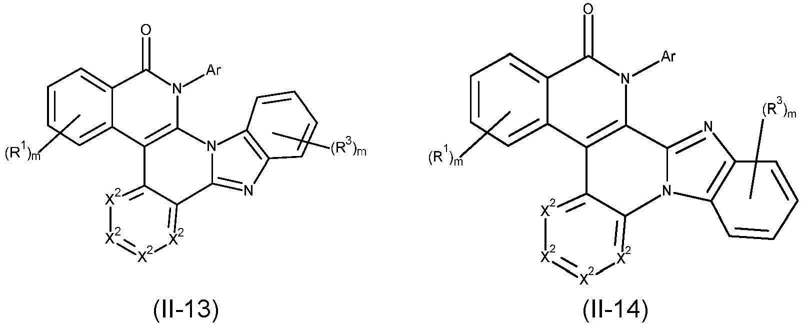 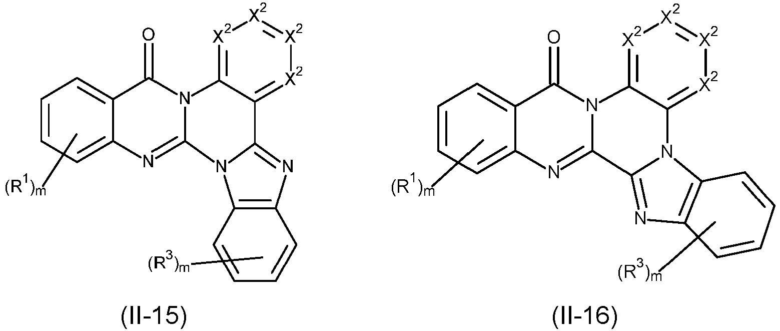 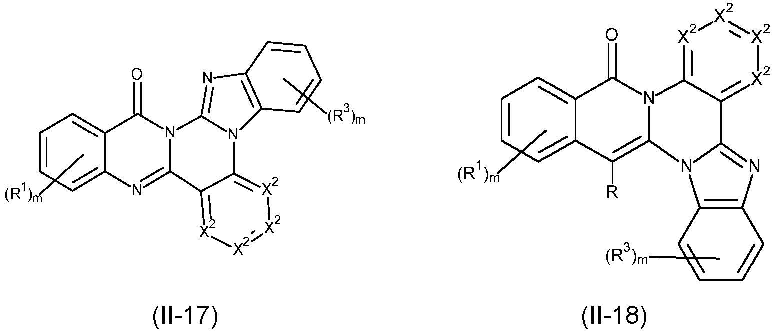 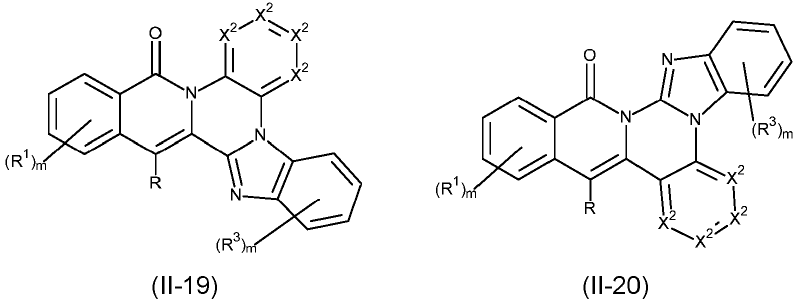 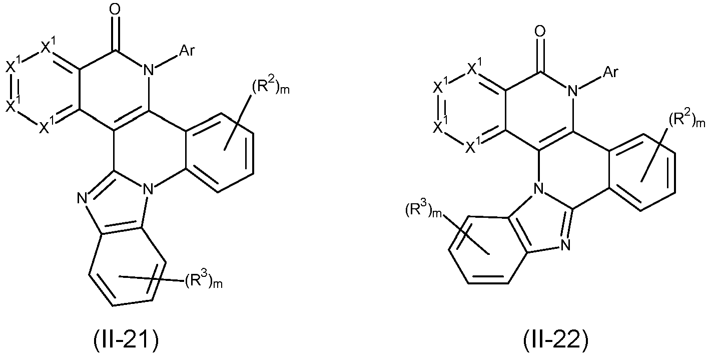 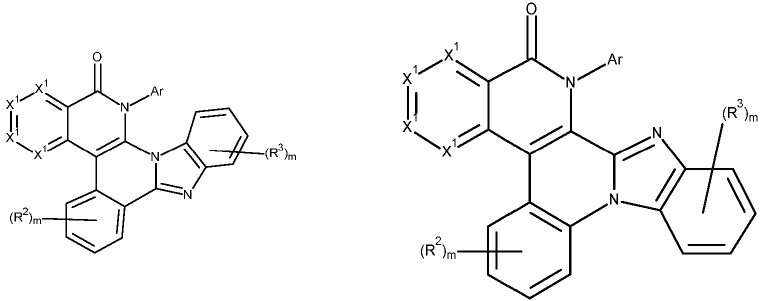 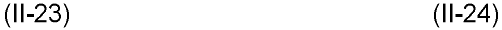 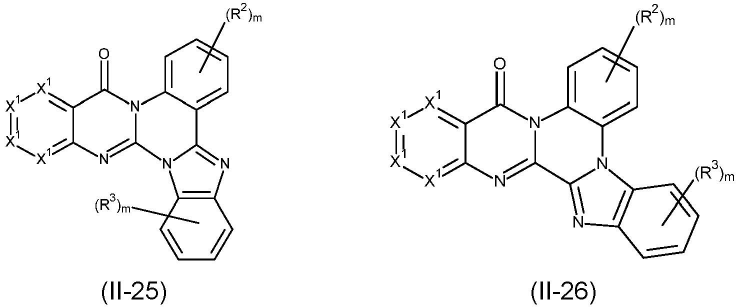 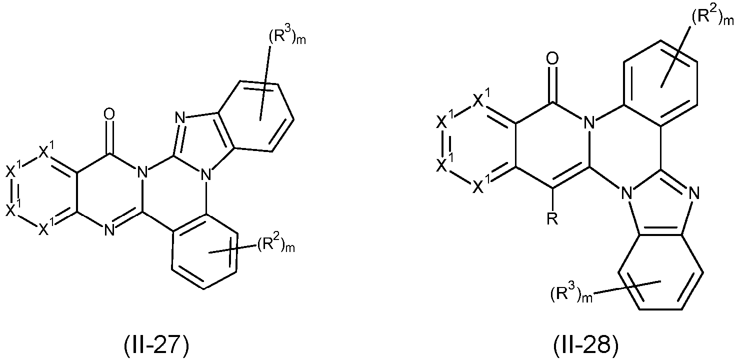 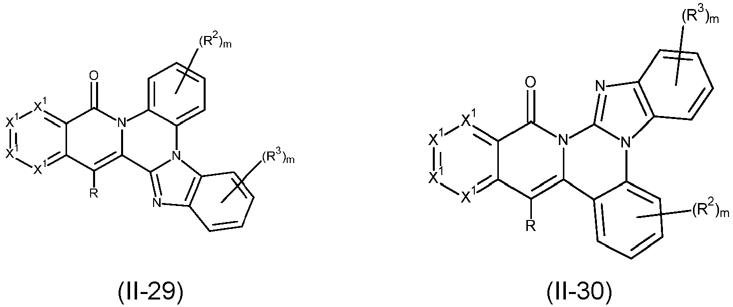 wobei die Symbole X¹, X², X³, R, R¹, R², R³ und Ar, die in Anspruch 1 genannten Bedeutungen aufweisen und der Index m 0, 1, 2, 3 oder 4, vorzugsweise 0, 1 oder 2 ist, wobei Strukturen/Verbindungen der Formeln (II-1) bis (II-7), (II-11) bis (II-17) und/oder (II-21) bis (II-27) bevorzugt sind.

4. Verbindung nach einem oder mehreren der Ansprüche 1 bis 3, umfassend mindestens eine Struktur der Formeln (III-1) bis (III-10), vorzugsweise ausgewählt aus den Verbindungen der Formeln (III-1) bis (III-10), 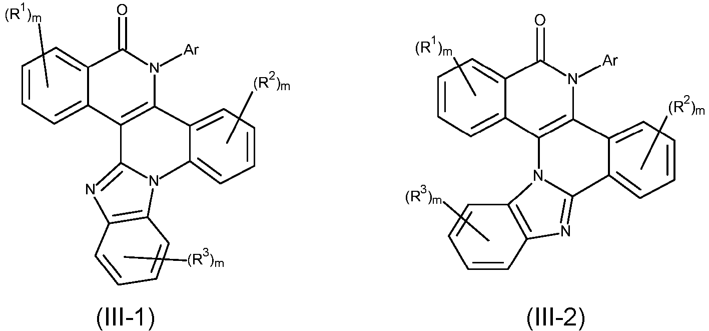 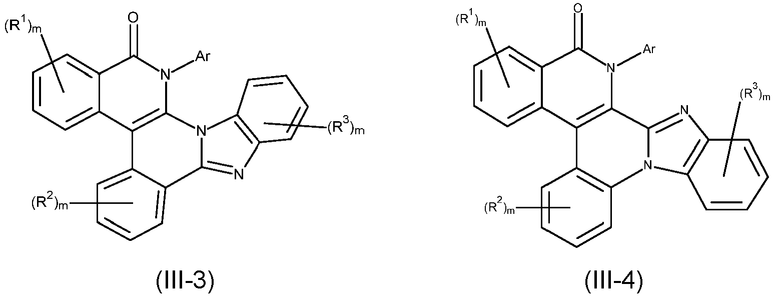 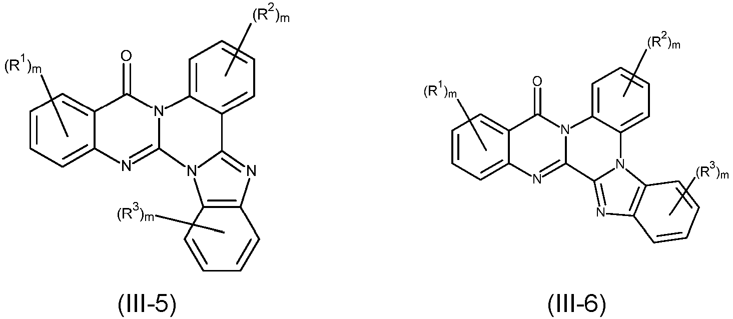 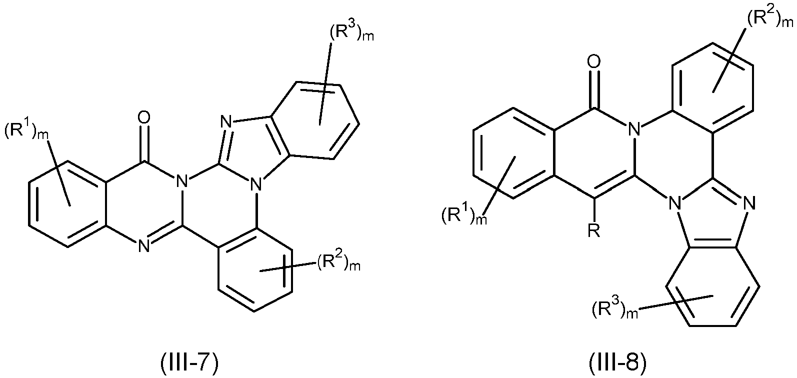 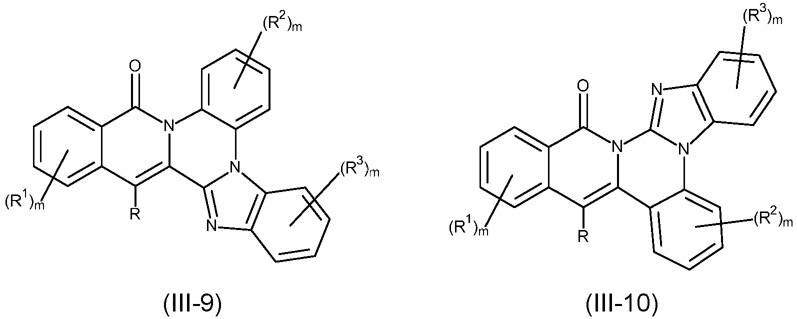 wobei die Symbole R, R¹, R², R³ und Ar, die in Anspruch 1 genannten Bedeutungen aufweisen und der Index m 0, 1, 2, 3 oder 4, vorzugsweise 0, 1 oder 2 ist, wobei Strukturen/Verbindungen der Formeln (III-1) bis (III-7) bevorzugt sind.

5. Verbindung nach einem oder mehreren der Ansprüche 1 bis 4, dass mindestens eine Gruppe R, R¹, R² und/oder R³ gleich oder verschieden ausgewählt ist aus den Resten der folgenden Formeln SAr-1 bis SAr-18 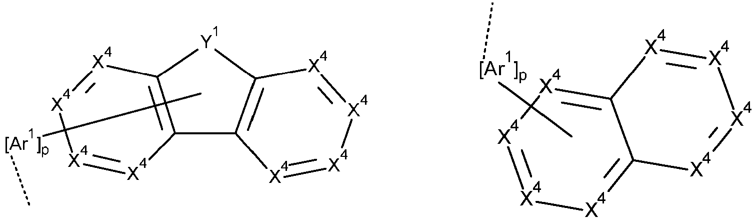 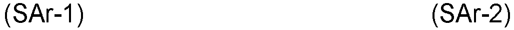 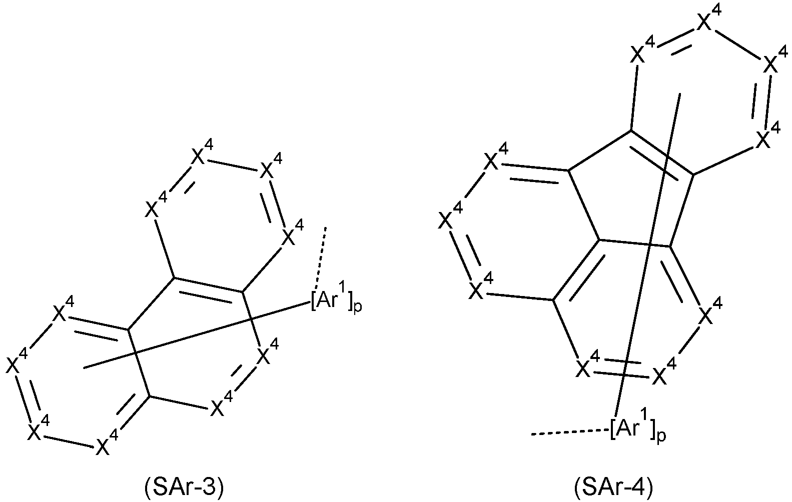 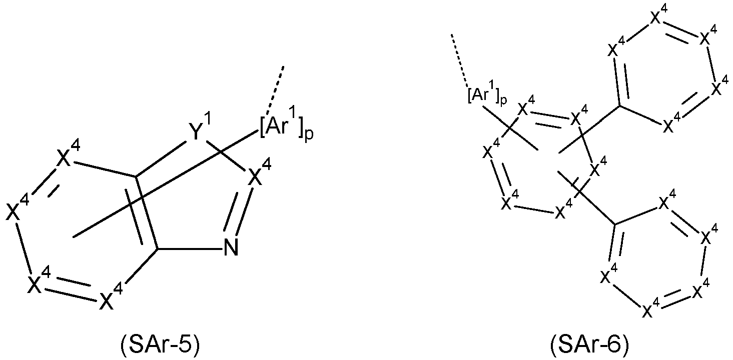 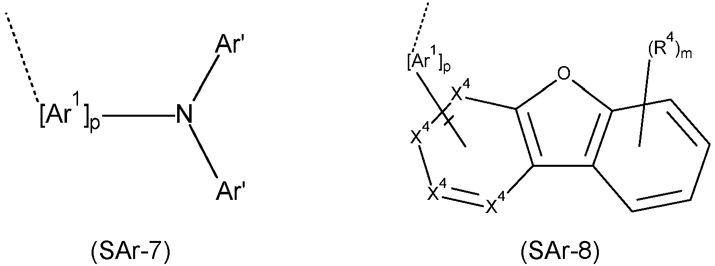 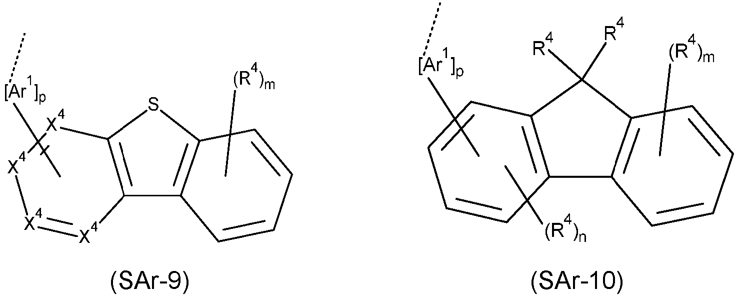 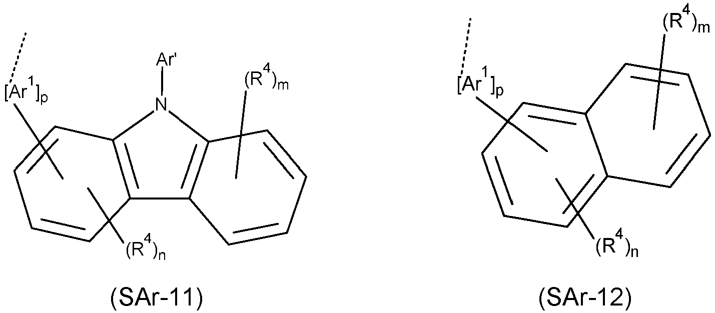 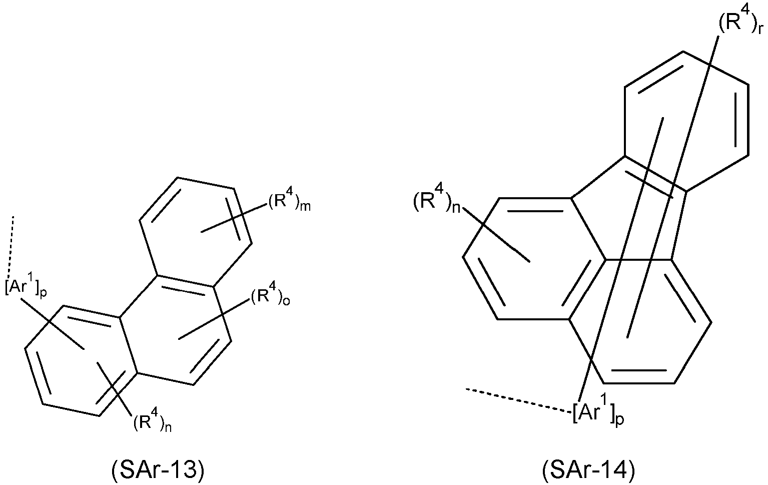 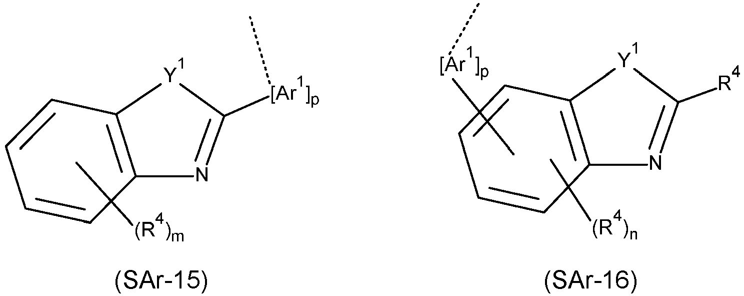 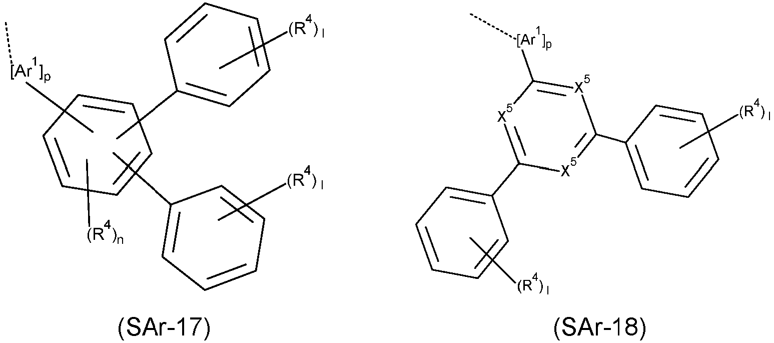 wobei R⁴ und Ar' die in Anspruch 1 genannten Bedeutungen aufweisen und für die weiteren Symbole und Indices gilt:
X⁴ ist bei jedem Auftreten gleich oder verschieden CR⁴, N oder C, falls hieran die Gruppe [Ar¹]ₚ bindet, vorzugsweise CR⁴, wobei bevorzugt keine N-N-Bindungen vorhanden sind;
X⁵ ist bei jedem Auftreten gleich oder verschieden CR⁴ oder N, vorzugsweise N;
Ar¹ ist bei jedem Auftreten gleich oder verschieden ein bivalentes aromatisches oder heteroaromatisches Ringsystem mit 6 bis 18 aromatischen Ringatomen, welches jeweils Reste R⁴ aufweist;
Y¹ ist bei jedem Auftreten gleich oder verschieden C(R⁴)₂, NR⁴, O, S oder N, falls hieran die Gruppe [Ar¹]ₚ bindet;
p ist 0 oder 1, wobei p = 0 bedeutet, dass die Gruppe Ar¹ nicht vorhanden ist und dass die entsprechende aromatische bzw. heteroaromatische Gruppe direkt an den entsprechenden Rest gebunden ist;
n ist 0, 1, 2 oder 3, vorzugsweise 0, 1 oder 2;
m ist 0, 1, 2, 3 oder 4, vorzugsweise 0, 1 oder 2;
l ist 0, 1, 2, 3, 4 oder 5, vorzugsweise 0, 1 oder 2;
r ist 0, 1, 2, 3, 4, 5 oder 6, vorzugsweise 0, 1 oder 2;
wobei Strukturen der Formeln (SAr-1), (SAr-4), (SAr-8), (SAr-11), (SAr-14), (SAr-18) bevorzugt sind.

6. Verbindung nach einem oder mehreren der Ansprüche 1 bis 9, **dadurch gekennzeichnet, dass** R, R¹, R² und/oder R³ gleich oder verschieden bei jedem Auftreten ausgewählt ist aus der Gruppe bestehend aus H, D oder einem aromatischen oder heteroaromatischen Ringsystem ausgewählt aus den Gruppen der folgenden Formeln Ar-1 bis Ar-79, vorzugsweise mindestens eine Gruppe R, R¹, R² und/oder R³ gleich oder verschieden ausgewählt ist aus den Gruppen der folgenden Formeln Ar-1 bis Ar-79 und/oder die Gruppe Ar gleich oder verschieden bei jedem Auftreten ausgewählt ist aus den Gruppen der folgenden Formeln Ar-1 bis Ar-79 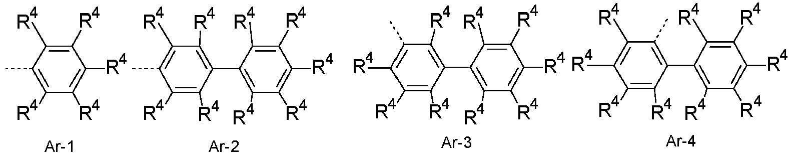 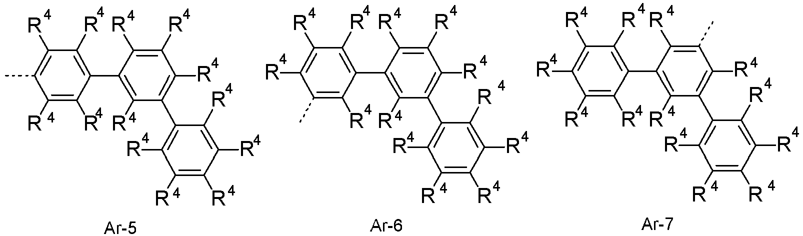 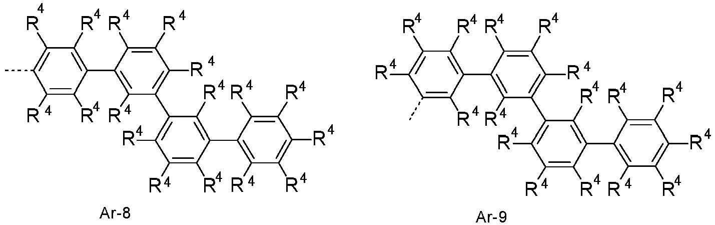 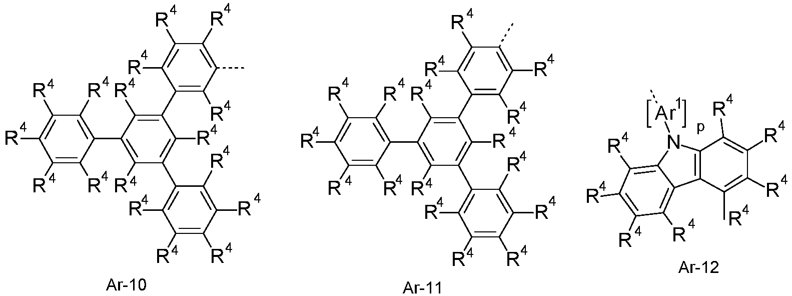 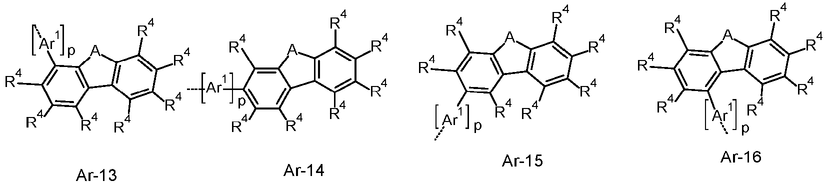 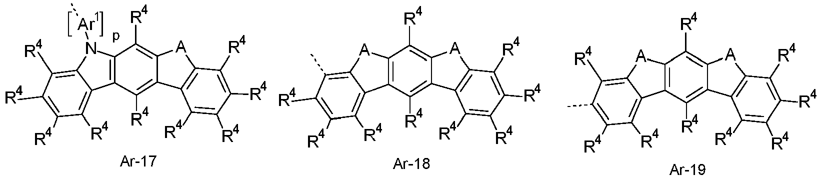 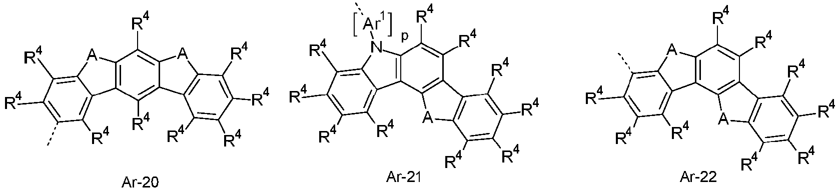 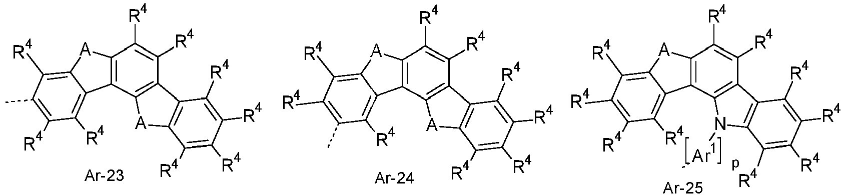 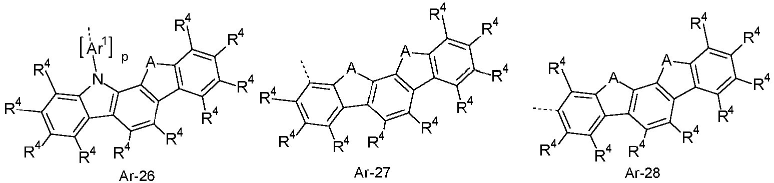 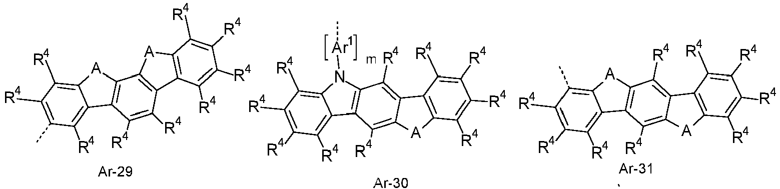 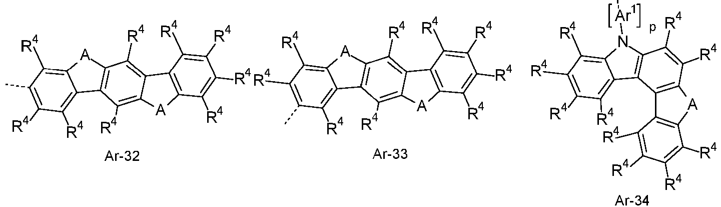 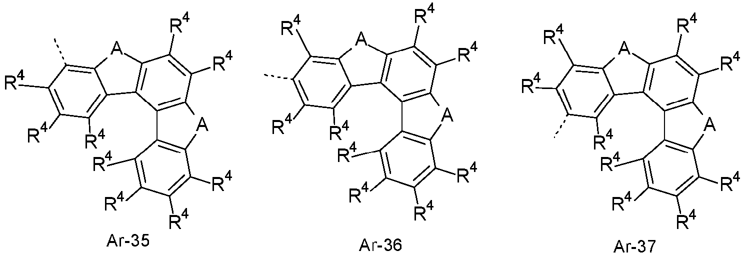 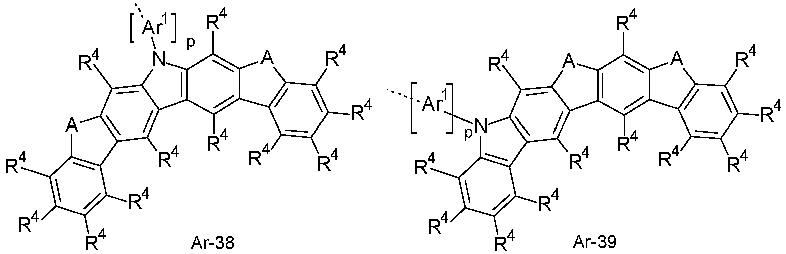 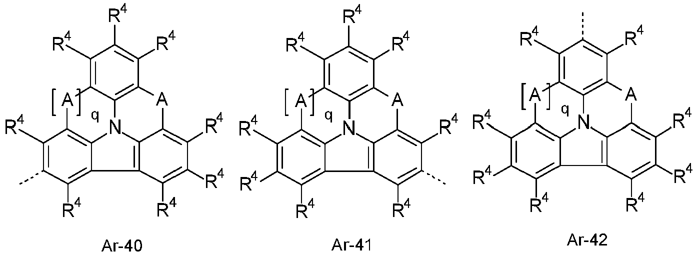 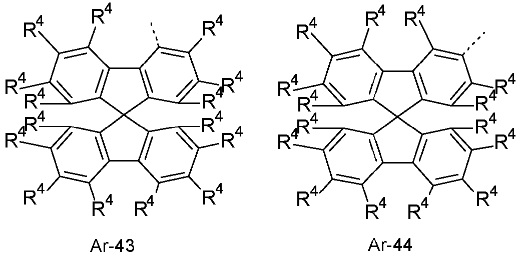 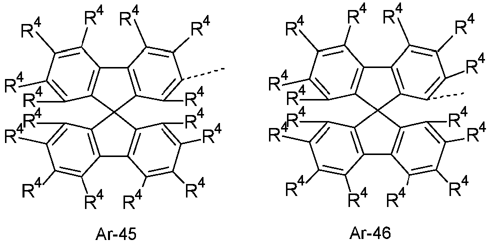 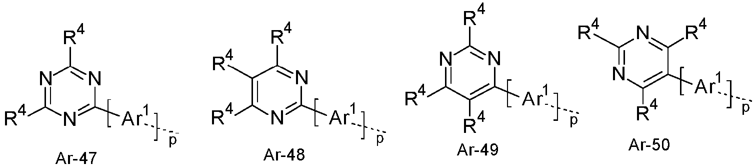 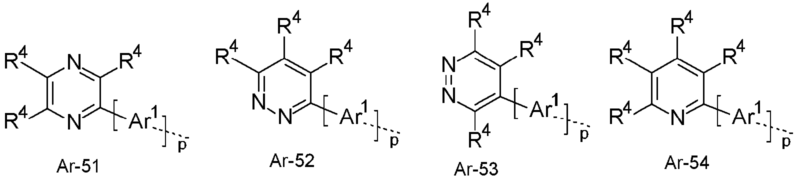 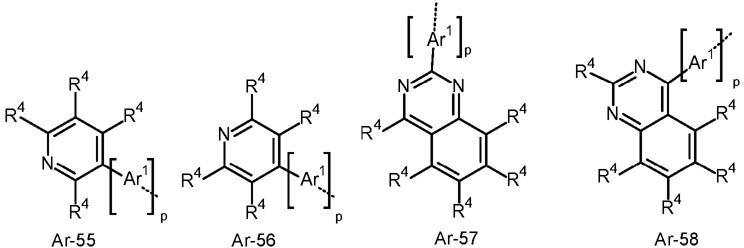 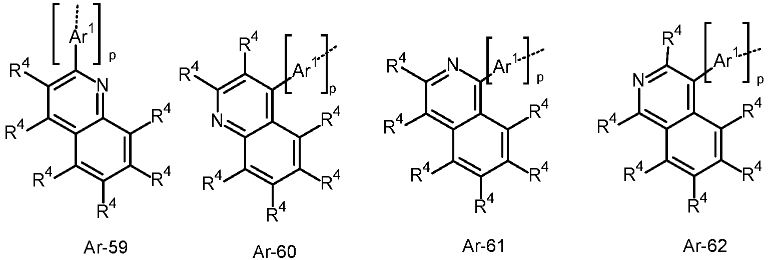 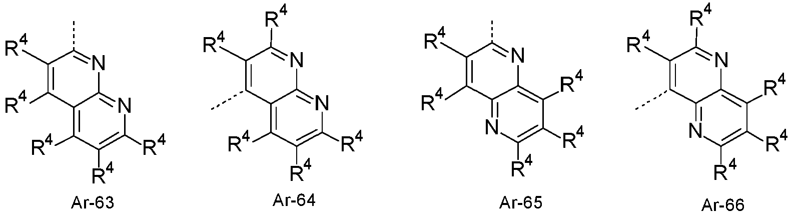 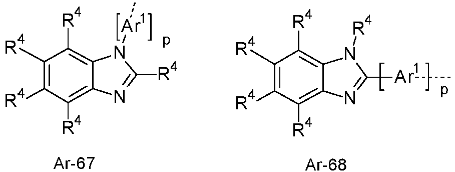 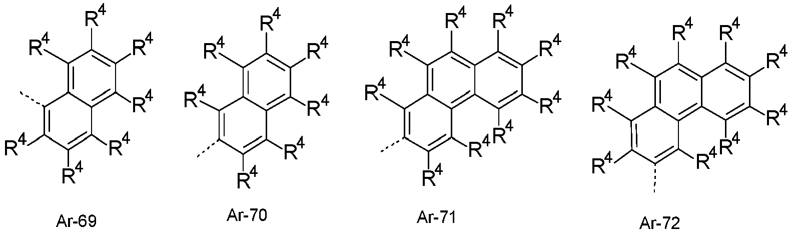 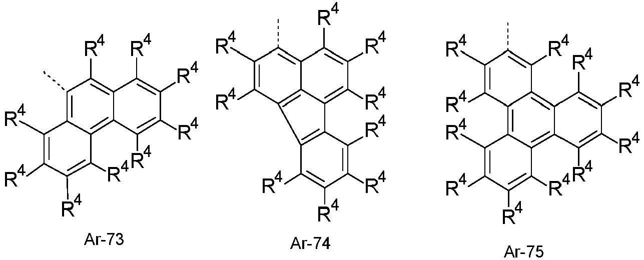 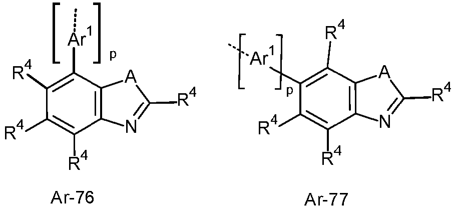 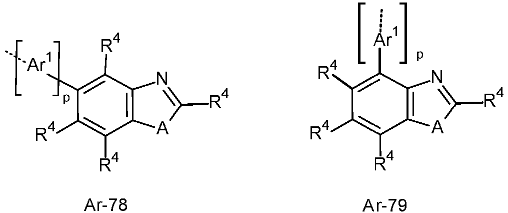 wobei R⁴ die oben genannten Bedeutungen aufweist, die gestrichelte Bindung die Bindung an die entsprechende Gruppe darstellt und weiterhin gilt:
Ar¹ ist bei jedem Auftreten gleich oder verschieden ein bivalentes aromatisches oder heteroaromatisches Ringsystem mit 6 bis 18 aromatischen Ringatomen, welches jeweils Reste R⁴ aufweist;
A ist bei jedem Auftreten gleich oder verschieden C(R⁴)₂, NR⁴, O oder S;
p ist 0 oder 1, wobei p = 0 bedeutet, dass die Gruppe Ar¹ nicht vorhanden ist und dass die entsprechende aromatische bzw. heteroaromatische Gruppe direkt an den entsprechenden Rest gebunden ist;
q ist 0 oder 1, wobei q = 0 bedeutet, dass an dieser Position keine Gruppe A gebunden ist und an die entsprechenden Kohlenstoffatome statt dessen Reste R⁴ gebunden sind, wobei Strukturen der Formeln (Ar-1), (Ar-2), (Ar-3), (Ar-12), (Ar-13), (Ar-14), (Ar-15), (Ar-16), (Ar-47), (Ar-70), (Ar-75), (Ar-76), (Ar-77), (Ar-78), (Ar-79), bevorzugt und Strukturen der Formeln (Ar-1), (Ar-2), (Ar-3), (Ar-12), (Ar-13), (Ar-14), (Ar-15), (Ar-16) besonders bevorzugt sind.

7. Verfahren zur Herstellung einer Verbindung nach einem oder mehreren der Ansprüche 1 bis 10, **dadurch gekennzeichnet, dass** eine stickstoffhaltige aromatische oder heteroaromatische Verbindung in einer Ringbildungsreaktion umgesetzt wird.

8. Zusammensetzung, enthaltend mindestens eine Verbindung umfassend mindestens eine Struktur mit mindestens drei miteinander kondensierten Strukturelementen gemäß den Formeln (A'), (B') und (C'), vorzugsweise Verbindung bestehend aus drei miteinander kondensierten Strukturelementen gemäß den Formeln (A'), (B') und (C') 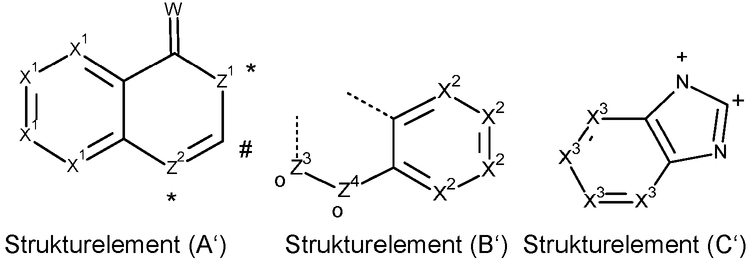
wobei das Strukturelement (A') mit Strukturelement (B') kondensiert ist und das Strukturelement (B') mit Strukturelement (C') kondensiert ist; wobei das Strukturelement (B') über die gestrichelt dargestellten Bindungen an das Strukturelement (A') bindet, wobei eine Bindung über die mit # markierte Bindungsstelle erfolgt und eine Bindung über eine mit * markierte Bindungsstelle erfolgt; wobei das Strukturelement (B') mit dem Strukturelement (C') über die mit o und + markierten Atome miteinander kondensiert ist und die jeweils markierten Atome von den Strukturelementen (B') und (C') geteilt sind, und
für die verwendeten Symbole und Indizes gilt:
W steht für O oder S, vorzugsweise für O;
Z¹ steht für NAr oder N, für den Fall, dass das Strukturelement (A') mit dem Strukturelement (B') über Z¹ kondensiert ist;
Z² steht für X oder C, für den Fall, dass das Strukturelement (A') mit dem Strukturelement (B') über Z² kondensiert ist;
Z³, Z⁴ steht für N oder C, wobei einer der Reste Z³, Z⁴ für N und einer der Reste Z³, Z⁴ für C steht;
X steht für N oder CR, vorzugsweise für N;
X¹ steht bei jedem Auftreten gleich oder verschieden für N oder CR¹, vorzugsweise für CR¹, mit der Maßgabe, dass nicht mehr als zwei der Gruppen X¹ in einem Cyclus für N stehen;
X² steht bei jedem Auftreten gleich oder verschieden für N oder CR², mit der Maßgabe, dass nicht mehr als zwei der Gruppen X² in einem Cyclus für N stehen;
X³ steht bei jedem Auftreten gleich oder verschieden für N oder CR³, vorzugsweise für CR³, mit der Maßgabe, dass nicht mehr als zwei der Gruppen X³ in einem Cyclus für N stehen;
Ar ist bei jedem Auftreten gleich oder verschieden ein aromatisches oder heteroaromatisches Ringsystem mit 5 bis 40 aromatischen Ringatomen, welches Reste R aufweist, vorzugsweise eine Arylgruppe mit 6 bis 30 aromatischen Ringatomen oder eine Heteroarylgruppe mit 5 bis 14 aromatischen Ringatomen, welche Reste R aufweist;
R ist bei jedem Auftreten gleich oder verschieden H, D, F, Cl, Br, I, R⁴C=C(R⁴)₂, N(R⁴)₂, N(Ar')₂^{,} CN, NO₂, OR⁴, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, eine geradkettige Alkyl-, Alkoxy- oder Thioalkylgruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkylgruppe mit 3 bis 20 C-Atomen, wobei die Alkyl-, Alkoxy- oder Thioalkyl-, Alkenyl- oder Alkinylgruppe jeweils Reste R⁴ aufweist und wobei eine oder mehrere nicht benachbarte CH₂-Gruppen durch R⁴C=CR⁴, C=C, Si(R⁴)₂, C=O, C=S, C=Se, C=NR⁴, -C(=O)O-, -C(=O)NR⁴-, NR⁴, P(=O)( R⁴), -O-, -S-, SO oder SO₂ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils Reste R⁴ aufweist, oder eine Aryloxy- oder Heteroaryloxygruppe mit 5 bis 40 aromatischen Ringatomen, die jeweils Reste R⁴ aufweist, oder eine Diarylamino-, Arylheteroarylamino-, Diheteroarylaminogruppe mit 5 bis 60 aromatischen Ringatomen im jeweiligen aromatischen oder heteroaromatischen Rest, wobei die Diarylamino-, Arylheteroarylamino-, Diheteroarylaminogruppe Reste R⁴ aufweist; dabei kann ein Rest R mit einem weiteren Rest, vorzugsweise einer Gruppe R¹, R², R³ ein aliphatisches, heteroaliphatisches, aromatisches oder heteroaromatisches Ringsystem, vorzugsweise ein aliphatisches, heteroaliphatisches oder heteroaromatisches Ringsystem bilden, besonders bevorzugt bildet der Rest R kein solches Ringsystem;
R¹ ist bei jedem Auftreten gleich oder verschieden H, D, F, Cl, Br, I, R⁴C=C(R⁴)₂, N(R⁴)₂, N(Ar')₂^{,} CN, NO₂, OR⁴, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, eine geradkettige Alkyl-, Alkoxy- oder Thioalkylgruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkylgruppe mit 3 bis 20 C-Atomen, wobei die Alkyl-, Alkoxy- oder Thioalkyl-, Alkenyl- oder Alkinylgruppe jeweils Reste R⁴ aufweist und wobei eine oder mehrere nicht benachbarte CH₂-Gruppen durch R⁴C=CR⁴, C=C, Si(R⁴)₂, C=O, C=S, C=Se, C=NR⁴, -C(=O)O-, -C(=O)NR⁴-, NR⁴, P(=O)( R⁴), -O-, -S-, SO oder SO₂ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils Reste R⁴ aufweist, oder eine Aryloxy- oder Heteroaryloxygruppe mit 5 bis 40 aromatischen Ringatomen, die jeweils Reste R⁴ aufweist, oder eine Diarylamino-, Arylheteroarylamino-, Diheteroarylaminogruppe mit 5 bis 60 aromatischen Ringatomen im jeweiligen aromatischen oder heteroaromatischen Rest, wobei die Diarylamino-, Arylheteroarylamino-, Diheteroarylaminogruppe Reste R⁴ aufweist; dabei können zwei Reste R¹ oder ein Rest R¹ mit einem weiteren Rest, vorzugsweise einer Gruppe R, R², R³ auch miteinander ein aromatisches, heteroaromatisches, aliphatisches oder heteroaliphatisches Ringsystem, vorzugsweise ein aliphatisches, heteroaliphatisches oder heteroaromatisches Ringsystem bilden, besonders bevorzugt bilden die Reste R¹ kein solches Ringsystem;
R² ist bei jedem Auftreten gleich oder verschieden H, D, F, Cl, Br, I, R⁴C=C(R⁴)₂, N(R⁴)₂, N(Ar')₂^{,} CN, NO₂, OR⁴, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, eine geradkettige Alkyl-, Alkoxy- oder Thioalkylgruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkylgruppe mit 3 bis 20 C-Atomen, wobei die Alkyl-, Alkoxy- oder Thioalkyl-, Alkenyl- oder Alkinylgruppe jeweils Reste R⁴ aufweist und wobei eine oder mehrere nicht benachbarte CH₂-Gruppen durch R⁴C=CR⁴, C=C, Si(R⁴)₂, C=O, C=S, C=Se, C=NR⁴, -C(=O)O-, -C(=O)NR⁴-, NR⁴, P(=O)( R⁴), -O-, -S-, SO oder SO₂ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils Reste R⁴ aufweist, oder eine Aryloxy- oder Heteroaryloxygruppe mit 5 bis 40 aromatischen Ringatomen, die jeweils Reste R⁴ aufweist, oder eine Diarylamino-, Arylheteroarylamino-, Diheteroarylaminogruppe mit 5 bis 60 aromatischen Ringatomen im jeweiligen aromatischen oder heteroaromatischen Rest, wobei die Diarylamino-, Arylheteroarylamino-, Diheteroarylaminogruppe Reste R⁴ aufweist; dabei können zwei Reste R² oder ein Rest R² mit einem weiteren Rest, vorzugsweise einer Gruppe R, R¹, R³ auch miteinander ein aromatisches, heteroaromatisches, aliphatisches oder heteroaliphatisches Ringsystem, vorzugsweise ein aliphatisches, heteroaliphatisches oder heteroaromatisches Ringsystem bilden, besonders bevorzugt bilden die Reste R² kein solches Ringsystem;
R³ ist bei jedem Auftreten gleich oder verschieden H, D, F, Cl, Br, I, R⁴C=C(R⁴)₂, N(R⁴)₂, N(Ar')₂^{,} CN, NO₂, OR⁴, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, eine geradkettige Alkyl-, Alkoxy- oder Thioalkylgruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkylgruppe mit 3 bis 20 C-Atomen, wobei die Alkyl-, Alkoxy- oder Thioalkyl-, Alkenyl- oder Alkinylgruppe jeweils Reste R⁴ aufweist und wobei eine oder mehrere nicht benachbarte CH₂-Gruppen durch R⁴C=CR⁴, C=C, Si(R⁴)₂, C=O, C=S, C=Se, C=NR⁴, -C(=O)O-, -C(=O)NR⁴-, NR⁴, P(=O)( R⁴), -O-, -S-, SO oder SO₂ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils Reste R⁴ aufweist, oder eine Aryloxy- oder Heteroaryloxygruppe mit 5 bis 40 aromatischen Ringatomen, die jeweils Reste R⁴ aufweist, oder eine Diarylamino-, Arylheteroarylamino-, Diheteroarylaminogruppe mit 5 bis 60 aromatischen Ringatomen im jeweiligen aromatischen oder heteroaromatischen Rest, wobei die Diarylamino-, Arylheteroarylamino-, Diheteroarylaminogruppe Reste R⁴ aufweist; dabei können zwei Reste R³ oder ein Rest R³ mit einem weiteren Rest, vorzugsweise einer Gruppe R, R¹, R² auch miteinander ein aromatisches, heteroaromatisches, aliphatisches oder heteroaliphatisches Ringsystem, vorzugsweise ein aliphatisches, heteroaliphatisches oder heteroaromatisches Ringsystem bilden, besonders bevorzugt bilden die Reste R³ kein solches Ringsystem;
Ar' ist bei jedem Auftreten gleich oder verschieden ein aromatisches oder heteroaromatisches Ringsystem mit 5 bis 40 aromatischen Ringatomen, welches Reste R⁴ aufweist, vorzugsweise eine Arylgruppe mit 6 bis 30 aromatischen Ringatomen oder eine Heteroarylgruppe mit 5 bis 14 aromatischen Ringatomen, welche Reste R⁴ aufweist;
R⁴ ist bei jedem Auftreten gleich oder verschieden H, D, F, Cl, Br, I, R⁵C=C(R⁵)₂, N(R⁵)₂, CN, NO₂, OR⁵, SR⁵, Si(R⁵)₃, B(OR⁵)₂, C(=O)R⁵, P(=O)(R⁵)₂, S(=O)R⁵, S(=O)₂R⁵, OSO₂R⁵, eine gerad- kettige Alkyl-, Alkoxy- oder Thioalkylgruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkylgruppe mit 3 bis 20 C-Atomen, wobei die Alkyl-, Alkoxy- oder Thioalkyl-, Alkenyl- oder Alkinylgruppe jeweils Reste R⁵ aufweist, wobei eine oder mehrere nicht benachbarte CH₂-Gruppen durch R⁵C=CR⁵, C=C, Si(R⁵)₂, C=O, C=S, C=Se, C=NR⁵, -C(=O)O-, -C(=O)NR⁵-, NR⁵, P(=O)( R⁵), -O-, -S-, SO oder SO₂ ersetzt sein können, oder ein aromatisches oder heteroaromatisches Ringsystem mit 5 bis 40 aromatischen Ringatomen, das jeweils Reste R⁵ aufweist, oder eine Aryloxy- oder Heteroaryloxygruppe mit 5 bis 40 aromatischen Ringatomen, die jeweils Reste R⁵ aufweist, oder eine Diarylamino-, Arylheteroarylamino-, Diheteroarylaminogruppe mit 5 bis 60 aromatischen Ringatomen im jeweiligen aromatischen oder heteroaromatischen Rest, wobei die Diarylamino-, Arylheteroarylamino-, Diheteroarylaminogruppe Reste R⁵ aufweist; dabei können zwei oder mehrere Reste R⁴ miteinander ein aromatisches, heteroaromatisches, aliphatisches oder heteroaliphatisches Ringsystem bilden, vorzugsweise ein aliphatisches Ringsystem, besonders bevorzugt bilden die Reste R⁴ kein solches Ringsystem;
-R⁵ ist bei jedem Auftreten gleich oder verschieden H, D, F oder ein aliphatischer, aromatischer oder heteroaromatischer organischer Rest, insbesondere ein Kohlenwasserstoffrest, mit 1 bis 20 C-Atomen, in dem auch ein oder mehrere H-Atome durch F ersetzt sein können, dabei können zwei oder mehrere Reste R⁵ miteinander ein Ringsystem bilden;
und
mindestens ein weiteres Matrixmaterial, wobei das weitere Matrixmaterial ausgewählt ist aus Verbindungen gemäß einer der Formeln (H-1), (H-2), (H-3), (H-4) oder (H-5), 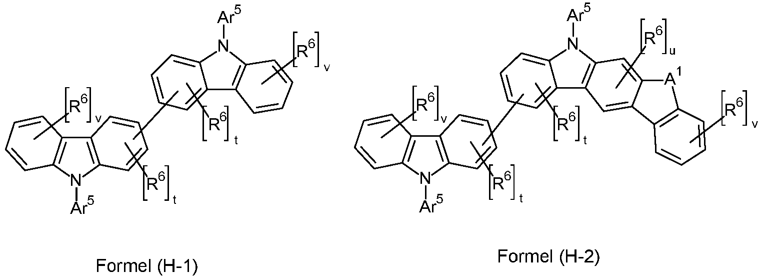 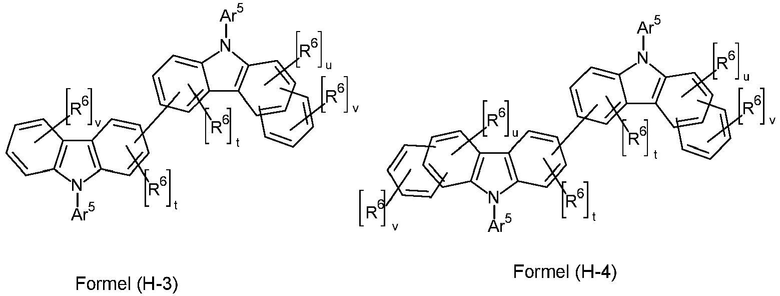 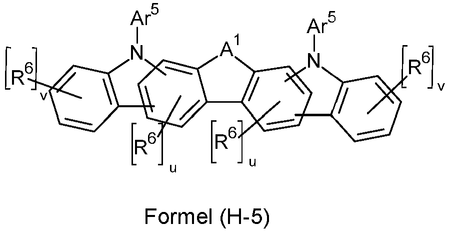
wobei für die verwendeten Symbole und Indizes gilt:
R⁶ ist bei jedem Auftreten gleich oder verschieden H, D, F, Cl, Br, I, N(R⁷)₂, N(Ar")₂, CN, NO₂, OR⁷, SR⁷, COOR⁷, C(=O)N(R')₂, Si(R⁷)₃, B(OR⁷)₂, C(=O)R⁷, P(=O)(R⁷)₂, S(=O)R⁷, S(=O)₂R⁷, OSO₂R⁷, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils Reste R⁷ aufweisen und wobei eine oder mehrere nicht benachbarte CH₂-Gruppen durch Si(R⁷)₂, C=O, NR⁷, O, S oder CONR⁷ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils Reste R⁷ aufweist; dabei können zwei Reste R⁶ auch miteinander ein aromatisches, heteroaromatisches, aliphatisches oder heteroaliphatisches Ringsystem bilden, vorzugsweise bilden die Reste R⁶ kein solches Ringsystem;
Ar" ist bei jedem Auftreten gleich oder verschieden ein aromatisches oder heteroaromatisches Ringsystem mit 5 bis 40 aromatischen Ringatomen, welches Reste R⁷ aufweist;
A¹ ist C(R⁷)₂, NR⁷, O oder S;
Ar⁵ steht gleich oder verschieden bei jedem Auftreten für ein aromatisches oder heteroaromatisches Ringsystem mit 5 bis 40 aromatischen Ringatomen steht, welches Reste R⁷ aufweist;
R⁷ ist bei jedem Auftreten gleich oder verschieden H, D, F, Cl, Br, I, N(R⁸)₂, CN, NO₂, OR⁸, SR⁸, Si(R⁸)₃, B(OR⁸)₂, C(=O)R⁸, P(=O)(R⁸)₂, S(=O)R⁸, S(=O)₂R⁸, OSO₂R⁸, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils Reste R⁸ aufweisen, wobei eine oder mehrere nicht benachbarte CH₂-Gruppen durch Si(R⁸)₂, C=O, NR⁸, O, S oder CONR⁸ ersetzt sein können, oder ein aromatisches oder heteroaromatisches Ringsystem mit 5 bis 40 aromatischen Ringatomen, das jeweils Reste R⁸ aufweist; dabei können zwei oder mehrere Reste R⁷ miteinander ein aromatisches, heteroaromatisches, aliphatisches oder heteroaliphatisches Ringsystem bilden, vorzugsweise bilden die Reste R⁷ kein solches Ringsystem;
R⁸ ist bei jedem Auftreten gleich oder verschieden H, D, F oder ein aliphatischer, aromatischer oder heteroaromatischer organischer Rest, insbesondere ein Kohlenwasserstoffrest, mit 1 bis 20 C-Atomen, in dem auch ein oder mehrere H-Atome durch F ersetzt sein können;
v ist bei jedem Auftreten gleich oder verschieden 0, 1, 2, 3 oder 4, vorzugsweise 0 oder 1 und ganz bevorzugt 0;
t ist bei jedem Auftreten gleich oder verschieden 0, 1, 2, oder 3, vorzugsweise 0 oder 1 und ganz bevorzugt 0;
u ist bei jedem Auftreten gleich oder verschieden 0, 1 oder 2, vorzugsweise 0 oder 1 und ganz bevorzugt 0.

9. Zusammensetzung nach Anspruch 8, **dadurch gekennzeichnet, dass** die Zusammensetzung aus mindestens einer Verbindung, enthaltend mindestens eine Verbindung umfassend mindestens eine Struktur mit mindestens drei miteinander kondensierten Strukturelementen gemäß den Formeln (A'), (B') und (C'), und mindestens einer Verbindungen gemäß einer der Formeln (H-1), (H-2), (H-3), (H-4) oder (H-5) besteht.

10. Zusammensetzung nach Anspruch 8 oder 9, **dadurch gekennzeichnet, dass** die Verbindungen gemäß einer der Formeln (H-1), (H-2), (H-3), (H-4) oder (H-5) in der Zusammensetzung einen Massenanteil im Bereich von 5 Gew.-% bis 90 Gew.-%, bevorzugt im Bereich von 10 Gew.-% bis 85 Gew.-%, mehr bevorzugt im Bereich von 20 Gew.-% bis 85 Gew.-%, noch mehr bevorzugt im Bereich von 30 Gew.-% bis 80 Gew.-%, ganz besonders bevorzugt im Bereich von 20 Gew.-% bis 60 Gew.-% und am meisten bevorzugt im Bereich von 30 Gew.-% bis 50 Gew.-% aufweist, bezogen auf die gesamte Zusammensetzung.

11. Formulierung, enthaltend mindestens eine Verbindung nach einem oder mehreren der Ansprüche 1 bis 6 und/oder mindestens eine Zusammensetzung nach Anspruch 8 bis 10 und mindestens eine weitere Verbindung, wobei die weitere Verbindung bevorzugt ausgewählt ist aus einem oder mehreren Lösemitteln.

12. Verwendung einer Verbindung, enthaltend mindestens eine Verbindung umfassend mindestens eine Struktur mit mindestens drei miteinander kondensierten Strukturelementen gemäß den Formeln (A'), (B') und (C'), vorzugsweise Verbindung bestehend aus drei miteinander kondensierten Strukturelementen gemäß den Formeln (A'), (B') und (C'), 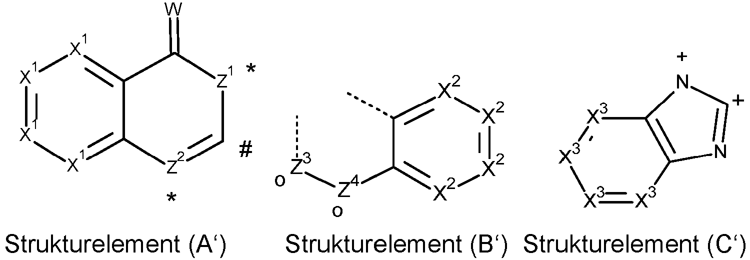
wobei das Strukturelement (A') mit Strukturelement (B') kondensiert ist und das Strukturelement (B') mit Strukturelement (C') kondensiert ist; wobei das Strukturelement (B') über die gestrichelt dargestellten Bindungen an das Strukturelement (A') bindet, wobei eine Bindung über die mit # markierte Bindungsstelle erfolgt und eine Bindung über eine mit * markierte Bindungsstelle erfolgt; wobei das Strukturelement (B') mit dem Strukturelement (C') über die mit o und + markierten Atome miteinander kondensiert ist und die jeweils markierten Atome von den Strukturelementen (B') und (C') geteilt sind,
wobei die verwendeten Symbole und Indizes die in Anspruch 8 genannten Bedeutungen aufweisen,
in einer elektronischen Vorrichtung; und/oder
Verwendung einer Zusammensetzung nach einem der Ansprüche 8 bis 10 in einer elektronischen Vorrichtung.

13. Elektronische Vorrichtung enthaltend mindestens eine Verbindung umfassend mindestens eine Struktur mit mindestens drei miteinander kondensierten Strukturelementen gemäß den Formeln (A'), (B') und (C'), vorzugsweise Verbindung bestehend aus drei miteinander kondensierten Strukturelementen gemäß den Formeln (A'), (B') und (C'), 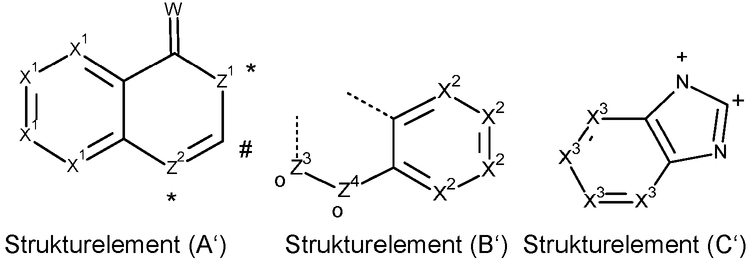
wobei das Strukturelement (A') mit Strukturelement (B') kondensiert ist und das Strukturelement (B') mit Strukturelement (C') kondensiert ist; wobei das Strukturelement (B') über die gestrichelt dargestellten Bindungen an das Strukturelement (A') bindet, wobei eine Bindung über die mit # markierte Bindungsstelle erfolgt und eine Bindung über eine mit * markierte Bindungsstelle erfolgt; wobei das Strukturelement (B') mit dem Strukturelement (C') über die mit o und + markierten Atome miteinander kondensiert ist und die jeweils markierten Atome von den Strukturelementen (B') und (C') geteilt sind,
wobei die verwendeten Symbole und Indizes die in Anspruch 8 genannten Bedeutungen aufweisen;
wobei es sich bei der elektronischen Vorrichtung vorzugsweise um eine Elektrolumineszenzvorrichtung handelt.

14. Elektronische Vorrichtung nach Anspruch 13, wobei es sich um eine organische Elektrolumineszenzvorrichtung handelt, **dadurch gekennzeichnet, dass** die Verbindung, umfassend mindestens eine Struktur mit mindestens drei miteinander kondensierten Strukturelementen gemäß den Formeln (A'), (B') und (C'), vorzugsweise Verbindung bestehend aus drei miteinander kondensierten Strukturelementen gemäß den Formeln (A'), (B') und (C'), als Matrixmaterial in einer emittierenden Schicht und/oder in einer Elektronentransportschicht und/oder in einer Lochblockierschicht, vorzugsweise als Matrixmaterial in einer emittierenden Schicht und/oder in einer Elektronentransportschicht, besonders bevorzugt als Matrixmaterial in einer emittierenden Schicht, speziell besonders bevorzugt als Matrixmaterial in einer emittierenden Schicht in Kombination mit einem rot oder grün phosphoreszierenden Emitter eingesetzt werden.

15. Elektronische Vorrichtung nach Anspruch 13 oder 14, **dadurch gekennzeichnet, dass** die Verbindung, umfassend mindestens eine Struktur mit mindestens drei miteinander kondensierten Strukturelementen gemäß den Formeln (A'), (B') und (C'), vorzugsweise Verbindung bestehend aus drei miteinander kondensierten Strukturelementen gemäß den Formeln (A'), (B') und (C'), als Matrixmaterial für phosphoreszierende Emitter eingesetzt wird in Kombination mit einem weiteren Matrixmaterial, welches ausgewählt ist aus Verbindungen gemäß einer der Formeln (H-1), (H-2), (H-3), (H-4) oder (H-5), 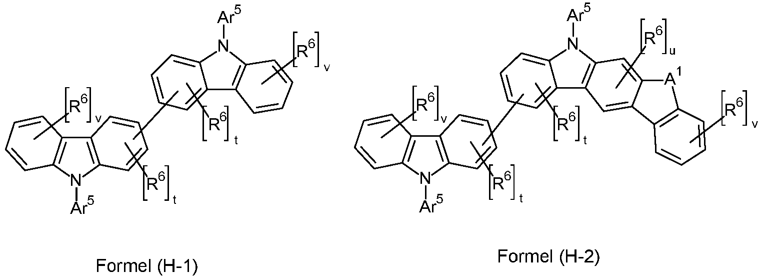 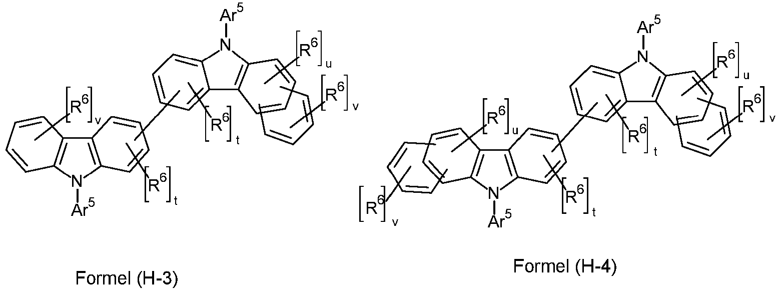 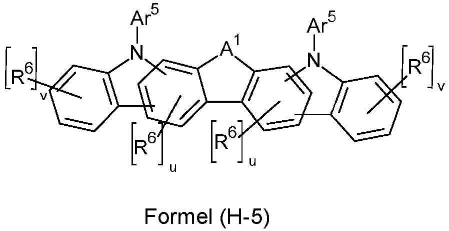 wobei die Symbole A¹, Ar⁵ und R⁶ und die Indices v, t und u die in Anspruch 8 genannten Bedeutungen aufweisen.

## Claims

1. Compound comprising at least one structure with at least three structural elements condensed together according to formulae (A), (B) and (C), preferably a compound consisting of three structural elements condensed together according to formulae (A), (B) and (C), 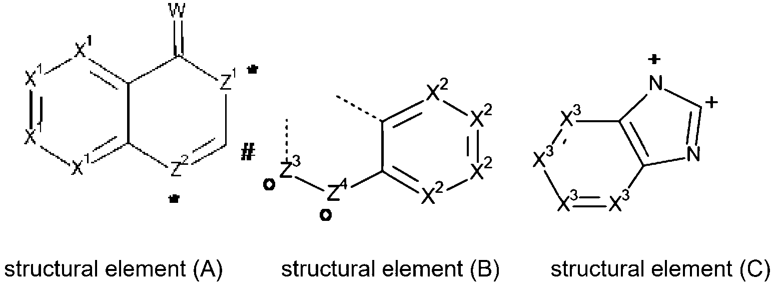
wherein the structural element (A) is condensed with structural element (B) and the structural element (B) is condensed with structural element (C); wherein the structural element (B) binds to the structural element (A) via the bonds shown in dashed lines, wherein a binding occurs via the binding site marked with # and a binding occurs via a binding site marked with *; wherein the structural element (B) is condensed with the structural element (C) via the atoms labelled with o and + and the respective labelled atoms are shared by the structural elements (B) and (C), and
applies to the symbols and indices used:
W stands for O or S, preferably for O;
Z¹ stands for NAr or N, in the event that the structural element (A) is condensed with the structural element (B) via Z¹;
Z² stands for X or C, in the event that the structural element (A) is condensed with the structural element (B) via Z²;
Z³, Z⁴ stands for N or C, wherein one of the residues Z³, Z⁴ stands for N and one of the residues Z³, Z⁴ stands for C;
X stands for N or CR, preferably for N;
X¹ stands for N or CR¹, preferably for CR¹, with the proviso that no more than two of the groups X¹ in a cycle stand for N;
X² in each occurrence is the same or different for N or CR², with the proviso that no more than two of the groups X² in a cycle are for N;
X³ is the same or different for each occurrence for N or CR³, preferably for CR³, with the proviso that no more than two of the groups X³ are in a cycle for N;
Ar in each occurrence is, identically or differently, an aromatic or heteroaromatic ring system having 5 to 40 aromatic ring atoms which has radicals R, preferably an aryl group having 6 to 30 aromatic ring atoms or a heteroaryl group having 5 to 14 aromatic ring atoms which has radicals R;
R in each occurrence is the same or different H, D, F, Cl, Br, I, R⁴C=C(R⁴)₂, N(R⁴)₂, N(Ar')₂^{,} CN, NO₂, OR⁴, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, a straight-chain alkyl, alkoxy or thioalkyl group having 1 to 20 carbon atoms or an alkenyl or alkynyl group having 2 to 20 carbon atoms or a branched or cyclic alkyl, alkoxy or thioalkyl group having 3 to 20 carbon atoms, wherein the alkyl, alkoxy or thioalkyl, alkenyl or alkynyl group each has radicals R⁴ and wherein one or more non-adjacent CH₂ groups are represented by R⁴C=CR⁴, C=C, Si(R⁴)₂, C=O, C=S, C=Se, C=NR⁴, - C(=O)O-, -C(=O)NR⁴-, NR⁴, P(=O)( R⁴), -O-, -S-, SO or SO₂, or an aromatic or heteroaromatic ring system with 5 to 60 aromatic ring atoms, preferably with 5 to 40 aromatic ring atoms, each of which has R⁴ radicals or an aryloxy or heteroaryloxy group having 5 to 40 aromatic ring atoms, each of which has radicals R⁴, or a diarylamino, arylheteroarylamino or diheteroarylamino group having 5 to 60 aromatic ring atoms in the respective aromatic or heteroaromatic radical, wherein the diarylamino, arylheteroarylamino or diheteroarylamino group has radicals R⁴; two radicals R or one radical R together with a further radical, preferably a group R¹, R², R³ can form an aliphatic, heteroaliphatic, aromatic or heteroaromatic ring system, preferably an aliphatic, heteroaliphatic or heteroaromatic ring system, particularly preferred the radical R does not form such a ring system;
R¹ is in each occurrence the same or different H, D, F, Cl, Br, I, R⁴C=C(R⁴)₂, N(R⁴)₂, N(Ar')₂^{,} CN, NO₂, OR⁴, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, a straight-chain alkyl, alkoxy or thioalkyl group having 1 to 20 carbon atoms or an alkenyl or alkynyl group having 2 to 20 carbon atoms or a branched or cyclic alkyl, alkoxy or thioalkyl group having 3 to 20 carbon atoms, wherein the alkyl, alkoxy or thioalkyl, alkenyl or alkynyl group each has radicals R⁴ and wherein one or more non-adjacent CH₂ groups are represented by R⁴C=CR⁴, C=C, Si(R⁴)₂, C=O, C=S, C=Se, C=NR⁴, - C(=O)O-, -C(=O)NR⁴-, NR⁴, P(=O)( R⁴), -O-, -S-, SO or SO₂, or an aromatic or heteroaromatic ring system with 5 to 60 aromatic ring atoms, preferably with 5 to 40 aromatic ring atoms, each of which has R⁴ radicals or an aryloxy or heteroaryloxy group having 5 to 40 aromatic ring atoms, each of which has radicals R⁴, or a diarylamino, arylheteroarylamino or diheteroarylamino group having 5 to 60 aromatic ring atoms in the respective aromatic or heteroaromatic radical, wherein the diarylamino, arylheteroarylamino or diheteroarylamino group has radicals R⁴; two radicals R¹ or a radical R¹ together with a further radical, preferably a group R, R², R³ can also form an aromatic, heteroaromatic, aliphatic or heteroaliphatic ring system, preferably an aliphatic, heteroaliphatic or heteroaromatic ring system, it being particularly preferred that the radicals R¹ do not form such a ring system;
R² is in each occurrence the same or different H, D, F, Cl, Br, I, R⁴C=C(R⁴)₂, N(R⁴)₂, N(Ar')₂^{,} CN, NO₂, OR⁴, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, a straight-chain alkyl, alkoxy or thioalkyl group having 1 to 20 carbon atoms or an alkenyl or alkynyl group having 2 to 20 carbon atoms or a branched or cyclic alkyl, alkoxy or thioalkyl group having 3 to 20 carbon atoms, wherein the alkyl, alkoxy or thioalkyl, alkenyl or alkynyl group each has radicals R⁴ and wherein one or more non-adjacent CH₂ groups are represented by R⁴C=CR⁴, C≡C, Si(R⁴)₂, C=O, C=S, C=Se, C=NR⁴, - C(=O)O-, -C(=O)NR⁴-, NR⁴, P(=O)( R⁴), -O-, -S-, SO or SO₂, or an aromatic or heteroaromatic ring system with 5 to 60 aromatic ring atoms, preferably with 5 to 40 aromatic ring atoms, each of which has R⁴ radicals or an aryloxy or heteroaryloxy group having 5 to 40 aromatic ring atoms, each of which has radicals R⁴, or a diarylamino, arylheteroarylamino or diheteroarylamino group having 5 to 60 aromatic ring atoms in the respective aromatic or heteroaromatic radical, wherein the diarylamino, arylheteroarylamino or diheteroarylamino group has radicals R⁴; two radicals R² or a radical R² together with a further radical, preferably a group R, R¹, R³ can also form an aromatic, heteroaromatic, aliphatic or heteroaliphatic ring system, preferably an aliphatic, heteroaliphatic or heteroaromatic ring system, it is particularly preferred that the radicals R² do not form such a ring system;
R³ is in each occurrence the same or different H, D, F, Cl, Br, I, R⁴C=C(R⁴)₂, N(R⁴)₂, N(Ar')₂^{,} CN, NO₂, OR⁴, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, a straight-chain alkyl, alkoxy or thioalkyl group having 1 to 20 carbon atoms or an alkenyl or alkynyl group having 2 to 20 carbon atoms or a branched or cyclic alkyl, alkoxy or thioalkyl group having 3 to 20 carbon atoms, wherein the alkyl, alkoxy or thioalkyl, alkenyl or alkynyl group each has radicals R⁴ and wherein one or more non-adjacent CH₂ groups are represented by R⁴C=CR⁴, C=C, Si(R⁴)₂, C=O, C=S, C=Se, C=NR⁴, - C(=O)O-, -C(=O)NR⁴-, NR⁴, P(=O)( R⁴), -O-, -S-, SO or SO₂, or an aromatic or heteroaromatic ring system with 5 to 60 aromatic ring atoms, preferably with 5 to 40 aromatic ring atoms, each of which has R⁴ radicals or an aryloxy or heteroaryloxy group having 5 to 40 aromatic ring atoms, each of which has radicals R⁴, or a diarylamino, arylheteroarylamino or diheteroarylamino group having 5 to 60 aromatic ring atoms in the respective aromatic or heteroaromatic radical, wherein the diarylamino, arylheteroarylamino or diheteroarylamino group has radicals R⁴; two radicals R³ or a radical R³ together with a further radical, preferably a group R, R¹, R² can also form an aromatic, heteroaromatic, aliphatic or heteroaliphatic ring system, preferably an aliphatic, heteroaliphatic or heteroaromatic ring system, and particularly preferred the radicals R³ do not form such a ring system;
Ar' is in each occurrence the same or different an aromatic or heteroaromatic ring system with 5 to 40 aromatic ring atoms, which has radicals R⁴, preferably an aryl group with 6 to 30 aromatic ring atoms or a heteroaryl group with 5 to 14 aromatic ring atoms, which has radicals R⁴;
R⁴ is the same or different in each occurrence H, D, F, Cl, Br, I, R⁵C=C(R⁵)₂, N(R⁵)₂, CN, NO₂, OR⁵, SR⁵, Si(R⁵)₃, B(OR⁵)₂, C(=O)R⁵, P(=O)(R⁵)₂, S(=O)R⁵, S(=O)₂R⁵, OSO₂R⁵, a straight-chain alkyl, alkoxy or thioalkyl group having 1 to 20 carbon atoms or an alkenyl or alkynyl group having 2 to 20 carbon atoms or a branched or cyclic alkyl, alkoxy or thioalkyl group having 3 to 20 carbon atoms, wherein the alkyl, alkoxy or thioalkyl, alkenyl or alkynyl group each has radicals R⁵, wherein one or more non-adjacent CH₂ groups are represented by R⁵C=CR⁵, C=C, Si(R⁵)₂, C=O, C=S, C=Se, C=NR⁵, -C(=O)O-, -C(=O)NR⁵-, NR⁵, P(=O)( R⁵), -O-, -S-, SO or SO₂, or an aromatic or heteroaromatic ring system with 5 to 40 aromatic ring atoms, each of which has R⁵ radicals, or an aryloxy or heteroaryloxy group with 5 to 40 aromatic ring atoms, each of which has radicals R⁵, or a diarylamino, arylheteroarylamino or diheteroarylamino group having 5 to 60 aromatic ring atoms in the respective aromatic or heteroaromatic radical, wherein the diarylamino, arylheteroarylamino or diheteroarylamino group has radicals R⁵; two or more radicals R⁴ can form an aromatic, heteroaromatic, aliphatic or heteroaliphatic ring system with one another, preferably an aliphatic ring system, and particularly preferred the radicals R⁴ do not form such a ring system;
R⁵ in each occurrence is identical or different H, D, F or an aliphatic, aromatic or heteroaromatic organic radical, in particular a hydrocarbon radical, having 1 to 20 carbon atoms, in which one or more H atoms can also be replaced by F, two or more radicals R⁵ can form a ring system with one another;
wherein at least one of the radicals R, R¹, R², R³ is selected from the group consisting of a heteroaromatic ring system with 6 to 60 aromatic ring atoms, which has radicals R⁴, an aromatic ring system with 10 to 60 aromatic ring atoms, which has residues R⁴, an aryloxy group with 10 to 60 aromatic ring atoms or heteroaryloxy group with 6 to 40 aromatic ring atoms, each of which has residues R⁴, a diarylamino group having 6 to 60 aromatic ring atoms in the respective aromatic radical, an arylheteroarylamino group having 6 to 60 aromatic ring atoms in the respective aromatic or heteroaromatic radical and a diheteroarylamino group having 6 to 60 aromatic ring atoms in the respective heteroaromatic radical, wherein the diarylamino, arylheteroarylamino, diheteroarylamino group has radicals R⁴.

2. The compound according to claim 1, comprising at least one structure of formulae (I-1) to (I-7), preferably selected from the compounds of formulae (1-1) to (I-7), 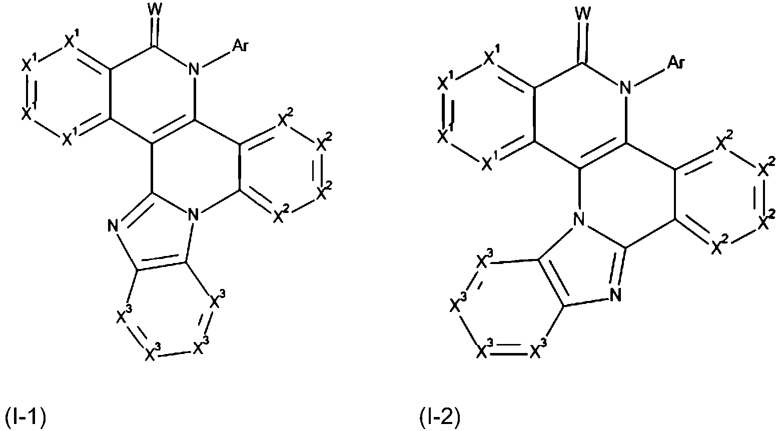 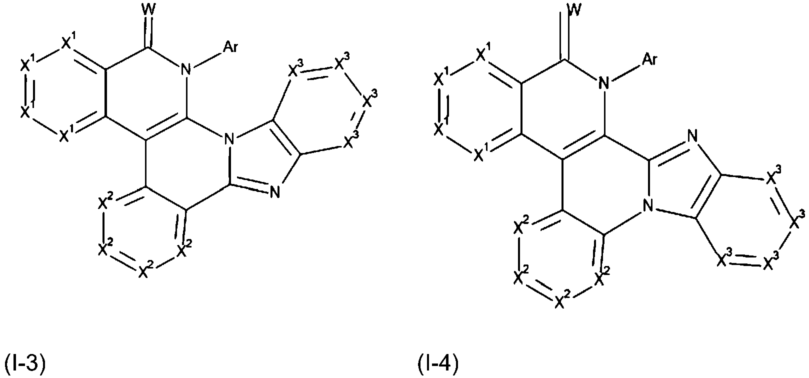 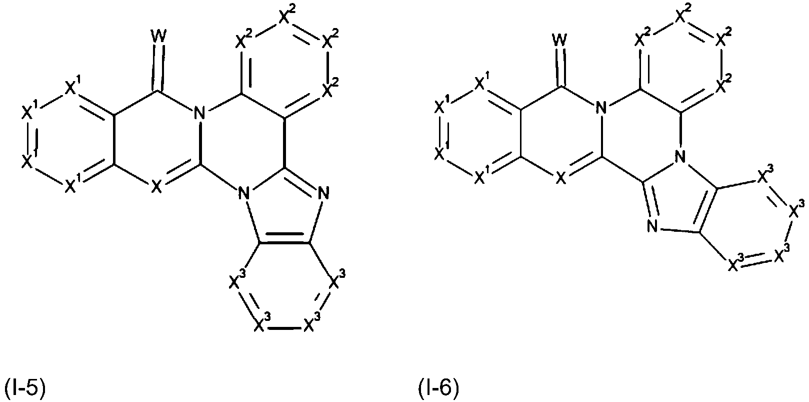 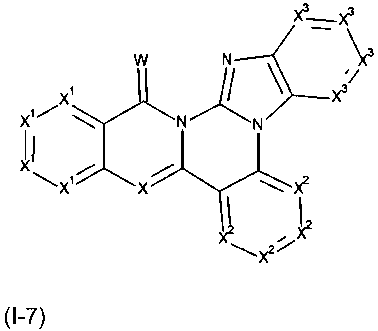 wherein symbols W, X, X¹, X², X³ and Ar, have the meanings set forth in claim 1.

3. The compound according to claim 1 or 2, comprising at least one structure of formulae (II-1) to (II-30), preferably selected from the compounds of formulae (II-1) to (II-30), 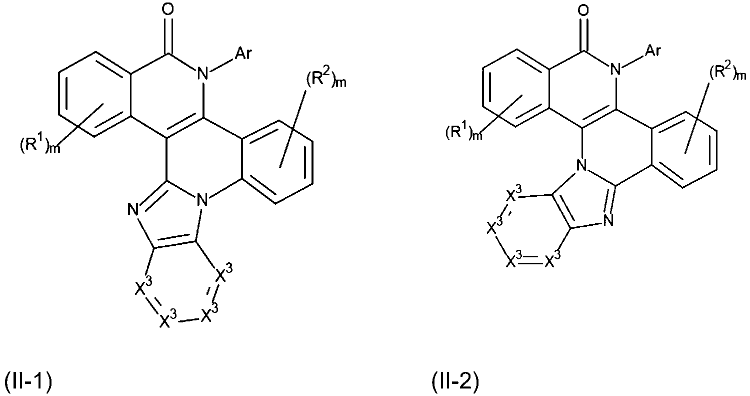 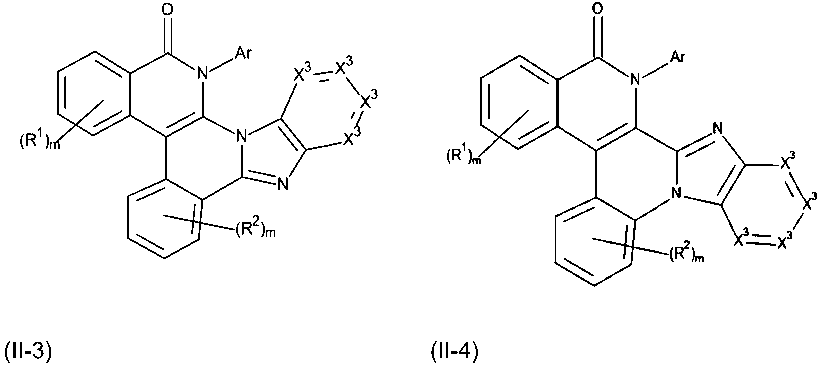 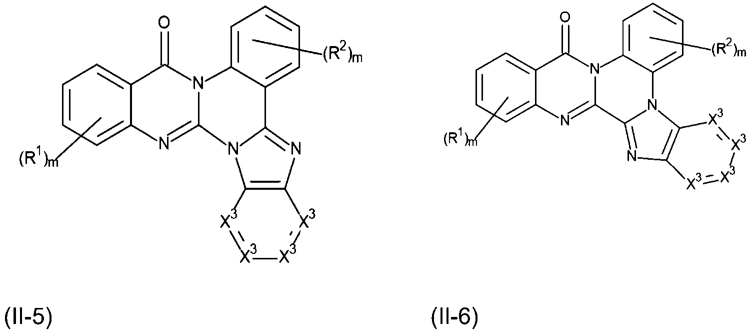 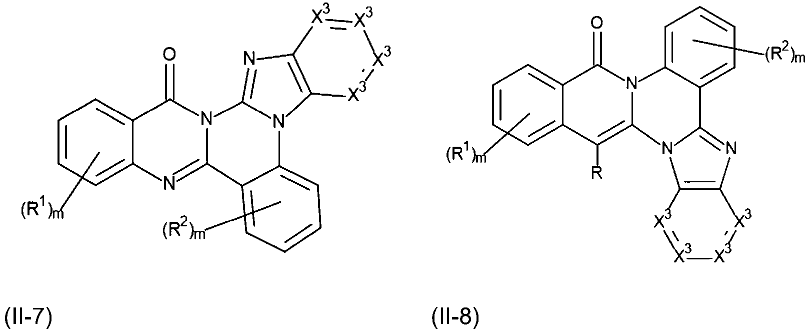 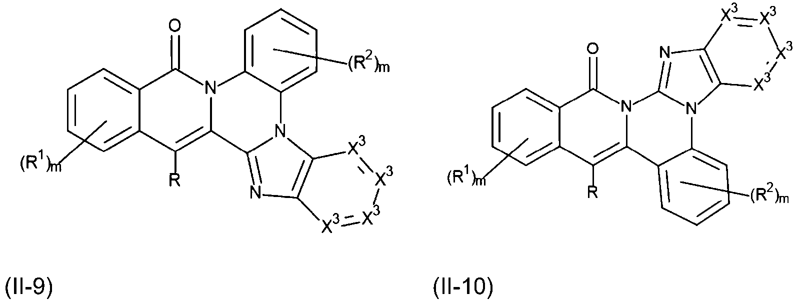 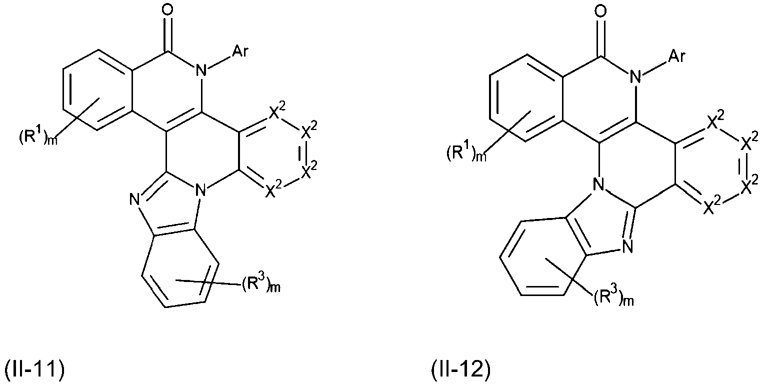 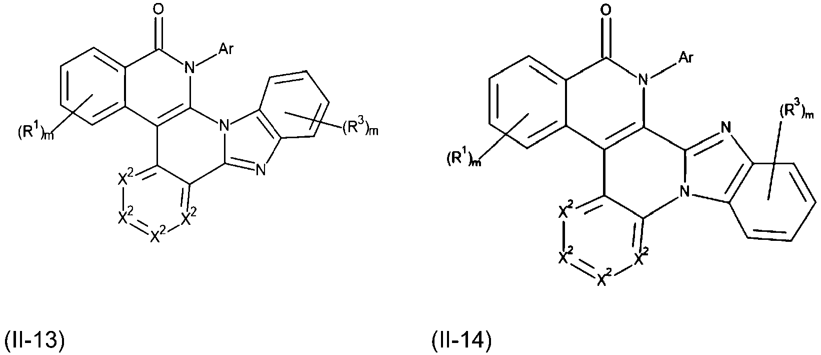 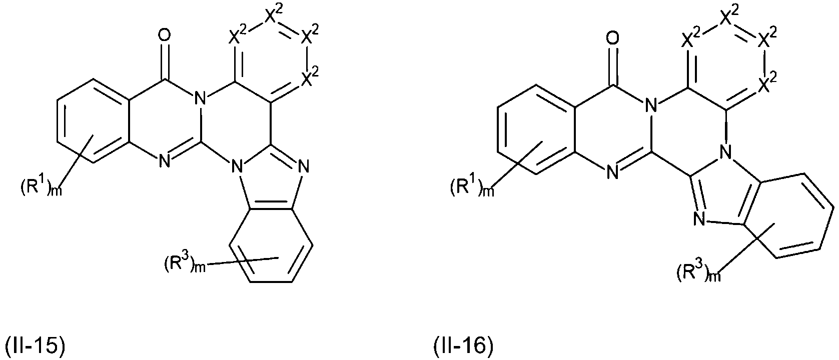 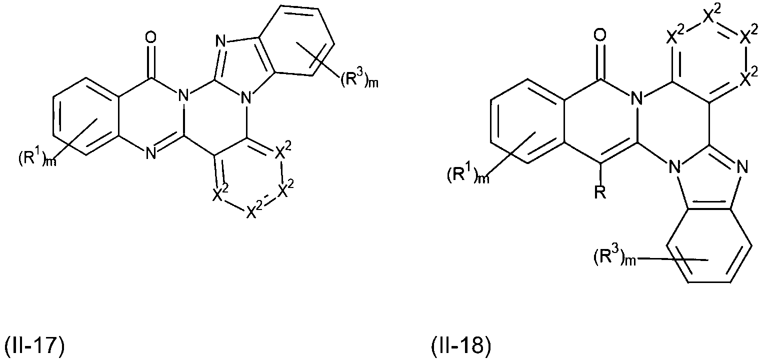 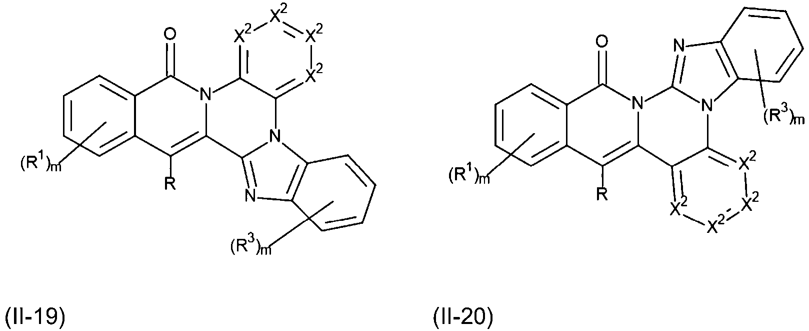 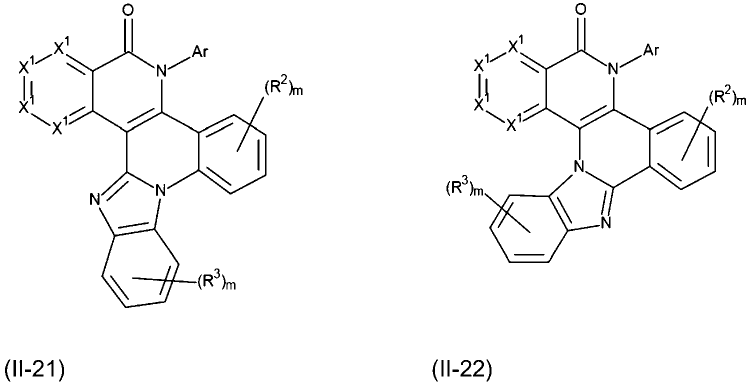 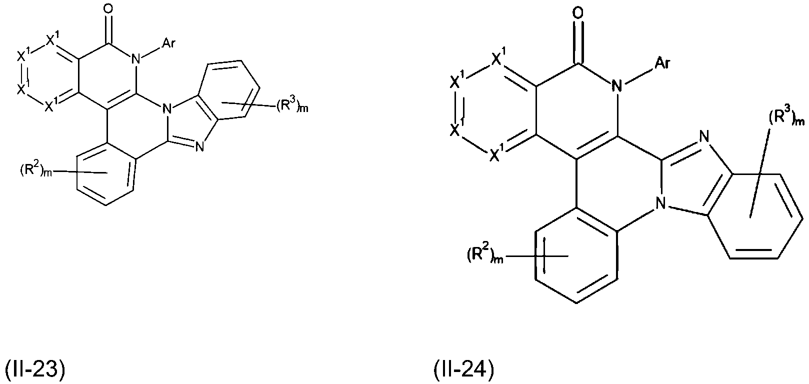 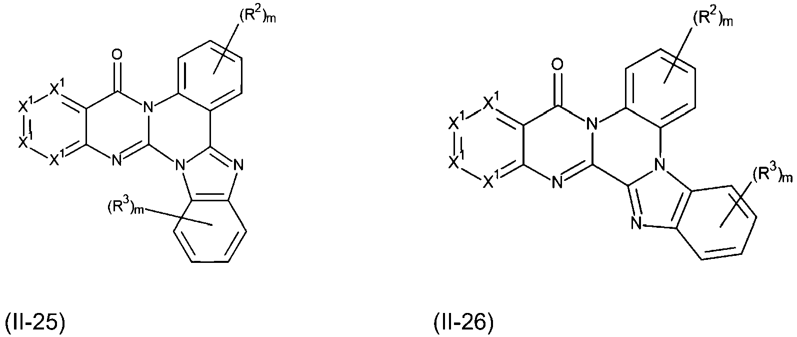 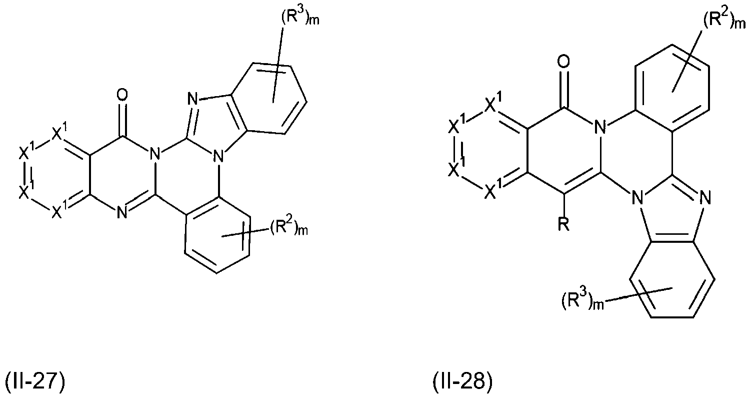 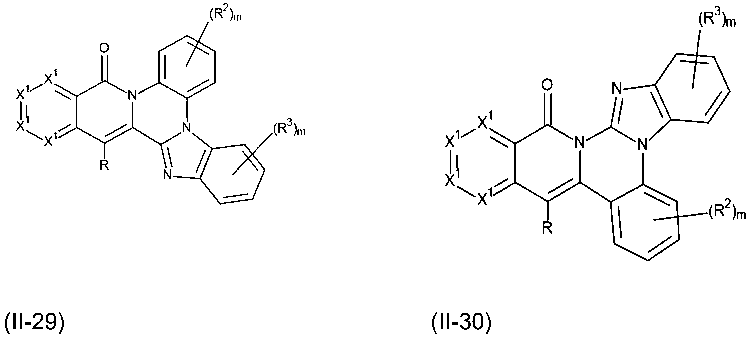 wherein the symbols X¹, X², X³, R, R¹, R², R³ and Ar, have the meanings mentioned in claim 1 and the index m is 0, 1, 2, 3 or 4, preferably 0, 1 or 2, wherein structures/compounds of the formulae (II-1) to (II-7), (II-11) to (II-17) and/or (II-21) to (II-27) are preferred.

4. The compound according to one or more of claims 1 to 3, comprising: at least one structure of formulae (III-1) to (III-10), preferably selected from the compounds of formulae (III-1) to (III-10), 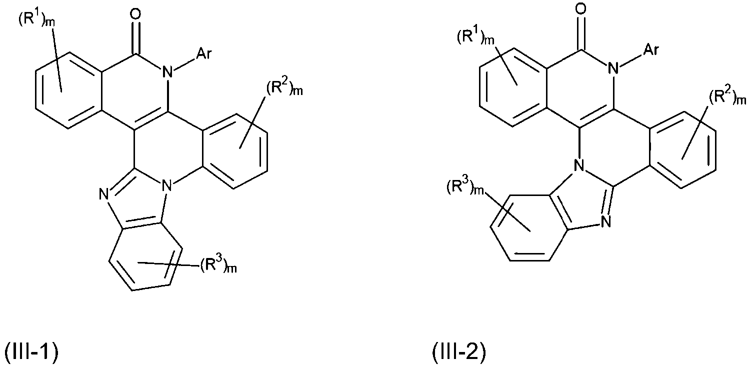 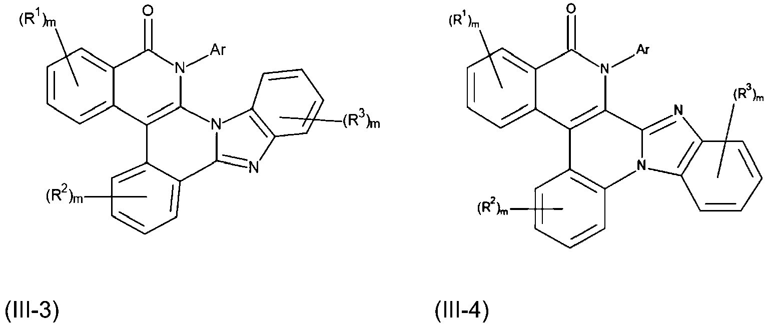 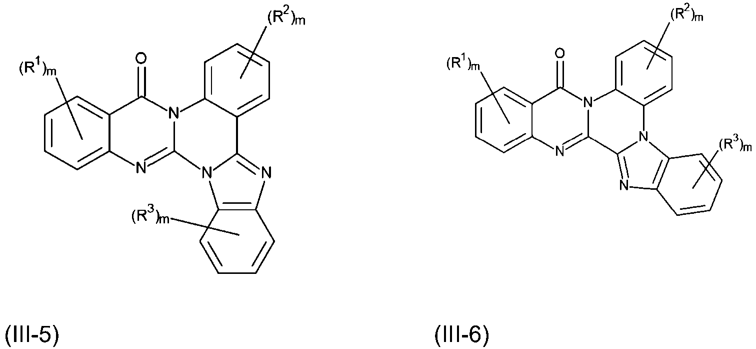 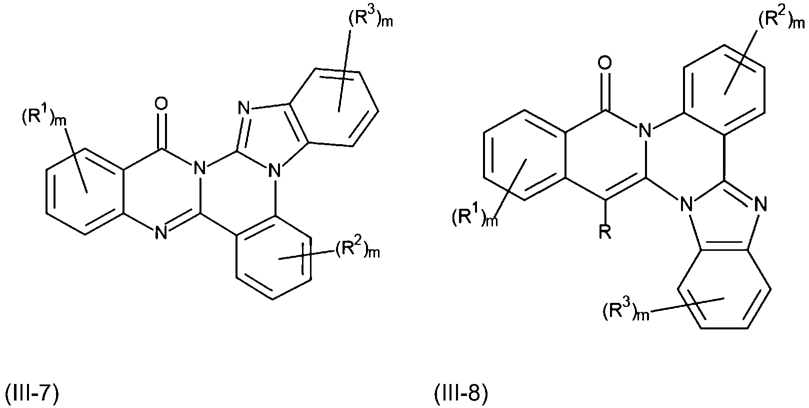 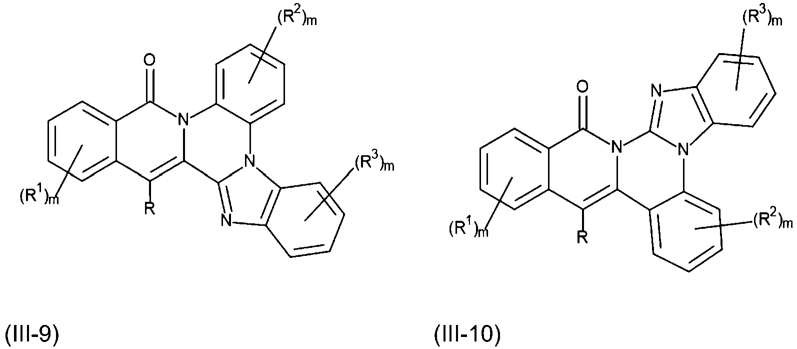 wherein the symbols R, R¹, R², R³ and Ar have the meanings given in claim 1 and the index m is 0, 1, 2, 3 or 4, preferably 0, 1 or 2, wherein structures/compounds of formulae (III-1) to (III-7) are preferred.

5. The compound according to one or more of claims 1 to 4, in that at least one group R, R¹, R² and/or R³ is selected, identically or differently, from the radicals of the following formulae SAr-1 to SAr-18 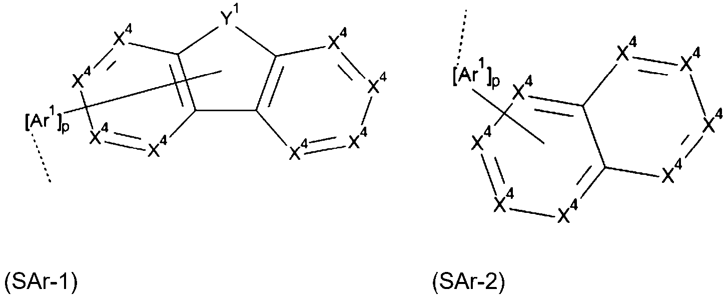 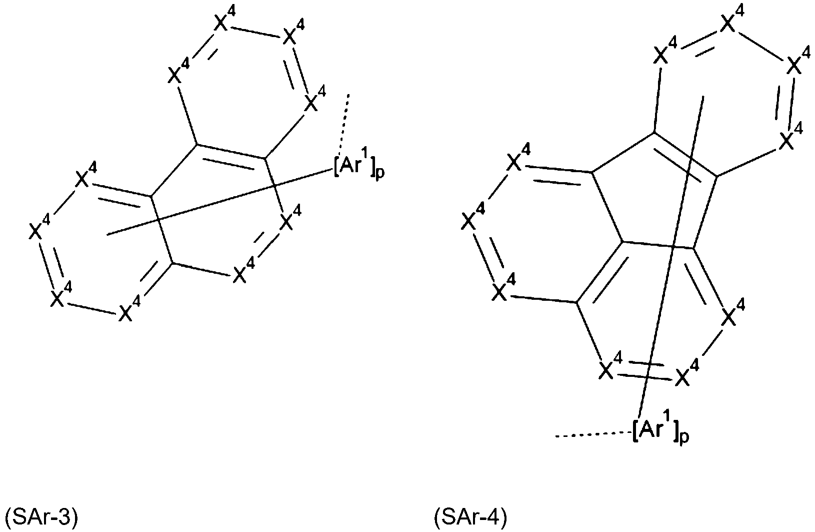 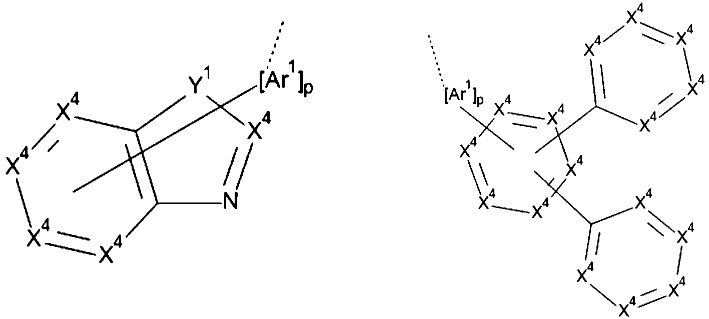 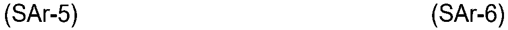 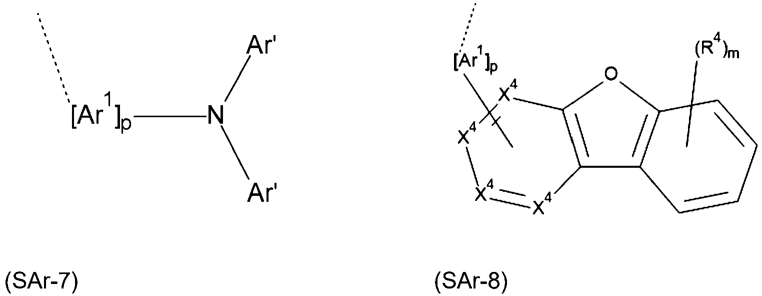 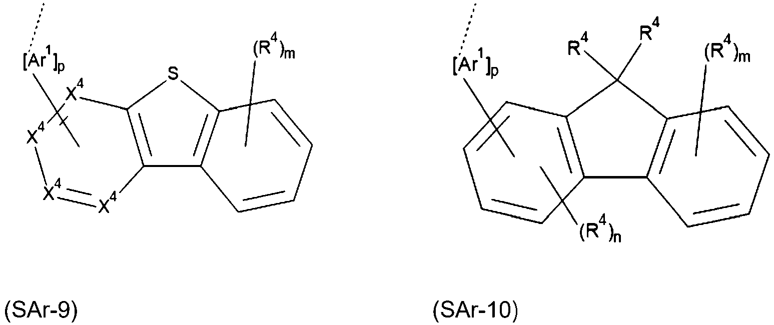 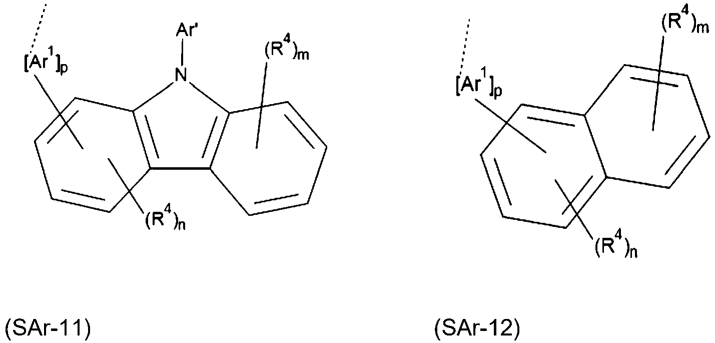 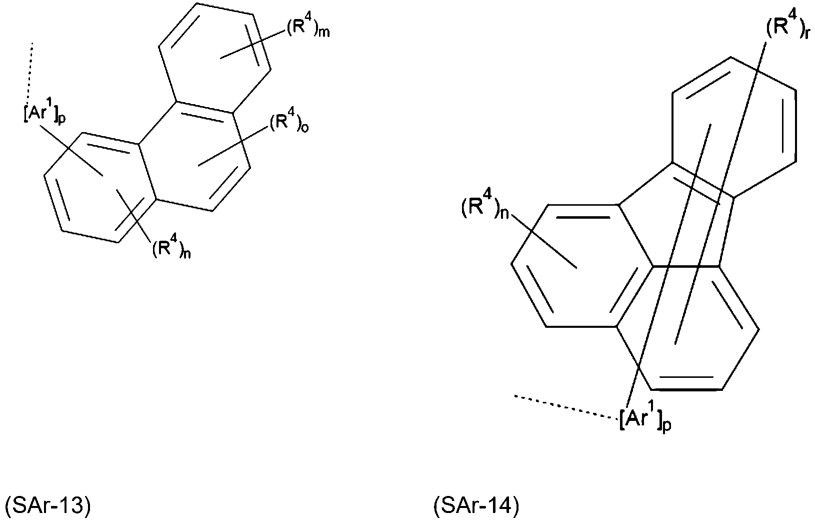 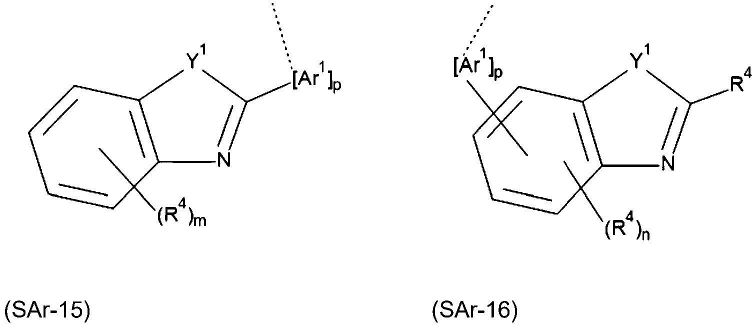 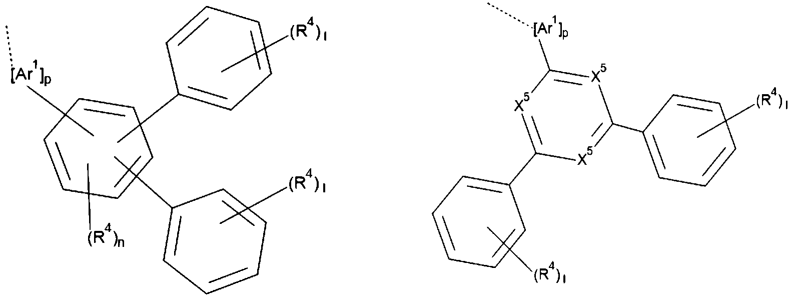 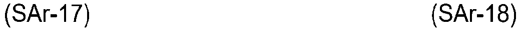 wherein R⁴ and Ar' have the meanings given in claim 1 and the following applies to the other symbols and indices:
X⁴ is in each occurrence the same or different CR⁴, N or C, if the group [Ar¹]ₚ binds thereto, preferably CR⁴, wherein preferably no N-N bonds are present;
X⁵ is the same or different in each occurrence CR⁴ or N, preferably N;
Ar¹ is, in each occurrence, identically or differently, a bivalent aromatic or heteroaromatic ring system with 6 to 18 aromatic ring atoms, each of which has R⁴ radicals;
Y¹ is in each occurrence the same or different C(R⁴)₂, NR⁴, O, S or N, if the group [Ar¹]ₚ binds to it;
p is 0 or 1, wherein p = 0 means that the group Ar¹ is not present and that the corresponding aromatic or heteroaromatic group is directly bonded to the corresponding radical;
n is 0, 1, 2 or 3, preferably 0, 1 or 2;
m is 0, 1, 2, 3 or 4, preferably 0, 1 or 2;
l is 0, 1, 2, 3, 4 or 5, preferably 0, 1 or 2;
r is 0, 1, 2, 3, 4, 5 or 6, preferably 0, 1 or 2;
wherein structures of the formulae (SAr-1), (SAr-4), (SAr-8), (SAr-11), (SAr-14), (SAr-18) are preferred.

6. The compound according to one or more of claims 1 to 9, **characterised in that** R, R¹, R² and/or R³ are the same or different at each occurrence and are selected from the group consisting of H, D or an aromatic or heteroaromatic ring system selected from the groups of the following formulae Ar-1 to Ar-79, preferably at least one group R, R¹, R² and/or R³ is selected, identically or differently at each occurrence, from the groups of the following formulae Ar-1 to Ar-79 and/or the group Ar is selected, identically or differently at each occurrence, from the groups of the following formulae Ar-1 to Ar-79 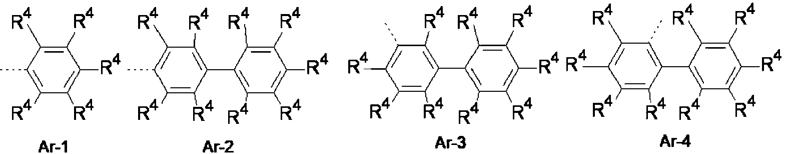 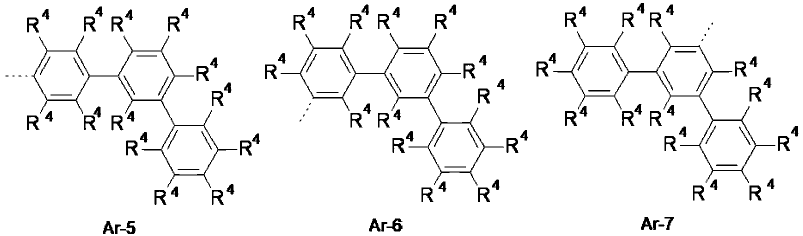 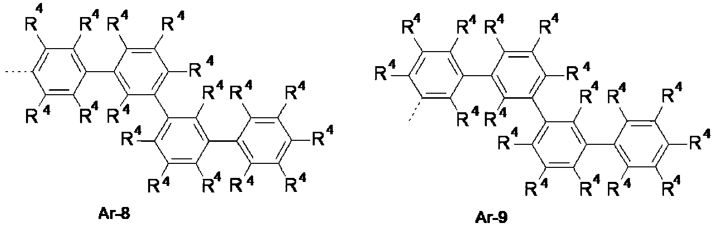 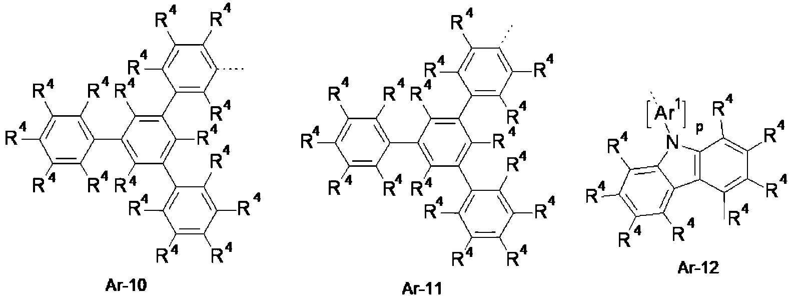 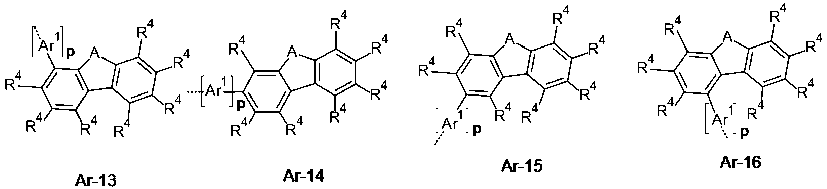 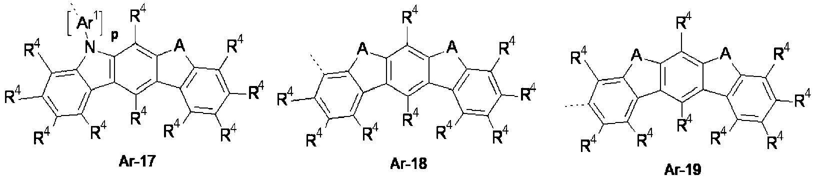 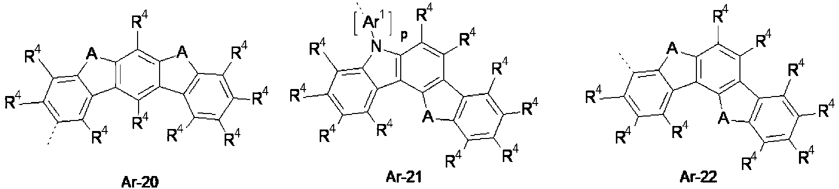 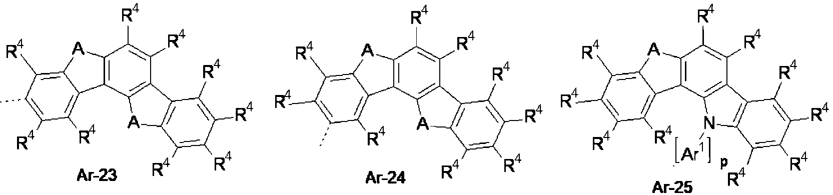 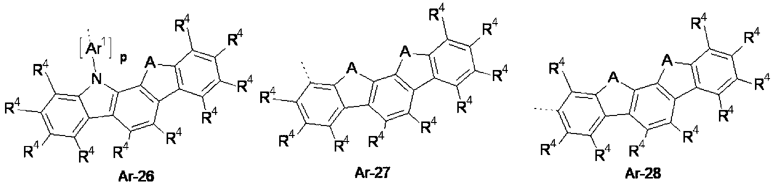 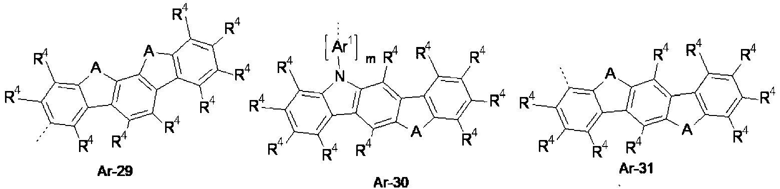 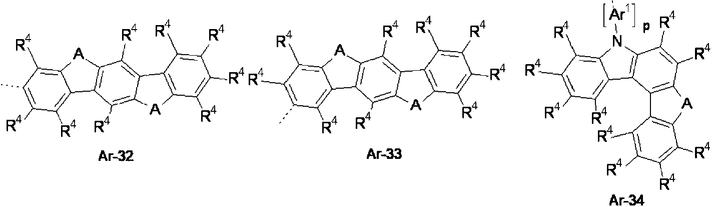 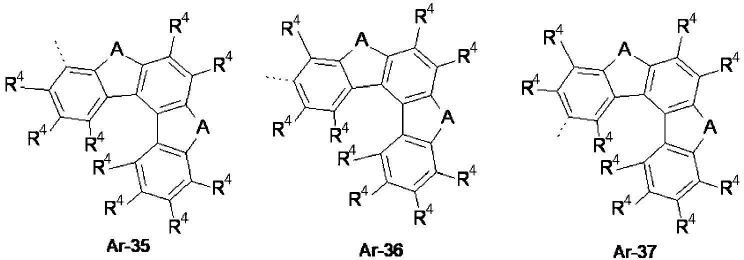 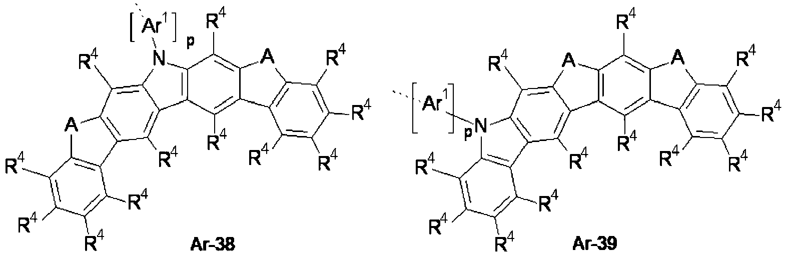 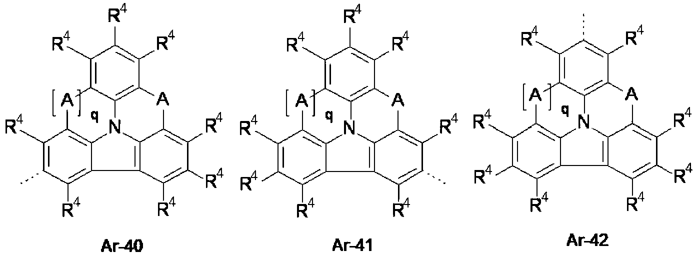 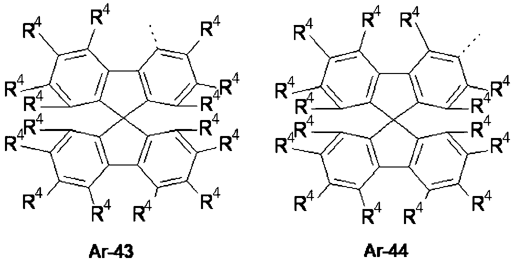 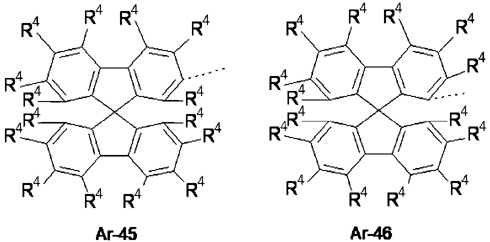 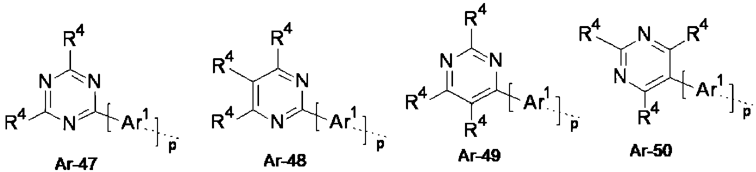 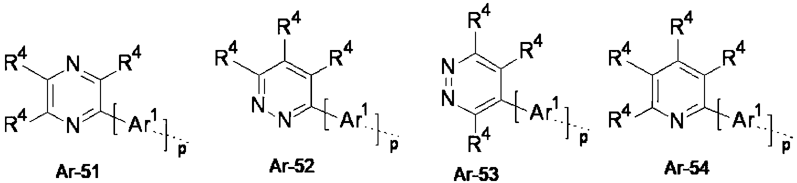 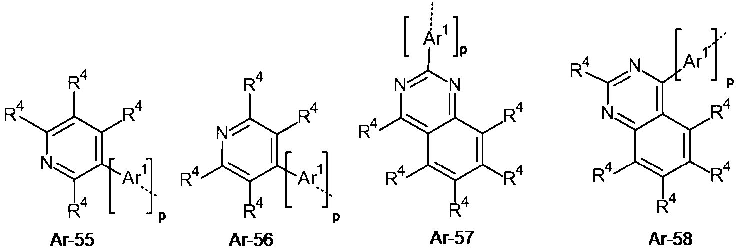 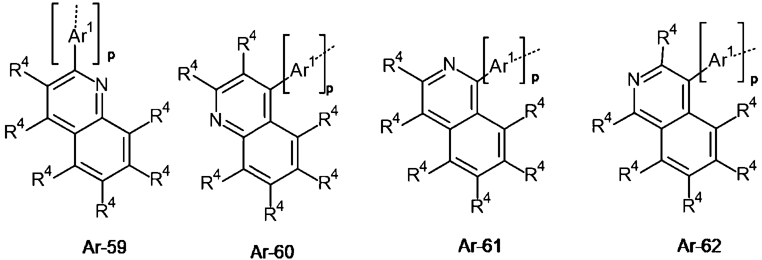 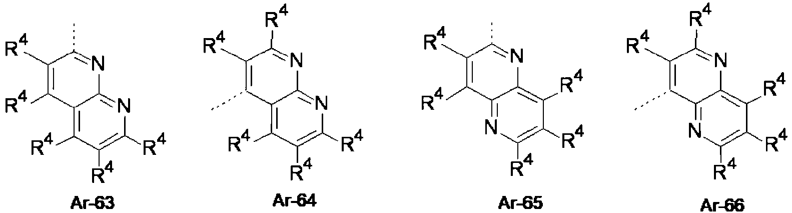 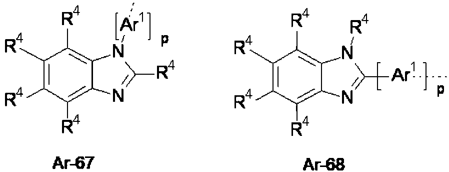 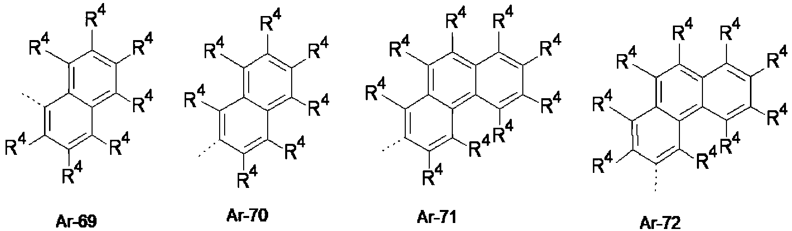 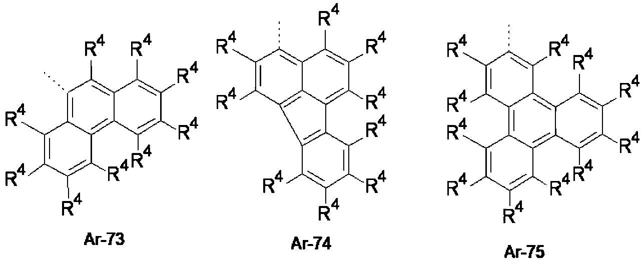 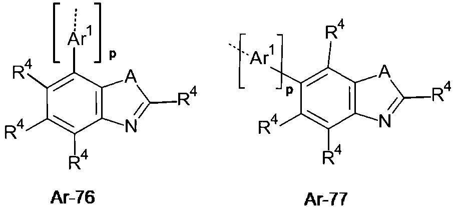 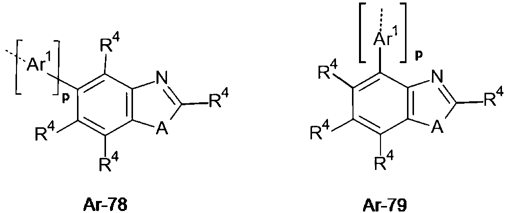 wherein R⁴ is as defined above, the dotted bond represents the bond to the corresponding group, and further wherein
Ar¹ is in each occurrence the same or different a bivalent aromatic or heteroaromatic ring system with 6 to 18 aromatic ring atoms, each of which has R⁴ radicals;
A is C(R⁴)₂, NR⁴, O or S, the same or different in each occurrence;
p is 0 or 1, wherein p = 0 means that the group Ar¹ is not present and that the corresponding aromatic or heteroaromatic group is directly bonded to the corresponding radical;
q is 0 or 1, wherein q = 0 means that no group A is bonded at this position and that residues R⁴ are bonded to the corresponding carbon atoms instead,
wherein structures of the formulae (Ar-1), (Ar-2), (Ar-3), (Ar-12), (Ar-13), (Ar-14), (Ar-15), (Ar-16), (Ar-47), (Ar-70), (Ar-75), (Ar-76), (Ar-77), (Ar-78), (Ar-79), are preferred and structures of the formulae (Ar-1), (Ar-2), (Ar-3), (Ar-12), (Ar-13), (Ar-14), (Ar-15), (Ar-16) are particularly preferred.

7. A process for the preparation of a compound according to one or more of claims 1 to 10, **characterised in that** a nitrogen-containing aromatic or heteroaromatic compound is reacted in a ring-forming reaction.

8. Composition comprising at least one compound comprising at least one structure with at least three structural elements condensed together according to formulae (A'), (B') and (C'), preferably a compound consisting of three structural elements condensed together according to formulae (A'), (B') and (C') 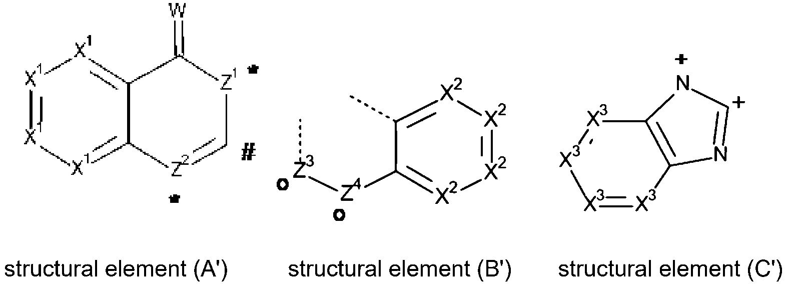
wherein the structural element (A') is condensed with structural element (B') and the structural element (B') is condensed with structural element (C'); wherein the structural element (B') binds to the structural element (A') via the bonds shown as dashed lines, wherein binding takes place via the binding site marked with # and binding takes place via a binding site marked with *; wherein the structural element (B') is condensed with the structural element (C') via the atoms labelled with o and + and the respective labelled atoms are shared by the structural elements (B') and (C'), and
applies to the symbols and indices used:
W stands for O or S, preferably for O;
Z¹ stands for NAr or N, in the event that the structural element (A') is condensed with the structural element (B') via Z¹;
Z² stands for X or C, for the case that the structural element (A') is condensed with the structural element (B') via Z²;
Z³, Z⁴ stands for N or C, wherein one of the residues Z³, Z⁴ stands for N and one of the residues Z³, Z⁴ stands for C;
X stands for N or CR, preferably for N;
X¹ stands for N or CR¹, preferably for CR¹, with the proviso that no more than two of the groups X¹ in a cycle stand for N;
X² in each occurrence is the same or different for N or CR², with the proviso that no more than two of the groups X² in a cycle are for N;
X³ is the same or different for each occurrence for N or CR³, preferably for CR³, with the proviso that no more than two of the groups X³ are in a cycle for N;
Ar is, in each occurrence, identical or different, an aromatic or heteroaromatic ring system having 5 to 40 aromatic ring atoms, which has radicals R, preferably an aryl group having 6 to 30 aromatic ring atoms or a heteroaryl group having 5 to 14 aromatic ring atoms, which has radicals R;
R in each occurrence is the same or different H, D, F, Cl, Br, I, R⁴C=C(R⁴)₂, N(R⁴)₂, N(Ar')₂^{,} CN, NO₂, OR⁴, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, a straight-chain alkyl, alkoxy or thioalkyl group having 1 to 20 carbon atoms or an alkenyl or alkynyl group having 2 to 20 carbon atoms or a branched or cyclic alkyl, alkoxy or thioalkyl group having 3 to 20 carbon atoms, wherein the alkyl, alkoxy or thioalkyl, alkenyl or alkynyl group each has radicals R⁴ and wherein one or more non-adjacent CH₂ groups are represented by R⁴C=CR⁴, C≡C, Si(R⁴)₂, C=O, C=S, C=Se, C=NR⁴, - C(=O)O-, -C(=O)NR⁴-, NR⁴, P(=O)( R⁴), -O-, -S-, SO or SO₂, or an aromatic or heteroaromatic ring system with 5 to 60 aromatic ring atoms, preferably with 5 to 40 aromatic ring atoms, each of which has R⁴ radicals or an aryloxy or heteroaryloxy group having 5 to 40 aromatic ring atoms, each of which has radicals R⁴, or a diarylamino, arylheteroarylamino or diheteroarylamino group having 5 to 60 aromatic ring atoms in the respective aromatic or heteroaromatic radical, wherein the diarylamino, arylheteroarylamino or diheteroarylamino group has radicals R⁴; a radical R can form an aliphatic, heteroaliphatic, aromatic or heteroaromatic ring system, preferably an aliphatic, heteroaliphatic or heteroaromatic ring system, with a further radical, preferably a group R¹, R², R³; particularly preferred is that the radical R does not form such a ring system;
R¹ is in each occurrence the same or different H, D, F, Cl, Br, I, R⁴C=C(R⁴)₂, N(R⁴)₂, N(Ar')₂^{,} CN, NO₂, OR⁴, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, a straight-chain alkyl, alkoxy or thioalkyl group having 1 to 20 carbon atoms or an alkenyl or alkynyl group having 2 to 20 carbon atoms or a branched or cyclic alkyl, alkoxy or thioalkyl group having 3 to 20 carbon atoms, wherein the alkyl, alkoxy or thioalkyl, alkenyl or alkynyl group each has radicals R⁴ and wherein one or more non-adjacent CH₂ groups are represented by R⁴C=CR⁴, C=C, Si(R⁴)₂, C=O, C=S, C=Se, C=NR⁴, - C(=O)O-, -C(=O)NR⁴-, NR⁴, P(=O)( R⁴), -O-, -S-, SO or SO₂, or an aromatic or heteroaromatic ring system with 5 to 60 aromatic ring atoms, preferably with 5 to 40 aromatic ring atoms, each of which has R⁴ radicals or an aryloxy or heteroaryloxy group having 5 to 40 aromatic ring atoms, each of which has radicals R⁴, or a diarylamino, arylheteroarylamino or diheteroarylamino group having 5 to 60 aromatic ring atoms in the respective aromatic or heteroaromatic radical, wherein the diarylamino, arylheteroarylamino or diheteroarylamino group has radicals R⁴; two radicals R¹ or a radical R¹ together with a further radical, preferably a group R, R², R³ can also form an aromatic, heteroaromatic, aliphatic or heteroaliphatic ring system, preferably an aliphatic, heteroaliphatic or heteroaromatic ring system, it being particularly preferred that the radicals R¹ do not form such a ring system;
R² is in each occurrence the same or different H, D, F, Cl, Br, I, R⁴C=C(R⁴)₂, N(R⁴)₂, N(Ar')₂^{,} CN, NO₂, OR⁴, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, a straight-chain alkyl, alkoxy or thioalkyl group having 1 to 20 carbon atoms or an alkenyl or alkynyl group having 2 to 20 carbon atoms or a branched or cyclic alkyl, alkoxy or thioalkyl group having 3 to 20 carbon atoms, wherein the alkyl, alkoxy or thioalkyl, alkenyl or alkynyl group each has radicals R⁴ and wherein one or more non-adjacent CH₂ groups are represented by R⁴C=CR⁴, C≡C, Si(R⁴)₂, C=O, C=S, C=Se, C=NR⁴, - C(=O)O-, -C(=O)NR⁴-, NR⁴, P(=O)( R⁴), -O-, -S-, SO or SO₂, or an aromatic or heteroaromatic ring system with 5 to 60 aromatic ring atoms, preferably with 5 to 40 aromatic ring atoms, each of which has R⁴ radicals or an aryloxy or heteroaryloxy group having 5 to 40 aromatic ring atoms, each of which has radicals R⁴, or a diarylamino, arylheteroarylamino or diheteroarylamino group having 5 to 60 aromatic ring atoms in the respective aromatic or heteroaromatic radical, wherein the diarylamino, arylheteroarylamino or diheteroarylamino group has radicals R⁴; two radicals R² or a radical R² together with a further radical, preferably a group R, R¹, R³ can also form an aromatic, heteroaromatic, aliphatic or heteroaliphatic ring system, preferably an aliphatic, heteroaliphatic or heteroaromatic ring system, it is particularly preferred that the radicals R² do not form such a ring system;
R³ is in each occurrence the same or different H, D, F, Cl, Br, I, R⁴C=C(R⁴)₂, N(R⁴)₂, N(Ar')₂^{,} CN, NO₂, OR⁴, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, a straight-chain alkyl, alkoxy or thioalkyl group having 1 to 20 carbon atoms or an alkenyl or alkynyl group having 2 to 20 carbon atoms or a branched or cyclic alkyl, alkoxy or thioalkyl group having 3 to 20 carbon atoms, wherein the alkyl, alkoxy or thioalkyl, alkenyl or alkynyl group each has radicals R⁴ and wherein one or more non-adjacent CH₂ groups are represented by R⁴C=CR⁴, C≡C, Si(R⁴)₂, C=O, C=S, C=Se, C=NR⁴, - C(=O)O-, -C(=O)NR⁴-, NR⁴, P(=O)( R⁴), -O-, -S-, SO or SO₂, or an aromatic or heteroaromatic ring system with 5 to 60 aromatic ring atoms, preferably with 5 to 40 aromatic ring atoms, each of which has R⁴ radicals or an aryloxy or heteroaryloxy group having 5 to 40 aromatic ring atoms, each of which has radicals R⁴, or a diarylamino, arylheteroarylamino or diheteroarylamino group having 5 to 60 aromatic ring atoms in the respective aromatic or heteroaromatic radical, wherein the diarylamino, arylheteroarylamino or diheteroarylamino group has radicals R⁴; two radicals R³ or a radical R³ together with a further radical, preferably a group R, R¹, R² can also form an aromatic, heteroaromatic, aliphatic or heteroaliphatic ring system, preferably an aliphatic, heteroaliphatic or heteroaromatic ring system, and particularly preferred the radicals R³ do not form such a ring system;
Ar' is in each occurrence the same or different an aromatic or heteroaromatic ring system with 5 to 40 aromatic ring atoms, which has radicals R⁴, preferably an aryl group with 6 to 30 aromatic ring atoms or a heteroaryl group with 5 to 14 aromatic ring atoms, which has radicals R⁴;
R⁴ is the same or different in each occurrence H, D, F, Cl, Br, I, R⁵C=C(R⁵)₂, N(R⁵)₂, CN, NO₂, OR⁵, SR⁵, Si(R⁵)₃, B(OR⁵)₂, C(=O)R⁵, P(=O)(R⁵)₂, S(=O)R⁵, S(=O)₂R⁵, OSO₂R⁵, a straight-chain alkyl, alkoxy or thioalkyl group having 1 to 20 carbon atoms or an alkenyl or alkynyl group having 2 to 20 carbon atoms or a branched or cyclic alkyl, alkoxy or thioalkyl group having 3 to 20 carbon atoms, wherein the alkyl, alkoxy or thioalkyl, alkenyl or alkynyl group each has radicals R⁵, wherein one or more non-adjacent CH₂ groups are represented by R⁵C=CR⁵, C=C, Si(R⁵)₂, C=O, C=S, C=Se, C=NR⁵, -C(=O)O-, -C(=O)NR⁵-, NR⁵, P(=O)( R⁵), -O-, -S-, SO or SO₂, or an aromatic or heteroaromatic ring system with 5 to 40 aromatic ring atoms, each of which has R⁵ radicals, or an aryloxy or heteroaryloxy group with 5 to 40 aromatic ring atoms, each of which has radicals R⁵, or a diarylamino, arylheteroarylamino or diheteroarylamino group having 5 to 60 aromatic ring atoms in the respective aromatic or heteroaromatic radical, wherein the diarylamino, arylheteroarylamino or diheteroarylamino group has radicals R⁵; two or more radicals R⁴ can form an aromatic, heteroaromatic, aliphatic or heteroaliphatic ring system with one another, preferably an aliphatic ring system, and particularly preferred the radicals R⁴ do not form such a ring system;
R⁵ is in each occurrence the same or different H, D, F or an aliphatic, aromatic or heteroaromatic organic radical, in particular a hydrocarbon radical, with 1 to 20 carbon atoms, in which one or more H atoms can also be replaced by F, two or more radicals R⁵ can form a ring system with one another;
and
at least one further matrix material, wherein the further matrix material is selected from compounds according to one of the formulae (H-1), (H-2), (H-3), (H-4) or (H-5), 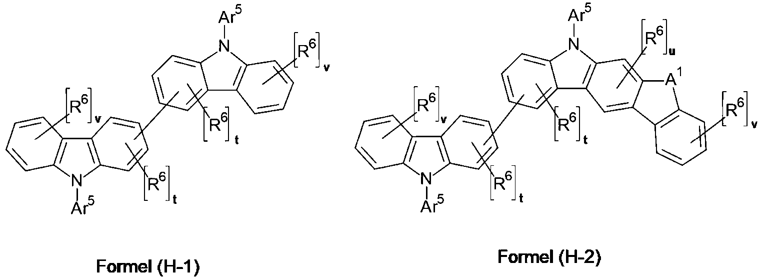 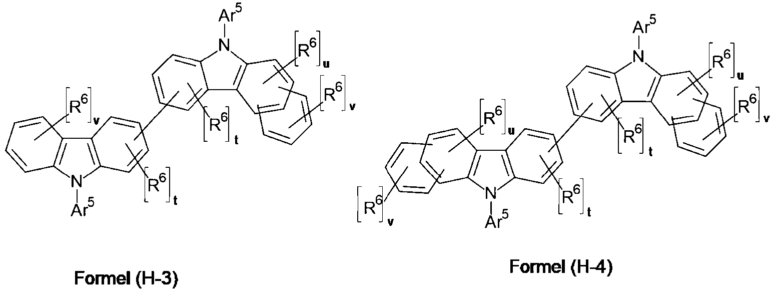 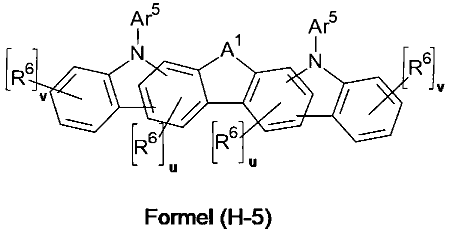
wherein the symbols and indices used are
R⁶ is the same or different for each occurrence H, D, F, Cl, Br, I, N(R⁷)₂, N(Ar")₂, CN, NO₂, OR⁷, SR⁷, COOR⁷, C(=O)N(R⁷)₂, Si(R⁷)₃, B(OR⁷)₂, C(=O)R⁷, P(=O)(R⁷)₂, S(=O)R⁷, S(=O)₂R⁷, OSO₂R⁷, a straight-chain alkyl group with 1 to 20 C atoms or an alkenyl or alkynyl group with 2 to 20 C atoms or a branched or cyclic alkyl group with 3 to 20 C atoms, wherein the alkyl, alkenyl or alkynyl group each have R⁷ radicals and wherein one or more non-adjacent CH₂ groups are replaced by Si(R⁷)₂, C=O, NR⁷, O, S or CONR⁷, or an aromatic or heteroaromatic ring system with 5 to 60 aromatic ring atoms, preferably with 5 to 40 aromatic ring atoms, each of which has R⁷ radicals; two radicals R⁶ can also together form an aromatic, heteroaromatic, aliphatic or heteroaliphatic ring system, preferably the radicals R⁶ do not form such a ring system;
Ar" is an aromatic or heteroaromatic ring system with 5 to 40 aromatic ring atoms, which has radicals R⁷;
A¹ is C(R⁷)₂, NR⁷, O or S;
Ar⁵ is the same or different in each occurrence for an aromatic or heteroaromatic ring system with 5 to 40 aromatic ring atoms, which has residues R⁷;
R⁷ is the same or different for each occurrence H, D, F, Cl, Br, I, N(R⁸)₂, CN, NO₂, OR⁸, SR⁸, Si(R⁸)₃, B(OR⁸)₂, C(=O)R⁸, P(=O)(R⁸)₂, S(=O)R⁸, S(=O)₂R⁸, OSO₂R⁸, a straight-chain alkyl group with 1 to 20 C atoms or an alkenyl or alkynyl group with 2 to 20 C atoms or a branched or cyclic alkyl group with 3 to 20 C atoms, wherein the alkyl, alkenyl or alkynyl group each have radicals R⁸, wherein one or more non-adjacent CH₂ groups may be replaced by Si(R⁸)₂, C=O, NR⁸, O, S or CONR⁸, or an aromatic or heteroaromatic ring system with 5 to 40 aromatic ring atoms, each of which has radicals R⁸; two or more radicals R⁷ can together form an aromatic, heteroaromatic, aliphatic or heteroaliphatic ring system, preferably the radicals R⁷ do not form such a ring system;
R⁸ in each occurrence is identical or different H, D, F or an aliphatic, aromatic or heteroaromatic organic radical, in particular a hydrocarbon radical, having 1 to 20 carbon atoms, in which one or more H atoms may also be replaced by F;
v in each occurrence is equal to or different from 0, 1, 2, 3 or 4, preferably 0 or 1 and most preferably 0;
t is equal to or different from 0, 1, 2 or 3, preferably 0 or 1 and most preferred 0;
u is the same or different for each occurrence 0, 1 or 2, preferably 0 or 1 and most preferred 0.

9. The composition according to claim 8, **characterised in that** the composition consists of at least one compound comprising at least one structure with at least three structural elements fused together according to formulae (A'), (B') and (C'), and at least one compound according to one of formulae (H-1), (H-2), (H-3), (H-4) or (H-5).

10. The composition according to claim 8 or 9, **characterized in that** the compounds according to one of the formulae (H-1), (H-2), (H-3), (H-4) or (H-5) have a mass fraction in the range of 5% by weight to 90% by weight, preferably in the range of 10% by weight to 85% by weight, more preferably in the range from 20% by weight to 85% by weight, even more preferably in the range from 30% by weight to 80% by weight, most preferably in the range from 20% by weight to 60% by weight and most preferably in the range from 30% by weight to 50% by weight, based on the total composition.

11. A formulation containing at least one compound according to one or more of claims 1 to 6 and/or at least one composition according to claims 8 to 10 and at least one further compound, wherein the further compound is preferably selected from one or more solvents.

12. Use of a compound comprising at least one compound comprising at least one structure with at least three mutually condensed structural elements according to the formulae (A'), (B') and (C'), preferably a compound consisting of three mutually condensed structural elements according to the formulae (A'), (B') and (C'), 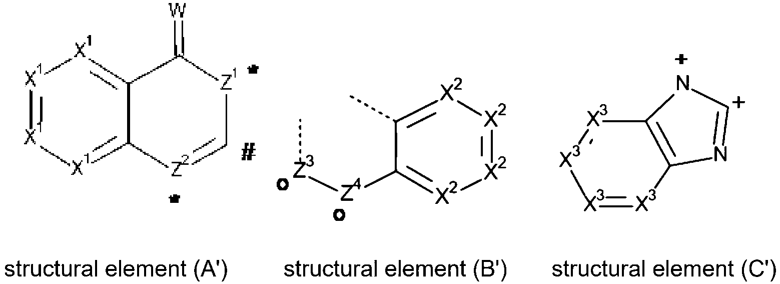
wherein the structural element (A') is condensed with structural element (B') and the structural element (B') is condensed with structural element (C'); wherein the structural element (B') binds to the structural element (A') via the bonds shown as dashed lines, wherein binding takes place via the binding site marked with # and binding takes place via a binding site marked with *; wherein the structural element (B') is condensed with the structural element (C') via the atoms marked with o and + and the respective marked atoms are shared by the structural elements (B') and (C'),
wherein the symbols and indices used have the meanings given in claim 8,
in an electronic device; and/or
use of a composition according to any one of claims 8 to 10 in an electronic device.

13. Electronic device comprising at least one compound comprising at least one structure with at least three mutually condensed structural elements according to the formulae (A'), (B') and (C'), preferably a compound consisting of three mutually condensed structural elements according to the formulae (A'), (B') and (C'), 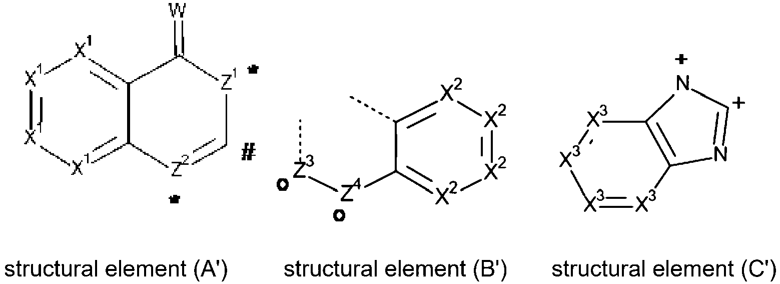
wherein the structural element (A') is condensed with structural element (B') and the structural element (B') is condensed with structural element (C'); wherein the structural element (B') binds to the structural element (A') via the bonds shown as dashed lines, wherein binding takes place via the binding site marked with # and binding takes place via a binding site marked with *; wherein the structural element (B') is condensed with the structural element (C') via the atoms marked with o and + and the respective marked atoms are shared by the structural elements (B') and (C'),
wherein the symbols and indices used have the meanings given in claim 8;
wherein the electronic device is preferably an electroluminescent device.

14. Electronic device according to claim 13, wherein it is an organic electroluminescent device, **characterised in that** the compound comprising at least one structure with at least three structural elements condensed together according to the formulae (A'), (B') and (C'), preferably compound consisting of three structural elements condensed together according to the formulae (A'), (B') and (C'), as matrix material in an emitting layer and/or in an electron transport layer and/or in a hole blocking layer, preferably as matrix material in an emitting layer and/or in an electron transport layer, particularly preferably as matrix material in an emitting layer, especially particularly preferably as matrix material in an emitting layer in combination with a red or green phosphorescent emitter.

15. Electronic device according to claim 13 or 14, **characterised in that** the compound comprising at least one structure with at least three structural elements condensed together according to the formulae (A'), (B') and (C'), preferably compound consisting of three structural elements condensed together according to the formulae (A'), (B') and (C'), is used as matrix material for phosphorescent emitters in combination with a further matrix material which is selected from compounds according to one of the formulae (H-1), (H-2), (H-3), (H-4) or (H-5), 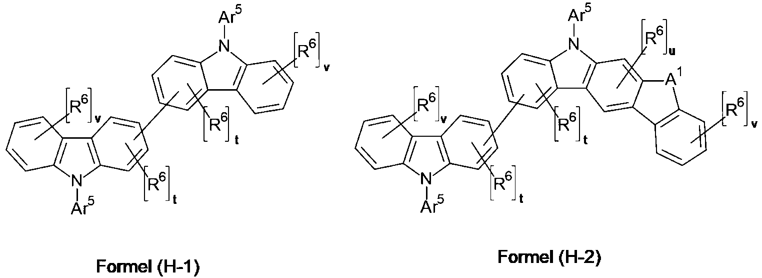 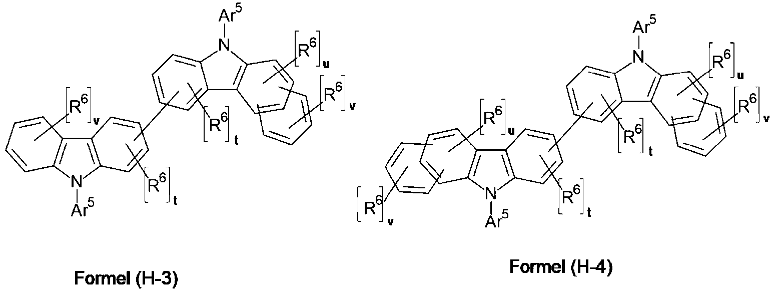 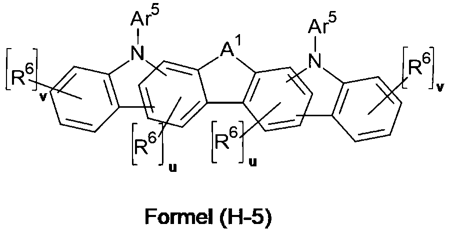 wherein the symbols A¹, Ar⁵ and R⁶ and the indices v, t and u have the meanings given in claim 8.

## Revendications

1. Composé comprenant au moins une structure ayant au moins trois éléments de structure condensés ensemble selon les formules (A), (B) et (C), de préférence composé constitué de trois éléments de structure condensés ensemble selon les formules (A), (B) et (C), 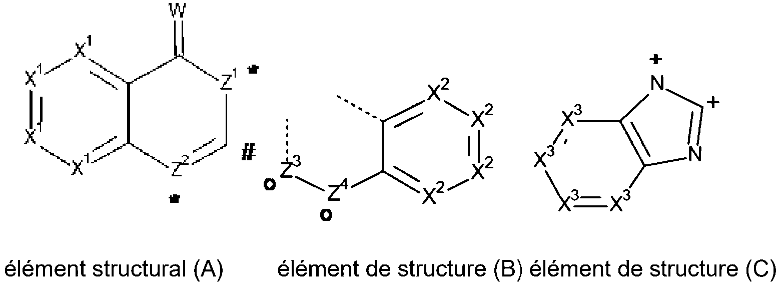
dans laquelle l'élément structural (A) est condensé avec l'élément structural (B) et l'élément structural (B) est condensé avec l'élément structural (C) ; l'élément structural (B) se liant à l'élément structural (A) par l'intermédiaire des liaisons représentées en pointillés, une liaison se faisant par le site de liaison marqué # et une liaison se faisant par un site de liaison marqué * ; dans lequel l'élément structural (B) est condensé avec l'élément structural (C) par l'intermédiaire des atomes marqués par o et +, et les atomes marqués respectivement sont partagés par les éléments structuraux (B) et (C), et
est valable pour les symboles et indices utilisés :
W représente O ou S, de préférence O ;
Z¹ représente NAr ou N, dans le cas où l'élément structural (A) est condensé avec l'élément structural (B) au-dessus de Z¹ ;
Z² représente X ou C, dans le cas où l'élément structural (A) est condensé avec l'élément structural (B) au-dessus de Z² ;
Z³, Z⁴ représente N ou C, dans lequel l'un des radicaux Z³, Z⁴ représente N et l'un des radicaux Z³, Z⁴ représente C ;
X représente N ou CR, de préférence N ;
X¹ est, à chaque occurrence, identique ou différent de N ou CR¹, de préférence CR¹, à condition que pas plus de deux des groupes X¹ dans un cycle ne soient N ;
X² est identique ou différent de N ou CR² à chaque occurrence, à condition que pas plus de deux des groupes X² dans un cycle soient N ;
X³ est identique ou différent à chaque occurrence de N ou CR³, de préférence CR³, à condition que pas plus de deux des groupes X³ dans un cycle soient N ;
Ar est, à chaque occurrence, identique ou différent, un système cyclique aromatique ou hétéroaromatique ayant de 5 à 40 atomes de cycle aromatique, qui présente des restes R, de préférence un groupe aryle ayant de 6 à 30 atomes de cycle aromatique ou un groupe hétéroaryle ayant de 5 à 14 atomes de cycle aromatique, qui présente des restes R ;
R est à chaque occurrence identique ou différente H, D, F, Cl, Br, I, R⁴C=C(R⁴)₂, N(R⁴)₂, N(Ar')₂, CN, NO₂, OR⁴, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, un groupe alkyle à chaîne droite, alcoxy ou thioalkyle ayant de 1 à 20 atomes de carbone ou un groupe alcényle ou alcynyle ayant de 2 à 20 atomes de carbone ou un groupe alkyle, alcoxy ou thioalkyle ramifié ou cyclique ayant de 3 à 20 atomes de carbone, dans lequel chaque groupe alkyle, alcoxy ou thioalkyle, alcényle ou alcynyle a des résidus R⁴ et dans lequel un ou plusieurs groupes CH₂ non adjacents sont remplacés par R⁴C=CR⁴, C≡C, Si(R⁴)₂, C=O, C=S, C=Se, C=NR⁴, - C(=O)O-, -C(=O)NR⁴-, NR⁴, P(=O)(R⁴), -O-, -S-, SO ou SO₂ peuvent être remplacés, ou un système cyclique aromatique ou hétéroaromatique ayant de 5 à 60 atomes aromatiques dans le cycle, de préférence de 5 à 40 atomes aromatiques dans le cycle, chacun ayant des résidus R⁴, ou un groupe aryloxy ou hétéroaryloxy avec 5 à 40 atomes de cycle aromatiques, qui présente respectivement des restes R⁴, ou un groupe diarylamino, arylhétéroarylamino, dihétéroarylamino avec 5 à 60 atomes de cycle aromatiques dans le reste aromatique ou hétéroaromatique respectif, dans lequel le groupe diarylamino, arylhétéroarylamino, dihétéroarylamino présente des restes R⁴ ; deux radicaux R ou un radical R avec un autre radical, de préférence un groupe R¹, R², R³ peuvent former un système cyclique aliphatique, hétéroaliphatique, aromatique ou hétéroaromatique, de préférence un système cyclique aliphatique, hétéroaliphatique ou hétéroaromatique, de manière particulièrement préférée le radical R ne forme pas un tel système cyclique ;
R¹ est à chaque occurrence identique ou différente H, D, F, Cl, Br, I, R⁴C=C(R⁴)₂, N(R⁴)₂, N(Ar')₂, CN, NO₂, OR⁴, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, un groupe alkyle à chaîne droite, alcoxy ou thioalkyle ayant de 1 à 20 atomes de carbone ou un groupe alcényle ou alcynyle ayant de 2 à 20 atomes de carbone ou un groupe alkyle, alcoxy ou thioalkyle ramifié ou cyclique ayant de 3 à 20 atomes de carbone, dans lequel chaque groupe alkyle, alcoxy ou thioalkyle, alcényle ou alcynyle a des résidus R⁴ et dans lequel un ou plusieurs groupes CH₂ non adjacents sont remplacés par R⁴C=CR⁴, C≡C, Si(R⁴)₂, C=O, C=S, C=Se, C=NR⁴, - C(=O)O-, -C(=O)NR⁴-, NR⁴, P(=O)( R⁴), -O-, -S-, SO ou SO₂ peuvent être remplacés, ou un système cyclique aromatique ou hétéroaromatique ayant de 5 à 60 atomes aromatiques dans le cycle, de préférence de 5 à 40 atomes aromatiques dans le cycle, chacun ayant des résidus R⁴, ou un groupe aryloxy ou hétéroaryloxy avec 5 à 40 atomes de cycle aromatiques, qui présente respectivement des restes R⁴, ou un groupe diarylamino, arylhétéroarylamino, dihétéroarylamino avec 5 à 60 atomes de cycle aromatiques dans le reste aromatique ou hétéroaromatique respectif, dans lequel le groupe diarylamino, arylhétéroarylamino, dihétéroarylamino présente des restes R⁴ ; deux radicaux R¹ ou un radical R¹ avec un autre radical, de préférence un groupe R, R², R³ peuvent également former ensemble un système cyclique aromatique, hétéroaromatique, aliphatique ou hétéroaliphatique, de préférence un système cyclique aliphatique, hétéroaliphatique ou hétéroaromatique, de manière particulièrement préférée les radicaux R¹ ne forment pas un tel système cyclique ;
R² est à chaque occurrence identique ou différente H, D, F, Cl, Br, I, R⁴C=C(R⁴)₂, N(R⁴)₂, N(Ar')₂, CN, NO₂, OR⁴, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, un groupe alkyle à chaîne droite, alcoxy ou thioalkyle ayant de 1 à 20 atomes de carbone ou un groupe alcényle ou alcynyle ayant de 2 à 20 atomes de carbone ou un groupe alkyle, alcoxy ou thioalkyle ramifié ou cyclique ayant de 3 à 20 atomes de carbone, dans lequel chaque groupe alkyle, alcoxy ou thioalkyle, alcényle ou alcynyle a des résidus R⁴ et dans lequel un ou plusieurs groupes CH₂ non adjacents sont remplacés par R⁴C=CR⁴, C≡C, Si(R⁴)₂, C=O, C=S, C=Se, C=NR⁴, - C(=O)O-, -C(=O)NR⁴-, NR⁴, P(=O)( R⁴), -O-, -S-, SO ou SO₂ peuvent être remplacés, ou un système cyclique aromatique ou hétéroaromatique ayant de 5 à 60 atomes aromatiques dans le cycle, de préférence de 5 à 40 atomes aromatiques dans le cycle, chacun ayant des résidus R⁴, ou un groupe aryloxy ou hétéroaryloxy avec 5 à 40 atomes de cycle aromatiques, qui présente respectivement des restes R⁴, ou un groupe diarylamino, arylhétéroarylamino, dihétéroarylamino avec 5 à 60 atomes de cycle aromatiques dans le reste aromatique ou hétéroaromatique respectif, dans lequel le groupe diarylamino, arylhétéroarylamino, dihétéroarylamino présente des restes R⁴ ; deux radicaux R² ou un radical R² avec un autre radical, de préférence un groupe R, R¹, R³ peuvent également former ensemble un système cyclique aromatique, hétéroaromatique, aliphatique ou hétéroaliphatique, de préférence un système cyclique aliphatique, hétéroaliphatique ou hétéroaromatique, de manière particulièrement préférée les radicaux R² ne forment pas un tel système cyclique ;
R³ est à chaque occurrence identique ou différente H, D, F, Cl, Br, I, R⁴C=C(R⁴)₂, N(R⁴)₂, N(Ar')₂, CN, NO₂, OR⁴, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, un groupe alkyle à chaîne droite, alcoxy ou thioalkyle ayant de 1 à 20 atomes de carbone ou un groupe alcényle ou alcynyle ayant de 2 à 20 atomes de carbone ou un groupe alkyle, alcoxy ou thioalkyle ramifié ou cyclique ayant de 3 à 20 atomes de carbone, dans lequel chaque groupe alkyle, alcoxy ou thioalkyle, alcényle ou alcynyle a des résidus R⁴ et dans lequel un ou plusieurs groupes CH₂ non adjacents sont remplacés par R⁴C=CR⁴, C≡C, Si(R⁴)₂, C=O, C=S, C=Se, C=NR⁴, - C(=O)O-, -C(=O)NR⁴-, NR⁴, P(=O)( R⁴), -O-, -S-, SO ou SO₂ peuvent être remplacés, ou un système cyclique aromatique ou hétéroaromatique ayant de 5 à 60 atomes aromatiques dans le cycle, de préférence de 5 à 40 atomes aromatiques dans le cycle, chacun ayant des résidus R⁴, ou un groupe aryloxy ou hétéroaryloxy avec 5 à 40 atomes de cycle aromatiques, qui présente respectivement des restes R⁴, ou un groupe diarylamino, arylhétéroarylamino, dihétéroarylamino avec 5 à 60 atomes de cycle aromatiques dans le reste aromatique ou hétéroaromatique respectif, dans lequel le groupe diarylamino, arylhétéroarylamino, dihétéroarylamino présente des restes R⁴ ; deux radicaux R³ ou un radical R³ avec un autre radical, de préférence un groupe R, R¹, R² peuvent également former ensemble un système cyclique aromatique, hétéroaromatique, aliphatique ou hétéroaliphatique, de préférence un système cyclique aliphatique, hétéroaliphatique ou hétéroaromatique, de manière particulièrement préférée les radicaux R³ ne forment pas un tel système cyclique ;
Ar' est, à chaque occurrence, identique ou différent, un système cyclique aromatique ou hétéroaromatique ayant de 5 à 40 atomes cycliques aromatiques, qui présente des restes R⁴, de préférence un groupe aryle ayant de 6 à 30 atomes cycliques aromatiques ou un groupe hétéroaryle ayant de 5 à 14 atomes cycliques aromatiques, qui présente des restes R⁴ ;
R⁴ est à chaque occurrence identique ou différente H, D, F, Cl, Br, I, R⁵C=C(R⁵)₂, N(R⁵)₂, CN, NO₂, OR⁵, SR⁵, Si(R⁵)₃, B(OR⁵)₂, C(=O)R⁵, P(=O)(R⁵)₂, S(=O)R⁵, S(=O)₂R⁵, OSO₂R⁵, un groupe alkyle à chaîne droite, alcoxy ou thioalkyle ayant de 1 à 20 atomes de carbone ou un groupe alcényle ou alcynyle ayant de 2 à 20 atomes de carbone ou un groupe alkyle, alcoxy ou thioalkyle ramifié ou cyclique ayant de 3 à 20 atomes de carbone, dans lequel le groupe alkyle, alcoxy ou thioalkyle, alcényle ou alcynyle a respectivement des résidus R⁵, un ou plusieurs groupes CH₂ non adjacents étant remplacés par R⁵C=CR⁵, C=C, Si(R⁵)₂, C=O, C=S, C=Se, C=NR⁵, -C(=O)O-, -C(=O)NR⁵-, NR⁵, P(=O)( R⁵), -O-, -S-, SO ou SO₂ peuvent être remplacés, ou un système cyclique aromatique ou hétéroaromatique ayant de 5 à 40 atomes de cycle aromatique, chacun ayant des résidus R⁵, ou un groupe aryloxy ou hétéroaryloxy ayant de 5 à 40 atomes de cycle aromatique, ayant chacun des résidus R⁵, ou un groupe diarylamino, arylhétéroarylamino, dihétéroarylamino ayant de 5 à 60 atomes de cycle aromatiques dans le résidu aromatique ou hétéroaromatique respectif, dans lequel le groupe diarylamino, arylhétéroarylamino, dihétéroarylamino a des résidus R⁵ ; deux ou plusieurs radicaux R⁴ peuvent former ensemble un système cyclique aromatique, hétéroaromatique, aliphatique ou hétéroaliphatique, de préférence un système cyclique aliphatique, de manière particulièrement préférée les radicaux R⁴ ne forment pas un tel système cyclique ;
R⁵ est, à chaque occurrence, identique ou différente, H, D, F ou un radical organique aliphatique, aromatique ou hétéroaromatique, en particulier un radical hydrocarboné, comportant de 1 à 20 atomes de carbone, dans lequel un ou plusieurs atomes de H peuvent également être remplacés par F, deux ou plusieurs radicaux R⁵ pouvant former ensemble un système cyclique ;
dans laquelle au moins l'un des radicaux R, R¹, R², R³ est choisi dans le groupe constitué d'un système cyclique hétéroaromatique comportant 6 à 60 atomes cycliques aromatiques, qui présente des radicaux R⁴, un système cyclique aromatique ayant de 10 à 60 atomes aromatiques dans le cycle et ayant des résidus R⁴, un groupe aryloxy ayant de 10 à 60 atomes aromatiques dans le cycle ou un groupe hétéroaryloxy ayant de 6 à 40 atomes aromatiques dans le cycle et ayant chacun des résidus R⁴, un groupe diarylamino ayant de 6 à 60 atomes de cycle aromatique dans le résidu aromatique respectif, un groupe arylhétéroarylamino ayant de 6 à 60 atomes de cycle aromatique dans le résidu aromatique ou hétéroaromatique respectif et un groupe dihétéroarylamino ayant de 6 à 60 atomes de cycle aromatique ayant de 6 à 60 atomes de cycle aromatique dans le résidu hétéroaromatique respectif, dans lequel le groupe diarylamino, arylhétéroarylamino, dihétéroarylamino a des résidus R⁴.

2. Composé selon la revendication 1, comprenant au moins une structure de formules (I-1) à (I-7), de préférence choisie parmi les composés de formules (I-1) à (I-7), 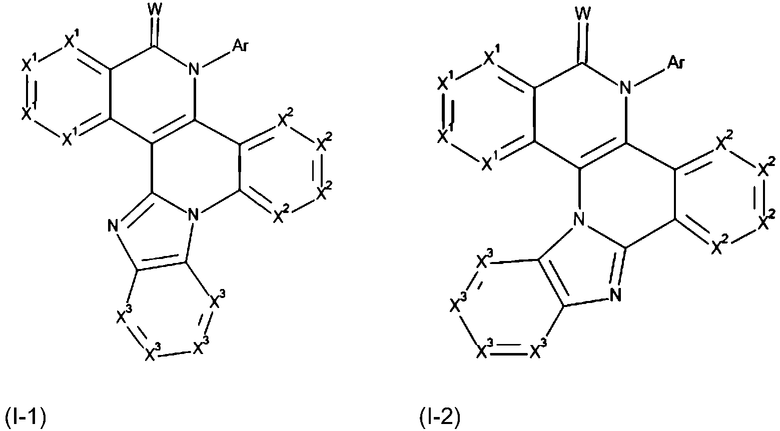 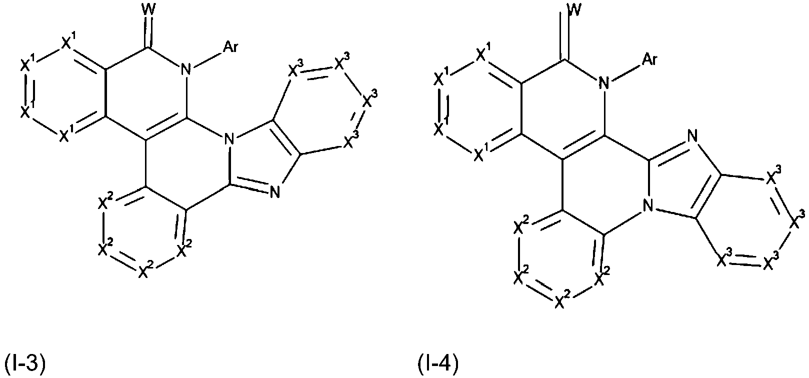 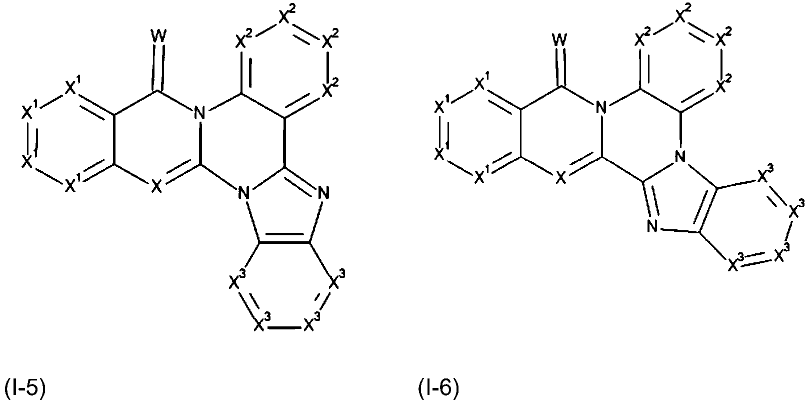 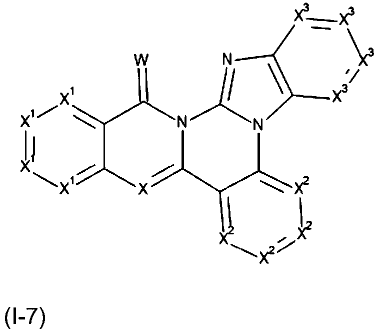 dans laquelle les symboles W, X, X¹, X², X³ et Ar, ont les significations données dans la revendication 1.

3. Composé selon la revendication 1 ou 2, comprenant au moins une structure de formules (II-1) à (II-30), de préférence choisie parmi les composés de formules (II-1) à (II-30), 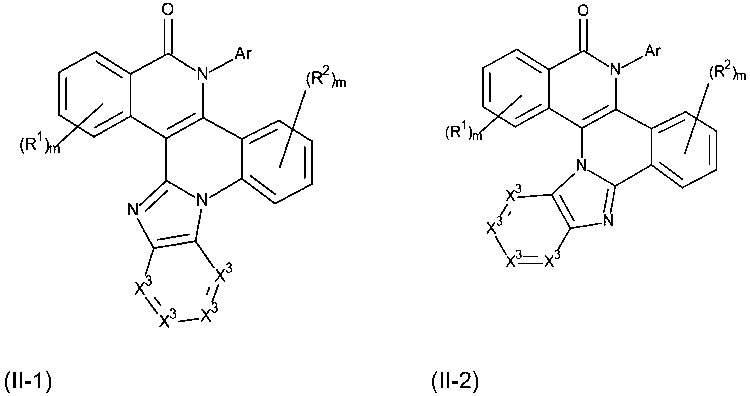 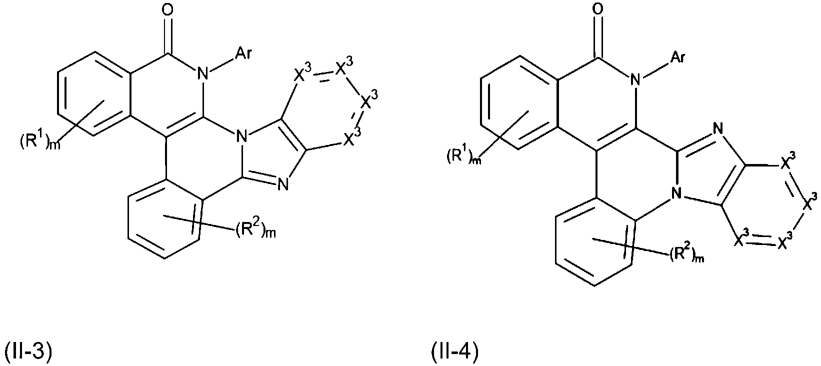 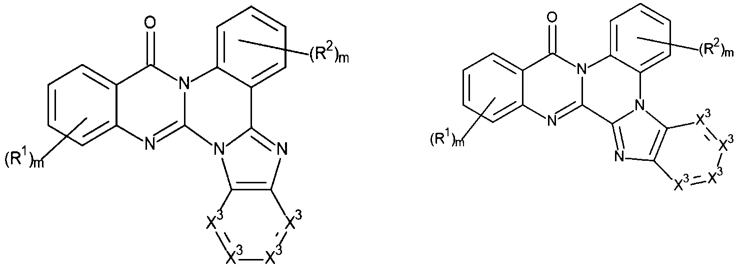 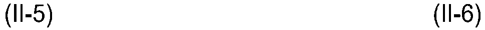 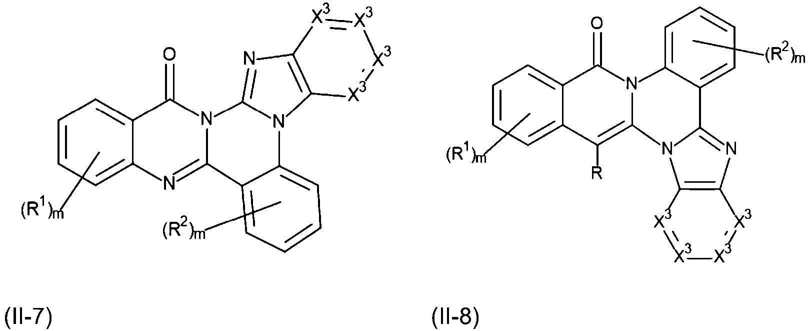 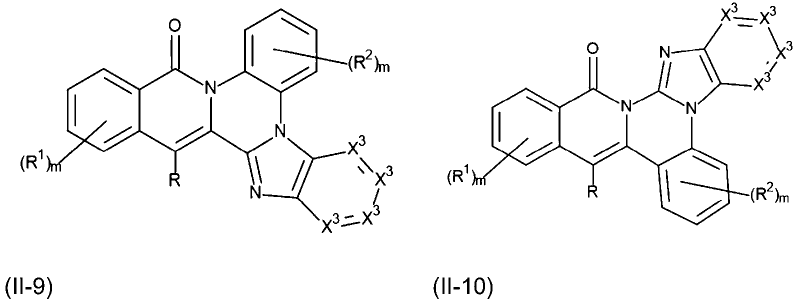 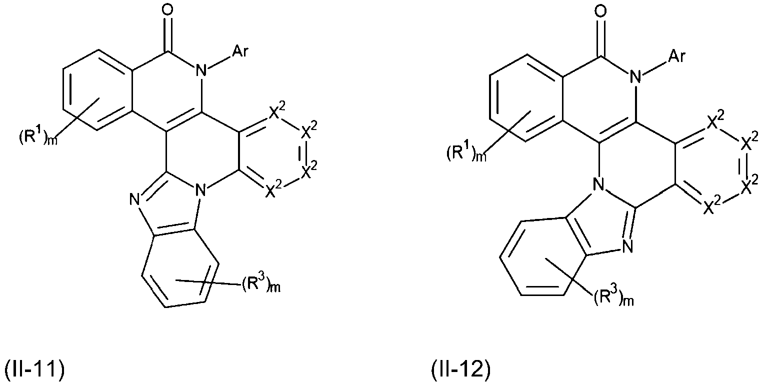 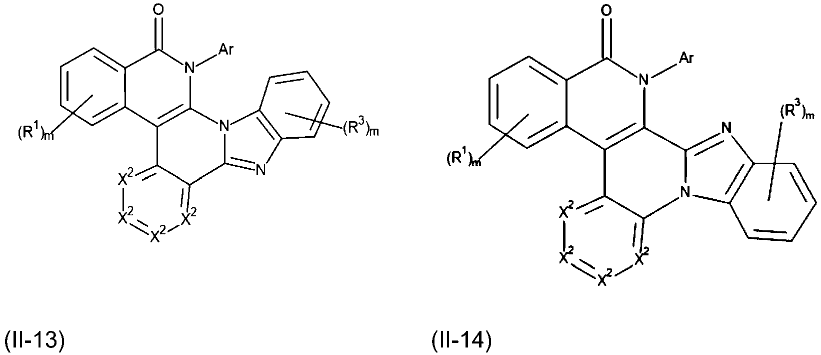 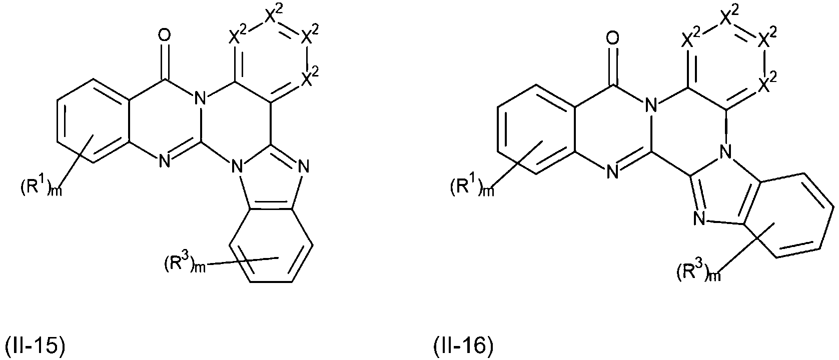 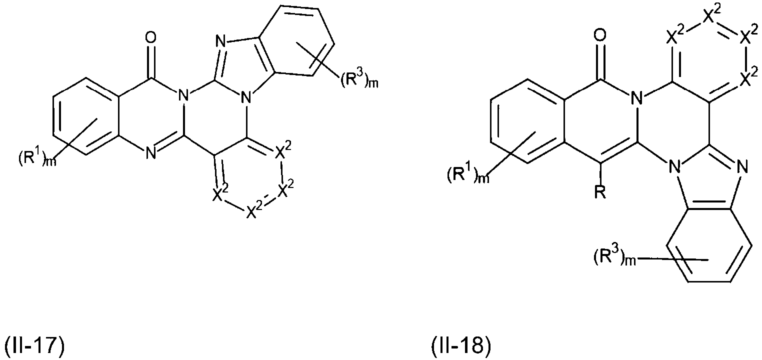 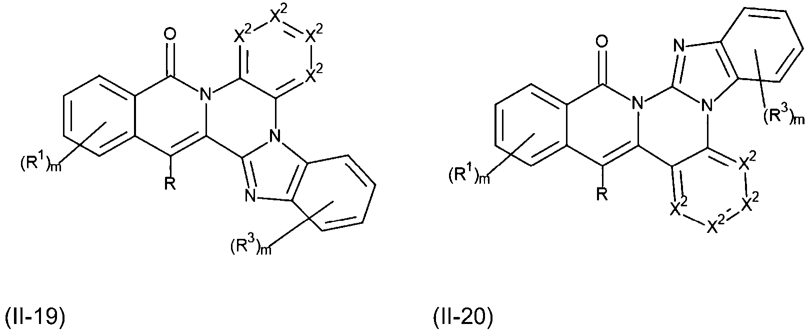 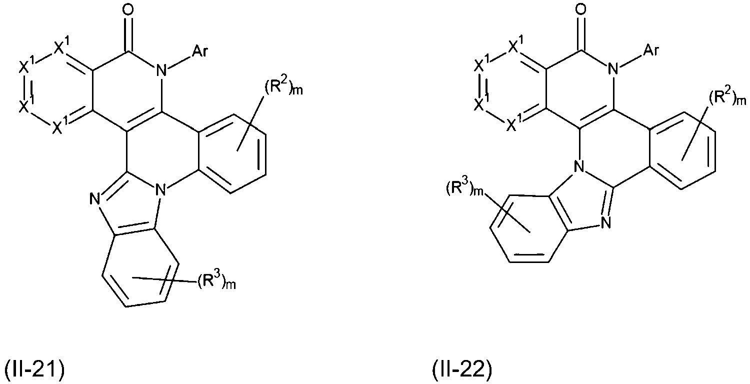 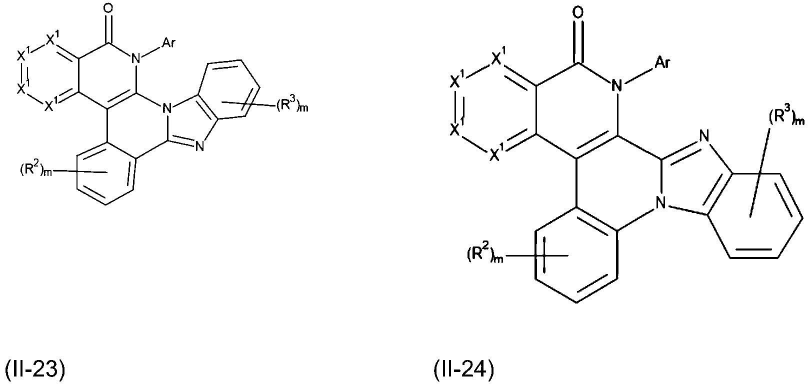 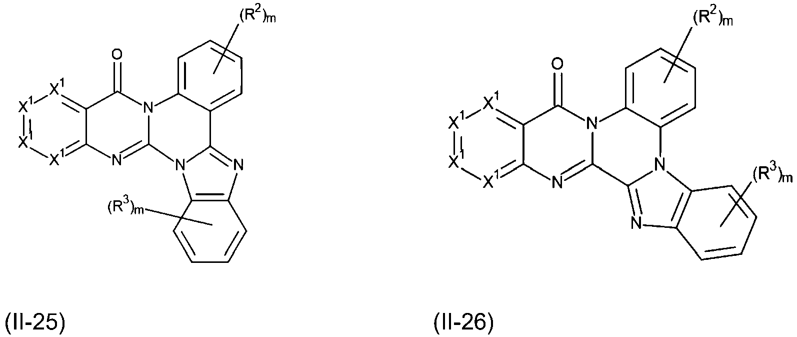 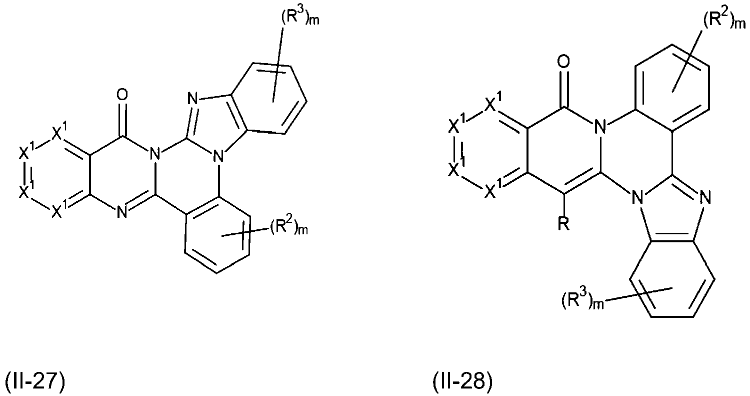 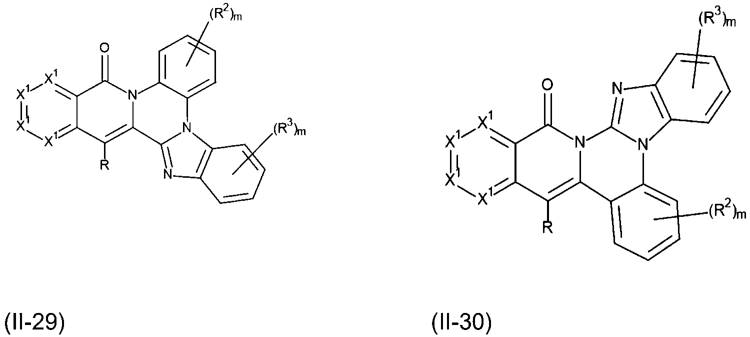 dans laquelle les symboles X¹, X², X³, R, R¹, R², R³ et Ar, ont les significations données dans la revendication 1 et l'indice m est 0, 1, 2, 3 ou 4, de préférence 0, 1 ou 2, les structures/composés de formules (II-1) à (II-7), (II-11) à (II-17) et/ou (II-21) à (II-27) étant préférés.

4. Composé selon une ou plusieurs des revendications 1 à 3, comprenant au moins une structure de formules (III-1) à (III-10), de préférence choisie parmi les composés de formules (III-1) à (III-10), 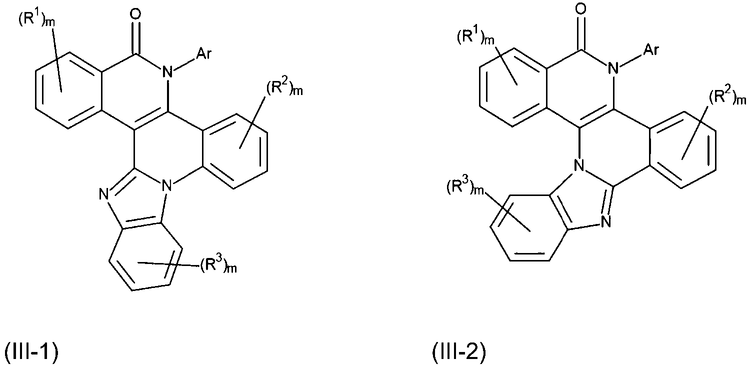 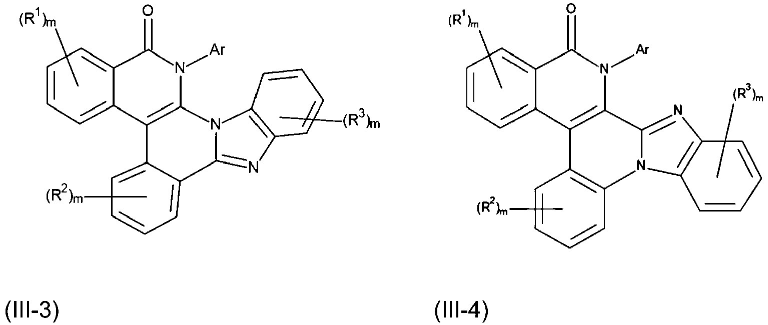 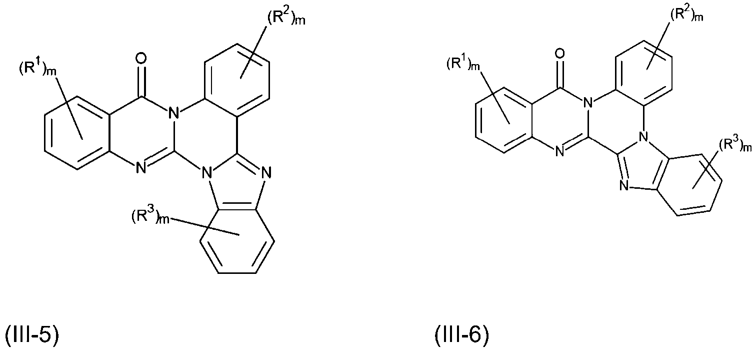 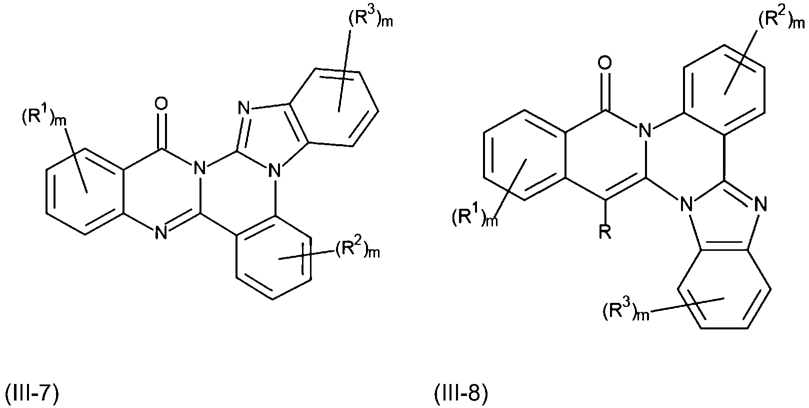 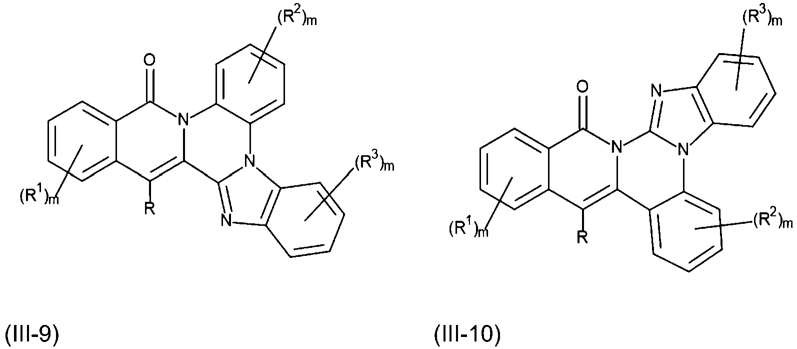 dans laquelle les symboles R, R¹, R², R³ et Ar, ont les significations données dans la revendication 1 et l'indice m est 0, 1, 2, 3 ou 4, de préférence 0, 1 ou 2, dans laquelle les structures/composés de formules (III-1) à (III-7) sont préférés.

5. Composé selon l'une ou plusieurs des revendications 1 à 4, dans lequel au moins un groupe R, R¹, R² et/ou R³, identiques ou différents, sont choisis parmi les radicaux de formules suivantes SAr-1 à SAr-18 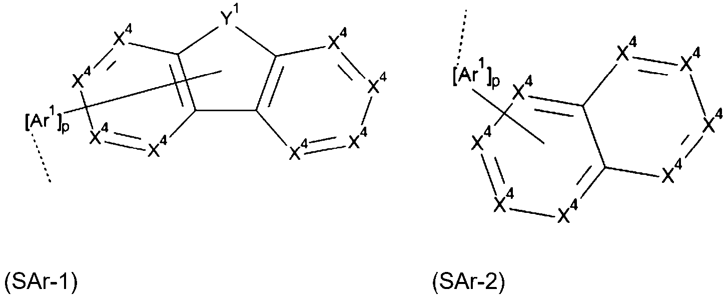 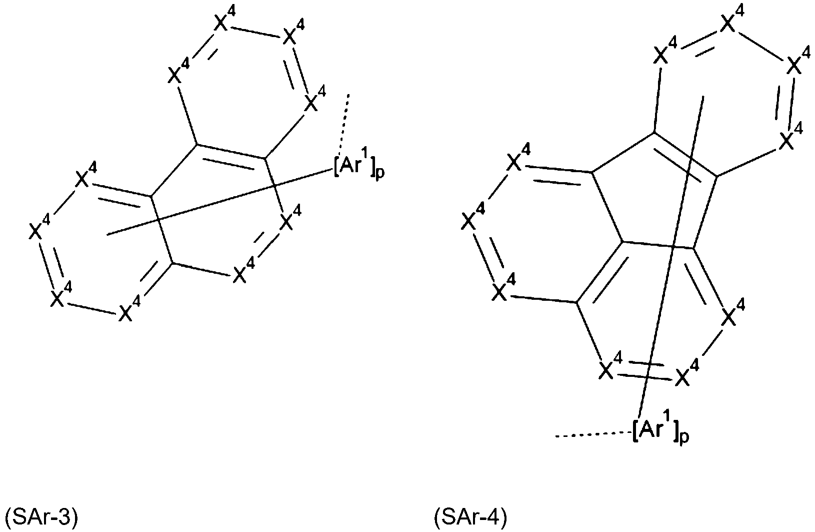 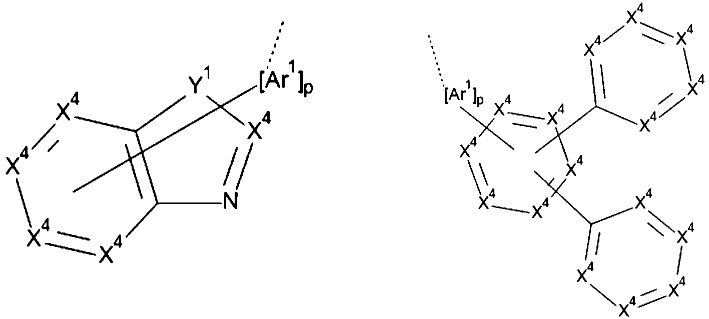 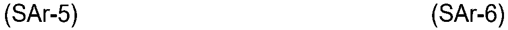 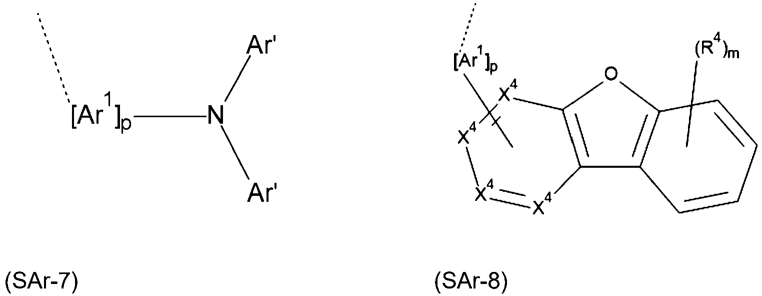 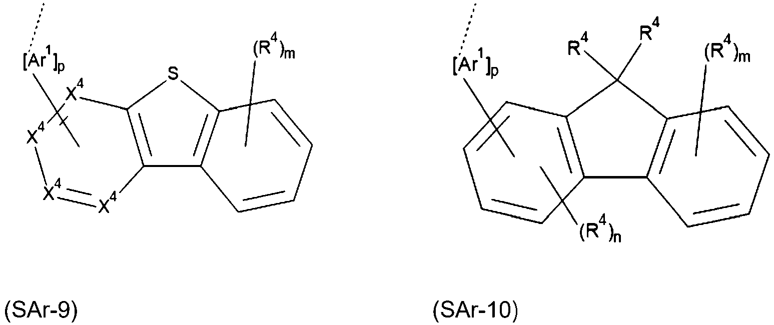 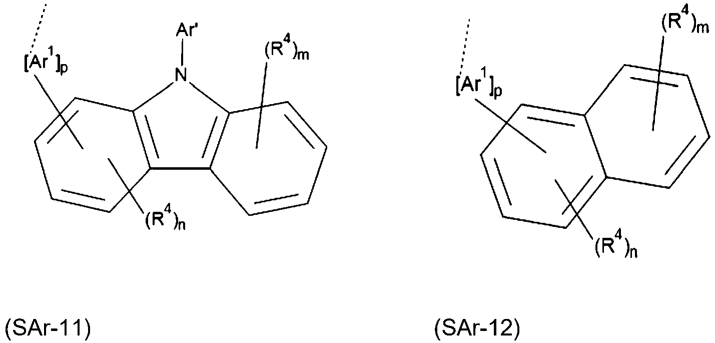 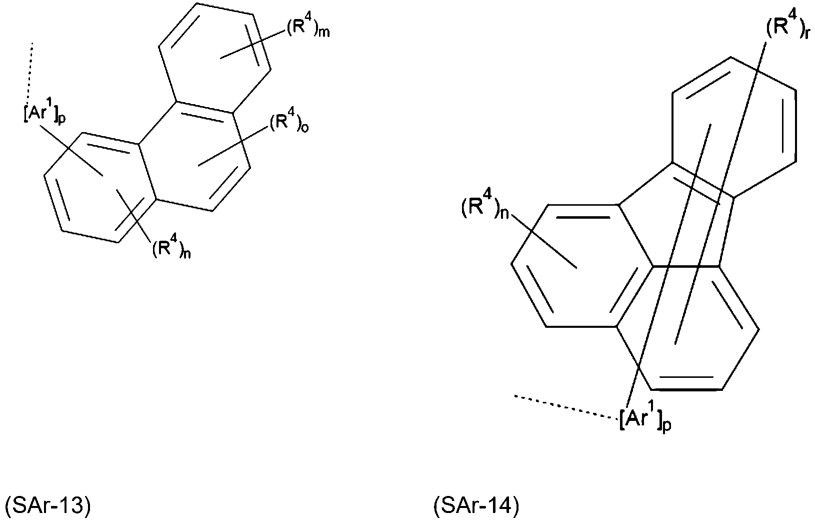 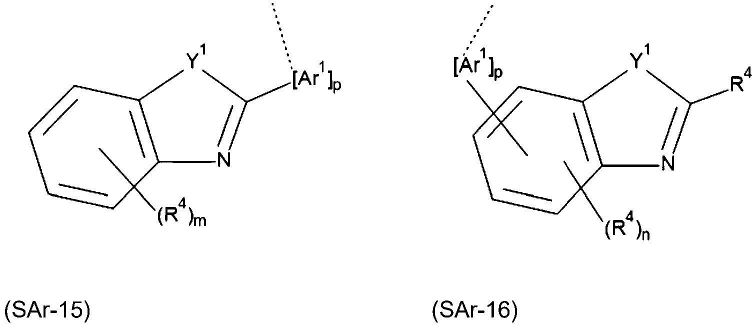 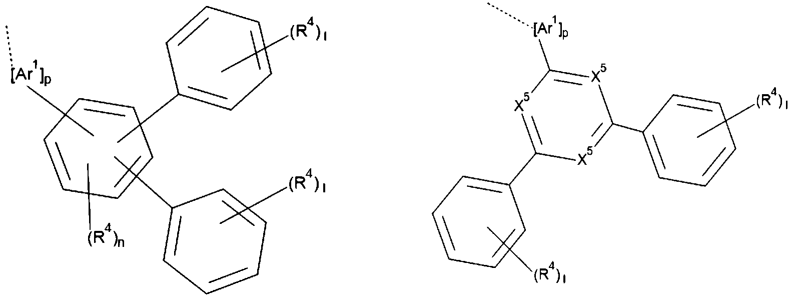 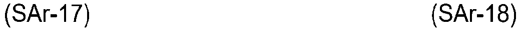 dans laquelle R⁴ et Ar' ont les significations données dans la revendication 1 et s'applique aux autres symboles et indices :
X⁴ est à chaque occurrence identique ou différente de CR⁴, N ou C, si le groupe [Ar¹]ₚ y est lié, de préférence CR⁴, dans lequel de préférence aucune liaison N-N n'est présente ;
X⁵ est à chaque occurrence identique ou différente de CR⁴ ou N, de préférence N ;
Ar¹ est, à chaque occurrence, identique ou différent, un système cyclique aromatique ou hétéroaromatique divalent ayant de 6 à 18 atomes de cycle aromatique, chacun ayant des résidus R⁴ ;
Y¹ est à chaque occurrence identique ou différente C(R⁴)₂, NR⁴, O, S ou N, si le groupe [Ar¹]ₚ y est lié ;
p est 0 ou 1, dans lequel p = 0 signifie que le groupe Ar¹ n'est pas présent et que le groupe aromatique ou hétéroaromatique correspondant est directement lié au résidu correspondant ;
n est égal à 0, 1, 2 ou 3, de préférence à 0, 1 ou 2 ;
m est égal à 0, 1, 2, 3 ou 4, de préférence à 0, 1 ou 2 ;
l est égal à 0, 1, 2, 3, 4 ou 5, de préférence à 0, 1 ou 2 ;
r est 0, 1, 2, 3, 4, 5 ou 6, de préférence 0, 1 ou 2 ;
dans laquelle les structures de formules (SAr-1), (SAr-4), (SAr-8), (SAr-11), (SAr-14), (SAr-18) sont préférées.

6. Composé selon une ou plusieurs des revendications 1 à 9, **caractérisé en ce que** R, R¹, R² et/ou R³, identiques ou différents à chaque occurrence, sont choisis dans le groupe constitué par H, D ou un système cyclique aromatique ou hétéroaromatique choisi parmi les groupes de formules Ar-1 à Ar-79 suivantes, de préférence, au moins un groupe R, R¹, R² et/ou R³, identiques ou différents, est choisi parmi les groupes des formules Ar-1 à Ar-79 suivantes et/ou le groupe Ar, identique ou différent à chaque occurrence, est choisi parmi les groupes des formules Ar-1 à Ar-79 suivantes 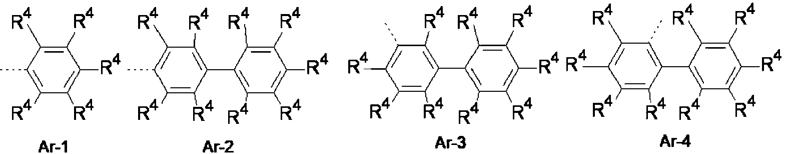 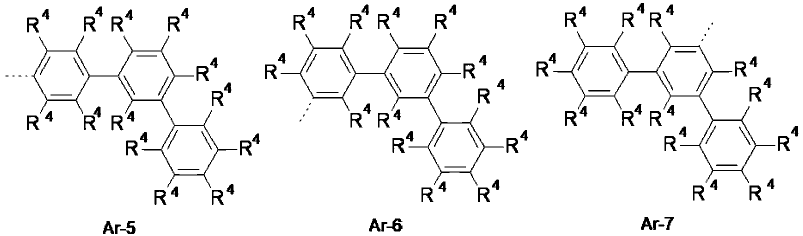 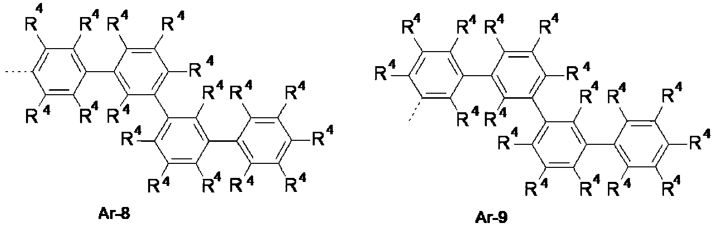 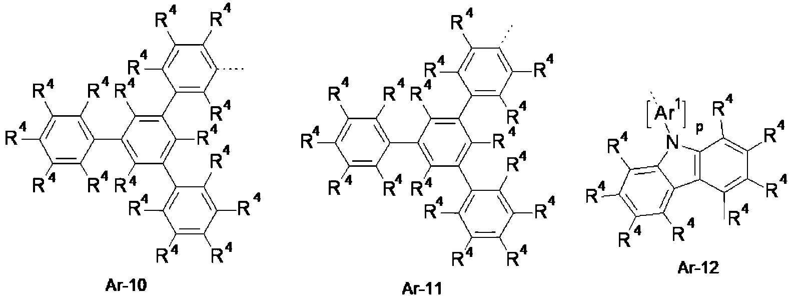 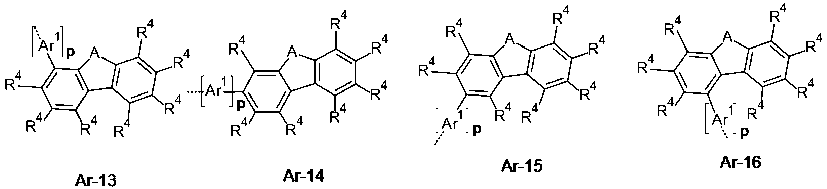 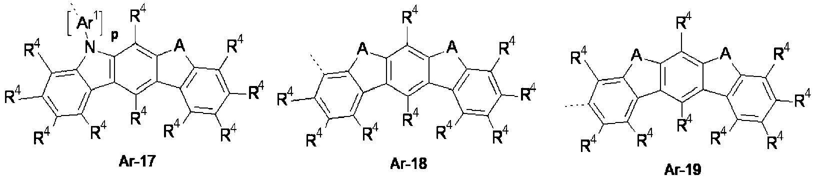 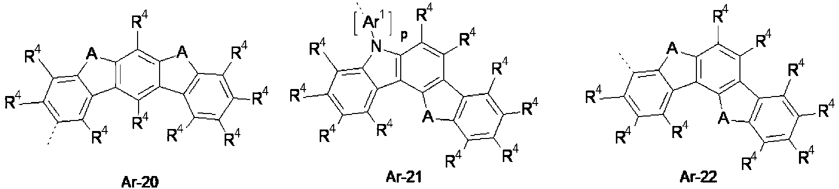 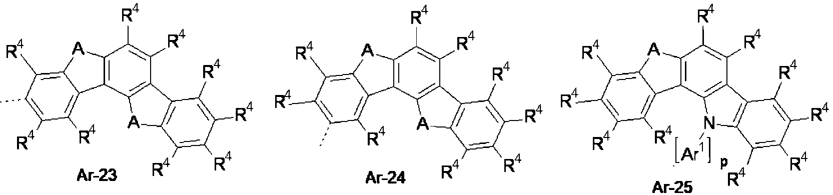 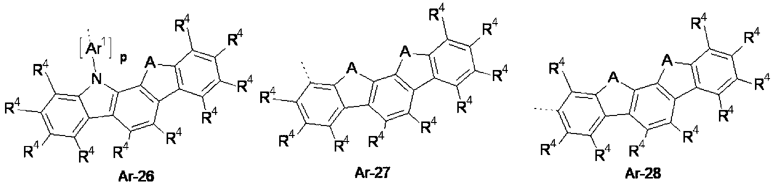 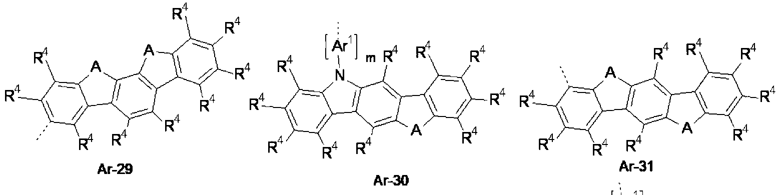 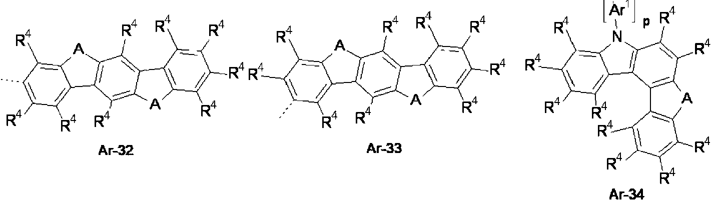 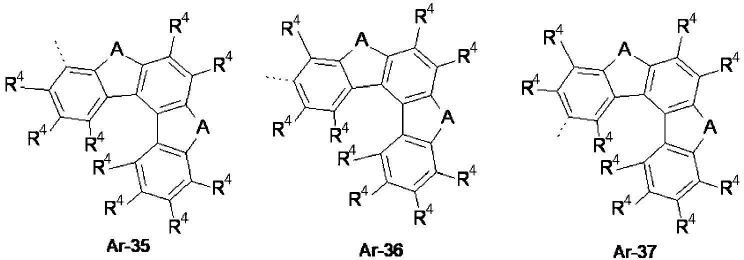 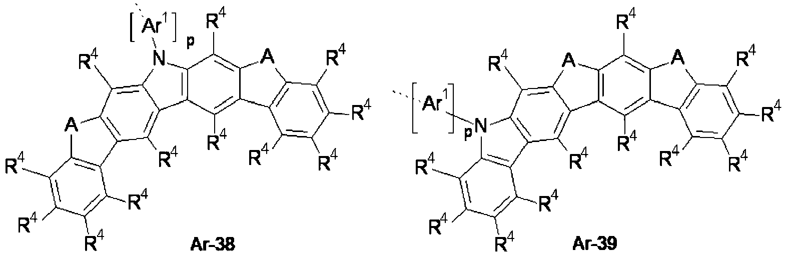 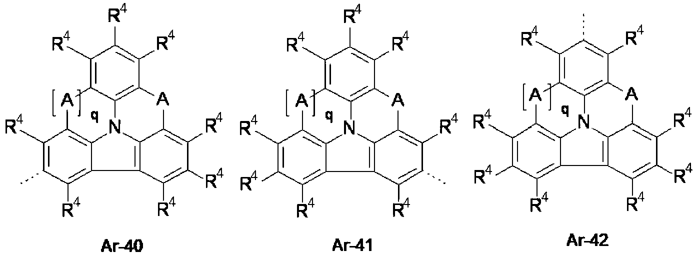 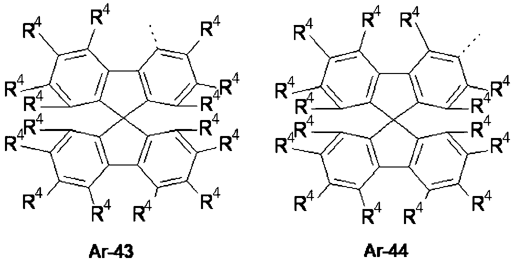 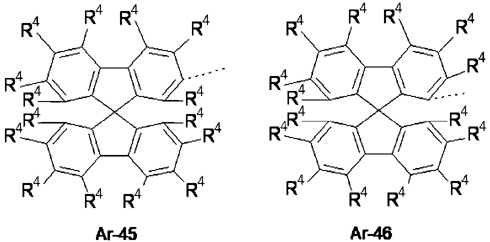 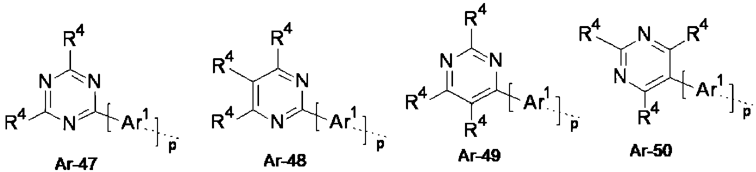 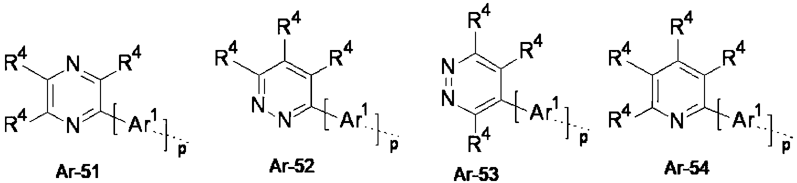 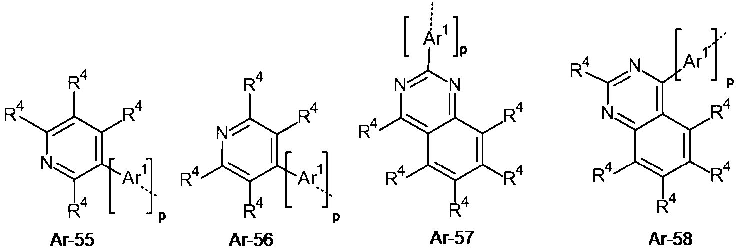 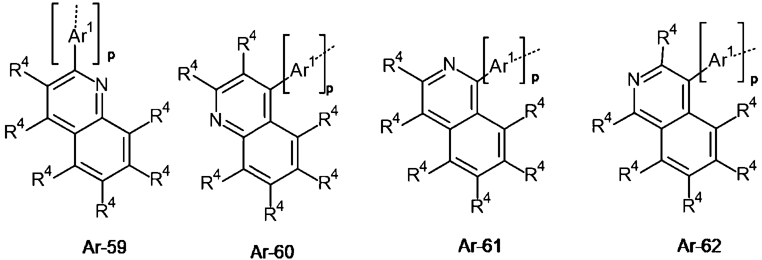 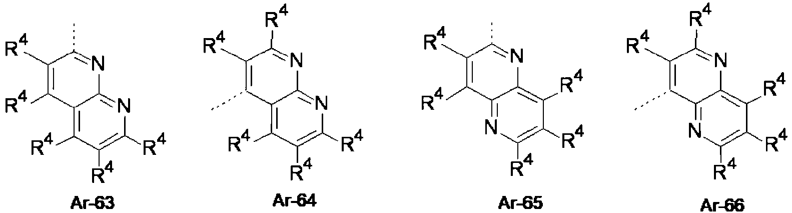 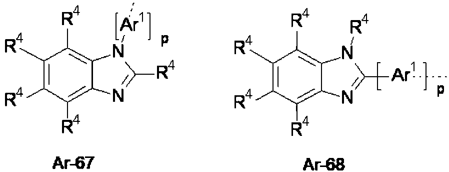 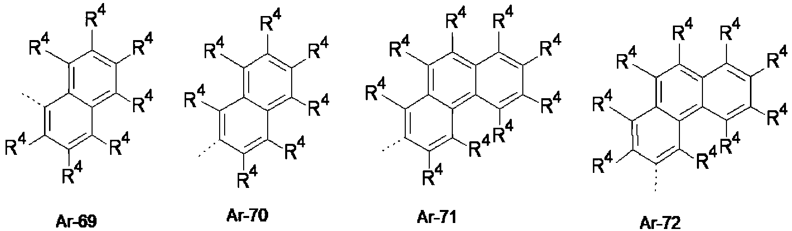 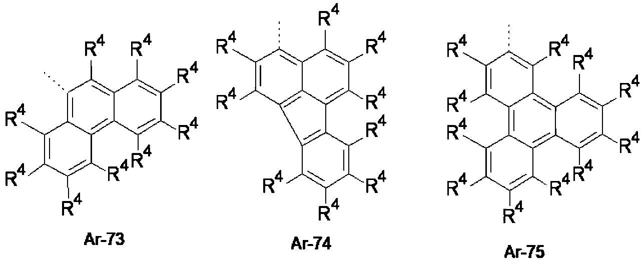 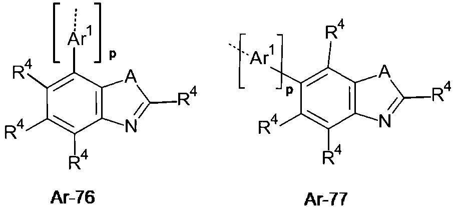 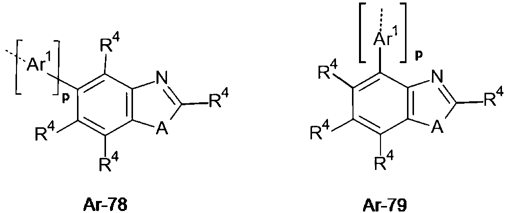 dans laquelle R⁴ a les significations indiquées ci-dessus, la liaison en pointillés représente la liaison au groupe correspondant et reste valable :
Ar¹ est à chaque occurrence, identique ou différente, un système cyclique aromatique ou hétéroaromatique bivalent avec 6 à 18 atomes cycliques aromatiques, qui présente à chaque fois des restes R⁴ ;
A est à chaque occurrence identique ou différente C(R⁴)₂, NR⁴, O ou S ;
p est 0 ou 1, dans lequel p = 0 signifie que le groupe Ar¹ n'est pas présent et que le groupe aromatique ou hétéroaromatique correspondant est directement lié au résidu correspondant ;
q est 0 ou 1, dans lequel q = 0 signifie qu'aucun groupe A n'est lié à cette position et que des radicaux R⁴ sont liés aux atomes de carbone correspondants à la place,
dans laquelle les structures de formules (Ar-1), (Ar-2), (Ar-3), (Ar-12), (Ar-13), (Ar-14), (Ar-15), (Ar-16), (Ar-47), (Ar-70), (Ar-75), (Ar-76), (Ar-77), (Ar-78), (Ar-79), sont préférées et les structures de formules (Ar-1), (Ar-2), (Ar-3), (Ar-12), (Ar-13), (Ar-14), (Ar-15), (Ar-16) sont particulièrement préférées.

7. Procédé de préparation d'un composé selon une ou plusieurs des revendications 1 à 10, **caractérisé en ce qu'**on fait réagir un composé aromatique ou hétéroaromatique contenant de l'azote dans une réaction de cyclisation.

8. Composition comprenant au moins un composé contenant au moins une structure ayant au moins trois éléments de structure condensés les uns avec les autres selon les formules (A'), (B') et (C'), de préférence un composé constitué de trois éléments de structure condensés les uns avec les autres selon les formules (A'), (B') et (C') 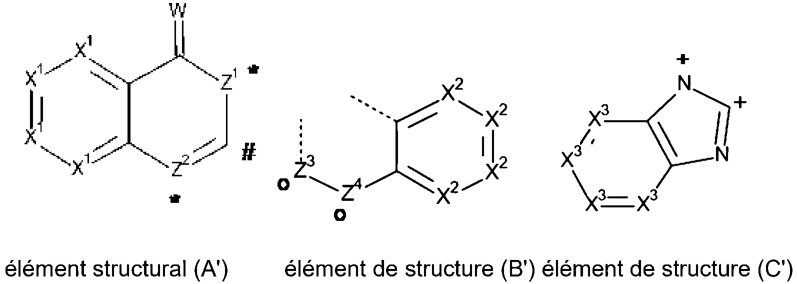
dans laquelle l'élément structural (A') est condensé avec l'élément structural (B') et l'élément structural (B') est condensé avec l'élément structural (C') ; dans laquelle l'élément structural (B') se lie à l'élément structural (A') par l'intermédiaire des liaisons représentées en pointillés, une liaison étant effectuée par l'intermédiaire du site de liaison marqué # et une liaison étant effectuée par l'intermédiaire d'un site de liaison marqué * ; dans lequel l'élément structural (B') est condensé avec l'élément structural (C') par l'intermédiaire des atomes marqués par o et +, et les atomes marqués respectivement sont partagés par les éléments structuraux (B') et (C'), et
est valable pour les symboles et indices utilisés :
W représente O ou S, de préférence O ;
Z¹ représente NAr ou N, dans le cas où l'élément structural (A') est condensé avec l'élément structural (B') au-dessus de Z¹ ;
Z² représente X ou C, dans le cas où l'élément structural (A') est condensé avec l'élément structural (B') au-dessus de Z² ;
Z³, Z⁴ représente N ou C, dans lequel l'un des radicaux Z³, Z⁴ représente N et l'un des radicaux Z³, Z⁴ représente C ;
X représente N ou CR, de préférence N ;
X¹ est, à chaque occurrence, identique ou différent de N ou CR¹, de préférence CR¹, à condition que pas plus de deux des groupes X¹ dans un cycle ne soient N ;
X² est identique ou différent de N ou CR² à chaque occurrence, à condition que pas plus de deux des groupes X² dans un cycle soient N ;
X³ est identique ou différent à chaque occurrence de N ou CR³, de préférence CR³, à condition que pas plus de deux des groupes X³ dans un cycle soient N ;
Ar est, à chaque occurrence, identique ou différent, un système cyclique aromatique ou hétéroaromatique ayant de 5 à 40 atomes de cycle aromatique, qui présente des restes R, de préférence un groupe aryle ayant de 6 à 30 atomes de cycle aromatique ou un groupe hétéroaryle ayant de 5 à 14 atomes de cycle aromatique, qui présente des restes R ;
R est à chaque occurrence identique ou différente H, D, F, Cl, Br, I, R⁴C=C(R⁴)₂, N(R⁴)₂, N(Ar')₂, CN, NO₂, OR⁴, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, un groupe alkyle à chaîne droite, alcoxy ou thioalkyle ayant de 1 à 20 atomes de carbone ou un groupe alcényle ou alcynyle ayant de 2 à 20 atomes de carbone ou un groupe alkyle, alcoxy ou thioalkyle ramifié ou cyclique ayant de 3 à 20 atomes de carbone, dans lequel chaque groupe alkyle, alcoxy ou thioalkyle, alcényle ou alcynyle a des résidus R⁴ et dans lequel un ou plusieurs groupes CH₂ non adjacents sont remplacés par R⁴C=CR⁴, C≡C, Si(R⁴)₂, C=O, C=S, C=Se, C=NR⁴, - C(=O)O-, -C(=O)NR⁴-, NR⁴, P(=O)( R⁴), -O-, -S-, SO ou SO₂ peuvent être remplacés, ou un système cyclique aromatique ou hétéroaromatique ayant de 5 à 60 atomes aromatiques dans le cycle, de préférence de 5 à 40 atomes aromatiques dans le cycle, chacun ayant des résidus R⁴, ou un groupe aryloxy ou hétéroaryloxy avec 5 à 40 atomes de cycle aromatiques, qui présente respectivement des restes R⁴, ou un groupe diarylamino, arylhétéroarylamino, dihétéroarylamino avec 5 à 60 atomes de cycle aromatiques dans le reste aromatique ou hétéroaromatique respectif, dans lequel le groupe diarylamino, arylhétéroarylamino, dihétéroarylamino présente des restes R⁴ ; un radical R pouvant former avec un autre radical, de préférence un groupe R¹, R², R³ un système cyclique aliphatique, hétéroaliphatique, aromatique ou hétéroaromatique, de préférence un système cyclique aliphatique, hétéroaliphatique ou hétéroaromatique, de manière particulièrement préférée le radical R ne forme pas un tel système cyclique ;
R¹ est à chaque occurrence identique ou différente H, D, F, Cl, Br, I, R⁴C=C(R⁴)₂, N(R⁴)₂, N(Ar')₂, CN, NO₂, OR⁴, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, un groupe alkyle à chaîne droite, alcoxy ou thioalkyle ayant de 1 à 20 atomes de carbone ou un groupe alcényle ou alcynyle ayant de 2 à 20 atomes de carbone ou un groupe alkyle, alcoxy ou thioalkyle ramifié ou cyclique ayant de 3 à 20 atomes de carbone, dans lequel chaque groupe alkyle, alcoxy ou thioalkyle, alcényle ou alcynyle a des résidus R⁴ et dans lequel un ou plusieurs groupes CH₂ non adjacents sont remplacés par R⁴C=CR⁴, C≡C, Si(R⁴)₂, C=O, C=S, C=Se, C=NR⁴, - C(=O)O-, -C(=O)NR⁴-, NR⁴, P(=O)( R⁴), -O-, -S-, SO ou SO₂ peuvent être remplacés, ou un système cyclique aromatique ou hétéroaromatique ayant de 5 à 60 atomes aromatiques dans le cycle, de préférence de 5 à 40 atomes aromatiques dans le cycle, chacun ayant des résidus R⁴, ou un groupe aryloxy ou hétéroaryloxy avec 5 à 40 atomes de cycle aromatiques, qui présente respectivement des restes R⁴, ou un groupe diarylamino, arylhétéroarylamino, dihétéroarylamino avec 5 à 60 atomes de cycle aromatiques dans le reste aromatique ou hétéroaromatique respectif, dans lequel le groupe diarylamino, arylhétéroarylamino, dihétéroarylamino présente des restes R⁴ ; deux radicaux R¹ ou un radical R¹ avec un autre radical, de préférence un groupe R, R², R³ peuvent également former ensemble un système cyclique aromatique, hétéroaromatique, aliphatique ou hétéroaliphatique, de préférence un système cyclique aliphatique, hétéroaliphatique ou hétéroaromatique, de manière particulièrement préférée les radicaux R¹ ne forment pas un tel système cyclique ;
R² est à chaque occurrence identique ou différente H, D, F, Cl, Br, I, R⁴C=C(R⁴)₂, N(R⁴)₂, N(Ar')₂, CN, NO₂, OR⁴, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, un groupe alkyle à chaîne droite, alcoxy ou thioalkyle ayant de 1 à 20 atomes de carbone ou un groupe alcényle ou alcynyle ayant de 2 à 20 atomes de carbone ou un groupe alkyle, alcoxy ou thioalkyle ramifié ou cyclique ayant de 3 à 20 atomes de carbone, dans lequel chaque groupe alkyle, alcoxy ou thioalkyle, alcényle ou alcynyle a des résidus R⁴ et dans lequel un ou plusieurs groupes CH₂ non adjacents sont remplacés par R⁴C=CR⁴, C≡C, Si(R⁴)₂, C=O, C=S, C=Se, C=NR⁴, - C(=O)O-, -C(=O)NR⁴-, NR⁴, P(=O)( R⁴), -O-, -S-, SO ou SO₂ peuvent être remplacés, ou un système cyclique aromatique ou hétéroaromatique ayant de 5 à 60 atomes aromatiques dans le cycle, de préférence de 5 à 40 atomes aromatiques dans le cycle, chacun ayant des résidus R⁴, ou un groupe aryloxy ou hétéroaryloxy avec 5 à 40 atomes de cycle aromatiques, qui présente respectivement des restes R⁴, ou un groupe diarylamino, arylhétéroarylamino, dihétéroarylamino avec 5 à 60 atomes de cycle aromatiques dans le reste aromatique ou hétéroaromatique respectif, dans lequel le groupe diarylamino, arylhétéroarylamino, dihétéroarylamino présente des restes R⁴ ; deux radicaux R² ou un radical R² avec un autre radical, de préférence un groupe R, R¹, R³ peuvent également former ensemble un système cyclique aromatique, hétéroaromatique, aliphatique ou hétéroaliphatique, de préférence un système cyclique aliphatique, hétéroaliphatique ou hétéroaromatique, de manière particulièrement préférée les radicaux R² ne forment pas un tel système cyclique ;
R³ est à chaque occurrence identique ou différente H, D, F, Cl, Br, I, R⁴C=C(R⁴)₂, N(R⁴)₂, N(Ar')₂, CN, NO₂, OR⁴, OAr', SR⁴, SAr', C(=O)OR⁴, C(=O)N(R⁴)₂, Si(R⁴)₃, B(OR⁴)₂, C(=O)R⁴, P(=O)(R⁴)₂, S(=O)R⁴, S(=O)₂R⁴, OSO₂R⁴, un groupe alkyle à chaîne droite, alcoxy ou thioalkyle ayant de 1 à 20 atomes de carbone ou un groupe alcényle ou alcynyle ayant de 2 à 20 atomes de carbone ou un groupe alkyle, alcoxy ou thioalkyle ramifié ou cyclique ayant de 3 à 20 atomes de carbone, dans lequel chaque groupe alkyle, alcoxy ou thioalkyle, alcényle ou alcynyle a des résidus R⁴ et dans lequel un ou plusieurs groupes CH₂ non adjacents sont remplacés par R⁴C=CR⁴, C≡C, Si(R⁴)₂, C=O, C=S, C=Se, C=NR⁴, - C(=O)O-, -C(=O)NR⁴-, NR⁴, P(=O)( R⁴), -O-, -S-, SO ou SO₂ peuvent être remplacés, ou un système cyclique aromatique ou hétéroaromatique ayant de 5 à 60 atomes aromatiques dans le cycle, de préférence de 5 à 40 atomes aromatiques dans le cycle, chacun ayant des résidus R⁴, ou un groupe aryloxy ou hétéroaryloxy avec 5 à 40 atomes de cycle aromatiques, qui présente respectivement des restes R⁴, ou un groupe diarylamino, arylhétéroarylamino, dihétéroarylamino avec 5 à 60 atomes de cycle aromatiques dans le reste aromatique ou hétéroaromatique respectif, dans lequel le groupe diarylamino, arylhétéroarylamino, dihétéroarylamino présente des restes R⁴ ; deux radicaux R³ ou un radical R³ avec un autre radical, de préférence un groupe R, R¹, R² peuvent également former ensemble un système cyclique aromatique, hétéroaromatique, aliphatique ou hétéroaliphatique, de préférence un système cyclique aliphatique, hétéroaliphatique ou hétéroaromatique, de manière particulièrement préférée les radicaux R³ ne forment pas un tel système cyclique ;
Ar' est, à chaque occurrence, identique ou différent, un système cyclique aromatique ou hétéroaromatique ayant de 5 à 40 atomes cycliques aromatiques, qui présente des restes R⁴, de préférence un groupe aryle ayant de 6 à 30 atomes cycliques aromatiques ou un groupe hétéroaryle ayant de 5 à 14 atomes cycliques aromatiques, qui présente des restes R⁴ ;
R⁴ est à chaque occurrence identique ou différente H, D, F, Cl, Br, I, R⁵C=C(R⁵)₂, N(R⁵)₂, CN, NO₂, OR⁵, SR⁵, Si(R⁵)₃, B(OR⁵)₂, C(=O)R⁵, P(=O)(R⁵)₂, S(=O)R⁵, S(=O)₂R⁵, OSO₂R⁵, un groupe alkyle à chaîne droite, alcoxy ou thioalkyle ayant de 1 à 20 atomes de carbone ou un groupe alcényle ou alcynyle ayant de 2 à 20 atomes de carbone ou un groupe alkyle, alcoxy ou thioalkyle ramifié ou cyclique ayant de 3 à 20 atomes de carbone, dans lequel le groupe alkyle, alcoxy ou thioalkyle, alcényle ou alcynyle a respectivement des résidus R⁵, un ou plusieurs groupes CH₂ non adjacents étant remplacés par R⁵C=CR⁵, C=C, Si(R⁵)₂, C=O, C=S, C=Se, C=NR⁵, -C(=O)O-, -C(=O)NR⁵-, NR⁵, P(=O)( R⁵), -O-, -S-, SO ou SO₂ peuvent être remplacés, ou un système cyclique aromatique ou hétéroaromatique ayant de 5 à 40 atomes de cycle aromatique, chacun ayant des résidus R⁵, ou un groupe aryloxy ou hétéroaryloxy ayant de 5 à 40 atomes de cycle aromatique, ayant chacun des résidus R⁵, ou un groupe diarylamino, arylhétéroarylamino, dihétéroarylamino ayant de 5 à 60 atomes de cycle aromatiques dans le résidu aromatique ou hétéroaromatique respectif, dans lequel le groupe diarylamino, arylhétéroarylamino, dihétéroarylamino a des résidus R⁵ ; deux ou plusieurs radicaux R⁴ peuvent former ensemble un système cyclique aromatique, hétéroaromatique, aliphatique ou hétéroaliphatique, de préférence un système cyclique aliphatique, de manière particulièrement préférée les radicaux R⁴ ne forment pas un tel système cyclique ;
R⁵ est, à chaque occurrence, identique ou différente, H, D, F ou un radical organique aliphatique, aromatique ou hétéroaromatique, en particulier un radical hydrocarboné, comportant de 1 à 20 atomes de carbone, dans lequel un ou plusieurs atomes de H peuvent également être remplacés par F, deux ou plusieurs radicaux R⁵ pouvant alors former ensemble un système cyclique ;
et
au moins un autre matériau de matrice, dans lequel l'autre matériau de matrice est choisi parmi les composés selon l'une des formules (H-1), (H-2), (H-3), (H-4) ou (H-5), 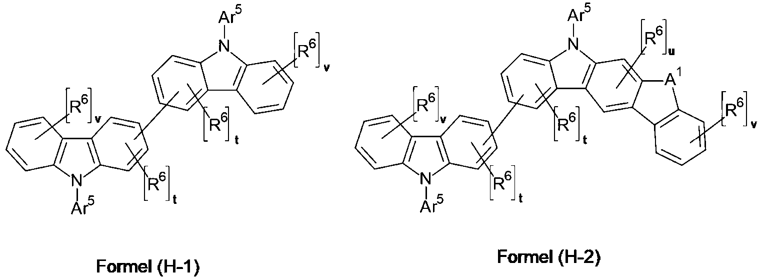 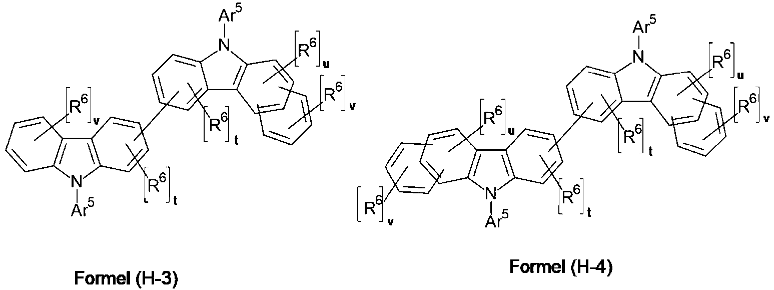 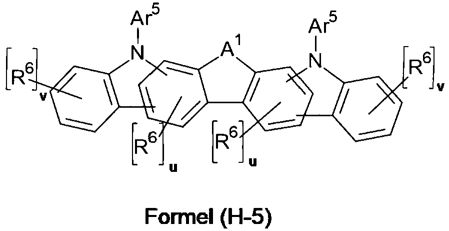
dans laquelle, pour les symboles et les indices utilisés, on a :
R⁶ est identique ou différent à chaque occurrence H, D, F, Cl, Br, I, N(R⁷)₂, N(Ar")₂, CN, NO₂, OR⁷, SR⁷, COOR⁷, C(=O)N(R⁷)₂, Si(R⁷)₃, B(OR⁷)₂, C(=O)R⁷, P(=O)(R⁷)₂, S(=O)R⁷, S(=O)₂R⁷, OSO₂R⁷, un groupe alkyle à chaîne droite ayant 1 à 20 atomes de carbone ou un groupe alcényle ou alcynyle ayant 2 à 20 atomes de carbone ou un groupe alkyle ramifié ou cyclique ayant 3 à 20 atomes de carbone, dans lequel les groupes alkyle, alcényle ou alcynyle ont chacun des résidus R⁷ et dans lequel un ou plusieurs groupes CH₂ non adjacents sont remplacés par Si(R⁷)₂, C=O, NR⁷, O, S ou CONR⁷, ou un système cyclique aromatique ou hétéroaromatique avec 5 à 60 atomes cycliques aromatiques, de préférence avec 5 à 40 atomes cycliques aromatiques, qui présentent chacun des restes R⁷ ; où deux radicaux R⁶ peuvent également former ensemble un système cyclique aromatique, hétéroaromatique, aliphatique ou hétéroaliphatique, de préférence les radicaux R⁶ ne forment pas un tel système cyclique ;
Ar" est, à chaque occurrence, identique ou différente, un système cyclique aromatique ou hétéroaromatique comportant de 5 à 40 atomes cycliques aromatiques, qui présente des radicaux R⁷ ;
A¹ est C(R⁷)₂, NR⁷, O ou S ;
Ar⁵ est identique ou différent à chaque occurrence et représente un système cyclique aromatique ou hétéroaromatique ayant de 5 à 40 atomes dans le cycle aromatique et ayant des résidus R⁷ ;
R⁷ est à chaque occurrence identique ou différente H, D, F, Cl, Br, I, N(R⁸)₂, CN, NO₂, OR⁸, SR⁸, Si(R⁸)₃, B(OR⁸)₂, C(=O)R⁸, P(=O)(R⁸)₂, S(=O)R⁸, S(=O)₂R⁸, OSO₂R⁸, un groupe alkyle à chaîne droite ayant de 1 à 20 atomes de carbone ou un groupe alcényle ou alcynyle ayant de 2 à 20 atomes de carbone ou un groupe alkyle ramifié ou cyclique ayant de 3 à 20 atomes de carbone, dans lequel le groupe alkyle, le groupe alcényle ou le groupe alcynyle présentent chacun des radicaux R⁸, un ou plusieurs groupes CH₂ non adjacents pouvant être remplacés par Si(R⁸)₂, C=O, NR⁸, O, S ou CONR⁸, ou un système cyclique aromatique ou hétéroaromatique avec 5 à 40 atomes cycliques aromatiques, qui présentent chacun des radicaux R⁸ ; où deux ou plusieurs radicaux R⁷ peuvent former ensemble un système cyclique aromatique, hétéroaromatique, aliphatique ou hétéroaliphatique, de préférence les radicaux R⁷ ne forment pas un tel système cyclique ;
R⁸ est à chaque occurrence identique ou différente H, D, F ou un radical organique aliphatique, aromatique ou hétéroaromatique, en particulier un radical hydrocarboné, ayant de 1 à 20 atomes de carbone, dans lequel un ou plusieurs atomes de H peuvent également être remplacés par F ;
v est, à chaque occurrence, identique ou différent de 0, 1, 2, 3 ou 4, de préférence 0 ou 1 et de manière tout à fait préférée 0 ;
t est à chaque occurrence identique ou différente 0, 1, 2 ou 3, de préférence 0 ou 1 et de manière préférée 0 ;
u est, à chaque occurrence, identique ou différent, 0, 1 ou 2, de préférence 0 ou 1, et de manière préférée 0.

9. Composition selon la revendication 8, **caractérisée en ce que** la composition est constituée d'au moins un composé contenant au moins une structure ayant au moins trois éléments de structure condensés entre eux selon les formules (A'), (B') et (C'), et d'au moins un composé selon l'une des formules (H-1), (H-2), (H-3), (H-4) ou (H-5).

10. Composition selon la revendication 8 ou 9, **caractérisée en ce que** les composés selon l'une des formules (H-1), (H-2), (H-3), (H-4) ou (H-5) représentent dans la composition une proportion en masse dans la plage de 5 % à 90 % en poids, de préférence dans la plage de 10 % à 85 % en poids.% en poids, plus préférentiellement de 20 % à 85 % en poids, plus préférentiellement encore de 30 % à 80 % en poids, plus préférentiellement de 20 % à 60 % en poids et plus préférentiellement encore de 30 % à 50 % en poids, par rapport à la composition totale.

11. Formulation contenant au moins un composé selon une ou plusieurs des revendications 1 à 6 et/ou au moins une composition selon les revendications 8 à 10 et au moins un autre composé, l'autre composé étant de préférence choisi parmi un ou plusieurs solvants.

12. Utilisation d'un composé contenant au moins un composé comprenant au moins une structure ayant au moins trois éléments de structure condensés entre eux selon les formules (A'), (B') et (C'), de préférence un composé constitué de trois éléments de structure condensés entre eux selon les formules (A'), (B') et (C'), 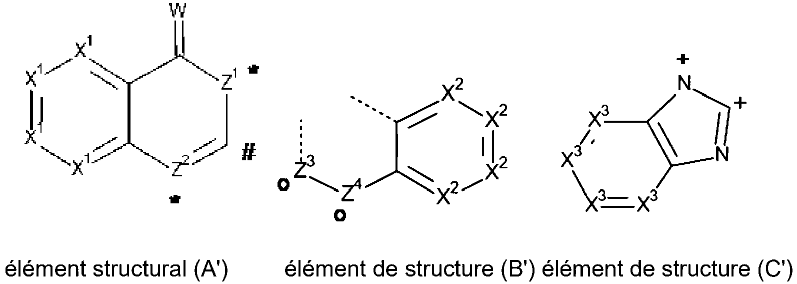
dans laquelle l'élément structural (A') est condensé avec l'élément structural (B') et l'élément structural (B') est condensé avec l'élément structural (C') ; dans laquelle l'élément structural (B') se lie à l'élément structural (A') par l'intermédiaire des liaisons représentées en pointillés, une liaison se faisant par l'intermédiaire du site de liaison marqué # et une liaison se faisant par l'intermédiaire d'un site de liaison marqué * ;
dans lequel l'élément structural (B') est condensé avec l'élément structural (C') par l'intermédiaire des atomes marqués par o et +, et les atomes marqués respectivement sont partagés par les éléments structuraux (B') et (C'),
dans lequel les symboles et indices utilisés ont les significations données dans la revendication 8,
dans un dispositif électronique ; et/ou
utilisation d'une composition selon l'une quelconque des revendications 8 à 10 dans un dispositif électronique.

13. Dispositif électronique contenant au moins un composé comprenant au moins une structure ayant au moins trois éléments de structure condensés entre eux selon les formules (A'), (B') et (C'), de préférence un composé constitué de trois éléments de structure condensés entre eux selon les formules (A'), (B') et (C'), 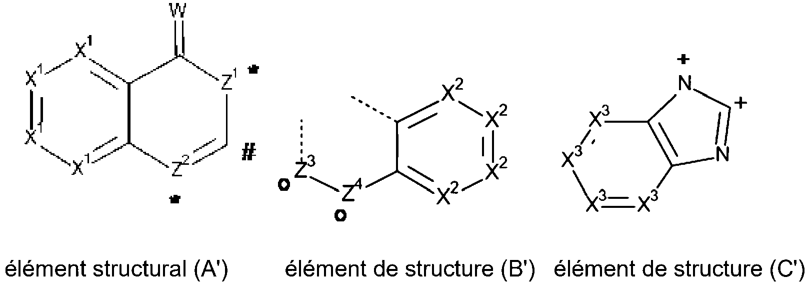
dans laquelle l'élément structural (A') est condensé avec l'élément structural (B') et l'élément structural (B') est condensé avec l'élément structural (C') ; dans laquelle l'élément structural (B') se lie à l'élément structural (A') par l'intermédiaire des liaisons représentées en pointillés, une liaison se faisant par l'intermédiaire du site de liaison marqué # et une liaison se faisant par l'intermédiaire d'un site de liaison marqué * ;
dans lequel l'élément structural (B') est condensé avec l'élément structural (C') par l'intermédiaire des atomes marqués par o et +, et les atomes marqués respectivement sont partagés par les éléments structuraux (B') et (C'),
dans lequel les symboles et indices utilisés ont les significations indiquées dans la revendication 8 ;
dans lequel le dispositif électronique est de préférence un dispositif électroluminescent.

14. Dispositif électronique selon la revendication 13, dans lequel il s'agit d'un dispositif électroluminescent organique, **caractérisé en ce que** le composé comprenant au moins une structure ayant au moins trois éléments de structure condensés ensemble selon les formules (A'), (B') et (C'), de préférence composé constitué de trois éléments de structure condensés ensemble selon les formules (A'), (B') et (C'), comme matériau de matrice dans une couche émettrice et/ou dans une couche de transport d'électrons et/ou dans une couche de blocage de trous, de préférence comme matériau de matrice dans une couche émettrice et/ou dans une couche de transport d'électrons, de manière particulièrement préférée comme matériau de matrice dans une couche émettrice, de manière particulièrement préférée comme matériau de matrice dans une couche émettrice en combinaison avec un émetteur phosphorescent rouge ou vert.

15. Dispositif électronique selon la revendication 13 ou 14, **caractérisé en ce que** le composé comprenant au moins une structure ayant au moins trois éléments structuraux condensés ensemble selon les formules (A'), (B') et (C'), de préférence le composé constitué de trois éléments structuraux condensés ensemble selon les formules (A'), (B') et (C'), est utilisé comme matériau de matrice pour des émetteurs phosphorescents en combinaison avec un autre matériau de matrice qui est choisi parmi des composés selon l'une des formules (H-1), (H-2), (H-3), (H-4) ou (H-5), 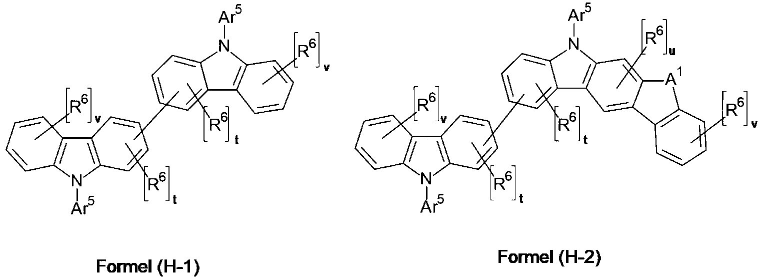 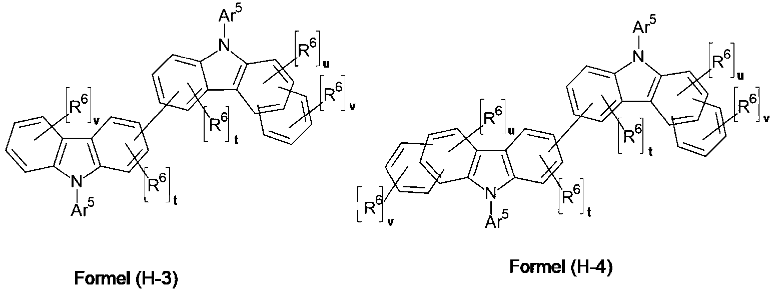 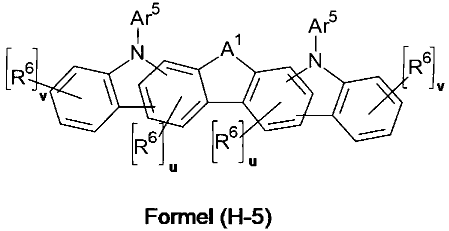 dans laquelle les symboles A¹, Ar⁵ et R⁶ et les indices v, t et u ont les significations indiquées dans la revendication 8.
